(19) **Europäisches Patentamt / European Patent Office / Office européen des brevets**

(11) **EP 4 098 662 A1**

(12) # EUROPEAN PATENT APPLICATION

(43) Date of publication:
**07.12.2022 Bulletin 2022/49**

(21) Application number: **22167890.7**

(22) Date of filing: **24.05.2018**

(51) International Patent Classification (IPC):
***C07K 16/28*** (2006.01)

(52) Cooperative Patent Classification (CPC):
**C07K 16/2896; C07K 16/2878;** C07K 2317/52;
C07K 2317/522; C07K 2317/524; C07K 2317/526;
C07K 2317/53; C07K 2317/55; C07K 2317/71;
C07K 2317/72; C07K 2317/75; C07K 2317/77;
C07K 2317/92; C07K 2317/94

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.05.2017 US 201762511178 P
15.12.2017 US 201762599221 P**

(62) Document number(s) of the earlier application(s) in
accordance with Art. 76 EPC:
**18731658.3 / 3 630 833**

(71) Applicant: **Bristol-Myers Squibb Company
Princeton, NJ 08543 (US)**

(72) Inventors:
  • **YAMNIUK, Aaron, P.
    Princeton (US)**
  • **KORMAN, Alan, J.
    Redwood City (US)**
  • **SELBY, Mark, J.
    Redwood City (US)**
  • **BARNHART, Bryan, C.
    Redwood City (US)**
  • **LONBERG, Nils
    Redwood City (US)**
  • **SRINIVASAN, Mohan
    Redwood City (US)**

  • **HENNING, Karla, A.
    Redwood City (US)**
  • **HAN, Michelle, Minhua
    Redwood City (US)**
  • **LEI, Ming
    Princeton (US)**
  • **SCHWEIZER, Liang
    Lawrenceville (US)**
  • **HATCHER, Sandra, V
    Princeton (US)**
  • **RAJPAL, Arvind
    Redwood City (US)**

(74) Representative: **Mewburn Ellis LLP
Aurora Building
Counterslip
Bristol BS1 6BX (GB)**

Remarks:
•The complete document including Reference
Table(s) and the Sequence Listing(s) can be
downloaded from the EPO website
•This application was filed on 12-04-2022 as a
divisional application to the application mentioned
under INID code 62.
•Claims filed after the date of filing of the application
(Rule 68(4) EPC).

(54) **ANTIBODIES COMPRISING MODIFIED HEAVY CONSTANT REGIONS**

(57)     Provided herein are heavy chain constant regions (referred to as "modified heavy chain constant regions"), or functionally equivalent fragments thereof, that enhance biological properties of antibodies relative to the same antibodies in unmodified form. An exemplary modified heavy chain constant region includes an IgG2 hinge and three constant domains (*i.e.,* CHI, CH2, and CH3 domains), wherein one or more of the constant region domains are of a non-IgG2 isotype (e.g., IgG1, IgG3 or IgG4). The heavy chain constant region may comprise wildtype human IgG domain sequences, or variants of these sequences. Also provided herein are methods for enhancing certain biological properties of antibodies that comprise a non-IgG2 hinge, such as internalization, agonism and antagonism, wherein the method comprises replacing the non-IgG2 hinge of the antibody with an IgG2 hinge.

EP 4 098 662 A1

**Description**

**CROSS-REFERENCE TO RELATED APPLICATIONS**

This application claims priority to U.S. Provisional Applications Nos. 62/511,178 (filed May 25, 2017) and 62/599,221 (filed December 15, 2017). The contents of any patents, patent applications, and references cited throughout this specification are hereby incorporated by reference in their entireties.

**BACKGROUND**

Antibody therapeutics is one of the fastest growing areas in the treatment of disease, such as cancer and immune disorders. Nevertheless, efficiently targeting an antigen by a therapeutic antibody remains a major challenge in health care. Therefore, antibody engineering has become a major focus in the pharmaceutical world. From this focus, a myriad of new engineered antibodies have emerged, such as antibody fragments, antibody drug conjugates (ADCs), antibodies with modified effector regions, and bispecific antibodies.

Antibodies facilitate their therapeutic properties through many different mechanisms. Antibodies may directly inhibit or activate a target antigen, thus regulating cell signaling. Antibodies may inhibit the binding of a ligand to a receptor. Antibodies may also induce or inhibit an immune response, for example, by boosting the subject's immune system to fight infection or cancer (e.g., as costimulators in the activation of T cells).

Furthermore, antibody-mediated internalization of a cell surface receptor/antigen is recognized as a major mechanism of action for therapeutic antibodies. In this instance, an antibody removes the target from the cell surface and from performing its function by inducing internalization into the cell. Indeed, one of the forerunners of antibody therapeutics is trastuzumab for the treatment of breast cancer. Trastuzumab targets the ErbB2 receptor and induces receptor/antibody internalization, thus inhibiting EGFR signaling. However, antibodies do not always display efficient internalization qualities, thus there is an ongoing need for antibodies with improved internalization functions. Accordingly, methods for improving the internalization of known therapeutic antibodies are highly desirable.

**SUMMARY**

The invention provides heavy chain constant regions (referred to as "modified heavy chain constant regions"), or functionally equivalent fragments thereof, that enhance or modify biological properties of antibodies relative to the same antibodies in unmodified form. For example, antibodies comprising modified constant regions exhibit increased internalization and/or agonistic or antagonistic activity. Accordingly, antibodies of the invention are optimized versions of the original unmodified antibody. In certain embodiments, a heavy chain comprises a modified constant region comprising one or more mutations or modifications relative to the wildtype heavy chain constant region. In certain embodiments, a modified heavy chain constant region includes an IgG2 hinge and three constant domains (i.e., CH1, CH2, and CH3 domains), wherein one or more of the constant region domains is a non-IgG2 human isotype (e.g., IgG1, IgG3 or IgG4), or functionally equivalent fragments thereof. The modified constant region can include the corresponding wildtype amino acid sequence, or a variant thereof, e.g., one or more (e.g., between 1-10, or more) amino acid substitutions or deletions within the hinge or the CH1, CH2, CH3 domains relative to the wildtype amino acid sequence. Accordingly, the amino acid sequence of the hinge and/or each constant domain is at least about 80%, 85%, 90%, 95%, or more (i.e., 96%, 97%, 98%, 99%, or 100%) identical to the corresponding wildtype amino acid sequence.

In one embodiment, the modified heavy chain constant region includes a wildtype human IgG2 hinge, or an amino acid sequence that is at least 95% identical to the amino acid sequence of a wildtype human IgG2 hinge. The hinge can further contain additional modifications, for example, to reduce disulfide bond formation. In one embodiment, the hinge includes the amino acid substitution C219S, relative to the wildtype human IgG2 hinge. In certain embodiments, the hinge comprises the amino acid sequence set forth in any of SEQ ID NO: 8, 21-23, 126-132 and 134-147 or one of these sequences that comprises 1-3 amino acids inserted between CVE and CPP.

In certain embodiments, the modified heavy chain constant region includes an IgG2 CH1 domain, e.g., a wildtype human IgG2 CH1 domain, or an amino acid sequence that is at least 95% identical to the amino acid sequence of a wildtype human IgG2 CH1 domain (SEQ ID NO: 7).

In certain embodiments, the modified heavy chain constant region includes an IgG1 CH2 domain, e.g., a wildtype human IgG1 CH2 domain, or an amino acid sequence that is at least 95% identical to the amino acid sequence of a wildtype human IgG1 CH2 domain. The CH2 domain may contain additional modifications (e.g., to reduce or eliminate effector functions). In certain embodiments, the CH2 domain comprises the amino acid substitutions A330S and P331S, relative to wildtype full-length human IgG1 CH2. In certain embodiments, the CH2 domain comprises SEQ ID NO: 24.

In certain embodiments, the modified heavy chain constant region includes an IgG1 CH3 domain, e.g., a wildtype human IgG1 CH3 domain, or an amino acid sequence that is at least 95% identical to the amino acid sequence of a wildtype human IgG1 CH3 domain. The CH3 domain can further contain additional modifications to confer a particular allotype. In one embodiment, the CH3 domain contains the amino acid residue E at position 356 and the amino acid M at position 358 ("f" allotype), relative to wildtype full-length human IgG1 of a different allotype (e.g., "fa" allotype, having D and L, respectively at those positions). In certain embodiments, the CH3 domain comprises SEQ ID NO: 5.

In a particular embodiment, the antibody comprises a modified heavy chain constant region wherein (a) the CH1 domain is a wildtype human IgG2 CH1 domain or a wildtype IgG1 CH1 domain, with or without additional modification, (b) the hinge is a wildtype IgG2 hinge with or without a C219S substitution, (c) the CH2 domain is a wildtype human IgG1 CH2 domain or a wildtype IgG2 CH2 domain, with or without additional modifications, and (d) the CH3 domain is a wildtype human IgG 1 CH3 domain or a wildtype human IgG2 CH3 domain, with or without amino acid E at position 356 and amino acid M at position 358 (e.g., of allotype f or fa). In a specific embodiment, the modified heavy chain constant region comprises an amino acid sequence described herein, e.g., set forth in any one of SEQ ID NOs: 26-37 and 78-93.

Antibodies of the invention (i.e., antibodies having a modified constant region) may be fully human antibodies or humanized antibodies, and further exhibit one or more enhanced or altered features, compared to the same antibodies without a modified heavy chain constant region. These features may include increased or altered internalization by a cell, agonistic activity, formation of large cross-linked complexes, ADCC, receptor mediated signaling, antagonist activity, immuno-modulating activity and anti-tumor activity; or introduction of a new property, e.g., agonist activity.

Bispecific molecules and immunoconjugates containing modified constant regions of the invention are also provided, as well as compositions which contain the antibodies, bispecifics, or immunoconjugates and an acceptable pharmaceutical carrier. Such compositions also may include one or more additional therapeutic agents, e.g., an agent that stimulates the immune system, such as a checkpoint inhibitor, a co-stimulatory molecule, an anti-CD39 antibody, or an anti-A2AR antibody.

Methods for preparing an antibody comprising a modified heavy chain constant region are also provided. Certain methods provided herein include methods of increasing internalization of an antibody by a cell, and methods for increasing the agonist activity of an antibody, compared to the same antibody comprising a hinge of a non-IgG2 isotype. Such methods comprise the steps of providing an antibody having a hinge that is not an IgG2 hinge, and replacing the hinge with an IgG2 hinge (such as a hinge that is a wildtype human IgG2 hinge, a hinge having an amino acid sequence that is at least 95% identical to the amino acid sequence of a wildtype human IgG2 hinge, or a hinge that is modified to reduce disulfide bond formation, e.g., a hinge that comprises amino acid substitution C219S). In one embodiment, internalization of the antibody is enhanced or increased by at least 10%, 30%, 50%, 75%, 2 fold, 3 fold, 5 fold or more, resulting in a reduction of the $T_{1/2}$ by at least 10%, 30%, 50%, 75%, 2 fold, 3 fold, 5 fold or more. In certain embodiments, agonist activity is increased or enhanced by at least 10%, 30%, 50%, 75%, 2 fold, 3 fold, 5 fold or more as defined by increased cytokine release or increased proliferation in effector T cells; reduced T regulatory cell activity if engagement on Tregs reduces Treg function; or increased depletion of Tregs.

In certain embodiments, the method further includes the step of replacing at least one of the CH1, CH2, or CH3 domains with a CH1, CH2, or CH3 domain of a different isotype. Such replacements include, for example: (a) replacing the CH1 domain with an IgG1 CH1 domain or an IgG2 CH1 domain; (b) replacing the CH2 domain with an IgG1 CH2 domain or an IgG2 CH2 domain; and/or (b) replacing the CH3 domain with an IgG1 CH3 domain or an IgG2 CH3 domain, wherein the replacement domain has the wildtype sequence or at least 95% identity the wildtype sequence. In certain embodiments, the CH1 domain comprises the amino acid sequence as set forth in SEQ ID NO: 7. In certain embodiments, the CH2 domain is modified to reduce or eliminate effector functions, e.g., the CH2 domain comprises amino acid substitutions A330S and P331S (SEQ ID NO:24). In certain embodiments, the CH3 domain comprises the amino acid residue E at position 356 and the amino acid M at position 358 (SEQ ID NO: 5, allotype "f") and in certain embodiments, the CH3 domain comprises allotype "fa."

Methods provided herein include methods of treating a subject by administering an antibody, bispecific molecule or immunoconjugate comprising a modified heavy chain constant region. One or more additional therapeutic agents, e.g., a therapeutic agent that stimulates the immune system, such as a checkpoint inhibitor, a co-stimulatory molecule also can be co-administered.

Provided herein are antibodies comprising a modified heavy chain constant region comprising a CH1 domain, a hinge, a CH2 domain, and a CH3 domain in order from N- to C-terminus, and wherein (a) the CH1 domain comprises the amino acid sequence of SEQ ID NO: 7 or an amino acid sequence that differs therefrom in at most 5 amino acids or which is at least 95% identical to SEQ ID NO: 7, and wherein at least one of C131, R133, E137, S138 or R217 are not substituted or deleted; (b) a hinge comprising any one of SEQ ID NO: 8, 21-23, 126-132 or 134-147 or a sequence that comprises 1-3 amino acids inserted between CVE and CPP, or which differs therefrom in at most 5 amino acids, wherein the hinge does not comprise a substitution or deletion at both C219 and C220; (c) the antibody has at least one enhanced property or a new introduced property relative to the same antibody that comprises an IgG1 hinge and CH1 domain; and (d) the modified heavy chain constant region is not a wildtype IgG2 constant region or an IgG2 constant region comprising C219S and/or C220S. The hinge may comprise the amino acid sequence ERKXCVECPPCPAP (SEQ ID NO: 129) or ERKCXVECPPCPAP (SEQ ID NO: 130), wherein X is any amino acid except cysteine. For example, the hinge may comprise the amino acid sequence ERKSCVECPPCPAP (SEQ ID NO: 131) or ERKCSVECPPCPAP (SEQ ID NO: 132). In certain embodiments at least one of, or all of, amino acid residues P233, V234, A235 and G237 are deleted or substituted with another amino acid residue, e.g,. the corresponding amino acid in an IgG1 hinge. In certain embodiments, none of amino acid residues R133, E137, S138 and R217 or none of C131, R133, E137, S138 and R217 are substituted or deleted. In certain embodiments, N192 and/or F193 are substituted with another amino acid. The antibody may comprise a CH2 domain that is at least 95% identical to that of wildtype IgG1. The antibody may comprise a CH3 domain that is at least 95% identical to that of wildtype IgG1. In certain embodiments, the CH2 and/or CH3 domain is not a wildtype IgG1 CH2 and/or CH3 domain, and the antibody has an effector function that is more potent than that of wildtype IgG 1. In certain embodiments, the CH2 and/or CH3 domain is not a wildtype IgG1 CH2 and/or CH3 domain, and the antibody has an effector function that less potent than that of wildtype IgG1. In certain embodiments, the antibody comprises a CH2 domain and/or CH1 domain that is at least 95% identical to that of wildtype IgG1 or IgG4. In certain embodiments, the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

In certain embodiments, an antibody comprises a modified heavy chain constant region, wherein (a) the CH1 domain is a wildtype human IgG2 CH1 domain; (b) the hinge comprises SEQ ID NO: any one of SEQ ID NO: 8, 21-23, 126-132 or 134-147 or a sequence that comprises 1-3 amino acids inserted between CVE and CPP; (c) the CH2 domain is a wildtype human IgG 1 CH2 domain or a modified CH2 domain conferring enhanced or reduced effector function to the antibody; and (d) the CH3 domain is a wildtype human IgG1 CH3 domain or a modified CH3 domain conferring enhanced or reduced effector function to the antibody. A modified heavy chain constant domain may comprise the amino acid sequence set forth in any one of SEQ ID NOs: 26-37, 54-56, 78-125, 152-232, 234-245 and 247-262, or an amino acid sequence that is at least 95% identical to one or more of SEQ ID NOs: 26-37, 54-56, 78-125, 152-232, 234-245 and 247-262. For heavy chains that comprise an Fc having an amino acid sequence that is at least 95% identical to any of these sequences, it is preferable that the specific amino acid mutations made to modulate biological activity in these sequences are not varied.

In certain embodiments, an antibody comprises a modified heavy chain constant region, wherein the heavy chain constant region comprises a CH1 domain and a hinge comprising the sequence ASTKGPSVFPLAPCS**R**STS**ES**TAALGCLVKDYFPEPVTVSWNSGALTSGVHTFPAVL QSSGLYSLSSVVTVPSS*NF*GTQTYTCNVDHKPSNTKVDKTVE**RK***CC*VECPPCPAP*PV AG* (SEQ ID NO: 133), or an amino acid sequence that differs from SEQ ID NO: 133 in at most 10 amino acids or is at least 90% identical to SEQ ID NO: 133, wherein (i) at least one of C131, R133, E137, S138 and R217 is not substituted with another amino acid or deleted; (ii) C219 and C220 may be substituted with another amino acid or deleted, but C219 and C220 may not both be substituted or deleted; (iii) 1-3 amino acids may be inserted between CVE and CPP in the hinge; (iv) the hinge optionally comprises an additional amino acid at the C-terminus, e.g., G; (v) one or more of amino acids P233, V234, A235 and G237 may be substituted with another amino acid (e.g., the corresponding amino acid from IgG1) or deleted; (vi) the CH2 and CH3 domains may be wildtype or modified IgG1, IgG2, IgG3 or IgG4 CH2 and CH3 domains; (vii) the modified heavy chain constant region is not a wildtype IgG2 heavy chain constant region or a wildtype IgG2 heavy constant domain with C219S or C220S; and (viii) the antibody has at least one enhanced property or a new introduced property relative to the same antibody that comprises an IgG1 hinge and CH1 domain. In certain embodiments, the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity. In certain embodiments, none of amino acids C131; R133; E137; S138; R217 are substituted with another amino acid or deleted. In certain embodiments, N192 and/or F193 are not substituted or are N192S and/or F193L, respectively. In certain embodiments, C219 is C219S, C220 is C220S, P233-G237 are substituted or deleted; V234-G237 are substituted or deleted; A235-G237 are substituted or deleted; G237 is substituted or deleted; P233 is substituted or deleted; P233-V234 are substituted or deleted; or P233-A235 are substituted or deleted. The antibody may have effector function, or be deprived of effector function. The antibody may comprise a wildtype or modified IgG1 CH2 domain and or a wildtype or modified IgG1 CH3 domain.

In certain embodiments, an antibody comprises a modified heavy chain constant region, wherein the heavy chain constant region comprises a CH1 domain comprising the sequence ASTKGPSVFPLAPCS**R**STS**ES**TAALGCLVKDYFPEPVTVSWNSGALTSGVHTFPAVL QSSGLYSLSSVVTVPSS*NF*GTQTYTCNVDHKPSNTKVDKTVE (SEQ ID NO: 7), or an amino acid sequence that differs from SEQ ID NO: 7 in at most 10 amino acids or is at least 90% identical to SEQ ID NO: 7, wherein (i) at least one of C131, R133, E137, S138 and R217 is not substituted or deleted; (ii) the modified heavy chain constant region is not a wildtype IgG2 heavy chain constant region or a wildtype IgG2 heavy constant domain with C219S or C220S; and (iii) the antibody has at least one enhanced property or a new introduced property relative to the same antibody that comprises an IgG1 hinge and CH1 domain. The antibody may have at least one enhanced property selected from agonist activity, antibody mediated receptor internalization, ADCC, receptor mediated signaling, antagonist activity, immuno-modulating activity or anti-tumor activity; or a newly introduced property, which is agonist activity. In certain embodiments, none of amino acids C131; R133; E137 and S138 are substituted with another amino acid or deleted. In certain embodiments, N192 and/or F193 are not substituted or are N192S and/or F193L, respectively. The antibody may have effector function, or be deprived of effector function. The antibody may comprise a wildtype or modified IgG1 CH2 domain and or a wildtype or modified IgG1 CH3 domain.

An antibody may comprise a modified heavy chain constant region, wherein the heavy chain constant region comprises a hinge comprising the sequence E**RK**C*C*VECPPCPAP*P*VAG (SEQ ID NO: 8), or an amino acid sequence that differs from SEQ ID NO: 8 in at most 5 amino acids, wherein (i) C219 and C220 may be substituted with another amino acid or deleted, but C219 and C220 may not both be substituted or deleted;(ii) one or more of amino acids P233, V234, A235 and G237 may be substituted or deleted; (iii) 1-3 amino acids may be inserted between CVE and CPP in the hinge; (iv) the hinge optionally comprises an additional amino acid at the C-terminus, e.g., G; (v) the CH2 and CH3 domains may be wildtype or modified IgG1, IgG2, IgG3 or IgG4 CH2 and CH3 domains; (vi) the modified heavy chain constant region is not a wildtype IgG2 heavy chain constant region or a wildtype IgG2 heavy constant domain with C219S or C220S; and (vii) the antibody has at least one enhanced property or a new introduced property relative to the same antibody that comprises an IgG1 hinge and CH1 domain. The antibody may have at least one enhanced property selected from agonist activity, antibody mediated receptor internalization, ADCC, receptor mediated signaling, antagonist activity, immuno-modulating activity or anti-tumor activity; or a newly introduced property, which is agonist activity. In certain embodiments, C219 is C219S, C220 is C220S, P233-G237 are substituted or deleted; V234-G237 are substituted or deleted; A235-G237 are substituted or deleted; G237 is substituted or deleted; P233 is substituted or deleted; P233-V234 are substituted or deleted; or P233-A235 are substituted or deleted. The antibody may have effector function, or be deprived of effector function. The antibody may comprise a wildtype or modified IgG1 CH2 domain and or a wildtype or modified IgG1 CH3 domain.

Also provided are antibodies comprising a modified heavy chain constant region, wherein the heavy chain constant region comprises an IgG1 or IgG2 hinge, and wherein the hinge is lacking 1-7 amino acids, and wherein the antibody has at least one enhanced property or a new introduced property relative to the same antibody that comprises an IgG1 hinge and CH1 domain. The antibody may have at least one enhanced property selected from agonist activity, antibody mediated receptor internalization, ADCC, receptor mediated signaling, antagonist activity, immuno-modulating activity or anti-tumor activity; or a newly introduced property, which is agonist activity. The hinge may be an IgG2 hinge that is lacking 1-4 amino acids, e.g., amino acids C219, C220, V222 and E224. The hinge is an IgG1 hinge that is lacking amino acids S219, C220, D221, K222, T223, H224 and T225. The antibody may comprise an IgG2 CH1 domain that is wildtype or modified; an IgG1 CH1 domain that is wildtype or modified, and an IgG1, IgG2 or IgG4 CH2 domain and an IgG1, IgG2 or IgG4 CH3 domain.

Antibodies with modified heavy chain constant regions may be human or humanized antibodies, or antigen binding portions thereof. In certain embodiments, the antibody binds specifically to an antigen that is involved in immune regulation. The antibody may be an agonist of a costimulatory receptor or an antagonist of an inhibitory receptor. For example, the antibody may bind to a costimulatory receptor, e.g., selected from the group of B7-1, B7-2, CD28, 4-1BB, GITR, OX40, ICOS, CD70, CD27, CD40, DR3 or CD28H, or the antibody may bind to an inhibitory receptor, e.g., selected from the group of CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1 and TIM-4. The antigen may be an antigen that is required to be internalized, e.g., CD73. The antigen may be CD39.

In certain embodiments, an antibody comprising a modified heavy chain constant region binds specifically to a costimulatory receptor, e.g.,.GITR, OX40, 4-1BB, CD28, ICOS, CD40, CD27 or any other TNFR superfamily member, and comprises a modified heavy chain constant region selected from the group of SEQ ID NOs: 26-37, 54-56, 78-125, 152-232 , 234-245 and 247-262. In certain embodiments, the antibody exhibits enhanced or altered agonist activity relative to an antibody having the same variable regions and light chain, but comprising an IgG1 heavy chain constant region.

In certain embodiments, an antibody comprising a modified heavy chain constant region binds specifically to a cell surface molecule, e.g., CD73, and triggers antibody mediated internalization of the cell surface molecule, and comprises a modified heavy chain constant region selected from the group of SEQ ID NOs: 26-37, 54-56, 78-125 and 152-232. In certain embodiments, the antibody possesses enhanced or altered internalization properties relative to an antibody having the same variable regions and light chain, but comprising an IgG1 heavy chain constant region. Anti-CD73 antibodies may also be linked to an Fc having any an amino acid sequence selected from the group consisting of SEQ ID NOs: 234-245 and 247-262.

In certain embodiments, an antibody comprising a modified heavy chain constant region binds specifically to an inhibitory receptor, e.g., CTLA-4, PD-1, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1 and TIM-4, and comprises a modified heavy chain constant region selected from the group of SEQ ID NOs: 26-37, 54-56, 78-125, 152-232, 234-245 and 247-262. In certain embodiments, the antibody exhibits more potent or altered antagonist activity or introduces a new activity relative to the same antibody having an IgG1 heavy chain constant region. In certain embodiments, the Fc comprises one or more mutations to modulate, e.g., reduce, effector function.

In certain embodiments, an antibody comprising a modified heavy chain constant region binds specifically to a cell surface molecule and triggers intracellular signaling, wherein the antibody comprises a modified heavy chain constant region selected from the group of SEQ ID NOs: 26-37, 54-56, 78-125, 152-232, 234-245 and 247-262. In certain embodiments, intracellular signaling mediates agonist activity, antagonist activity, internalization of the cell surface molecule, or ADCC. In certain embodiments, the antibody triggers more potent intracellular signaling relative to to an antibody having the same variable regions and light chain, but comprising an IgG1 heavy chain constant region.

In certain embodiments, an antibody comprising a modified heavy chain constant region binds specifically to a cell surface molecule and triggers formation of high molecular weight antibody-cell surface molecule complexes, wherein the antibody comprises a modified heavy chain constant region selected from the group of SEQ ID NOs: 26-37, 54-56, 78-125, 152-232, 234-245 and 247-262. In certain embodiments, the antibody triggers formation of higher molecular weight complexes relative to an antibody having the same variable regions and light chain, but comprising an IgG1 heavy chain constant region.

In certain embodiments, an antibody comprising a modified heavy chain constant region binds specifically to a cell surface molecule and triggers clustering or oligomerization of the cell surface molecule, wherein the antibody comprises a modified heavy chain constant region selected from the group of SEQ ID NOs: 26-37, 54-56, 78-125, 152-232, 234-245 and 247-262. In certain embodiments, the antibody triggers more clustering or oligomerization of the cell surface molecule relative to an antibody having the same variable regions and light chain, but comprising an IgG1 heavy chain constant region.

Also provided herein are bispecific molecules comprising an antibody comprising a modified heavy chain constant region linked to a molecule having a second binding specificity. Also provided herein are immunoconjugates comprising an antibody comprising a modified heavy chain constant region, linked to a second agent. Composition comprising an antibody, bispecific or immunoconjugate described herein and a carrier are also provided. Compositions may comprise one or more additional therapeutic agents, e.g., a therapeutic agent stimulates the immune system, and is, e.g., an antagonist of a checkpoint inhibitor or a co-stimulatory receptor.

Also provided herein are methods of preparing an antibody comprising a modified heavy chain constant region, wherein the antibody comprises a CH1 domain, a hinge, a CH2 domain, and a CH3 domain in order from N- to C-terminus, comprising the steps of: (a) providing an antibody comprising a hinge and/or a CH1 domain that is not an IgG2 hinge and/or IgG2 CH1 domain; and (b) replacing the hinge and/or the CH1 domain with an IgG2 hinge and/or IgG2 CH1 domain, respectively. Further provided herein are methods of increasing internalization of an antibody by a cell, comprising: (a) providing an antibody comprising a hinge and/or a CH1 domain that is not an IgG2 hinge and/or IgG2 CH1 domain; and (b) replacing the hinge and/or the CH1 domain with an IgG2 hinge and/or IgG2 CH1 domain, respectively. Internalization of the antibody may be increased compared to internalization of the same antibody comprising a hinge of a non-IgG2 isotype, e.g., an antibody comprising an IgG1 constant region. Also provided are methods of increasing the agonist activity of an antibody, comprising: (a) providing an antibody comprising a hinge and/or a CH1 domain that is not an IgG2 hinge and/or IgG2 CH1 domain; and (b) replacing the hinge and/or the CH1 domain with an IgG2 hinge and/or IgG2 CH1 domain, respectively. The agonist activity may be increased compared to agonist activity of the same antibody comprising a hinge of a non-IgG2 isotype, e.g., an antibody comprising an IgG1 constant region. An IgG2 hinge may be a wildtype human IgG2 hinge, or comprises an amino acid sequence that is at least 95% identical to the amino acid sequence of a wildtype human IgG2 hinge and may comprise, e.g., a sequence set forth in Table 4. A method may comprise the step of replacing at least one of the CH1, CH2, or CH3 domains with a CH1, CH2, or CH3 domain of a different isotype, respectively. A method may comprise the steps of (a) replacing the CH1 domain with an IgG2 CH1 domain; (b) replacing the CH2 domain with an IgG1 CH2 domain; and/or (b) replacing the CH3 domain with an IgG1 CH3 domain. A method may comprise the steps of (a) replacing the CH1 domain with a wildtype human IgG2 CH1 domain, or a domain at least 95% identical thereto; (b) replacing the CH2 domain with a wildtype human IgG1 CH2 domain, or a domain at least 95% identical thereto; and/or (b) replacing the CH3 domain with a wildtype human IgG1 CH3 domain, or a domain at least 95% identical thereto. A method may comprise the step of replacing the heavy chain constant region with a modified heavy chain constant region comprising any one of SEQ ID NOs: 26-37, 54-56, 78-125, 152-232, 234-245 and 247-262 or a region at least 95% identical to SEQ ID NOs: 26-37, 54-56, 78-125, 152-232, 234-245 and 247-262 (or introducing into the Fc the amino acid mutations of these sequences). The hinge may be modified to reduce or alter disulfide bond formation. The hinge may comprise amino acid substitution C219S. The hinge may comprise an amino acid sequence set forth in any one of SEQ ID NO: 8, 21-23, 126-132 or 134-147 or a sequence that comprises 1-3 amino acids inserted between CVE and CPP. The CH1 domain may comprise the amino acid sequence ASTKGPSVFPLAPCSRSTSESTAALGCLVKDYFPEPVTVSWNSGALTSGVHTFPAVLQ SSGLYSLSSVVTVPSSNFGTQTYTCNVDHKPSNTKVDKTV (SEQ ID NO: 7). The CH2 domain may be modified to reduce or eliminate effector functions. The CH2 domain may comprise amino acid substitutions A330S and P331S. The CH2 domain may comprise the amino acid sequence PSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAK (SEQ ID NO: 4). The CH2 domain may comprise amino acid substitutions A330S and P331S. The CH3 domain may comprise the amino acid sequence

GQPREPQVYTLPPSREEMTKNQVSLTCLVKGFYPSDIAVEWESNGQPENNYKTTPPV LDSDGSFFLYSKLTVDKSRWQQGNVFSCSVMHEALHNHYTQKSLSLSPGK (SEQ ID NO: 5).

Also provided herein are modified heavy chain constant region with reduced or undetectable binding to one or more FcγRs (e.g., CD16, CD32, CD64). Such modified heavy chain constant regions may have 1-5, 1-3, 1-2 or a single mutation (e.g., substitution) relative to the wildtype heavy chain constant region.

Also provided are antibodies, or antigen binding portion thereof, produced by the methods described herein, e.g., set forth above, e.g., human or humanized antibodies. Methods of treating a subject, e.g., a subject having cancer, with any of the antibodies described herein are also encompassed herein. The methods may comprise administering one or more additional therapeutic agents, e.g., therapeutic agents that stimulate the immune system. For example, a therapeutic agent may target a checkpoint inhibitor or a co-stimulatory molecule. Methods may include administering a composition, bispecific molecule, or immunoconjugate described herein.

**BRIEF DESCRIPTION OF THE DRAWINGS**

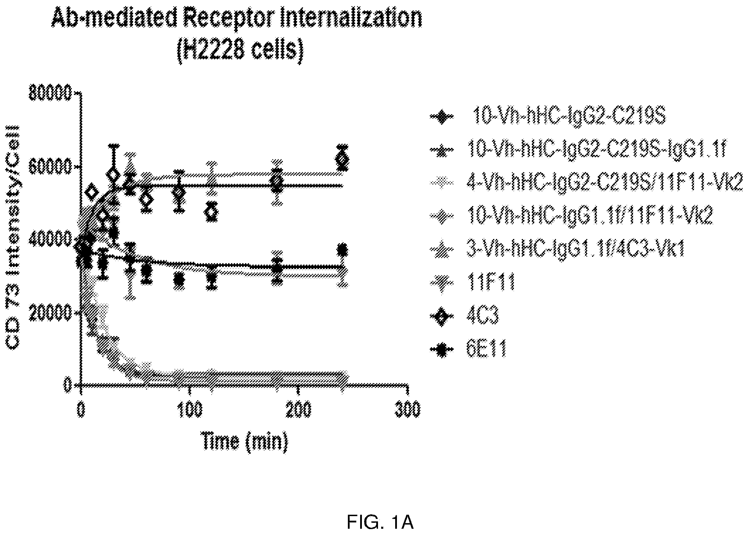

FIG. 1A shows the kinetics of antibody mediated internalization of CD73 in H2228 cells (non-small cell lung carcinoma cell line) by the following antibodies: 11F11, 4C3, 6D11, CD73.3-IgG1.1f with the 4C3Vk1 light chain ("3-Vh-hHC-IgG1.1f/4C3Vk1"), CD73.4-IgG2CS with the 11F11 Vk2 light chain ("4-Vh-hHC-IgG2-C219S/11F11-Vk2"), CD73.10-IgG2CS ("CD73.10-Vh-hHC-IgG2-C219S"), CD73.10-IgG2CS-IgG1.1f ("CD73.10-Vh-hHC-IgG2-C219S-IgG1.1f"), and CD73.10-IgG1.1f ("CD73.10-Vh-hHC-IgG1.1f") antibodies in H2228 cells. The 11F11 (which is of an IgG2 isotype), CD73.4-IgG2CS, CD73.10-IgG2CS and CD73.10-IgG2CS-IgG1.1f antibodies are internalized faster and to a higher degree than the other tested antibodies, which are of an IgG1 isotype.

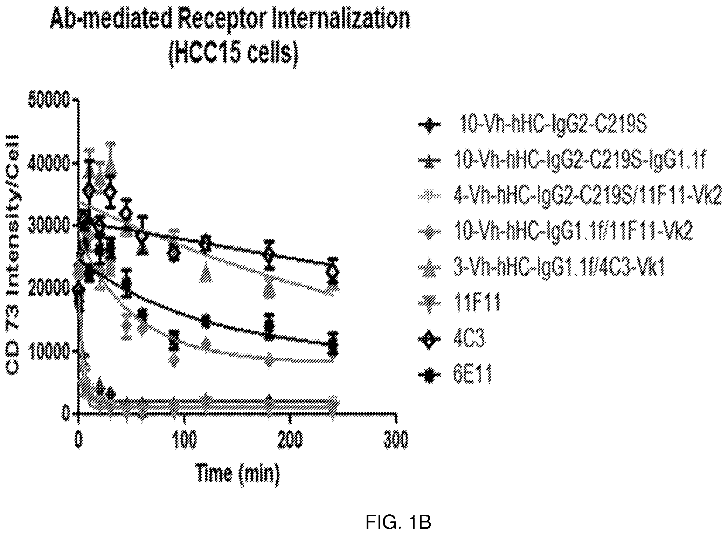

FIG. 1B shows the kinetics of antibody mediated CD73 internalization of the same antibodies as those shown in Figure 1A in HCC15 cells (non-small cell lung carcinoma cell line), showing similar results to those obtained in H2228 cells.

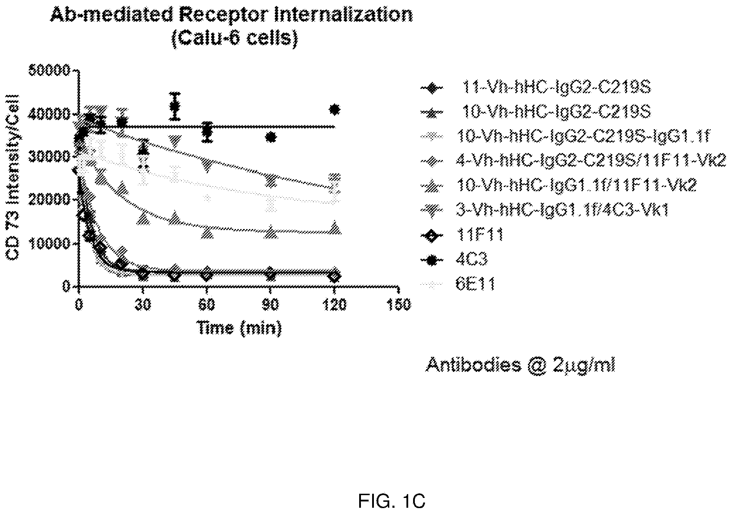

FIG. 1C shows the kinetics of antibody mediated CD73 internalization of the same antibodies as those shown in Figures 1A and 1B, as well as CD73.11-IgG2CS ("11-Vh-hVC-IgG2-C219S"), in Calu6 cells, showing similar results to those obtained in H2228 and HCC15 cells.

FIG. ID shows the kinetics of antibody mediated CD73 internalization of the same antibodies as those shown in Figure 1C in NCI-2030 cells (non-small cell lung carcinoma cell line), showing similar results to those obtained in H2228, HCC15, and Calu6 cells.

FIG. IE shows the kinetics of antibody mediated CD73 internalization of the indicated antibodies in Calu6 cells, as measured by flow cytometry.

FIG. IF shows the kinetics of antibody mediated CD73 internalization of the indicated antibodies in NCI-H292 cells (mucoepidermoid pulmonary carcinoma cell line), as measured by flow cytometry, but where the antibodies were not washed out after the first incubation of the cells with the antibodies.

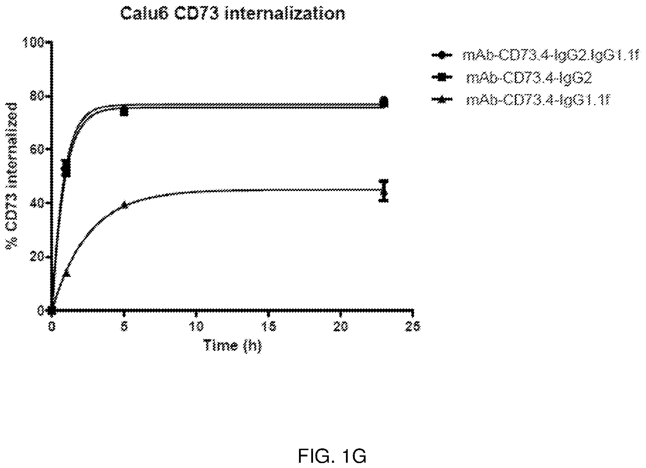

FIG. 1G shows the percentage of CD73 internalized in Calu6 cells treated with the indicated antibodies, showing antibody mediated CD73 internalization of the indicated antibodies in Calu6 cells over time.

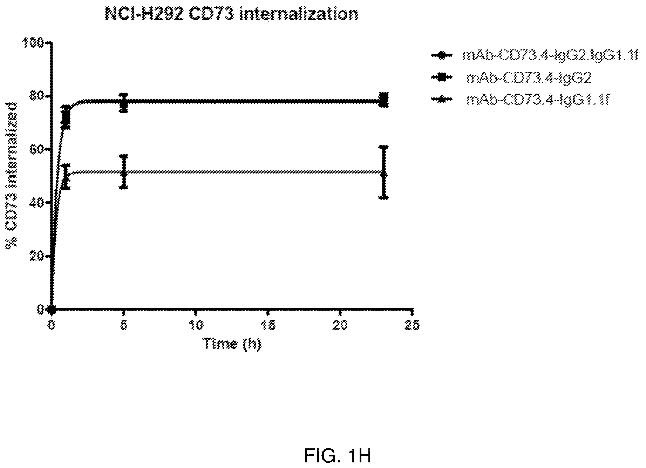

FIG. 1H shows the percentage of CD73 internalized in NCI-H292 cells treated with the indicated antibodies over time, showing antibody mediated CD73 internalization of the indicated antibodies in NCI-H292 cells over time.

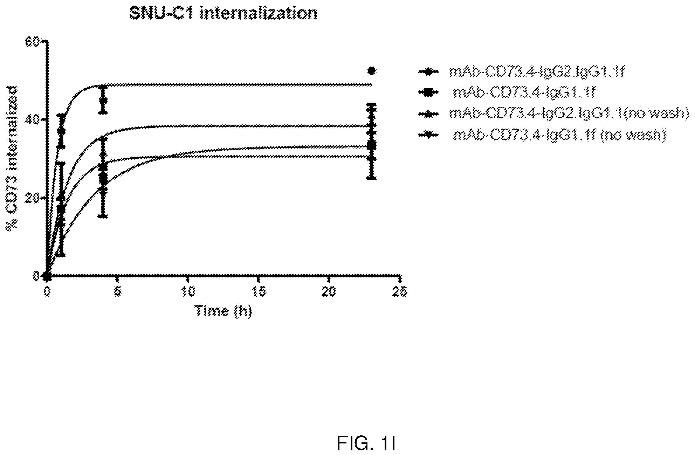

FIG. 1I shows the percentage of CD73 internalized in SNU-C1 cells (colon carcinoma cell line) treated with the indicated antibodies over time, showing antibody mediated CD73 internalization of the indicated antibodies in SNU-C1 cells over time.

FIG. 1**J** shows the percentage of CD73 internalized in NCI-H1437 cells (non-small cell lung carcinoma cell line) treated with the indicated antibodies over time, showing antibody mediated CD73 internalization of the indicated antibodies in NCI-H1437 cells over time.

FIG. 2 shows the binding kinetics of the indicated anti-human GITR antibodies to anti-CD3 (plate coated) and CD28-activated human CD4 T cells and their corresponding EC50 values derived from the graph.

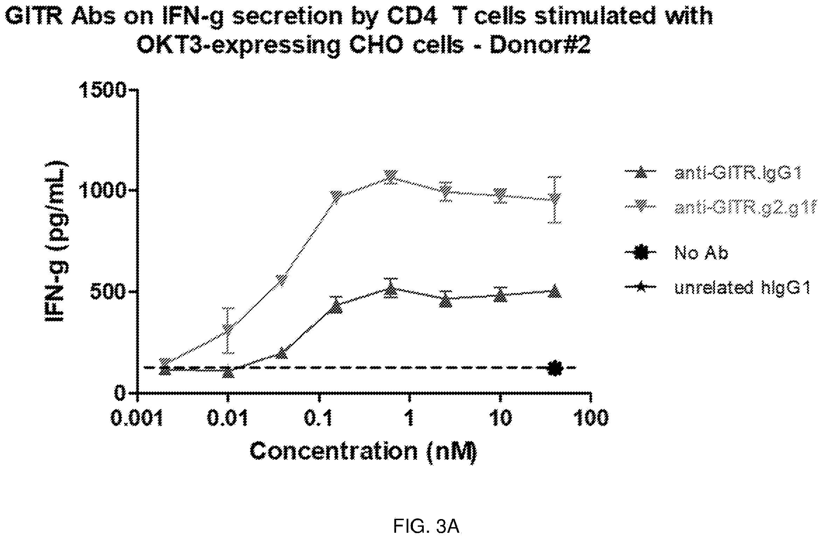

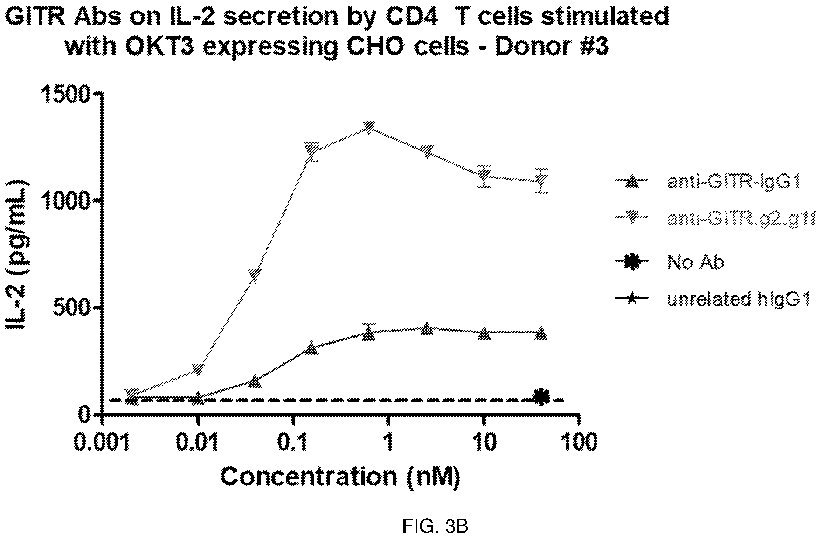

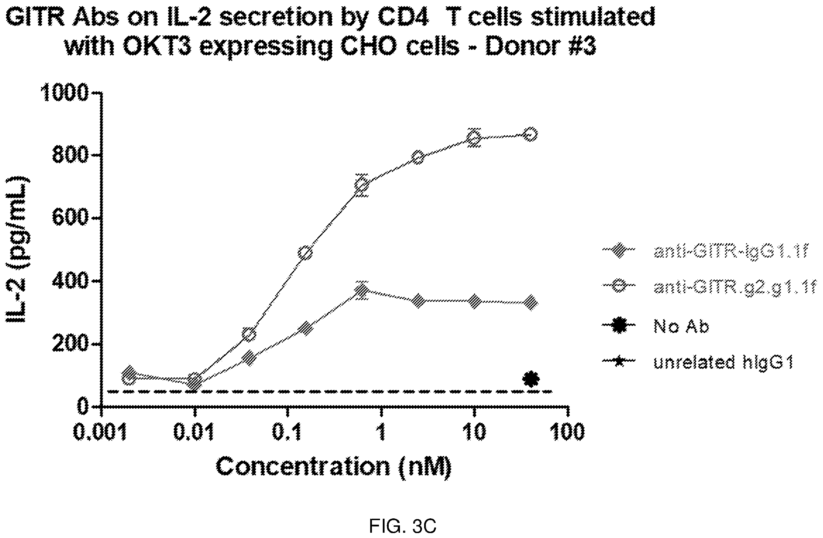

FIG. 3A, 3B and 3C show the secretion of IFN-γ and IL-2 from donor CD4 T cells stimulated with soluble anti-human GITR antibodies with different heavy chain constant regions. FIG. 3A shows IFN-γ secretion from donor CD4 T cells stimulated with OKT3 expressing CHO cells and various concentrations of anti-human GITR antibodies with an IgG2-IgG1 constant region. FIG. 3B shows IL-2 secretion from donor CD4 T cells stimulated with OKT3 expressing CHO cells and various concentrations of an IgG1 heavy chain constant domain or an IgG2-IgG1 hybrid heavy chain constant domain. FIG. 3C shows IL-2 secretion from donor CD4 T cells stimulated with OKT3 expressing CHO cells and various concentrations of effectorless versions (IgG1.1) of the antibodies in FIG. 3A and B.

FIG. 4 shows IL-2 secretion from 3A9-hGITR cells cultured on anti-CD3 monoclonal antibody-coated plates in the presence of increasing amounts of the indicated anti-human GITR antibodies: the hybridoma anti-GITR (IgG2) and recombinant derivatives as IgG1f, IgG1.1 (effectorless), or as chimera with the IgG2 hinge.

FIG. 5A, 5B, 5C and 5D show the effect of an IgG2 hinge on the size of antibody/antigen complexes. FIG. 5A, 5B and 5C show SEC chromatogram data, DLS data and MALS data, for complexes of hCD73-his with the antibody CD73.4 containing different constant regions. FIG. 5D shows a schematic model of the hCD73-his/mAb complexes derived from the MALS-determined masses in FIG. 5C.

FIG. 6 shows SEC-MALS data for CD73/mAb complexes.

FIG. 7 shows DLS data for CD73/mAb complexes.

FIG. 8A shows the percentage of CD73 internalized in Calu6 cells treated with the indicated antibodies over time, showing antibody mediated CD73 internalization of the indicated antibodies in Calu6 cells over time.

FIG. 8B shows the percentage of CD73 internalized in NCI-H292 cells treated with the indicated antibodies over time, showing antibody mediated CD73 internalization of the indicated antibodies in Calu6 cells over time.

FIG. 8C shows the level of CD73 on the surface of Calu6 cells treated with 5 $\mu$g/ml of the indicated antibodies for 0, 5, 15 or 30 minutes.

FIG. 9 shows the level of IL-2 secreted by CD4+ T cells co-cocultured with CHO-OKT3 cells in the presence of an anti-GITR antibody having the indicated constant regions.

FIG. 10 shows the percentage of antibody mediated CD73 internalization at 1, 4 or 21 hours after the addition of each of the shown antibodies. The bars for each antibody are shown in the order of 21 hours (on the left), 4 hours (middle) and 1 hour (right).

FIG. 11A shows overlay of SEC chromatogram data for 1:1 molar complexes of hCD73-his with 16 different CD73.4 antibodies containing different constant region sequences.

FIG. 11B shows an expansion of the chromatogram data from 11 - 19.5 min of the chromatogram of Figure 10A, with 4 distinct elution species indicated.

FIG. 11C shows the percentage of the UV chromatogram signal area for peak 2 of Figure 11B, plotted for the 16 different antibody/CD73-his complexes. Data is sorted from left to right in order of increasing peak area.

FIG. 12 shows antibody binding to anti-his Fab captured FcyR-his proteins. Binding responses are plotted as a percentage of the theoretical Rmax assuming a 1:1 mAb:FcyR binding stoichiometry. The bars for each antibody are shown in the order provided by the color legends at the bottom of the slide.

FIG. 13 shows antibody binding to anti-his Fab captured FcgR-his proteins. Binding responses are plotted as a percentage of the theoretical Rmax assuming a 1:1 mAb:FcyR binding stoichiometry. The bars for each antibody are shown in the order provided by the color legends at the bottom of the slide.

FIG. 14A shows antibody binding to anti-his Fab captured FcyR-his proteins. Binding responses are plotted as a percentage of the theoretical Rmax assuming a 1:1 mAb:FcyR binding stoichiometry. The bars for each antibody are shown in the order provided by the color legends at the bottom of the slide.

FIG. 14B shows antibody binding to anti-his Fab captured FcyR-his proteins. Binding responses are plotted as a percentage of the theoretical Rmax assuming a 1:1 mAb:FcyR binding stoichiometry. The bars for each antibody are shown in the order provided by the color legends at the bottom of the slide.

FIG. 15 shows an internalization time course analysis of anti-GITR antibodies.

FIG. 16A shows GITR and early endosome marker EEA2 co-localization analysis at time zero.

FIG. 16B shows GITR and early endosome marker EEA2 co-localization analysis at time 30 and 120 minutes.

FIG. 16C shows the results of quantification of endosomal co-localization shown in FIG. 16A and 16B plotted as the ratio of colocalized pixel intensity relative to total staining.

FIG. 17A shows NFkB signaling activation in CD8+ T cells treated with the indicated anti-GITR antibodies.

FIG. 17B shows NFkB signaling activation in CD4+ T cells treated with the indicated anti-GITR antibodies.

FIG. 18 shows P38 activation in CD4+ T cells treated with the indicated anti-GITR antibodies.

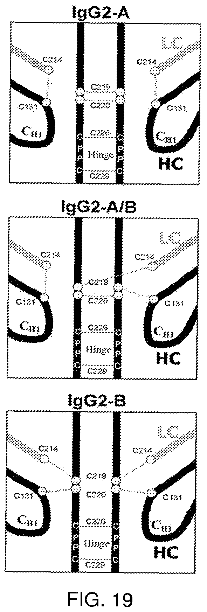

FIG. 19 shows the configuation of the disulfide bonds in IgG2 antibodies having conformation A, B or A/B.

FIG. 20A shows the level of IL-2 secreted by CD4+ T cells co-cocultured with CHO-OKT3 cells in the presence of different concentrations of an anti-GITR antibody having the indicated constant regions.

FIG. 20B shows the level of IL-2 secreted by CD4+ T cells co-cocultured with CHO-OKT3 cells in the presence of 5 $\mu$g/ml of an anti-GITR antibody having the indicated constant regions (same experiment as that in FIG. 20A).

FIG. 20C shows the level of IL-2 secreted by CD4+ T cells co-cocultured with CHO-OKT3 cells in the presence of 1.25 $\mu$g/ml of an anti-GITR antibody having the indicated constant regions (same experiment as that in FIG. 20A).

FIG. 20D shows the level of IL-2 secreted by CD4+ T cells co-cocultured with CHO-OKT3 cells in the presence of 0.313 μg/ml of an anti-GITR antibody having the indicated constant regions (same experiment as that in FIG. 20A).

FIG. 21 shows the amino acid sequence of a portion of hIgG1f, wherein the underlined sequences are reproduced below and show the location of the mutations in the hIgG1, hIgG1.1f, hIgG1.3f and hIgG1-P238K amino acid sequences relative to wild-type IgG1.

FIG. 22A, 22B, 22C, 22D, 22E, 22F, 22G, 22H, 22I, 22J, 22K, and 22L show a comparison of the dissociation rates of the antibody Y1238 in the context of different Fc regions from the indicated Fc receptors based on sensorgram data.

FIG. 23A, 23B, 23C, 23D, 23E, and 23F show the charge profiles for dAb-Fc molecules as characterized by icIEF.

## DETAILED DESCRIPTION

In certain embodiments, the invention is based, at least in part, on the findings that the following properties of antibodies are enhanced or altered when the antibodies comprise an IgG2 hinge relative to the same antibodies that comprise a non-IgG2 hinge (or relative to the same antibodies comprising an IgG1 constant region): (i) internalization; (ii) agonist function; (iii) receptor mediated intracellular signaling; (iv) ADCC; and (v) weight of antibody/antigen complexes. In addition, these enhanced or altered features of antibodies are further enhanced or altered when the antibodies comprise, in addition to an IgG2 hinge, an IgG2 CH1 domain. It has also been observed that antibodies having an IgG2 CH1 domain, but not an IgG2 hinge, have enhanced or altered activities compared to the same antibodies having an IgG1 CH1 domain. Without wanting to be limited to a particular mechanism of action, the enhancing effects of an IgG2 hinge has been found to correlate with an increase in size of antibody/antigen complexes. The enhanced size of antibody/antigen complexes when the antibody has an IgG2 hinge may result from a higher rigidity of IgG2 hinges relative to that of other isotypes. Furthermore, it has been shown that specific regions or amino acid residues of the IgG2 hinge and CH1 domain may be modified, whereas others are preferably not modified, to preserve the enhanced or altered activities.

As further described herein, these modified heavy chain constant regions conferring onto antibodies (or antigen binding regions thereof) enhanced or modified activities may have effector function. Thus, it was shown that antibodies may be created that have the advantageous properties conferred by an IgG2 hinge and/or CH1 domain and also have effector function.

The invention is also based at least in part on the finding that deletion of certain portions of a hinge in an IgG1 or IgG2 antibody results in the antibody having enhanced or altered properties relative to the antibody with an IgG1 constant region.

Also described herein are modified heavy chain regions that have mutations that reduce ADCC and/or CDC effector function, e.g., a P238 mutation, e.g., P238K, and in some some embodiments, such one or more mutation is combined with a mutation that enhances (i) internalization; (ii) agonist function; (iii) receptor mediated intracellular signaling; (iv) ADCC; and/or (v) weight of antibody/antigen complexes.

Accordingly, provided herein are (i) antibodies having modified heavy chain constant regions conferring to the antigen binding regions of the antibodies enhanced or altered properties and methods of using them, and (ii) methods for enhancing or altering certain biological properties of antibodies that comprise a non-IgG2 hinge and/or CH1 domain, such as internalization, agonism and antagonism, wherein the method comprises replacing the non-IgG2 hinge and/or CH1 domain of the antibody with an IgG2 hinge and/or IgG2 CH1 domain or portion thereof.

Provided herein are "modified heavy chain constant regions" that enhance certain biological properties of antibodies, e.g., antibodies that have a non-IgG2 hinge and/or a non-IgG2 CH1 domain, relative to the same antibodies having different constant regions. Exemplary modified heavy chain constant regions include an IgG2 hinge, a CH1 domain, a CH2 domain and a CH3 domain, wherein at least one of these constant domains is not of the IgG2 isotype and may be, *e.g.,* of an IgG1, IgG3 or IgG4. In certain embodiments, a modified heavy chain constant region comprises an IgG2 hinge and IgG1 CH2 and CH3 domains. In certain embodiments, a modified heavy chain constant region comprises an IgG2 CH1 domain and an IgG2 hinge. In certain embodiments, a modified heavy chain constant region comprises an IgG2 CH1 domain, an IgG2 hinge, an IgG1 CH2 domain and an IgG1 CH3 domain. A modified heavy chain constant region may have effector function similar to that of wild-type IgG1, or may be engineered to have reduced or enhanced effector function relative to that of the wildtype IgG. A modified heavy chain constant region may comprise a wildtype CH1, hinge, CH2 and/or CH3 domain, or a variant thereof, e.g., a CH1, hinge, CH2 and/or CH3 domain having one or more amino acid substitutions, deletions or additions relative to the corresponding wildtype domain, and/or having an amino acid sequence that is at least 90% identical, or more, to the corresponding wildtype sequence.

Also provided are antibodies and fusion proteins comprising an IgG1.3 heavy chain constant region. An antibody comprising an IgG1.3 heavy chain constant region may be an antagonist or an agonist antibody, such as a an antagonist antibody to a checkpoint inhibitor or an agonist antibody to a checkpoint stimulator.

**Definitions**

In order that the present description may be more readily understood, certain terms are first defined. Additional definitions are set forth throughout the detailed description.

The term "antibody" as used herein may include whole antibodies and any antigen binding fragments *(e.g.,* an antigen binding fragment that includes a hinge, an antigen binding fragment that includes a hinge and a CH1 domain, an antigen binding fragment that includes a hinge and CH2 domain, or an antigen binding fragment that includes a hinge, a CH2 domain and a portion of a CH3 domain) or single chains thereof. In one embodiment, an "antibody" refers to a protein, e.g., a glycoprotein, comprising at least two heavy (H) chains and two light (L) chains inter-connected by disulfide bonds, or an antigen binding portion thereof. Each heavy chain is comprised of a heavy chain variable region (abbreviated herein as $V_H$) and a heavy chain constant region. In certain naturally occurring IgG, IgD and IgA antibodies, the heavy chain constant region is comprised of a hinge, a CH1 domain, a CH2 domain and a CH3 domain. In certain naturally occurring antibodies, each light chain is comprised of a light chain variable region (abbreviated herein as $V_L$) and a light chain constant region. The light chain constant region is comprised of one domain, CL. The $V_H$ and $V_L$ regions can be further subdivided into regions of hypervariability, termed complementarity determining regions (CDR), interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

An immunoglobulin may be from any of the commonly known isotypes, including but not limited to IgA, secretory IgA, IgG and IgM. The IgG isotype is divided in subclasses in certain species: IgG1, IgG2, IgG3 and IgG4 in humans, and IgG1, IgG2a, IgG2b and IgG3 in mice. In certain embodiments, the antibodies described herein are of the human IgG1 or IgG2 subtype. Immunoglobulins, e.g., human IgG1, exist in several allotypes, which differ from each other in at most a few amino acids. "Antibody" may include, by way of example, both naturally occurring and non-naturally occurring antibodies; monoclonal and polyclonal antibodies; chimeric and humanized antibodies; human and nonhuman antibodies; wholly synthetic antibodies; and single chain antibodies.

In certain embodiments, a heavy chain of an antibody comprises a C-terminal lysine; a C-terminal glycine (having lost the C-terminal lysine), or is lacking GK or is lacking K. When referring to antibodies comprising a modified heavy chain constant region described herein, the antibody may comprise a provided sequence having the C-terminal GK or K, or alternatively, lacking GK or K.

Amino acid numbering is according to the EU index as in Kabat. Kabat et al. (1991) Sequences of Proteins of Immunological Interest, National Institutes of Health, Bethesda, MD, and according to FIGs. 3c-3f of U.S. Pat. App. Pub. No. 2008/0248028.

The term "antigen-binding portion" of an antibody, as used herein, refers to one or more fragments of an antibody that retain the ability to specifically bind to an antigen. An antigen-binding portion of an antibody can be a "hinge containing antigen binding portion." It has been shown that the antigen-binding function of an antibody can be performed by fragments of a full-length antibody. Examples of binding fragments encompassed within the term "antigen-binding portion" of an antibody described herein, include (i) a Fab fragment, a monovalent fragment consisting of the $V_L$, $V_H$, CL and CH1 domains; (ii) a F(ab')$_2$ fragment, a bivalent fragment comprising two Fab fragments linked by a disulfide bridge at the hinge region; (iii) a Fd fragment consisting of the $V_H$ and CH1 domains; (iv) a Fv fragment consisting of the $V_L$ and $V_H$ domains of a single arm of an antibody, (v) a dAb fragment (Ward et al., (1989) Nature 341:544-546), which consists of a $V_H$ domain; and (vi) an isolated complementarity determining region (CDR) or (vii) a combination of two or more isolated CDRs which may optionally be joined by a synthetic linker. Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These and other potential constructs are described at Chan & Carter (2010) Nat. Rev. Immunol. 10:301. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

A "CDR" of a variable domain are amino acid residues within the hypervariable region that are identified in accordance with the definitions of the Kabat, Chothia, the combination of both Kabat and Chothia, AbM, contact, and/or conformational definitions or any method of CDR determination well known in the art. Antibody CDRs may be identified as the hypervariable regions originally defined by Kabat et al. See, e.g., Kabat et al., 1992, Sequences of Proteins of Immunological Interest, 5th ed., Public Health Service, NIH, Washington D.C. The positions of the CDRs may also be identified as the structural loop structures originally described by Chothia and others. See, e.g., Chothia et al., 1989, Nature 342:877-883. Other approaches to CDR identification include the "AbM definition," which is a compromise between Kabat and Chothia and is derived using Oxford Molecular's AbM antibody modeling software (now Accelrys®), or the "contact definition" of CDRs based on observed antigen contacts, set forth in MacCallum et al., 1996, J. Mol. Biol., 262:732-745. In another approach, referred to herein as the "conformational definition" of CDRs, the positions of the CDRs may be identified as the residues that make enthalpic contributions to antigen binding. See, e.g., Makabe et al., 2008, Journal of Biological Chemistry, 283:1156-1166. Still other CDR boundary definitions may not strictly follow one of the above approaches, but will nonetheless overlap with at least a portion of the Kabat CDRs, although they may be shortened or lengthened in light of prediction or experimental findings that particular residues or groups of residues or even entire CDRs do not significantly impact antigen binding. As used herein, a CDR may refer to CDRs defined by any approach known in the art, including combinations of approaches. The methods used herein may utilize CDRs defined according to any of these approaches. For any given embodiment containing more than one CDR, the CDRs may be defined in accordance with any of Kabat, Chothia, extended, AbM, contact, and/or conformational definitions.

As used herein, "isotype" refers to the antibody class (e.g., IgG1, IgG2, IgG3, IgG4, IgM, IgA1, IgA2, IgD, and IgE antibody) that is encoded by the heavy chain constant domain genes. The full-length amino acid sequence of each wild type human IgG constant region (including all domains, i.e., CH1 domain, hinge, CH2 domain, and CH3 domain) is cataloged in the UniProt database available on-line, e.g., as P01857 (IgG1), P01859 (IgG2), P01860 (IgG3), and P01861 (IgG4), or different allotypes thereof (SEQ ID NOs: 1, 6, 11, and 16, respectively). As used herein, a domain of a heavy chain constant region, e.g., the hinge, is of an "IgG1 isotype," "IgG2 isotype," "IgG3 isotype," or "IgG4 isotype," if the domain comprises the amino acid sequence of the corresponding domain of the respective isotype, or a variant thereof (that has a higher homology to the corresponding domain of the respective isotype than it does to that of the other isotypes).

"Allotype" refers to naturally occurring variants within a specific isotype group, which variants differ in a few amino acids (see, e.g., Jefferies et al. (2009) mAbs 1:1). Antibodies described herein may be of any allotype.

A "wildtype" protein or portion thereof is a version of the protein as it is found in nature. An amino acid sequence of a wildtype protein, e.g., a heavy chain constant region, is the amino acid sequence of the protein as it occurs in nature. Due to allotypic differences, there can be more than one amino acid sequence for a wildtype protein. For example, there are several allotypes of naturally occurring human IGg1 heavy chain constant regions (see, e.g., Jeffries et al. (2009) mAbs 1:1).

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (C1q) of the classical complement system. Thus, an Fc region of an antibody of isotype IgG comprises the heavy chain constant region of the antibody excluding the first constant region immunoglobulin domain (CH1). In IgG, IgA and IgD antibody isotypes, the Fc region comprises $C_{H2}$ and $C_{H3}$ constant domains in each of the antibody's two heavy chains; IgM and IgE Fc regions comprise three heavy chain constant domains ($C_H$ domains 2-4) in each polypeptide chain. For IgG, the Fc region comprises immunoglobulin domains consisting of the hinge, CH2 and CH3. For purposes herein, the Fc region is defined as starting at amino acid 216 and ending at amino acid 447, wherein the numbering is according to the EU index as in Kabat. Kabat et al. (1991) Sequences of Proteins of Immunological Interest, National Institutes of Health, Bethesda, MD, and according to FIGs. 3c-3f of U.S. Pat. App. Pub. No. 2008/0248028. The Fc may be a native (or naturally-occurring or wildtype) Fc, including any allotypic variant, or a variant Fc (e.g., a non-naturally occurring Fc), comprising, e.g., 1, 2, 3, 4, 5, 1-5, 1-10 or 5-10 or more amino acid mutations, e.g., substitutions, additions or deletions. For example a variant Fc may comprise an amino acid sequence that is at least 75%, 80%, 85%, 90%, 95%, 96%, 97%, 98% or 99% identical to a wildtype Fc. Modified or mutated Fcs may have enhanced or reduced effector function and/or half-life. The CH2 and CH3 regions are the primary site of effector functions and FcRn binding. Fc may refer to this region in isolation or in the context of an Fc-comprising protein polypeptide such as a "binding protein comprising an Fc region," also referred to as an "Fc fusion protein" (e.g., an antibody or immunoadhesin).

An "effector function" refers to the interaction of an antibody Fc region with an Fc receptor or ligand, or a biochemical event that results therefrom. Exemplary "effector functions" include C1q binding, complement dependent cytotoxicity (CDC), Fc receptor binding, FcγR-mediated effector functions such as ADCC and antibody dependent cell-mediated phagocytosis (ADCP), and downregulation of a cell surface receptor (e.g., the B cell receptor; BCR). Such effector functions generally require the Fc region to be combined with a binding domain (e.g., an antibody variable domain).

An "Fc receptor" or "FcR" is a receptor that binds to the Fc region of an immunoglobulin. FcRs that bind to an IgG antibody comprise receptors of the FcγR family, including allelic variants and alternatively spliced forms of these receptors. The FcγR family consists of three activating (FcγRI, FcγRIII, and FcγRIV in mice; FcγRIA, FcγRIIA, and FcγRIIIA in humans) and one inhibitory (FcγRIIB) receptor. Various properties of human FcγRs are summarized in Table 1. The majority of innate effector cell types coexpress one or more activating FcγR and the inhibitory FcγRIIB, whereas natural killer (NK) cells selectively express one activating Fc receptor (FcγRIII in mice and FcγRIIIA in humans) but not

the inhibitory FcγRIIB in mice and humans. Human IgG1 binds to most human Fc receptors and is considered equivalent to murine IgG2a with respect to the types of activating Fc receptors that it binds to.

**Table 1.** Properties of human FcγRs

| Fcγ | Allelic variants | Affinity for human IgG | Isotype preference | Cellular distribution |
|---|---|---|---|---|
| FcγRI (CD64) | None described | High ($K_D$ -10 nM) | IgG 1=3>4>>2 | Monocytes, macrophages, activated neutrophils, dendritic cells? |
| FcγRIIA (CD32a) | H131 | Low to medium | IgGl>3>2>4 | Neutrophils, monocytes, macrophages, eosinophils, dendritic cells, platelets |
| | R131 | Low | IgG 1>3>4>2 | |
| FcγRIIIA CD16a) | V158 | Medium | IgG 1=3>>4>2 | NK cells, monocytes, macrophages, mast cells, eosinophils, dendritic cells? |
| | F158 | Low | IgG 1=3>>4>2 | |
| FcγRIIB (CD32b) | I232 | Low | IgG 1=3=4>2 | B cells, monocytes, macrophages, dendritic cells, mast cells |
| | T232 | Low | IgG 1=3=4>2 | |

A "hinge", "hinge domain" or "hinge region" or "antibody hinge region" refers to the domain of a heavy chain constant region that joins the CH1 domain to the CH2 domain and includes the upper, middle, and lower portions of the hinge (Roux et al. J. Immunol. 1998 161:4083). The hinge provides varying levels of flexibility between the binding and effector regions of an antibody and also provides sites for intermolecular disulfide bonding between the two heavy chain constant regions. As used herein, a hinge starts at Glu216 and ends at Gly237 for all IgG isotypes (Roux et al., 1998 J Immunol 161:4083). The sequences of wildtype IgG1, IgG2, IgG3 and IgG4 hinges are shown in Table 2.

**Table 2.**

*Hinge region amino acids*

| Ig Type | C-terminal $C_H1^*$ | Upper Hinge | Middle Hinge | Lower Hinge |
|---|---|---|---|---|
| IgG1 | VDKRV (SEQID NO:57) | EPKSCDKTHT (SEQ ID NO:59) | CPPCP (SEQ ID NO:64) | APELLGG (SEQ ID NO:70) |
| IgG2 | VDKTV (SEQ ID NO:58) | ERK (SEQ ID NO: 60) | CCVECPPCP (SEQ ID NO:65) | APPVAG (SEQ ID NO:71) |
| IgG3 (17-15-15-15) | VDKRV | ELKTPLGDTTHT (SEQ ID NO:61) | CPRCP (SEQ ID NO:66) (EPKSCDTPPPCPRCP)3 (SEQ ID NO:67) | APELLGG |
| IgG3 (17-15-15) | VDKRV | ELKTPLGDTTHT | CPRCP (EPKS CDTPPPCPRCP)2 | APELLGG |
| IgG3 (17-15) | VDKRV | ELKTPLGDTTHT | CPRCP (EPKSCDTPPPCPRCP)1 | APELLGG |
| IgG3 (15-15-15) | VDKRV | EPKS (SEQ ID NO:62) | CDTPPPCPRCP (SEQ ID NO:68) (EPKSCDTPPPCPRCP)2 | APELLGG |
| IgG3 (15) | VDKRV | EPKS | CDTPPPCPRCP | APELLGG |

(continued)

*Hinge region amino acids*

| Ig Type | C-terminal C$_H$1* | Upper Hinge | Middle Hinge | Lower Hinge |
|---------|-------------------|-------------|--------------|-------------|
| IgG4 | VDKRV | ESKYGPP (SEQ ID NO:63) | CPSCP(SEQ ID NO:69) | APEFLGG |

\* C-terminal amino acid sequences of the CH1 domains.

The term "hinge" includes wildtype hinges (such as those set forth in Table 3), as well as variants thereof (e.g., non-naturally-occurring hinges or modified hinges). For example, the term "IgG2 hinge" includes wildtype IgG2 hinge, as shown in Table 3, and variants having 1, 2, 3, 4, 5, 1-3, 1-5, 3-5 and/or at most 5, 4, 3, 2, or 1 mutations, e.g., substitutions, deletions or additions. Exemplary IgG2 hinge variants include IgG2 hinges in which 1, 2, 3 or all 4 cysteines (C219, C220, C226 and C229) are changed to another amino acid. In a specific embodiment, an IgG2 hinge comprises a C219X or C220X substitution, wherein X is any amino acid, except cysteine. An IgG2 hinge may comprise a substitution, which alone, or together with one or more substitutions in other regions of the heavy or light chain will cause the antibody comprising the hinge to adopt form A or B (see, e.g., Allen et al. (2009) Biochemistry 48:3755). In certain embodiments, a hinge is a hybrid hinge that comprises sequences from at least two isotypes. For example, a hinge may comprise the upper, middle or lower hinge from one isotype and the remainder of the hinge from one or more other isotypes. For example, a hinge can be an IgG2/IgG1 hinge, and may comprise, e.g., the upper and middle hinges of IgG2 and the lower hinge of IgG1. A hinge may have effector function or be deprived of effector function. For example, the lower hinge of wildtype IgG 1 provides effector function.

A "non-IgG2" hinge refers to a hinge that is not of the IgG2 isotype.

The term "CH1 domain" refers to the heavy chain constant region linking the variable domain to the hinge in a heavy chain constant domain. As used herein, a CH1 domain starts at A118 and ends at V215. The term "CH1 domain" includes wildtype CH1 domains (such as having SEQ ID NO: 2 for IgG1 and SEQ ID NO: 7 for IgG2; Table 3), as well as variants thereof (e.g., non-naturally-occurring CH1 domains or modified CH1 domains). For example, the term "CH1 domain" includes wildtype CH1 domains and variants thereof having 1, 2, 3, 4, 5, 1-3, 1-5, 3-5 and/or at most 5, 4, 3, 2, or 1 mutations, e.g., substitutions, deletions or additions. Exemplary CH1 domains include CH1 domains with mutations that modify a biological activity of an antibody, such as ADCC, CDC or half-life. Modifications to the CH1 domain that affect a biological activity of an antibody are provided herein.

The term "CH2 domain" refers to the heavy chain constant region linking the hinge to the CH3 domain in a heavy chain constant domain. As used herein, a CH2 domain starts at P238 and ends at K340. The term "CH2 domain" includes wildtype CH2 domains (such as having SEQ ID NO: 4 for IgG1; Table 3), as well as variants thereof (e.g., non-naturally-occurring CH2 domains or modified CH2 domains). For example, the term "CH2 domain" includes wildtype CH2 domains and variants thereof having 1, 2, 3, 4, 5, 1-3, 1-5, 3-5 and/or at most 5, 4, 3, 2, or 1 mutations, e.g., substitutions, deletions or additions. Exemplary CH2 domains include CH2 domains with mutations that modify a biological activity of an antibody, such as ADCC, CDC or half-life. In certain embodiments, a CH2 domain comprises the substitutions A330S/P331S that reduce effector function. Other modifications to the CH2 domain that affect a biological activity of an antibody are provided herein.

The term "CH3 domain" refers to the heavy chain constant region that is C-terminal to the CH2 domain in a heavy chain constant domain. As used herein, a CH3 domain starts at G341 and ends at K447. The term "CH3 domain" includes wildtype CH3 domains (such as having SEQ ID NO: 5 for IgG1; Table 3), as well as variants thereof (e.g., non-naturally-occurring CH3 domains or modified CH3 domains). For example, the term "CH3 domain" includes wildtype CH3 domains and variants thereof having 1, 2, 3, 4, 5, 1-3, 1-5, 3-5 and/or at most 5, 4, 3, 2, or 1 mutations, e.g., substitutions, deletions or additions. Exemplary CH3 domains include CH3 domains with mutations that modify a biological activity of an antibody, such as ADCC, CDC or half-life. Modifications to the CH3 domain that affect a biological activity of an antibody are provided herein.

**Table 3**

| Domain | Amino acid sequence | SEQ ID NO: |
|--------|---------------------|------------|
| IgG1 CH1 | ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKV | 2 |

(continued)

| Domain | Amino acid sequence | SEQ ID NO: |
|---|---|---|
| IgG1 Hinge | EPKSCDKTHTCPPCPAPELLGG | 3 |
| IgG1 CH2 | PSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAK | 4 |
| IgG1 CH3 | GQPREPQVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPGK | 5 |
| IgG2 CH1 | ASTKGPSVFPLAPCSRSTSESTAALGCLVKDYFPEPVTVSWNSGALTSGVHTFPAVLQS SGLYSLSSVVTVPSSNFGTQTYTCNVDHKPSNTKVDKTV | 7 |
| IgG2 Hinge | ERKCCVECPPCPAPPVAG | 8 |
| IgG2 CH2 | PSVFLFPPKPKDTLMISRTPEVTCVVVDVSHEDPEVQFNWYVDGVEVHNAKTKPREE QFNSTFRVVSVLTVVHQDWLNGKEYKCKVSNKGLPAPIEKTISKTK | 9 |
| IgG2 CH3 | GQPREPQVYTLPPSREEMTKNQVSLTCLVKGFYPSDIAVEWESNGQPENNYKTTPPML DSDGSFFLYSKLTVDKSRWQQGNVFSCSVMHEALHNHYTQKSLSLSPGK | 10 |
| IgG3 CH1 | ASTKGPSVFPLAPCSRSTSGGTAALGCLVKDYFPEPVTVSWNSGALTSGVHTFPAVLQ SSGLYSLSSVVTVPSSSLGTQTYTCNVNHKPSNTKVDKRV | 12 |
| IgG3 Hinge | ELKTPLGDTTHTCPRCPE | 13 |
| IgG3 CH2 | PKSCDTPPPCPRCPEPKSCDTPPPCPRCPEPKSCDTPPPCPRCPAPELLGGPSVFLFPPKP KDTLMISRTPEVTCVVVDVSHEDPEVQFKWYVDGVEVHNAKTKPREEQYNSTFRVV SVLTVLHQDWLNGKEYKCKVSNKALPAPIEKTISKTK | 14 |
| IgG3 CH3 | GQPREPQVYTLPPSREEMTKNQVSLTCLVKGFYPSDIAVEWESSGQPENNYNTTPPML DSDGSFFLYSKLTVDKSRWQQGNIFSCSVMHEALHNRFTQKSLSLSPGK | 15 |
| IgG4 CH1 | ASTKGPSVFPLAPCSRSTSESTAALGCLVKDYFPEPVTVSWNSGALTSGVHTFPAVLQS SGLYSLSSVVTVPSSSLGTKTYTCNVDHKPSNTKVDKRV | 17 |
| IgG4 Hinge | ESKYGPPCPSCPAPEFLGG | 18 |
| IgG4 CH2 | PSVFLFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAK | |
| IgG4 CH3 | GQPREPQVYTLPPSQEEMTKNQVSLTCLVKGFYPSDIAVEWESNGQPENNYKTTPPVL DSDGSFFLYSRLTVDKSRWQEGNVFSCSVMHEALHNHYTQKSLSLSLGK | 20 |

The term "monoclonal antibody," as used herein, refers to an antibody that displays a single binding specificity and affinity for a particular epitope or a composition of antibodies in which all antibodies display a single binding specificity and affinity for a particular epitope. Typically such monoclonal antibodies will be derived from a single cell or nucleic acid encoding the antibody, and will be propagated without intentionally introducing any sequence alterations. Accordingly, the term "human monoclonal antibody" refers to a monoclonal antibody that has variable and optional constant regions derived from human germline immunoglobulin sequences. In one embodiment, human monoclonal antibodies are produced by a hybridoma, for example, obtained by fusing a B cell obtained from a transgenic or transchromosomal non-human animal *(e.g.,* a transgenic mouse having a genome comprising a human heavy chain transgene and a light chain transgene), to an immortalized cell.

The term "recombinant human antibody," as used herein, includes all human antibodies that are prepared, expressed, created or isolated by recombinant means, such as (a) antibodies isolated from an animal *(e.g.,* a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies comprise variable and constant regions that utilize particular human germline immunoglobulin sequences are encoded by the germline genes, but include subsequent rearrangements and mutations that occur, for example, during antibody maturation. As known in the art (see, *e.g.,* Lonberg (2005) Nature Biotech. 23(9):1117-1125), the variable region contains the antigen binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen *(i.e.,* isotype switch). Therefore, the rearranged and somatically mutated nucleic acid sequences that encode the light chain and heavy chain immunoglobulin polypeptides in response to an antigen may not be identical to the original germline sequences, but instead will be substantially identical or similar *(i.e.,* have at least 80% identity).

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The antibodies described herein may include amino acid residues not encoded by human germline immunoglobulin sequences *(e.g.,* mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized" antibody refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

A "bispecific" or "bifunctional antibody" is an artificial hybrid antibody having two different heavy/light chain pairs, giving rise to two antigen binding sites with specificity for different antigens. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148, 1547-1553 (1992).

The phrases "an antibody recognizing an antigen" and "an antibody specific for an antigen" are used interchangeably herein with the term "an antibody which binds specifically to an antigen."

An "isolated antibody," as used herein, is intended to refer to an antibody that is substantially free of other antibodies having different antigenic specificities (e.g., an isolated antibody that specifically binds to antigen "x" is substantially free of antibodies that specifically bind antigens other than antigen "x"). An isolated antibody that specifically binds to an epitope of antigen "x" may, however, have cross-reactivity to other antigen "x" proteins from different species.

As used herein, an "agonist antibody" refers to an antibody that is an agonist of a co-stimulatory receptor, e.g., an antibody that is capable of boosting the immune system (or an immune response) of a subject by stimulating the activity of a protein that, in turn, stimulates an immune cell, e.g., a T cell, such as a B7-1, B7-2, CD28, 4-1BB (CD137), 4-1BBL, GITR, ICOS, ICOS-L, OX40, OX40L, CD70, or CD27, DR3, or CD28H protein. In certain embodiments, an agonist antibody is an antibody that enhances the activity of an inhibitory receptor, e.g., CTLA-4, PD-1, PD-L1, PD-L2, or LAG-3, TIM-3, Galectin 9, CEACAM-1, BTLA, CD69, Galectin-1, TIGIT, CD113, GPR56, VISTA, 2B4, CD48, GARP, CD73, PD1H, LAIR1, TIM-1, or TIM-4, and thereby inhibits an immune response.

As used herein, an "antagonist antibody" refers to an antibody that is an antagonist of an inhibitory signal on an immune cell, e.g., a T cell, e.g., an antibody that is capable of inhibiting or blocking a protein that inhibits T cell activation (e.g., immune checkpoint inhibitors), such as a CTLA-4, PD-1, PD-L1, PD-L2, or LAG-3, TIM-3, Galectin 9, CEACAM-1, BTLA, CD69, Galectin-1, TIGIT, CD113, GPR56, VISTA, 2B4, CD48, GARP, CD73, PD1H, LAIR1, TIM-1, or TIM-4, and thereby stimulates an immune response. In certain embodimetns, an antagonist antibody is an antibody that inhibits the activity of a stimulatory receptor, e.g., B7-1, B7-2, CD28, 4-1BB (CD137), 4-1BBL, GITR, ICOS, ICOS-L, OX40, OX40L, CD70, or CD27, DR3, or CD28H, and thereby inhibits an immune response.

Both agonist and antagonist antibodies result in amplifying antigen-specific T cell responses, or in inhibiting antigen-specific T cell responses (immune checkpoint regulators).

The term "epitope" or "antigenic determinant" refers to a site on an antigen (e.g., GITR) to which an immunoglobulin or antibody specifically binds. Epitopes within protein antigens can be formed both from contiguous amino acids (usually a linear epitope) or noncontiguous amino acids juxtaposed by tertiary folding of the protein (usually a conformational epitope). Epitopes formed from contiguous amino acids are typically, but not always, retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods for determining what epitopes are bound by a given antibody (*i.e.*, epitope mapping) are well known in the art and include, for example, immunoblotting and immunoprecipitation assays, wherein overlapping or contiguous peptides from are tested for reactivity with a given antibody. Methods of determining spatial conformation of epitopes include techniques in the art and those described herein, for example, x-ray crystallography, 2-dimensional nuclear magnetic resonance and HDX-MS (see, *e.g.*, Epitope Mapping Protocols in Methods in Molecular Biology, Vol. 66, G. E. Morris, Ed. (1996)).

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

A "polypeptide" refers to a chain comprising at least two consecutively linked amino acid residues, with no upper limit on the length of the chain. One or more amino acid residues in the protein may contain a modification such as, but not limited to, glycosylation, phosphorylation or a disulfide bond. A "protein" may comprise one or more polypeptides.

The term "nucleic acid molecule," as used herein, is intended to include DNA molecules and RNA molecules. A nucleic acid molecule may be single-stranded or doublestranded, and may be cDNA.

Also provided are "conservative sequence modifications" of the sequences set forth herein include, for example, conservative nucleotide and amino acid substitutions, as well as, nucleotide and amino acid additions and deletions. For example, modifications can be introduced into SEQ ID NOs: 1 - 74 by standard techniques known in the art, such as site-directed mutagenesis and PCR-mediated mutagenesis. Conservative sequence modifications include conservative amino acid substitutions, in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine, tryptophan), nonpolar side chains (*e.g.,* alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

In one embodiment, amino acid sequence modifications to a heavy chain constant region or domain thereof do not modify or abrogate certain properties of the heavy chain constant region. These properties include, e.g., the rigidity or stiffness of the hinge, as well as agonist or antagonist activity of the antibody. In certain embodiments, amino acid sequence modifications to a heavy chain constant region or domain thereof do modify or abrogate certain properties of the heavy chain constant region.

Methods of identifying amino acid conservative substitutions that do and do not abrogate antibody and/or constant region properties are well-known in the art, e.g., as described herein in the Examples section.

For nucleic acids, the term "substantial homology" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial homology exists when the segments will hybridize under selective hybridization conditions, to the complement of the strand.

For polypeptides, the term "substantial homology" indicates that two polypeptides, or designated sequences thereof, when optimally aligned and compared, are identical, with appropriate amino acid insertions or deletions, in at least about 80% of the amino acids, usually at least about 90% to 95%, and more preferably at least about 98% to 99.5% of the amino acids.

The percent identity between two sequences is a function of the number of identical positions shared by the sequences when the sequences are optimally aligned (*i.e.,* % homology = # of identical positions/total # of positions x 100), with optimal alignment determined taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The nucleic acid and protein sequences described herein can further be used as a "query sequence" to perform a search against public databases to, for example, identify related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to the nucleic acid molecules described herein. BLAST protein searches can be performed with the XBLAST program, score = 50, wordlength = 3 to obtain amino acid sequences homologous to the protein molecules described herein. To obtain gapped alignments for comparison purposes, Gapped BLAST can be utilized as described in Altschul et $\alpha$l., (1997) Nucleic Acids Res. 25(17):3389-3402. When utilizing BLAST and Gapped BLAST programs, the default parameters of the respective programs (e.g., XBLAST and NBLAST) can be used. See www.ncbi.nlm.nih.gov.

As used herein, the term "antigen" refers to any natural or synthetic immunogenic substance, such as a protein, peptide, or hapten. An antigen may be a full-length or mature protein, or a fragment thereof.

An "immune response" refers to a biological response within a vertebrate against foreign agents, which response protects the organism against these agents and diseases caused by them. An immune response is mediated by the action of a cell of the immune system (for example, a T lymphocyte, B lymphocyte, natural killer (NK) cell, macrophage, eosinophil, mast cell, dendritic cell or neutrophil) and soluble macromolecules produced by any of these cells or the liver (including antibodies, cytokines, and complement) that results in selective targeting, binding to, damage to, destruction of, and/or elimination from the vertebrate's body of invading pathogens, cells or tissues infected with pathogens, cancerous or other abnormal cells, or, in cases of autoimmunity or pathological inflammation, normal human cells or tissues. An immune reaction includes, e.g., activation or inhibition of a T cell, e.g., an effector T cell or a Th cell, such as a CD4+ or CD8+ T cell, or the inhibition of a Treg cell.

An "immunomodulator" or "immunoregulator" refers to an agent, e.g., a component of a signaling pathway, that may be involved in modulating, regulating, or modifying an immune response. "Modulating," "regulating," or "modifying" an immune response refers to any alteration in a cell of the immune system or in the activity of such cell (e.g., an effector T cell). Such modulation includes stimulation or suppression of the immune system which may be manifested by an increase or decrease in the number of various cell types, an increase or decrease in the activity of these cells, or any other changes which can occur within the immune system. Both inhibitory and stimulatory immunomodulators have been identified, some of which may have enhanced function in a tumor microenvironment. In preferred embodiments, the immunomodulator is located on the surface of a T cell. An "immunomodulatory target" or "immunoregulatory target" is an immunomodulator that is targeted for binding by, and whose activity is altered by the binding of, a substance, agent, moiety, compound or molecule. Immunomodulatory targets include, for example, receptors on the surface of a cell ("immunomodulatory receptors") and receptor ligands ("immunomodulatory ligands").

"Immunotherapy" refers to the treatment of a subject afflicted with, or at risk of contracting or suffering a recurrence of, a disease by a method comprising inducing, enhancing, suppressing or otherwise modifying an immune response.

"Immunostimulating therapy" or "immunostimulatory therapy" refers to a therapy that results in increasing (inducing or enhancing) an immune response in a subject for, e.g., treating cancer.

"Potentiating an endogenous immune response" means increasing the effectiveness or potency of an existing immune response in a subject. This increase in effectiveness and potency may be achieved, for example, by overcoming mechanisms that suppress the endogenous host immune response or by stimulating mechanisms that enhance the endogenous host immune response.

"T effector" ("$T_{eff}$") cells refers to T cells (e.g., CD4+ and CD8+ T cells) with cytolytic activities as well as T helper (Th) cells, which secrete cytokines and activate and direct other immune cells, but does not include regulatory T cells (Treg cells).

As used herein, the term "linked" refers to the association of two or more molecules. The linkage can be covalent or non-covalent. The linkage also can be genetic (*i.e.,* recombinantly fused). Such linkages can be achieved using a wide variety of art recognized techniques, such as chemical conjugation and recombinant protein production.

As used herein, "administering" refers to the physical introduction of a composition comprising a therapeutic agent to a subject, using any of the various methods and delivery systems known to those skilled in the art. Preferred routes of administration for antibodies described herein include intravenous, intraperitoneal, intramuscular, subcutaneous, spinal or other parenteral routes of administration, for example by injection or infusion. The phrase "parenteral administration" as used herein means modes of administration other than enteral and topical administration, usually by injection, and includes, without limitation, intravenous, intraperitoneal, intramuscular, intraarterial, intrathecal, intralymphatic, intralesional, intracapsular, intraorbital, intracardiac, intradermal, transtracheal, subcutaneous, subcuticular, intraarticular, subcapsular, subarachnoid, intraspinal, epidural and intrasternal injection and infusion, as well as *in vivo* electroporation. Alternatively, an antibody described herein can be administered via a non-parenteral route, such as a topical, epidermal or mucosal route of administration, for example, intranasally, orally, vaginally, rectally, sublingually or topically. Administering can also be performed, for example, once, a plurality of times, and/or over one or more extended periods.

As used herein, the term "T cell-mediated response" refers to a response mediated by T cells, including effector T cells (*e.g.,* CD8+ cells) and helper T cells (*e.g.,* CD4+ cells). T cell mediated responses include, for example, T cell cytotoxicity and proliferation.

As used herein, the term "cytotoxic T lymphocyte (CTL) response" refers to an immune response induced by cytotoxic T cells. CTL responses are mediated primarily by CD8+ T cells.

As used herein, the terms "inhibits" or "blocks" (e.g., referring to inhibition/blocking of a ligand to its receptor or to a subsequent intracellular response) are used interchangeably and encompass both partial and complete inhibition/blocking. In some embodiments, the antibody inhibits binding by at least about 50%, for example, at least about 60%, 70%, 80%, 90%, 95%, 99%, or 100%, determined, e.g., as further described herein.

As used herein, "cancer" refers a broad group of diseases characterized by the uncontrolled growth of abnormal cells in the body. Unregulated cell division may result in the formation of malignant tumors or cells that invade neighboring tissues and may metastasize to distant parts of the body through the lymphatic system or bloodstream.

The terms "treat," "treating," and "treatment," as used herein, refer to any type of intervention or process performed on, or administering an active agent to, the subject with the objective of reversing, alleviating, ameliorating, inhibiting, or slowing down or preventing the progression, development, severity or recurrence of a symptom, complication, condition or biochemical indicia associated with a disease. Prophylaxis refers to administration to a subject who does not have a disease, to prevent the disease from occurring or minimize its effects if it does.

A "hematological malignancy" includes a lymphoma, leukemia, myeloma or a lymphoid malignancy, as well as a cancer of the spleen and the lymph nodes. Exemplary lymphomas include both B cell lymphomas and T cell lymphomas. B-cell lymphomas include both Hodgkin's lymphomas and most non-Hodgkin's lymphomas. Non-limiting examples of B cell lymphomas include diffuse large B-cell lymphoma, follicular lymphoma, mucosa-associated lymphatic tissue lymphoma, small cell lymphocytic lymphoma (overlaps with chronic lymphocytic leukemia), mantle cell lymphoma (MCL), Burkitt's lymphoma, mediastinal large B cell lymphoma, Waldenström macroglobulinemia, nodal marginal zone B cell lymphoma, splenic marginal zone lymphoma, intravascular large B-cell lymphoma, primary effusion lymphoma, lymphomatoid granulomatosis. Non-limiting examples of T cell lymphomas include extranodal T cell lymphoma, cutaneous T cell lymphomas, anaplastic large cell lymphoma, and angioimmunoblastic T cell lymphoma. Hematological malignancies also include leukemia, such as, but not limited to, secondary leukemia, chronic lymphocytic leukemia, acute myelogenous leukemia, chronic myelogenous leukemia, and acute lymphoblastic leukemia. Hematological malignancies further include myelomas, such as, but not limited to, multiple myeloma and smoldering multiple myeloma. Other hematological and/or B cell- or T-cell-associated cancers are encompassed by the term hematological malignancy.

The term "effective dose" or "effective dosage" is defined as an amount sufficient to achieve or at least partially achieve a desired effect. A "therapeutically effective amount" or "therapeutically effective dosage" of a drug or therapeutic agent is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A "prophylactically effective amount" or a "prophylactically effective dosage" of a drug is an amount of the drug that, when administered alone or in combination with another therapeutic agent to a subject at risk of developing a disease or of suffering a recurrence of disease, inhibits the development or recurrence of the disease. The ability of a therapeutic or prophylactic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

By way of example, an anti-cancer agent is a drug that slows cancer progression or promotes cancer regression in a subject. In preferred embodiments, a therapeutically effective amount of a drug promotes cancer regression to the point of eliminating the cancer. "Promoting cancer regression" means that administering an effective amount of the drug, alone or in combination with an anti-neoplastic agent, results in a reduction in tumor growth or size, necrosis of the tumor, a decrease in severity of at least one disease symptom, an increase in frequency and duration of disease symptom-free periods, a prevention of impairment or disability due to the disease affliction, or otherwise amelioration of disease symptoms in the patient. Pharmacological effectiveness refers to the ability of the drug to promote cancer regression in the patient. Physiological safety refers to an acceptably low level of toxicity, or other adverse physiological effects at the cellular, organ and/or organism level (adverse effects) resulting from administration of the drug.

By way of example for the treatment of tumors, a therapeutically effective amount or dosage of the drug preferably inhibits cell growth or tumor growth by at least about 20%, more preferably by at least about 40%, even more preferably by at least about 60%, and still more preferably by at least about 80% relative to untreated subjects. In the most preferred embodiments, a therapeutically effective amount or dosage of the drug completely inhibits cell growth or tumor growth, i.e., preferably inhibits cell growth or tumor growth by 100%. The ability of a compound to inhibit tumor growth can be evaluated using the assays described *infra*. Alternatively, this property of a composition can be evaluated by examining the ability of the compound to inhibit cell growth, such inhibition can be measured *in vitro* by assays known to the skilled practitioner. In other preferred embodiments described herein, tumor regression may be observed and may continue for a period of at least about 20 days, more preferably at least about 40 days, or even more preferably at least about 60 days.

The terms "patient" and "subject" refer to any human or non-human animal that receives either prophylactic or therapeutic treatment. For example, the methods and compositions described herein can be used to treat a subject having cancer. The term "non-human animal" includes all vertebrates, e.g., mammals and non-mammals, such as non-human primates, sheep, dog, cow, chickens, amphibians, reptiles, *etc.*

Various aspects described herein are described in further detail in the following subsections.

## I. Modified heavy chain constant regions

Described herein are "modified heavy chain constant regions," which, when present in antibodies, enhance or alter certain biological properties or features of the antibodies, relative to the same antibodies that do not have a modified heavy chain constant region, such as antibodies that contain a non-IgG2 hinge, e.g., IgG1 antibodies. Enhanced or altered biological properties of antibodies include:

(a) increased or altered internalization by a cell;
(b) increased or altered agonist activity;
(c) increased or altered antagonist or blocking activity;
(d) enhanced ADCC;
(d) generation of a new property;
(e) increased or altered signal transduction;
(f) formation of larger antibody/antigen cross-linked complexes;
(g) increased clustering or oligomerization of the target cell surface molecule;
(h) increased stimulation or enhancement of an immune response; and/or
(i) increased inhibition of an immune response.

Also provided herein are antibodies comprising heavy chains comprising one or more amino acid mutation that modulates effector function, e.g., reduces effector function.

In certain embodiments, an antibody comprising a modified heavy chain constant region mediates antibody dependent receptor (or ligand or surface molecule) internalization more effectively, e.g., the antibody internalizes a target or surface molecule (e.g., a receptor or ligand) and/or is internalized itself with a higher rate and/or extent of internalization into a cell after the antibody binds to its target on the cell membrane, relative to the same antibody that does not comprise a modified heavy chain constant region, and comprises, e.g., an IgG1 heavy chain. The rate and extent of internalization of an antibody can be determined, e.g., as shown in the Examples. The rate of internalization, as measured, e.g., by $T_{1/2}$ of internalization, e.g., as shown in the Examples, can be enhanced or increased by at least 10%, 30%, 50%, 75%, 2 fold, 3 fold, 5 fold or more, resulting in a reduction of the $T_{1/2}$ by at least 10%, 30%, 50%, 75%, 2 fold, 3 fold, 5 fold or more. For example, instead of having a $T_{1/2}$ of 10 minutes, a modified heavy chain constant region may increase the rate of internalization and thereby reduce the $T_{1/2}$ to 5 minutes (i.e., a two fold increase in rate of internalization or a two-fold decrease in $T_{1/2}$). "$T_{1/2}$" is defined as the time at which half of the maximal internalization is achieved, as measured from the time the antibody is added to the cells. In certain embodiments, $T_{1/2}$ is reduced by at least 10 minutes, 30 minutes, or 1 hour. The maximal level of internalization can be the level of internalization at the plateau of a graph representing the internalization plotted against antibody concentrations or time. A modified heavy chain constant region may increase the maximal level of internalization of an antibody by at least 10%, 30%, 50%, 75%, 2 fold, 3 fold, 5 fold or more. Another way of comparing internalization efficacies of different antibodies, such as an antibody with, and the same antibody without, a modified heavy chain constant region, is by comparing their level of internalization at a given antibody concentration (e.g., 100nM) and/or at a given time (e.g., 2 minutes, 5 minutes, 10 minutes or 30 minutes). Comparing levels of internalization can also be done by comparing the $EC_{50}$ levels of internalization. The level of internalization of one antibody can be defined relative to that of a given (reference) antibody, e.g., an antibody described herein, e.g., 11F11 or CD73.4-IgG2CS-IgG1, and, can be indicated as a percentage of the value obtained with the given (reference) antibody. The extent of internalization can be enhanced by at least 10%, 30%, 50%, 75%, 2 fold, 3 fold, 5 fold or more, as compared by any one of these methods.

In certain embodiments, an antibody comprising a modified heavy chain constant region has more potent agonist activity, relative to the same antibody that does not comprise a modified heavy chain constant region, and comprises, e.g., an IgG1 heavy chain. In certain embodiments, the enhanced agonist activity enhances the stimulatory activity of a target molecule, e.g., GITR, or other moleceules that stimulate or co-stimulate an immune response, e.g., T cell activity. In certain embodiments, the enhanced agonist activity enhances the inhibitory activity of a target molecule that inhibits an immune response, e.g., T cell activity (e.g., a checkpoint inhibitor). The enhanced agonist activity of an antibody that modulates T cell activity can be determined, e.g., as shown in the Examples, e.g., by measuring the level of IFN-$\gamma$ or IL-2 secretion from T cells that are contacted with the antibody. The agonist activity of an antibody that binds to a stimulatory target may be enhanced by at least 10%, 30%, 50%, 75%, 2 fold, 3 fold, 5 fold or more as defined by increased cytokine release or increased proliferation of effector T cells; reduced T regulatory cell activity if engagement on Tregs reduces Treg function; or increased depletion of Tregs. For example, the amount of IFN-$\gamma$ or IL-2 secreted from T cells stimulated with an antibody that binds to a stimulatory target comprising a modified heavy chain constant region may be at least 10%, 30%, 50%, 75%, 2 fold, 3 fold, 5 fold or more higher than that of T cells simulated with the same antibody that does not comprise a modified heavy chain constant region. The agonist activity of an antibody that binds to an inhibitory target may be enhanced by at least 10%, 30%, 50%, 75%, 2 fold, 3 fold, 5 fold or more as defined by reduced cytokine release or reduced proliferation of effector T cells; increased T regulatory cell activity; or decreased depletion of Tregs. For example, the amount of IFN-$\gamma$ or IL-2 secreted from T cells stimulated with an antibody that binds to an inhibitory target comprising a modified heavy chain constant region may be at least 10%, 30%, 50%, 75%, 2 fold, 3 fold, 5 fold or more lower than that of T cells simulated with the same antibody that does not comprise a modified heavy chain constant region.

In certain embodiments, an antibody comprising a modified heavy chain constant region has more potent antagonist or blocking activity, relative to the same antibody that does not comprise a modified heavy chain constant region, and comprises, e.g., an IgG1 heavy chain. The enhanced antagonist activity of an antibody can be determined, e.g., by measuring cytokine release and/or proliferation in contexts that include conditions of T cell activation. The antagonist activity may be enhanced by at least 10%, 30%, 50%, 75%, 2 fold, 3 fold, 5 fold or more.

In certain embodiments, an antibody comprising a modified heavy chain constant region has enhanced ADCC activity, relative to the same antibody that does not comprise a modified heavy chain constant region, and comprises, e.g., an IgG1 heavy chain. Enhanced ADCC may be determined according to methods known in the art. ADCC may be enhanced by at least 10%, 30%, 50%, 2 fold, 5 fold or more.

In certain embodiments, an antibody comprising a modified heavy chain constant region has the ability to form larger antibody/antigen cross-linked complexes, relative to the same antibody that does not comprise a modified heavy chain constant region, and comprises, e.g., an IgG1 heavy chain. The ability to form complexes can be determined as described, e.g., in the Examples. Antibody/antigen complexes formed with an antibody that comprises a modified heavy chain constant region may be at least 50%, 2 fold, 3 fold, 5 fold or 10 folder larger than complexes formed with the same antibody that does not comprise a modified heavy chain constant region. In certain embodiments, complexes of at least 2,000 kDa; 3,000 kDa; 5000 kDa; 10,000 kDa, 50,000kDa or 100,000 kDa are formed with antibodies having a modified heavy chain constant region.

In certain embodiments, an antibody comprising a modified heavy chain constant region triggers more clustering or oligomerization of the target molecule on the cell surface, relative to the same antibody that does not comprise a modified heavy chain constant region, and comprises, e.g., an IgG1 heavy chain. The extent of clustering an oligomerization can be determined, e.g., by measuring the size of antibody/antigen complexes.

In certain embodiments, an antibody comprising a modified heavy chain constant region transduces a higher level or different type of signaling or signal transduction, relative to the same antibody that does not comprise a modified heavy chain constant region, and comprises, e.g., an IgG1 heavy chain. Signal transduction can be monitored by determining the level of activation of one or more proteins in signal transduction pathways. In certain embodiments, signal transduction is determined by measuring the activity (or phosphorylation) of a signal transduction protein, e.g., NFkB or p38, as described, e.g., in the Examples. Signal transduction triggered by an antibody that comprises a modified heavy chain constant region may be higher or lower by at least 10%, 20%, 50%, 2 fold, 5 fold or more than signal transduction with the same antibody that does not comprise a modified heavy chain constant region. For example, signal transduction triggered by an antibody that binds to a stimulatory molecule (e.g,. GITR) and comprises a modified heavy chain constant region may be enhanced by at least 10% relative to that obtained with the same antibody having an IgG1 heavy chain. For example, $EC_{50}$ of NFkB or p38 activity (e.g., phosphorylation) may be reduced by at least 50%, 2 fold, 5 fold or more.

In certain embodiments, an antibody comprising a modified heavy chain constant region has an increased ability to stimulate or enhance an immune response or the immune system, relative to the same antibody that does not comprise a modified heavy chain constant region, and comprises, e.g., an IgG1 heavy chain. An increased ability to stimulate an immune response or the immune system, can result from an enhanced agonist activity of T cell costimulatory receptors and/or an enhanced antagonist activity of inhibitory receptors. An increased ability to stimulate an immune response or the immune system may be reflected by a fold increase of the $EC_{50}$ or maximal level of activity in an assay that measures an immune response, e.g., an assay that measures changes in cytokine or chemokine release, cytolytic activity (determined directly on target cells or indirectly via detecting CD107a or granzymes) and proliferation. The ability to stimulate an immune response or the immune system activity may be enhanced by at least 10%, 30%, 50%, 75%, 2 fold, 3 fold, 5 fold or more.

In certain embodiments, an antibody comprising a modified heavy chain constant region has an increased anti-proliferative or anti-tumor activity, relative to the same antibody that does not comprise a modified heavy chain constant region, and comprises, e.g., an IgG1 heavy chain. The enhanced anti-tumor activity of an antibody can be determined, e.g., by the growth of a tumor in an animal that has been treated with the antibody. The anti-tumor activity may be enhanced by at least 10%, 30%, 50%, 75%, 2 fold, 3 fold, 5 fold or more. Anti-tumor activity can be measured, e.g., as a decrease in tumor burden, e.g., manifested by decreased tumor growth kinetics and complete tumor regressions.

In certain embodiments, an antibody comprising a modified heavy chain constant region has an increased ability to inhibit or suppress an immune response or the immune system, relative to the same antibody that does not comprise a modified heavy chain constant region, and comprises, e.g., an IgG1 heavy chain. An increased ability to inhibit or suppress an immune response or the immune system, can result from an enhanced antagonist activity of T cell costimulatory receptors and/or an enhanced agonist activity of inhibitory receptors. An increased ability to stimulate an immune response or the immune system may be reflected by a fold increase of the $EC_{50}$ or maximal level of activity in an assay that measures an immune response, e.g., an assay that measures changes in cytokine or chemokine release, cytolytic activity (determined directly on target cells or indirectly via detecting CD107a or granzymes) and proliferation. The ability to inhibit or suppress an immune response or the immune system activity may be enhanced by at least 10%, 30%, 50%, 75%, 2 fold, 3 fold, 5 fold or more.

In certain embodiments, a modified heavy chain constant region or portion thereof, e.g., the hinge, is more rigid, compared to other heavy chain constant regions, e.g., IgG1, IgG2, IgG3 and/or IgG4 heavy chain constant regions. For example, a modified heavy chain constant region is a non-naturally occurring heavy chain constant region that is more rigid than, or has a portion, e.g., the hinge, that is more rigid than a naturally-occurring heavy chain constant region or hinge thereof. The rigidity of a heavy chain constant region or portion thereof, such as the hinge, can be determined by e.g., by computer modeling, electron microscopy, spectroscopy such as Nuclear Magnetic Resonance (NMR), X-ray crystallography (B-factors), or Sedimentation Velocity Analytical ultracentrifugation (AUC) to measure or compare the radius of gyration of antibodies comprising the hinge. Alternatively, the rigidity of a heavy chain constant region or portion thereof can be determined by measuring the sizes of antibody/antigen complexes, e.g., as further described herein.

An antibody comprising a modified heavy chain constant region and exhibiting an enhanced functional property as determined according to methodologies known in the art and described herein, will be understood to relate to a statistically significant difference in the particular activity relative to that seen in the same antibody but with a different heavy chain constant region.

In certain embodiments, a modified heavy chain constant region comprises a hinge of the IgG2 isotype (an "IgG2 hinge") and a CH1, CH2 and CH3 domain. In certain embodiments, a modified heavy chain constant region comprises an IgG2 hinge and a CH1, CH2 and CH3 domain, wherein at least one of the CH1, CH2 and CH3 domains is not of the IgG2 isotype. In certain embodiments, a modified heavy chain constant region comprises an IgG2 hinge and a CH1, CH2 and CH3 domain, wherein the heavy chain constant domain is not a wild-type IgG2 constant region or is not an IgG2 constant region with a mutation at amino acid 219 or 220. The IgG2 hinge may be a wildtype IgG2 hinge, e.g., a wildtype human IgG2 hinge (e.g., having SEQ ID NO: 8) or a variant thereof, provided that the IgG2 hinge retains the ability to confer to the antibody an enhanced activity relative to that of the same antibody that comprises a non-IgG2 hinge or comprises an IgG1 heavy chain. In certain embodiments, an IgG2 hinge variant retains similar rigidity or stiffness to that of a wildtype IgG2 hinge. The rigidity of a hinge can be determined, e.g., by computer modeling, electron microscopy, spectroscopy such as Nuclear Magnetic Resonance (NMR), X-ray crystallography (B-factors), or Sedimentation Velocity Analytical ultracentrifugation (AUC) to measure or compare the radius of gyration of antibodies comprising the hinge. A hinge has similar or higher rigidity relative to that of another hinge if an antibody comprising the hinge has a value obtained from one of the tests described in the previous sentence that differs from the value of the same antibody with a different hinge, e.g., an IgG1 hinge, in less than 5%, 10%, 25%, 50%, 75%, or 100%. A person of skill in the art would be able to determine from the tests whether a hinge has at least similar rigidity to that of another hinge by interpreting the results of these tests.

An exemplary human IgG2 hinge variant is an IgG2 hinge that comprises a substitution of one or more of the four cysteine residues (i.e., C219, C220, C226 and C229) with another amino acid. A cysteine may be replaced by a serine. An exemplary IgG2 hinge is a human IgG2 hinge comprising a C219X mutation or a C220X mutation, wherein X is any amino acid exept cysteine. In a certain embodiments, an IgG2 hinge does not comprise both a C219X and a C220X substitution. In certain embodimetns, an IgG2 hinge comprises C219S or C220S, but not both C219S and C22S. Other IgG2 hinge variants that may be used include human IgG2 hinges comprising a C220, C226 and/or C229 substitution, e.g., a C220S, C226S or C229S mutation (which may be combined with a C219S mutation). An IgG2 hinge may also be an IgG2 hinge in which a portion of the hinge is that of another isotype (i.e., it is a chimeric or hybrid hinge), provided that the rigidity of the chimeric hinge is at least similar to that of a wildtype IgG2 hinge. For example, an IgG2 hinge may be an IgG2 hinge in which the lower hinge (as defined in Table 2) is of an IgG1 isotype, and is, e.g., a wildtype IgG1 lower hinge.

A "hybrid" or "chimeric" hinge is referred to as being of a specific isotype if more than half of the consecutive amino acids of the hinge are from that isotype. For example, a hinge having an upper and middle hinge of IgG2 and the lower hinge of IgG1 is considered to be an IgG2 hybrid hinge.

In certain embodiments, an antibody comprises a modified heavy chain constant region that comprises an IgG2 hinge comprising a sequence set forth in Table 4, e.g., one of the following amino acid sequences: 8, 21, 22, 23, 126-129, and 134-147. In certain embodiments, the hinge comprises SEQ ID NO: 8, 21, 126, 134 or 135, wherein 1, 2, 3 or all 4 amino acids P233,V234, A235 and G237 (corresponding to the C-terminal 4 amino acids "PVAG" (SEQ ID NO: 148) are deleted or substituted with another amino acid, e.g., the amino acids of the C-terminus of the IgG1 hinge (ELLG (SEQ ID NO: 149) or ELLGG (SEQ ID NO: 150). In certain embodiments, the hinge comprises SEQ ID NO: 8, 21, 126, 134 or 135, wherein V234, A235 and G237 are deleted or substituted with another amino acid. In certain embodiments, the hinge comprises SEQ ID NO: 8, 21, 126, 134 or 135, wherein A235 and G237 are deleted or substituted with another amino acid. In certain embodiments, the hinge comprises SEQ ID NO: 8, 21, 126, 134 or 135, wherein G237 is deleted or substituted with another amino acid. In certain embodiments, the hinge comprises SEQ ID NO: 8, 21, 126, 134 or 135, wherein V234 and A235 are deleted or substituted with another amino acid. Substitution of PVAG (SEQ ID NO: 143) in an IgG2 with the corresponding amino acids of an IgG1 hinge, i.e., (ELLG (SEQ ID NO: 144) or ELLGG (SEQ ID NO: 145)) to obtain a hybrid hinge having SEQ ID NO: 22 or 138 or variants thereof (see, e.g., Table 4) provides a hinge having the advantages of an IgG2 hinge and the effector function of IgG1 hinges.

In certain embodiments, a modified heavy chain constant region comprises a hinge that consists of or consists essentially of one of the sequences in Table 4, e.g., SEQ ID NOs: 8, 21, 22, 23, 127-132, and 134-141, and, in certain embodiments, does not comprise additional hinge amino acid residues.

**Table 4:** Exemplary IgG2 hinges

| IgG2 Hinge description | Amino acid sequence | SEQ ID NO: |
|---|---|---|
| Wildtype IgG2 | ERKCCVECPPCPAPPVAG | 8 |
| IgG2 with C219S | ERKSCVECPPCPAPPVAG | 21 |
| IgG2 with C220S | ERKCSVECPPCPAPPVAG | 126 |
| IgG2 with C219X | ERKXCVECPPCPAPPVAG | 134 |
| IgG2 with C220X | ERKCXVECPPCPAPPVAG | 135 |
| Wildtype IgG2 with C-terminal X | ERKCCVECPPCPAPPVAGX | 143 |
| IgG2 with C219S with C-terminal X | ERKSCVECPPCPAPPVAGX | 144 |
| IgG2 with C220S with C-terminal X | ERKCSVECPPCPAPPVAGX | 145 |
| IgG2 with C219X with C-terminal X | ERKXCVECPPCPAPPVAGX | 146 |
| IgG2 with C220X with C-terminal X | ERKCXVECPPCPAPPVAGX | 147 |
| IgG2/IgG1 hybrid | ERKCCVECPPCPAPELLGG | 22 |
| IgG2/IgG1 hybrid with C219S | ERKSCVECPPCPAPELLGG | 23 |
| IgG2/IgG1 hybrid with C220S | ERKCSVECPPCPAPELLGG | 127 |
| IgG2/IgG1 hybrid with C219X | ERKXCVECPPCPAPELLGG | 136 |
| IgG2/IgG1 hybrid with C220X | ERKCXVECPPCPAPELLGG | 137 |
| IgG2/IgG1 hybrid deltaG | ERKCCVECPPCPAPELLG | 138 |
| IgG2/IgG1 hybrid with C219S deltaG | ERKSCVECPPCPAPELLG | 139 |
| IgG2/IgG1 hybrid with C220S deltaG | ERKCSVECPPCPAPELLG | 140 |

(continued)

| IgG2 Hinge description | Amino acid sequence | SEQ ID NO: |
|---|---|---|
| IgG2/IgG1 hybrid with C219X deltaG | ERKXCVECPPCPAPELLG | 141 |
| IgG2/IgG1 hybrid with C220X deltaG | ERKCXVECPPCPAPELLG | 142 |
| Truncated wiltype IgG2 | ERKCCVECPPCPAP | 128 |
| Truncated wiltype IgG2 with C219S | ERKSCVECPPCPAP | 129 |
| Truncated wiltype IgG2 with C220S | ERKCSVECPPCPAP | 130 |
| Truncated wiltype IgG2 with C219X | ERKXCVECPPCPAP | 131 |
| Truncated wiltype IgG2 with C220X | ERKCXVECPPCPAP | 132 |
| X is any amino acid, except cysteine. | | |

In certain embodiments, a modified heav chain constant region comprises an IgG2 hinge set forth in Table 4, in which 1-5, 1-3, 1-2 or 1 amino acid is inserted between amino acid residues CVE and CPP. In certain embodiments, THT or GGG is inserted. In certain embodiments, 1, 1-2 or 1-3 amino acids may be inserted between the hinge and CH2 domain. For example, an additional glycine may be inserted between the hinge and the CH2 domain.

In certain embodiments a modified heavy chain constant region is an IgG1 or IgG2 constant region, wherein the hinge comprises a deletion of 1-10 amino acids. As shown in the Examples, an IgG1 antibody lacking amino acid residues SCDKTHT (S219, C220, D221, K222, T223, H224 and T225; SEQ ID NO: 151) conferred antibody mediated CD73 internalization more effectively than the same antibody having a wildtype IgG1 constant region. Similarly, in the context of an IgG2 antibody, an IgG2 antibody lacking amino acid residues CCVE (C219, C220, V222, and E224; SEQ ID NO: 152) conferred antibody mediated CD73 internalization more effectively than the same antibody having a wildtype IgG1 constant region. Accordingly, provided herein are modified heavy chain constant region in which the hinge comprises a deletion of 1, 2, 3, 4, 5, 6, or 7 amino acid residues, selected from residues S219, C220, D221, K222, T223, H224 and T225 for an IgG1 antibody, and residues C219, C220, V222, and E224 for an IgG2 antibody.

In certain embodiments, a modified heavy chain constant region comprises a CH1 domain that is a wildtype CH1 domain of the IgG1 or IgG2 isotype ("IgG1 CH1 domain" or "IgG2 CH1 domain," respectively). CH1 domains of the isotypes IgG3 and IgG4 ("IgG3 CH1 domain and "IgG2 CH1 domain," respectively) may also be used. A CH1 domain may also be a variant of a wildtype CH1 domain, e.g., a variant of a wildtype IgG1, IgG2, IgG3 or IgG4 CH1 domain. Exemplary variants of CH1 domains include A114C, C131S and/or T173C. A CH1 domain, e.g., an IgG2 CH1 domain, may comprise the substitution C131S, which substitution confers onto an IgG2 antibody or antibody having an IgG2 CH1 and hinge the B form (or conformation).

In certain embodiments, a modified heavy chain constant region comprises a CH1 domain that is of the IgG2 isotype. In certain embodiments, the CH1 domain is wildtype IgG2 CH1 domain, e.g., having the amino acid sequence: ASTKGPSVFPLAP**CSR**STS**ES**TAALGCLVKDYFPEPVTVSWNSGALTSGVHTFPAVL QSSGLYSLSSVVTVPSS*NF*-GTQTYTCNVDHKPSNTKVDKTV (SEQ ID NO: 7). In certain embodiments, the CH1 domain is a variant of SEQ ID NO: 7 and comprises 1-10, 1-5, 1-2 or 1 amino acid substitutions or deletions relative to SEQ ID NO: 7. As further described in the Examples, it has been shown herein that an IgG2 CH1 domain or variants thereof confer enhanced properties to antibodies relative to IgG1 antibodies and even more enhanced properties when the antibodies also comprise an IgG2 hinge. In certain embodiments, IgG2 CH1 variants do not comprise an amino acid substitution or deletion at one or more of the following amino acid residues: C131, R133, E137 and S138, which amino acid residues are shown in bold and underlined in SEQ ID NO: 7 shown above. For example, a modified heavy chain constant region may comprise an IgG2 CH1 domain in which neither of R133, E137 and S138 are substituted with another amino acid or are deteled or in which neither of C131, R133, E137 and S138 are substituted with another amino acid or are deteled. In certain embodiments, C131 is substituted with another amino acid, e.g., C131S, which substitution triggers the antibody to adopt conformation B. Both conformation A and conformation B antibodies having modified heavy chain constant regions have been shown herein to have enhanced activities relative to the same antibody with an IgG1 constant region.

In certain embodiments, N192 and/or F193 (shown as italicized and underlined residues in SEQ ID NO: 7 shown above) are substituted with another amino acid, e.g., with the corresponding amino acids in IgG1, i.e., N192S and/or F193L.

In certain embodiments, one or more amino acid residues of an IgG2 CH1 domain are substituted with the corresponding amino acid residues in IgG4. For example, N192 may be N192S; F193 may be F193L; C131 may be C131K; and/or T214 may be T214R.

An antibody may comprise a modified heavy chain constant region comprising an IgG2 CH1 domain or variant thereof and IgG2 hinge or variant thereof. The hinge and CH1 domain may be a combination of any IgG2 hinge and IgG2 CH1 domain described herein. In certain embodiments, the IgG2 CH1 and hinge comprise the following amino acid sequence ASTKGPSVFPLAP**CSR**STS**ES**TAALGCLVKDYFPEPVTVSWNSGALTSGVHTFPAVL QSSGLYSLSSVVTVPSS*NF*GTQTYTCNVDHKPSN̲T̲KVDKTVE̲RKCCVECPPCPAPPV A̲G(SEQ ID NO: 133), or an amino acid sequence that differs therefrom in at most 1-10 amino acids. The amino acid variants are as described for the hinge and CH1 domains above.

In certain embodiments, antibodies comprise at least an IgG2 hinge, and optionally also an IgG2 CH1 domain or fragment or derivative of the hinge and/or CH1 domain and the antibody has adopted form (of conformation) A (see, e.g., Allen et al. (2009) Biochemistry 48:3755). In certain embodiments, antibodies comprise at least an IgG2 hinge, and optionally also an IgG2 CH1 domain or fragment or derivative of the hinge and/or CH1 domain and the antibody has adopted form B (see, e.g., Allen et al. (2009) Biochemistry 48:3755).

In certain embodiments, a modified heavy chain constant region comprises a CH2 domain that is a wildtype CH2 domain of the IgG1, IgG2, IgG3 or IgG4 isotype ("IgG1 CH2 domain," "IgG2 CH2 domain," "IgG3 CH2 domain," or "IgG4 CH2 domain," respectively). A CH2 domain may also be a variant of a wildtype CH2 domain, e.g., a variant of a wildtype IgG1, IgG2, IgG3 or IgG4 CH2 domain. Exemplary variants of CH2 domains include variants that modulate a biological activity of the Fc region of an antibody, such as ADCC or CDC or modulate the half-life of the antibody or its stability. In one embodiment, the CH2 domain is a human IgG1 CH2 domain with an A330S and/or P331S mutation, wherein the CH2 domain has reduced effector function relative to the same CH2 mutation without the mutations. A CH2 domain may have enhanced effector function. CH2 domains may comprise one or more of the following mutations: SE (S267E), SELF (S267E/L328F), SDIE (S239D/I332E), SEFF, GASDALIE (G236A/S239D/A330L/I332E), and/or one or more mutations at the following amino acids: E233, L235, G237, P238, H268, P271, L328, A330 and K322. Note that some of these mutations are actually part of the hinge, rather than the CH2 domain as defined herein. Other mutations are further set forth herein elsewhere.

In certain embodiments, a modified heavy chain constant region comprises a CH3 domain that is a wildtype CH3 domain of the IgG1, IgG2, IgG3 or IgG4 isotype ("IgG1 CH3 domain," "IgG2 CH3 domain," "IgG3 CH3 domain," or "IgG4 CH3 domain," respectively). A CH3 domain may also be a variant of a wildtype CH3 domain, e.g., a variant of a wildtype IgG1, IgG2, IgG3 or IgG4 CH3 domain. Exemplary variants of CH3 domains include variants that modulate a biological activity of the Fc region of an antibody, such as ADCC or CDC or modulate the half-life of the antibody or its stability.

Generally, variants of the CH1, hinge, CH2 or CH3 domains may comprise 1, 2, 3, 4, 5, 6, 7, 8, 9, 10 or more mutations, and/or at most 10, 9, 8, 7, 6, 5, 4, 3, 2 or 1 mutation, or 1-10 or 1-5 mutations, or comprise an amino acid sequence that is at least about 75%, 80%, 85%, 90%, 95%, 96%, 97%, 98%, or 99% identical to that of the corresponding wildtype domain (CH1, hinge, CH2, or CH3 domain, respectively), provided that the heavy chain constant region comprising the specific variant retains the necessary biological activity.

Table 5 sets forth exemplary human heavy chain constant regions comprising a human CH1, hinge, CH2 and/or CH3 domains, wherein each domain is either a wildtype domain or a variant thereof that provides the desired biological activity to the heavy chain constant region. An unfilled cell in Table 5 indicates that the domain is present or not, and if present can be of any isotype, e.g., IgG1, IgG2, IgG3 or IgG4. For example, an antibody comprising the heavy chain constant region 1 in Table 5 is an antibody that comprises a heavy chain constant region comprising at least an IgG2 hinge, and which may also comprise a CH1, CH2 and/or CH3 domain, and if present, which CH1, CH2 and/or CH3 domain is of an IgG1, IgG2, IgG3 or IgG4 isotype. As another example for understanding Table 5, an antibody comprising a heavy chain constant region 8 is an antibody comprising a heavy chain constant region comprising an IgG1 CH1 domain, and IgG2 hinge, an IgG1 CH2 domain, and which may or may not also comprise an CH3 domain, which is present, may be of an IgG1, IgG2, IgG3 or IgG4 isotype.

**Table 5**

| MHCCR* | CH1 | Hinge | CH2 | CH3 |
|--------|-----|-------|-----|-----|
| 1 | | IgG2 | | |
| 2 | IgG1 | IgG2 | | |
| 3 | IgG2 | IgG2 | | |
| 4 | | IgG2 | IgG1 | |
| 5 | | IgG2 | IgG2 | |
| 6 | | IgG2 | | IgG1 |
| 7 | | IgG2 | | IgG2 |

(continued)

| MHCCR* | CH1 | Hinge | CH2 | CH3 |
|---|---|---|---|---|
| 8 | IgG1 | IgG2 | IgG1 | |
| 9 | IgG1 | IgG2 | IgG2 | |
| 10 | IgG2 | IgG2 | IgG1 | |
| 11 | IgG2 | IgG2 | IgG2 | |
| 12 | IgG1 | IgG2 | | IgG1 |
| 13 | IgG1 | IgG2 | | IgG2 |
| 14 | IgG2 | IgG2 | | IgG1 |
| 15 | IgG2 | IgG2 | | IgG2 |
| 16 | | IgG2 | IgG1 | IgG1 |
| 17 | | IgG2 | IgG1 | IgG2 |
| 18 | | IgG2 | IgG2 | IgG1 |
| 19 | | IgG2 | IgG2 | IgG2 |
| 20 | IgG1 | IgG2 | IgG1 | IgG1 |
| 21 | IgG1 | IgG2 | IgG1 | IgG2 |
| 22 | IgG1 | IgG2 | IgG2 | IgG1 |
| 23 | IgG1 | IgG2 | IgG2 | IgG2 |
| 24 | IgG2 | IgG2 | IgG1 | IgG1 |
| 25 | IgG2 | IgG2 | IgG1 | IgG2 |
| 26 | IgG2 | IgG2 | IgG2 | IgG1 |
| 27 | IgG2 | IgG2 | IgG2 | IgG2 |
| * Modified heavy chain constant region | | | | |

In certain embodiments, an antibody comprising a heavy chain constant region shown in Table 5 has an enhanced biological activity relative to the same antibody comprising a heavy chain constant region that does not comprise that specific heavy chain constant region or relative to the same antibody that comprises an IgG1 constant region.

In certain embodiments, a method for improving the biological activity of an antibody that comprises a non-IgG2 hinge and/or non-IgG2 CH1 domain comprises providing an antibody that comprises a non-IgG2 hinge and/or a non-IgG2 CH1 domain, and replacing the non-IgG2 hinge and the non-IgG2 CH1 domain with an IgG2 hinge and an IgG2 CH1 domain, respectively. A method for improving the biological activity of an antibody that does not comprise a modified heavy chain constant region, may comprise providing an antibody that does not comprise a modified heavy chain constant region, and replacing its heavy chain constant region with a modified heavy chain constant region.

Exemplary modified heavy chain constant regions are provided in Table 6, which sets forth the identity of each of the domains.

**Table 6**

| Modified heavy chain constant region | CH1 | Hinge | CH2 | CH3 | SEQ ID NO of whole MHCCR |
|---|---|---|---|---|---|
| IgGl-IgG2-IgGl | IgG1 wildtype SEQ ID NO:2 | IgG2/IgG1 SEQ ID NO:22 | IgG1 wildtype SEQ ID NO:4 | IgG1 wildtype SEQ ID NO:5 | SEQ ID NO:26 |

(continued)

| Modified heavy chain constant region | CH1 | Hinge | CH2 | CH3 | SEQ ID NO of whole MHCCR |
|---|---|---|---|---|---|
| IgG1-IgG2-IgG12 | IgG1 wildtype SEQ ID NO:2 | IgG2 wildtype SEQ ID NO:8 | IgG1 wildtype SEQ ID NO:4 | IgG1 wildtype SEQ ID NO:5 | SEQ ID NO:27 |
| IgG1-IgG2CS-IgG1 | IgG1 wildtype SEQ ID NO:2 | IgG2C219S/IgG1 SEQ ID NO:23 | IgG1 wildtype SEQ ID NO:4 | IgG1 wildtype SEQ ID NO:5 | SEQ ID NO:32 |
| IgGl-IgG2CS-IgG12 | IgG1 wildtype SEQ ID NO:2 | IgG2 C219S SEQ ID NO:21 | IgG1 wildtype SEQ ID NO:4 | IgG1 wildtype SEQ ID NO:5 | SEQ ID NO:33 |
| IgG2-IgGl | IgG2 wildtype SEQ ID NO:7 | IgG2/IgG1 SEQ ID NO:22 | IgG1 wildtype SEQ ID NO:4 | IgG1 wildtype SEQ ID NO:5 | SEQ ID NO:28 |
| IgG2-IgG12 | IgG2 wildtype SEQ ID NO:7 | IgG2 wildtype SEQ ID NO:8 | IgG1 wildtype SEQ ID NO:4 | IgG1 wildtype SEQ ID NO:5 | SEQ ID NO:29 |
| IgG2CS-IgGl | IgG2 wildtype SEQ ID NO:7 | IgG2C219S/IgG1 SEQ ID NO:23 | IgG1 wildtype SEQ ID NO:4 | IgG1 wildtype SEQ ID NO:5 | SEQ ID NO:34 |
| IgG2CS-IgG12 | IgG2 wildtype SEQ ID NO:7 | IgG2 C219S SEQ ID NO:21 | IgG1 wildtype SEQ ID NO:4 | IgG1 wildtype SEQ ID NO:5 | SEQ ID NO:35 |
| IgG1CH1-IgG2Hinge-IgG1CH2 (A330S, P331S)-IgG1CH3 or IgG1-IgG2-IgG1.1 | IgG1 wildtype SEQ ID NO:2 | IgG2 wildtype SEQ ID NO:8 | IgG1 A330S/P331S SEQ ID NO:24 | IgG1 wildtype SEQ ID NO:5 | SEQ ID NO:30 |
| IgG1CH1-IgG2Hinge (C219S)-IgGlCH2(A330S, P331S)-IgG1CH3 or IgG1-IgG2CS-IgG1.1 | IgG1 wildtype SEQ ID NO:2 | IgG2 C219S SEQ ID NO:21 | IgG1 A330S/P331S SEQ ID NO:24 | IgG1 wildtype SEQ ID NO:5 | SEQ ID NO:36 |
| IgG2-IgG1.1 | IgG2 wildtype SEQ ID NO:7 | IgG2 wildtype SEQ ID NO:8 | IgG1 A330S/P331S SEQ ID NO:24 | IgG1 wildtype SEQ ID NO:5 | SEQ ID NO:31 |
| IgG2CS-IgG1.1 | IgG2 wildtype SEQ ID NO:7 | IgG2 C219S SEQ ID NO:21 | IgG1 A330S/P331S SEQ ID NO:24 | IgG1 wildtype SEQ ID NO:5 | SEQ ID NO:37 |

In certain embodiments, an antibody comprises a modified heavy chain constant region comprising an IgG2 hinge comprising any one of SEQ ID NO: 8, 21, 22, 23, 126-132, 134-136 and 137 or a variant thereof, such as an IgG2 hinge comprising an amino acid sequence that (i) differs from any one of SEQ ID NO: 8, 21, 22, 23, 126-132, 134-136 and 137 in 1, 2, 3, 4 or 5 amino acids substitutions, additions or deletions; (ii) differs from any one of SEQ ID NO: 8, 21, 22, 23, 126-132, 134-136 and 137 in at most 5, 4, 3, 2, or 1 amino acids substitutions, additions or deletions; (iii) differs from any one of SEQ ID NO: 8, 21, 22, 23, 126-132, 134-136 and 137 in 1-5, 1-3, 1-2, 2-5 or 3-5 amino acids substitutions, additions or deletions and/or (iv) comprises an amino acid sequence that is at least about 75%, 80%, 85%, 90%, 95%, 96%, 97%, 98% or 99% identical to any one of SEQ ID NO: 8, 21, 22, 23, 126-132, 134-136 or 137, wherein in any of (i)-(iv), an amino acid substitution may be a conservative amino acid substitution or a non-conservative amino acid substitution; and wherein the modified heavy chain constant region has an enhanced biological activity relative to that of another heavy chain constant region, e.g., a heavy chain constant region that comprises a non-IgG2 hinge or relative to the same modified heavy chain constant region that comprises a non-IgG2 hinge.

In certain embodiments, a hinge comprises a sequence that is a variant of any one of SEQ ID NO: 8, 21, 22, 23, 126-132, 134-136 and 137, wherein R217 (second amino acid in wildtype IgG2 hinge (SEQ ID NO: 8) is not deleted or substituted with another amino acid. In certain embodiments in which a hinge is a variant of any one of SEQ ID NO: 8, 21, 22, 23, 126-132, 134-136 and 137, the hinge has a stiffness that is similar to that of wildtype IgG2.

In certain embodiments, an antibody comprises a modified heavy chain constant region comprising an IgG1 CH1 domain comprising SEQ ID NO: 2 or an IgG2 CH1 domain comprising SEQ ID NO: 7, or a variant of SEQ ID NO: 2 or 7, which variant (i) differs from SEQ ID NO: 2 or 7 in 1, 2, 3, 4 or 5 amino acids substitutions, additions or deletions; (ii) differs from SEQ ID NO: 2 or 7 in at most 5, 4, 3, 2, or 1 amino acids substitutions, additions or deletions; (iii) differs from SEQ ID NO: 2 or 7 in 1-5, 1-3, 1-2, 2-5 or 3-5 amino acids substitutions, additions or deletions and/or (iv) comprises an amino acid sequence that is at least about 75%, 80%, 85%, 90%, 95%, 96%, 97%, 98% or 99% identical to SEQ ID NO: 2 or 7, wherein in any of (i)-(iv), an amino acid substitution may be a conservative amino acid substitution or a non-conservative amino acid substitution; and wherein the modified heavy chain constant region has an enhanced biological activity relative to that of another heavy chain constant region, e.g., a heavy chain constant region that comprises a non-IgG2 hinge or relative to the same modified heavy chain constant region that comprises a non-IgG2 hinge.

In certain embodiments, an antibody comprises a modified heavy chain constant region comprising an IgG1 CH2 domain comprising SEQ ID NO: 4 or 24, or a variant of SEQ ID NO: 4 or 24, which variant (i) differs from SEQ ID NO: 4 or 24 in 1, 2, 3, 4 or 5 amino acids substitutions, additions or deletions; (ii) differs from SEQ ID NO: 4 or 24 in at most 5, 4, 3, 2, or 1 amino acids substitutions, additions or deletions; (iii) differs from SEQ ID NO: 4 or 24 in 1-5, 1-3, 1-2, 2-5 or 3-5 amino acids substitutions, additions or deletions and/or (iv) comprises an amino acid sequence that is at least about 75%, 80%, 85%, 90%, 95%, 96%, 97%, 98% or 99% identical to SEQ ID NO: 4 or 24, wherein in any of (i)-(iv), an amino acid substitution may be a conservative amino acid substitution or a non-conservative amino acid substitution; and wherein the modified heavy chain constant region has an enhanced biological activity relative to that of another heavy chain constant region, e.g., a heavy chain constant region that comprises a non-IgG2 hinge or relative to the same modified heavy chain constant region that comprises a non-IgG2 hinge.

In certain embodiments, an antibody comprises a modified heavy chain constant region comprising an IgG1 CH3 domain comprising SEQ ID NO: 5, or a variant of SEQ ID NO: 5, which variant (i) differs from SEQ ID NO: 5 in 1, 2, 3, 4 or 5 amino acids substitutions, additions or deletions; (ii) differs from SEQ ID NO: 5 in at most 5, 4, 3, 2, or 1 amino acids substitutions, additions or deletions; (iii) differs from SEQ ID NO: 5 in 1-5, 1-3, 1-2, 2-5 or 3-5 amino acids substitutions, additions or deletions and/or (iv) comprises an amino acid sequence that is at least about 75%, 80%, 85%, 90%, 95%, 96%, 97%, 98% or 99% identical to SEQ ID NO: 5, wherein in any of (i)-(iv), an amino acid substitution may be a conservative amino acid substitution or a non-conservative amino acid substitution; and wherein the modified heavy chain constant region has an enhanced biological activity relative to that of another heavy chain constant region, e.g., a heavy chain constant region that comprises a non-IgG2 hinge or relative to the same modified heavy chain constant region that comprises a non-IgG2 hinge.

Modified heavy chain constant regions may also comprise a combination of the CH1, hinge, CH2 and CH3 domains described above.

In certain embodiments, an antibody comprises a modified heavy chain constant region described herein or a variant of a modified heavy chain constant region described herein, which variant (i) differs from a modified heavy chain constant region described herein in 1, 2, 3, 4, 5, 6, 7, 8, 9, 10 or more amino acids substitutions, additions or deletions; (ii) differs from a modified heavy chain constant region described herein in at most 10, 9, 8, 7, 6,5, 4, 3, 2, or 1 amino acids substitutions, additions or deletions; (iii) differs from a modified heavy chain constant region described herein in 1-5, 1-3, 1-2, 2-5, 3-5, 1-10, or 5-10 amino acids substitutions, additions or deletions and/or (iv) comprises an amino acid sequence that is at least about 75%, 80%, 85%, 90%, 95%, 96%, 97%, 98% or 99% identical to a modified heavy chain constant region described herein, wherein in any of (i)-(iv), an amino acid substitution may be a conservative amino acid substitution or a non-conservative amino acid substitution; and wherein the modified heavy chain constant region has an enhanced biological activity relative to that of another heavy chain constant region, e.g., a heavy chain constant region that comprises a non-IgG2 hinge or relative to the same modified heavy chain constant region that comprises a non-IgG2 hinge.

In certain embodiments, an antibody comprises a modified heavy chain constant region comprising any one of SEQ ID NO: 26-37, 54-56, 78-125, 152-232, 234-245 and 247-262, or a variant of any one of SEQ ID NO: 26-37, 54-56, 78-125, 152-232, 234-245 and 247-262, which variant (i) differs from any one of SEQ ID NO: 26-37, 54-56, 78-125, 152-232, 234-245 and 247-262 in 1, 2, 3, 4, 5, 6, 7, 8, 9, 10 or more amino acids substitutions, additions or deletions; (ii) differs from any one of SEQ ID NO: 26-37, 54-56, 78-125, 152-232, 234-245 and 247-262 in at most 10, 9, 8, 7, 6,5, 4, 3, 2, or 1 amino acids substitutions, additions or deletions; (iii) differs from any one of SEQ ID NO: 26-37, 54-56, 78-125, 152-232, 234-245 and 247-262 in 1-5, 1-3, 1-2, 2-5, 3-5, 1-10, or 5-10 amino acids substitutions, additions or deletions and/or (iv) comprises an amino acid sequence that is at least about 75%, 80%, 85%, 90%, 95%, 96%, 97%, 98% or 99% identical to any one of SEQ ID NO: 26-37, 54-56, 78-125, 152-232, 234-245 and 247-262, wherein in any of (i)-(iv), an amino acid substitution may be a conservative amino acid substitution or a non-conservative amino acid substitution; and wherein the modified heavy chain constant region has an enhanced biological activity (and/or reduced effector function) relative to that of another heavy chain constant region, e.g., a heavy chain constant region that comprises a non-IgG2 hinge or relative to the same modified heavy chain constant region that comprises a non-IgG2 hinge.

Modified heavy chain constant regions may have (i) similar, reduced or increased effector function (e.g., binding to an FcγR) relative to a wildtype heavy chain constant region and or (ii) similar, reduced or increased half-life (or binding to the FcRn receptor) relative to a wildtype heavy chain constant region.

In certain embodiments, an antibody (or antigen binding fragment thereof) comprises a modified heavy chain constant region comprising SEQ ID NO: 198 or a portion thereof comprising P238K, or a variant of any one of SEQ ID NO: 198 or portion thereof, which variant (i) differs from SEQ ID NO: 198 or a portion thereof comprising P238K in 1, 2, 3, 4, 5, 6, 7, 8, 9, 10 or more amino acids substitutions, additions or deletions; (ii) differs from SEQ ID NO: 198 or a portion thereof comprising P238K in at most 10, 9, 8, 7, 6,5, 4, 3, 2, or 1 amino acids substitutions, additions or deletions; (iii) differs from SEQ ID NO: 198 or a portion thereof comprising P238K in 1-5, 1-3, 1-2, 2-5, 3-5, 1-10, or 5-10 amino acids substitutions, additions or deletions and/or (iv) comprises an amino acid sequence that is at least about 75%, 80%, 85%, 90%, 95%, 96%, 97%, 98% or 99% identical to SEQ ID NO: 198 or a portion thereof comprising P238K, wherein an amino acid substitution may be a conservative amino acid substitution or a non-conservative amino acid substitution; and wherein the modified heavy chain constant region has reduced effector function, e.g., undetectable binding to the low affinity Fc√Rs (e.g., CD32a, CD32b and CD16a) and optionally undetectable binding to the high affinity Fc√R (CD64), such as determined in an assay described herein.

In certain embodiments, an IgG1 Fc comprising a P238K mutation (e.g., comprising SEQ ID NO: 198 or a portion thereof), comprises no other mutations relative to a wild type IgG1 Fc, e.g., those described herein. In certain embodiments, an IgG1 Fc comprising a P238K mutation (e.g., comprising SEQ ID NO: 198 or a portion thereof), comprises 1-5 amino acid changes in addition to P238K relative to the wild type human IgG1 Fc, e.g., it comprises SEQ ID NO: 198 or a portion thereof and 1-5 amino acid changes relative to SEQ ID NO: 198 or the portion thereof, provided that the IgG1 Fc has reduced effector function.

In certain embodiments, an IgG1 Fc comprising a P238K mutation does not comprise any other mutation that reduces effector function. In certain embodiments, an IgG1 Fc comprising a P238K mutation comprises 1-5 mutations that reduces effector function.

In certain embodiments, an IgG Fc comprising a P238K mutation also comprises an L235E mutation and/or a K322A mutation, and may, in certain embodiments not contain any additional Fc mutation that modulates Fc effector function, e.g., it does not include a mutation at P330, P331, or a mutation in the lower hinge, e.g., at amino acids 234 and 236-237. The IgG may be an IgG1 or IgG2.

In certain embodiments, an antibody comprises a heavy chain constant region comprising an IgG2 constant domain, or at least the hinge thereof, wherein the IgG2 constant domain or hinge thereof comprises a mutation selected from the group consisting of P238A, P238K, L235A, K322A, and optionally a mutation at C219 and/or C220, e.g., C219S and/or C220S.

In certain embodiments, an antibody comprises a heavy chaing constant region comprising an IgG1 constant domain comprising one or more of L234A, L235E and G237A. As used herein "IgG1.3" refers to an IgG1 heavy chain comprising L234A, L235E and G237A (see, e.g., SEQ ID NO: 248). IgG1 constant regions comprising these three mutations may also comprise additional mutations, such as those described herein. Exemplary sequences comprising L234A, L235E and G237A mutations and additional mutations are provided herein in the Sequence Table. An IgG 1.3 Fc provides an antibody with significantly reduced effector function, such as ADCC and CDC. In certain embodiments, an Fc comprises the mutations of IgG1.3 and additional mutations, e.g., P238K.

In certain embodiments, an antibody comprises an IgG1.3 heavy chain constant region, which constant region does not comprise any other than mutation that modulates effector function, in addition to L234A, L235E and G237A. In certain embodiments, an antibody comprises an IgG1.3 heavy chain constant region, which constant region does not comprise any other than mutation, in addition to L234A, L235E and G237A.

Heavy chain constant regions are provided in the Sequence Table. In certain embodiments, an antibody comprises one of the heavy chain constant regions set forth in the Table, wherein the constant region does not comprise any mutation in addition to that in the sequence set forth in the Table. In certain embodiments, an antibody comprises one of the heavy chain constant regions set forth in the Table, wherein the constant region (i) differs from a sequence in the Sequence Table in 1, 2, 3, 4 or 5 amino acids substitutions, additions or deletions; (ii) differs from a sequence in the Sequence Table in at most 5, 4, 3, 2, or 1 amino acids substitutions, additions or deletions; (iii) differs from a sequence in the Sequence Table in 1-5, 1-3, 1-2, 2-5 or 3-5 amino acids substitutions, additions or deletions and/or (iv) comprises an amino acid sequence that is at least about 75%, 80%, 85%, 90%, 95%, 96%, 97%, 98% or 99% identical to a sequence in the Sequence Table, wherein in any of (i)-(iv), an amino acid substitution may be a conservative amino acid substitution or a nonconservative amino acid substitution; and wherein the bioligcal activity of the constant region is not significantly changed by these mutation(s).

Heavy chain constant regions may comprise a combination of mutations that confer onto an antibody comprising the heavy chain region a combination of the biological activities conferred by each individual mutation. For example, one or more mutation that enhances agonist activity formation of large cell surface complexes or that enhance internalization of the antibody can be combined with one or more mutation that modulate effector function. Exemplary constant chain sequences comprising a combination of mutations conferring different biological functions are set forth in the Sequence Table.

## II. Antibodies with modified heavy chain constant regions and target antigens thereof

Modified heavy chain constant regions can be used in a wide range of antibodies, such as antibodies that require internalization (e.g., antibody drug conjugates (ADCs), and anti-CD73 antibodies), agonist activity (e.g., antibodies that are effective in modulating immune responses, e.g., in stimulating T cell activation, such as agonist anti-GITR antibodies), antagonist activity (e.g., antibodies that inhibit or block a protein that inhibits an immune response, e.g., T cell activation, such as an antagonist PD-1 antibody), effector function, e.g., ADCC and CDC, or reduced effector function, signal transduction, or antitumor activity. For example, internalization of a cell surface inhibitory receptor may limit its ability to interact with its receptor(s) and decrease cell function(s).

In one embodiment, antibodies comprising a modified heavy chain constant domain are antibodies that require their internalization for activity (e.g., antibodies that are specific for cell surface receptors) by, e.g., inducing receptor-mediated endocytosis when they bind to the cell surface. Such antibodies may be used as vehicles for targeted delivery of drugs, toxins, enzymes or DNA for therapeutic applicationsTherefore, increasing the internalization properties of these antibodies is desirable. Exemplary antibodies that may benefit from effective internalization are antibody drug conjugates. Various assays for measuring the internalization properties of an antibody are known in the art and described herein. These assays utilize, for example, a wide range of dyes for antibody labeling that can be used in wash or quench-based assays to monitor internalization. Antibody internalization can also be monitored in no-wash assays which rely on fluorescent labels.

In one embodiment, antibodies comprising a modified heavy chain constant domain are antibodies that require the internalization of the antigen to which they bind, e.g., a cell surface molecule, such as a receptor or a ligand, for activity. Thus, antibodies to cell surface proteins that require to be downregulated for biological (e.g., therapeutic) activity can use a modified heavy chain constant region described herein.

In certain embodiments, antibodies comprising a modified heavy chain constant domain bind to cell surface molecules and agonize or antagonize the biological activity of the cell surface molecule, e.g., a cell suface molecule on an immune cell, e.g., a T cell, Teff cell, Th1 cell, Th2 cell, CD4+ T cell, CD8+ T cell, Treg cell, dendritic cell, macrophage, monocyte, Langerhans cell, NK cell, myeloid derived suppressor cell, B cell or any other immune cell. The cell suface molecule may be a stimulatory, e.g., co-stimulatory molecule (e.g., GITR, OX40, CD137, CD40, ICOS and other TNFR family members), and the antibody may further stimulate the activity (an agonist antibody) or the antibody may inhibit the activity (an antagonist antibody). The cell suface molecule may be an inhibitory molecule (e.g., CTLA-4, PD-1, PD-L1, LAG-3, TIM-3), and the antibody may further stimulate the activity (an agonist antibody) or the antibody may inhibit the activity (an antagonist antibody).

In certain embodiments, antibodies comprising a modified heavy chain constant domain are agonist antibodies of stimulatory (or co-stimulatory) molecules that, e.g., boost the immune system of a subject, e.g., by inducing IL-2 and/or IFN-γ secretion from T cells (e.g., anti-GITR antibodies). Other agonist antibodies have been shown to activate APCs, promote antitumor T-cell responses, and/or foster cytotoxic myeloid cells with the potential to control cancer in the absence of T-cell immunity. Agonist antibodies of stimulatory molecules are different from antagonist antibodies of inhibitory molecules, which block negative immune checkpoint such as anti-CTLA-4 or anti-PD-1. Agonist activity, such as T cell proliferation, can be measured using a variety of methods known in the art.

In certain embodiments, antibodies comprising a modified heavy chain constant domain are antagonist antibodies of checkpoint inhibitors boost the immune response of a subject by blocking or inhibiting negative immune checkpoint, such as anti-CTLA-4 or anti-PD-1 antibodies, e.g., by targeting the inhibitory receptor expressed on activated T-cells. Antagonist activity, such as inhibition of T cell proliferation can be measured using a variety of methods known in the art.

In one embodiment, the antibody is (i) an agonist of a co-stimulatory receptor or (ii) an antagonist of an inhibitory signal on, e.g., T cells, both of which may result in amplifying immune responses, e.g., antigen-specific T cell responses, (immune checkpoint regulators). In certain embodiments, an antibody is (i) an antagonist of a co-stimulatory receptor or (ii) an agonist of an inhibitory signal, e.g., on T cells. Co-stimulatory and co-inhibitory molecules may be members of the immunoglobulin super family (IgSF), and antibodies having modified heavy chain constant regions may bind to any of them. One important family of membranebound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6, and antibodies having modified heavy chain constant regions may bind to any of them. Another family of membrane bound ligands that bind to costimulatory or co-inhibitory receptors is the TNF family of molecules that bind to cognate TNF receptor (TNFR) family members, which include CD40 and CD40L, OX-40, OX-40L, CD70, CD27L, CD30, CD30L, 4-1BBL, CD137, TRAIL/Apo2-L, TRAILR1/DR4, TRAILR2/DR5, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNEβ, TNFR2, TNFα, LTα, LTβ, LTβR, Lymphotoxin α 1β2, FAS, FASL (CD 178), DR3 (TNFRSF25), RELT, DR6, TROY, NGFR (see, e.g., Tansey (2009) Drug Discovery Today 00:1). Thus, the antibodies described herein can bind to any of these surface molecules, and they can be, e.g., (i) agonists or antagonists (or inhibitors or blocking agents) of proteins of the IgSF family or B7 family or the TNFR family that inhibit T cell activation or antagonists of cytokines that inhibit T cell activation (e.g., IL-6, IL-10, TGF-β, VEGF; "immunosuppressive cytokines") and/or (ii) agonists or antagonists of stimulatory receptors of the IgSF family, B7 family or the TNF family or of cytokines that stimulate T cell activation, for modulating, e.g,. stimulating, an immune response, e.g., for treating proliferative diseases, such as cancer.

Accordingly, an antibody with a modified heavy chain constant domain may be used as one of the following agents:

(1) An agonist of a protein that stimulates, e.g., T cell activation, such as B7-1, B7-2, CD28, 4-1BB (CD137), 4-1BBL, GITR, ICOS, ICOS-L, OX40, OX40L, CD70, CD27, CD40, DR3 or CD28H; or
(2) An antagonist (inhibitor or blocking agent) of a protein that inhibits T cell activation (e.g., immune checkpoint inhibitors), such as CTLA-4, PD-1, PD-L1, PD-L2, and LAG-3, as described above, and any of the following proteins: TIM-3, Galectin 9, CEACAM-1, BTLA, CD69, Galectin-1, TIGIT, CD113, GPR56, VISTA, 2B4, CD48, GARP, CD73, PD1H, LAIR1, TIM-1 ,TIM-4, CD39.

Other antibodies include antagonists of inhibitory receptors on NK cells and agonists of activating receptors on NK cells, e.g., KIR, TIGIT, NKG2A.

Generally, antibodies that may benefit from a modified heavy chain constant region include, e.g., agonist antibodies that ligate positive costimulatory receptors, blocking antibodies that attenuate signaling through inhibitory receptors, antagonist antibodies, and antibodies that increase systemically the frequency of anti-tumor T cells, antibodies that overcome distinct immune suppressive pathways within the tumor microenvironment (e.g., block inhibitory receptor engagement (e.g., PD-L1/PD-1 interactions), deplete or inhibit Tregs (e.g., an anti-CD25 monoclonal antibody, inhibit metabolic enzymes such as IDO, or reverse/prevent T cell anergy or exhaustion) and antibodies that trigger innate immune activation and/or inflammation at tumor sites. An increased internalization of inhibitory receptors may translate into a lower level of a potential inhibitor.

In certain embodiments, an antibody comprising a modified heavy chain constant region is an antibody that is conjugated to a therapeutic agent to form an immunoconjugate, such as an antibody drug conjugate (ADC), which immunoconjugate requires internalization for its activity. In an ADC, the antibody functions as a targeting agent for directing the ADC to a target cell expressing its antigen, such as an antigen on a cancer cell. In this case, the antigen may be a tumor associated antigen, *i.e.,* one that is uniquely expressed or overexpressed by the cancer cell. Once there, the drug is released, either inside the target cell or in its vicinity, to act as a therapeutic agent. For a review on the mechanism of action and use of ADCs in cancer therapy, see Schrama et al., Nature Rev. Drug Disc. 2006, 5, 147.

For cancer treatment, the therapeutic agent or drug of an ADC preferably is a cytotoxic drug that causes death of the targeted cancer cell. Cytotoxic drugs that can be used in ADCs include the following types of compounds and their analogs and derivatives:

(a) enediynes such as calicheamicin (see, *e.g.,* Lee et al., J. Am. Chem. Soc. 1987, 109, 3464 and 3466) and uncialamycin (see, *e.g.,* Davies et al., WO 2007/038868 A2 (2007) and Chowdari *et al.,* US 8,709,431 B2 (2012));
(b) tubulysins (see, *e.g.,* Domling et al., US 7,778,814 B2 (2010); Cheng et al., US 8,394,922 B2 (2013); and Cong et al., US 2014/0227295 A1;
(c) CC-1065 and duocarmycin (see, *e.g.,* Boger, US 6,5458,530 B1 (2003); Sufi et al., US 8,461,117 B2 (2013);

and Zhang et al., US 2012/0301490 A1 (2012));

(d) epothilones (see, *e.g.,* Vite et al., US 2007/0275904 A1 (2007) and US RE42930 E (2011));

(e) auristatins (see, *e.g.,* Senter et al., US 6,844,869 B2 (2005) and Doronina et al., US 7,498,298 B2 (2009));

(f) pyrrolobezodiazepine (PBD) dimers (see, *e.g.,* Howard et al., US 2013/0059800 A1(2013); US 2013/0028919 A1 (2013); and WO 2013/041606 A1 (2013)); and

(g) maytansinoids such as DM1 and DM4 (see, *e.g.,* Chari et al., US 5,208,020 (1993) and Amphlett et al., US 7,374,762 B2 (2008)).

In ADCs, the antibody and therapeutic agent may be conjugated via a linker, e.g., a cleavable linker, such as a peptidyl, disulfide, or hydrazone linker. For example, the linker may be a peptidyl linker such as Val-Cit, Ala-Val, Val-Ala-Val, Lys-Lys, Pro-Val-Gly-Val-Val, Ala-Asn-Val, Val-Leu-Lys, Ala-Ala-Asn, Cit-Cit, Val-Lys, Lys, Cit, Ser, or Glu. The ADCs can be prepared as described in U.S. Pat. Nos. 7,087,600; 6,989,452; and 7,129,261; PCT Publications WO 02/096910; WO 07/038658; WO 07/051081; WO 07/059404; WO 08/083312; and WO 081103693; U.S. Patent Publications 20060024317; 20060004081; and 20060247295; the disclosures of which are incorporated herein by reference.

Exemplary targets of ADCs that may be enhanced with a modified heavy chain constant region include B7H4 (Korman et al., US 2009/0074660 A1); CD19 (Rao-Naik et al., 8,097,703 B2); CD22 (King et al., US 2010/0143368 A1); CD30 (Keler et al., US 7,387,776 B2 (2008); CD70 (Terrett et al., US 8,124,738 B2); CTLA-4 (Korman et al., US 6,984,720 B1 (2006)); PD-1 (Korman et al., US 8,008,449 B2 (2011); PSMA (Huang et al., US 2009/0297438 A1 and Cardarelli et al., US 7,875,278 B2); PTK7 (Terrett et al., US 2010/0034826 A1); glypican-3 (Terrett et al., US 2010/0209432 (A1)); RG1 (Harkins et al., US 7,335,748 B2(2008)); mesothelin (Terrett *et al.,* US 8,268,970 B2 (2012)); and CD44 (Xu et al., US 2010/0092484 A1).

The modified heavy chain constant domains may also be part of antibodies for uses outside of oncology, e.g., immunological diseases, such as rheumatoid artritis, lupus etc.

The modified heavy chain constant domains may also be fused to non antibody molecules (or antibody variants) or fragments thereof, and may be fused to any polypeptide that needs the presence of an Fc. A modified heavy chain constant domain may be fused to an antigen binding fragment of an antibody, as further defined herein (e.g., in the definition section).

In certain embodiments, a heavy chain constant domain or portion thereof comprising a P238K mutation, which is devoid of certain effector function, is fused to a polypeptide, e.g., the heavy chain portion of an antigen binding fragment of an antibody. As further described herein, an IgG, e.g., IgG1, Fc comprising a P238K mutation, and comprising, e.g., the amino acid sequence set forth in SEQ ID NO: 198, may be fused to a heavy chain variable domain of an antibody, wherein the antibody binds to any target, e.g., a target protein described herein (e.g., CD40 or CD40L). An IgG1 Fc with a P238K mutation (e.g., P238K IgG1 fa having the amino acid sequence SEQ ID NO: 198 or in the context of IgG1 having allotype f) may be used in any antibody or with any antigen binding fragment thereof for which effector function, in particular binding to FcγRs CD32a, CD32b and CD16a, is not desired. In addition to P238K, a heavy chain constant region may comprise an additional 1 or 2 mutations, e.g., substitutions, that reduce binding to FcγR CD64, or P238K may be used in the context of an IgG2 hinge, e.g., an IgG2 hinge comprising C219S, as further described herein.

## III. Methods of modifying the biological activity of antibodies

Provided herein are methods for enhancing the biological activity of certain antibodies, such as the one or more of the following biological activities:

(a) increased or altered internalization by a cell;

(b) increased or altered agonist activity;

(c) increased or altered antagonist or blocking activity;

(d) enhanced or reduced ADCC;

(d) generation of a new property;

(e) increased or altered signal transduction;

(f) formation of larger antibody/antigen cross-linked complexes;

(g) increased clustering or oligomerization of the target cell surface molecule;

(h) increased stimulation or enhancement of an immune response; and/or

(i) increased inhibition of an immune response.

A method for enhancing a biological activity of an antibody may comprise replacing the heavy chain constant region or a portion thereof, e.g., the hinge and/or CH1 domain, with a modified heavy chain constant region or portion thereof, e.g., an IgG2 hinge and/or IgG2 CH1 domain.

In certain embodiments, a method for improving the biological activity of an antibody comprises (i) providing an antibody that does not comprise a modified heavy chain constant region as described herein; and (ii) replacing the heavy chain constant region of the antibody with a modified heavy chain constant region, or a portion thereof, that enhances the biological activity of the antibody. In certain embodiments, a method for improving the biological activity of an antibody comprises (i) providing an antibody that comprises a non-IgG2 hinge (e.g., an IgG1 hinge, an IgG3 hinge or an IgG4 hinge); and (ii) replacing the non-IgG2 hinge of the antibody with an IgG2 hinge. In certain embodiments, a method for improving the biological activity of an antibody comprises (i) providing an antibody that comprises a non-enhancing IgG2 hinge; and (ii) replacing the non- enhancing IgG2 hinge of the antibody with an IgG2 hinge. A "non-enhancing IgG2 hinge" is a variant IgG2 hinge that differs from an IgG2 hinge in such a way that it no longer has the required characteristic for enhancing the biologic activity of an antibody, e.g., a variant hinge that no longer has the stiffness of a wildtype IgG2 hinge.

Exemplary methods for enhancing the biological activity of an antibody comprise (i) providing an antibody that comprises a non-IgG2 hinge or a non-enhancing IgG2 hinge, and (ii) replacing the hinge with a hinge comprising SEQ ID NO: 8, 21, 22, 23, 126-132, 134-136 or 137 or variants thereof, e.g., the variants described herein. Methods for enhancing the biological activity of an antibody may also comprise (i) providing an antibody that comprises heavy chain constant region that is not a modified heavy chain constant region, and (ii) replacing the heavy chain constant region with a modified heavy chain constant region. Replacing the heavy chain constant region may comprise replacing the CH1, hinge, CH2 and/or CH3 domain. For example, a heavy chain constant region may be modified, by replacing the hinge with an IgG2 hinge or variant thereof, and/or by replacing the CH1 domain with an IgG1 or IgG2 CH1 domain or variant thereof. In certain embodiments, the hinge is replaced with an IgG2 hinge and the CH2 domain is replaced with an IgG1 CH2 domain. In certain embodiments, the hinge is replaced with an IgG2 hinge and the CH3 domain is replaced with an IgG1 CH3 domain. In certain embodiments, the hinge is replaced with an IgG2 hinge, the CH1 is replaced with an IgG2 hinge, the CH2 domain is replaced with an IgG1 CH2 domain and the CH3 domain is replaced with an IgG1 CH3 domain. In certain embodiments, a heavy chain constant region is replaced with a modified heavy chain regions 1-27 set forth in Table 5 above or the heavy chain constant regions set forth in Table 6 or described herein.

Also provided herein are methods for enhancing the biological activity of an IgG1 or IgG2 antibody, comprising deleting 1-10 amino acids in the hinge of the IgG1 or IgG2 antibody, respectively. For example, one or more of amino acids S219, C22, D221, K222, T223, H224 and T225 can be deleted. In one embodiment, all of amino acids S219, C22, D221, K222, T223, H224 and T225 are deleted.

Further provided herein are methods for making and providing effectorless antibodies or antigen-binding fragments thereof, e.g., by mutating P238, e.g., to P238K, to eliminate or reduce the effector function of an antibody.

In certain embodiments, replacing the heavy chain constant region of an antibody, e.g., to modify its biological activity, is not accompanied by a reduction or a significant reduction of its binding activity to the target antigen. As described in the Examples, substituting the heavy chain constant region of anti-GITR and anti-CD73 antibodies did not significantly change their affinity for the human GITR and human CD73 antigens, respectively.

It will be understood that when referring to replacing a domain of a specific isotype with the same domain of a different isotype or with a domain including a mutation, e.g., a P238 mutation, it is not necessary to literally replace the domain, but rather, it may only be necessary to change the amino acids that are different between the two isotypes.

Standard assays to evaluate the binding ability of the antibodies toward an antigen of various species are known in the art and are further described herein, and include for example, ELISAs, Western blots, and RIAs. Suitable assays are described in detail in the Examples. The binding kinetics (e.g., binding affinity) of the antibodies also can be assessed by standard assays known in the art, such as by BIACORE® SPR analysis. Assays to evaluate the properties of antibodies having modified constant regions (e.g., ligand binding, T cell proliferation, cytokine production) are described in further detail *infra* and in the Examples.

Exemplary antibodies that can be modified as described herein include, e.g., antibodies for treating cancer, such as: Yervoy™ (ipilimumab) or Tremelimumab (to CTLA-4), galiximab (to B7.1), BMS-936558 (to PD-1), CT-011 (to PD-1), MK-3475 (to PD-1), AMP224 (to B7DC), BMS-936559 (to B7-H1), MPDL3280A (to B7-H1), MEDI-570 (to ICOS), AMG557 (to B7H2), MGA271 (to B7H3), IMP321 (to LAG-3), BMS-663513 (to CD137), PF-05082566 (to CD137), CDX-1127 (to CD27), anti-OX40 (Providence Health Services), huMAbOX40L (to OX40L), Atacicept (to TACI), CP-870893 (to CD40), Lucatumumab (to CD40), Dacetuzumab (to CD40), Muromonab-CD3 (to CD3), Ipilumumab (to CTLA-4).

Other antibodies that can be modified as described herein include PD-1 and PD-L1 antagonist antibodies. An exemplary anti-PD-1 antibody that may be modified as described herein is nivolumab (BMS-936558); an antibody that comprises the CDRs or variable regions of one of antibodies 17D8, 2D3, 4H1, 5C4, 7D3, 5F4 and 4A11 described in WO 2006/121168; MK-3475 (Lambrolizumab) described in WO2012/145493; AMP-514 described in WO 2012/145493; CT-011 (Pidilizumab; previously CT-AcTibody or BAT; see, e.g., Rosenblatt et al. (2011) J. Immunotherapy 34:409); those described in WO 2009/014708, WO 03/099196, WO 2009/114335, WO 2011/066389, WO 2011/161699, WO 2012/145493, WO2013/173223, U.S. Patent Nos. 7,635,757 and 8,217,149, and U.S. Patent Publication No. 2009/0317368.

Further antibodies that may be modified include anti-PD-LI antibodies, e.g., BMS-936559 (referred to as 12A4 in WO 2007/005874 and US Patent No. 7,943,743); an antibody that comprises the CDRs or variable regions of 3G10, 12A4, 10A5, 5F8, 10H10, 1B12, 7H1, 11E6, 12B7 and 13G4, which are described in PCT Publication WO 07/005874 and US Patent No. 7,943,743; MEDI4736 (also known as Anti-B7-H1); MPDL3280A (also known as RG7446); any of the anti-PD-LI antibodies disclosed in WO2013/173223, WO2011/066389, WO2012/145493, U.S. Patent Nos. 7,635,757 and 8,217,149 and U.S. Publication No. 2009/145493.

Other antibodies that may be modified include anti-CTLA-4 antibodies, e.g., Yervoy™ (ipilimumab or antibody 10D1, described in PCT Publication WO 01114424); tremelimumab (formerly ticilimumab, CP-675,206); monoclonal or an anti-CTLA-4 antibody described in any of the following publications: WO 98/42752; WO 00/37504; U.S. Pat. No. 6,207,156; Hurwitz et al. (1998) Proc. Natl. Acad. Sci. USA 95(17):10067-10071; Camacho et al. (2004) J. Clin. Oncology 22(145): Abstract No. 2505 (antibody CP-675206); and Mokyr et al. (1998) Cancer Res. 58:5301-5304; and any of the anti-CTLA-4 antibodies disclosed in WO2013/173223.

Other antibodies that may be modified include anti-LAG-3 antibodies, e.g., BMS-986016; IMP731 described in US 2011/007023; and IMP-321.

Other antibodies that may be modified include anti-GITR agonist antibodies, e.g., the anti-GITR antibody 6C8 or humanized versions thereof, described in WO2006/105021; an antibody described in WO2011/028683; and an antibody described in JP2008278814.

Antibodies that target other antigens, including those described elsewhere herein, may also be modified. For example, anti-Her2 antibodies that require internalization, e.g., trastuzumab (Herceptin), may be modified as described herein.

## IV. **Additional** heavy **chain constant domain modifications**

In addition to the modifications described herein to antibodies to enhance their biological activity or reduce effector function, further mutations can be made, e.g., to the CH1, hinge, CH2 or CH3 domain, e.g., to further reduce the effector function, binding to FcγRs, and/or the stability of the antibodies. For example, any of the modifications described herein, e.g., below, may be combined with a P238, e.g., P238K, mutation, such as in an IgG1 or IgG1-IgG2 hybrid Fc or portion thereof.

*Fcs and modified Fcs*

Antibodies described herein may comprise an Fc comprising one or more modifications, typically to alter one or more functional properties of the antibody, such as serum half-life, complement fixation, Fc receptor binding, and/or antigen-dependent cellular cytotoxicity. For example, one may make modifications in the Fc region in order to generate an Fc variant with (a) increased or decreased antibodydependent cell-mediated cytotoxicity (ADCC), (b) increased or decreased complement mediated cytotoxicity (CDC), (c) increased or decreased affinity for C1q and/or (d) increased or decreased affinity for a Fc receptor relative to the parent Fc. Such Fc region variants will generally comprise at least one amino acid modification in the Fc region. Combining amino acid modifications is thought to be particularly desirable. For example, the variant Fc region may include two, three, four, five, etc substitutions therein, e.g. of the specific Fc region positions identified herein. Exemplary Fc sequence variants are disclosed herein, and are also provided at U.S. Pat. Nos. 5,624,821; 6,277,375; 6,737,056; 6,194,551; 7,317,091; 8,101,720; PCT Patent Publications WO 00/42072; WO 01/58957; WO 04/016750; WO 04/029207; WO 04/035752; WO 04/074455; WO 04/099249; WO 04/063351; WO 05/070963; WO 05/040217, WO 05/092925 and WO 06/020114.

*Reducing Effector Function*

ADCC activity may be reduced by modifying the Fc region. In certain embodiments, sites that affect binding to Fc receptors may be removed (e.g., by mutation), preferably sites other than salvage receptor binding sites. In other embodiments, an Fc region may be modified to remove an ADCC site. ADCC sites are known in the art; see, for example, Sarmay et al. (1992) Molec. Immunol. 29 (5): 633-9 with regard to ADCC sites in IgG1. In one embodiment, the G236R and L328R variant of human IgG1 effectively eliminates FcγR binding. Horton et al. (2011) J. Immunol. 186:4223 and Chu et al. (2008) Mol. Immunol. 45:3926. In other embodiments, the Fc having reduced binding to FcγRs comprised the amino acid substitutions L234A, L235E and G237A. Gross et al. (2001) Immunity 15:289.

CDC activity may also be reduced by modifying the Fc region. Mutations at IgG1 positions D270, K322, P329 and P331, specifically alanine mutations D270A, K322A, P329A and P331A, significantly reduce the ability of the corresponding antibody to bind C1q and activate complement. Idusogie et al. (2000) J. Immunol. 164:4178; WO 99/51642. Modification of position 331 of IgG1 *(e.g.* P331S) has been shown to reduce complement binding. Tao et al. (1993) J. Exp. Med. 178:661 and Canfield & Morrison (1991) J. Exp. Med. 173:1483. In another example, one or more amino acid residues within amino acid positions 231 to 239 are altered to thereby reduce the ability of the antibody to fix complement.

WO 94/29351.

In some embodiments, the Fc with reduced complement fixation has the amino acid substitutions A330S and P331S. Gross et al. (2001) Immunity 15:289.

For uses where effector function is to be avoided altogether, e.g. when antigen binding alone is sufficient to generate the desired therapeutic benefit, and effector function only leads to (or increases the risk of) undesired side effects, IgG4 antibodies may be used, or antibodies or fragments lacking the Fc region or a substantial portion thereof can be devised, or the Fc may be mutated to eliminate glycosylation altogether (e.g. N297A). Alternatively, a hybrid construct of human IgG2 (C$_H$1 domain and hinge region) and human IgG4 (C$_H$2 and C$_H$3 domains) has been generated that is devoid of effector function, lacking the ability to bind the FcγRs (like IgG2) and unable to activate complement (like IgG4). Rother et al. (2007) Nat. Biotechnol. 25:1256. *See also* Mueller et al. (1997) Mol. Immunol. 34:441; Labrijn et al. (2008) Curr. Op. Immunol. 20:479 (discussing Fc modifications to reduce effector function generally).

In other embodiments, the Fc region is altered by replacing at least one amino acid residue with a different amino acid residue to reduce all effector function(s) of the antibody. For example, one or more amino acids selected from amino acid residues 234, 235, 236, 237, 297, 318, 320 and 322 can be replaced with a different amino acid residue such that the antibody has decreased affinity for an effector ligand but retains the antigen-binding ability of the parent antibody. The effector ligand to which affinity is altered can be, for example, an Fc receptor (residues 234, 235, 236, 237, 297) or the C1 component of complement (residues 297, 318, 320, 322). U.S. Patent Nos. 5,624,821 and 5,648,260, both by Winter *et al.*

WO 88/007089 proposed modifications in the IgG Fc region to decrease binding to FcγRI to decrease ADCC (234A; 235E; 236A; G237A) or block binding to complement component C1q to eliminate CDC (E318A or V/K320A and K322A/Q). *See also* Duncan & Winter (1988) Nature 332:563; Chappel et al. (1991) Proc. Nat'l Acad. Sci. (USA) 88:9036; and Sondermann et al. (2000) Nature 406:267 (discussing the effects of these mutations on FcγRIII binding).

Fc modifications reducing effector function also include substitutions, insertions, and deletions at positions 234, 235, 236, 237, 267, 269, 325, and 328, such as 234G, 235G, 236R, 237K, 267R, 269R, 325L, and 328R. An Fc variant may comprise 236R/328R. Other modifications for reducing FcγR and complement interactions include substitutions 297A, 234A, 235A, 237A, 318A, 228P, 236E, 268Q, 309L, 330S, 331 S, 220S, 226S, 229S, 238S, 233P, and 234V. These and other modifications are reviewed in Strohl (2009) Current Opinion in Biotechnology 20:685-691. Effector functions (both ADCC and complement activation) can be reduced, while maintaining neonatal FcR binding (maintaining half-life), by mutating IgG residues at one or more of positions 233 - 236 and 327 - 331, such as E233P, L234V, L235A, optionally G236Δ, A327G, A330S and P331S in IgG1; E233P, F234V, L235A, optionally G236Δ in IgG4; and A330S and P331S in IgG2. *See* Armour et al. (1999) Eur. J. Immunol. 29:2613; WO 99/58572. Other mutations that reduce effector function include L234A and L235A in IgG1 (Alegre et al. (1994) Transplantation 57:1537); V234A and G237A in IgG2 (Cole et al. (1997) J. Immunol. 159:3613; *see also* U.S. Pat. No. 5,834,597); and S228P and L235E for IgG4 (Reddy et al. (2000) J. Immunol. 164:1925). Another combination of mutations for reducing effector function in a human IgG1 include L234F, L235E and P331S. Oganesyan et al. (2008) Acta Crystallogr. D. Biol. Crystallogr. 64:700. *See generally* Labrijn et gal. (2008) Curr. Op. Immunol. 20:479. Additional mutations found to decrease effector function in the context of an Fc (IgG1) fusion protein (abatacept) are C226S, C229S and P238S (EU residue numbering). Davis et al. (2007) J. Immunol. 34:2204.

Other Fc variants having reduced ADCC and/or CDC are disclosed at Glaesner et al. (2010) Diabetes Metab. Res. Rev. 26:287 (F234A and L235A to decrease ADCC and ADCP in an IgG4); Hutchins et al. (1995) Proc. Nat'l Acad. Sci. (USA) 92:11980 (F234A, G237A and E318A in an IgG4); An et al. (2009) MAbs 1:572 and U.S. Pat. App. Pub. 2007/0148167 (H268Q, V309L, A330S and P331S in an IgG2); McEarchern et al. (2007) Blood 109:1185 (C226S, C229S, E233P, L234V, L235A in an IgG1); Vafa et al. (2014) Methods 65:114 (V234V, G237A, P238S, H268A, V309L, A330S, P331S in an IgG2).

In certain embodiments, an Fc is chosen that has essentially no effector function, i.e., it has reduced binding to FcγRs and reduced complement fixation. An exemplary Fc, e.g., IgG1 Fc, that is effectorless comprises the following five mutations: L234A, L235E, G237A, A330S and P331S. Gross et al. (2001) Immunity 15:289. These five substitutions maybe combined with N297A to eliminate glycosylation as well.

*Enhancing Effector Function*

Alternatively, ADCC activity may be increased by modifying the Fc region. With regard to ADCC activity, human IgG1 □ IgG3 □ IgG4 □ IgG2, so an IgG1 constant domain, rather than an IgG2 or IgG4, might be chosen for use in a drug where ADCC is desired. Alternatively, the Fc region may be modified to increase antibody dependent cellular cytotoxicity (ADCC) and/or to increase the affinity for an Fcγ receptor by modifying one or more amino acids at the following positions: 234, 235, 236, 238, 239, 240, 241, 243, 244, 245, 247, 248, 249, 252, 254, 255, 256, 258, 262, 263, 264, 265, 267, 268, 269, 270, 272, 276, 278, 280, 283, 285, 286, 289, 290, 292, 293, 294, 295, 296, 298, 299, 301, 303, 305, 307, 309, 312, 313, 315, 320, 322, 324, 325, 326, 327, 329, 330, 331, 332, 333, 334, 335, 337, 338, 340, 360, 373, 376, 378, 382, 388, 389, 398, 414, 416, 419, 430, 433, 434, 435, 436, 437, 438 or 439. *See* WO 2012/142515; *see also* WO 00/42072. Exemplary substitutions include 236A, 239D, 239E, 268D, 267E, 268E, 268F, 324T, 332D, and 332E. Exemplary variants include 239D/332E, 236A/332E, 236A/239D/332E, 268F/324T, 267E/268F, 267E/324T, and 267E/268F/324T. For example, human IgGIFcs comprising the G236A variant, which can optionally be combined with I332E, have been shown to increase the Fc$\gamma$IIA / Fc$\gamma$IIB binding affinity ratio approximately 15-fold. Richards et al. (2008) Mol. Cancer Therap. 7:2517; Moore et al. (2010) mAbs 2:181. Other modifications for enhancing FcyR and complement interactions include but are not limited to substitutions 298A, 333A, 334A, 326A, 247I, 339D, 339Q, 280H, 290S, 298D, 298V, 243L, 292P, 300L, 396L, 305I, and 396L. These and other modifications are reviewed in Strohl (2009) Current Opinion in Biotechnology 20:685-691. Specifically, both ADCC and CDC may be enhanced by changes at position E333 of IgG1, e.g. E333A. Shields et al. (2001)J. Biol. Chem. 276:6591. The use of P247I and A339D/Q mutations to enhance effector function in an IgG1 is disclosed at WO 2006/020114, and D280H, K290S $\pm$ S298D/V is disclosed at WO 2004/074455. The K326A/W and E333A/S variants have been shown to increase effector function in human IgG1, and E333S in IgG2. Idusogie et al. (2001) J. Immunol. 166:2571.

Specifically, the binding sites on human IgG1 for Fc$\gamma$R1, Fc$\gamma$RII, FcyRIII and FcRn have been mapped, and variants with improved binding have been described. Shields et al. (2001) J. Biol. Chem. 276:6591-6604. Specific mutations at positions 256, 290, 298, 333, 334 and 339 were shown to improve binding to FcyRIII, including the combination mutants T256A/S298A, S298A/E333A, S298A/K224A and S298A/E333A/K334A (having enhanced FcyRIIIa binding and ADCC activity). Other IgG1 variants with strongly enhanced binding to FcyRIIIa have been identified, including variants with S239D/I332E and S239D/I332E/A330L mutations which showed the greatest increase in affinity for Fc$\gamma$RIIIa, a decrease in FcyRIIb binding, and strong cytotoxic activity in cynomolgus monkeys. Lazar et al.(2006) Proc. Nat'l Acad Sci. (USA) 103:4005; Awan et al. (2010) Blood 115:1204; Desjarlais & Lazar (2011) Exp. Cell Res. 317:1278. Introduction of the triple mutations into antibodies such as alemtuzumab (CD52-specific), trastuzumab (HER2/neu-specific), rituximab (CD20-specific), and cetuximab (EGFR-specific) translated into greatly enhanced ADCC activity *in vitro,* and the S239D/I332E variant showed an enhanced capacity to deplete B cells in monkeys. Lazar et al.(2006) Proc. Nat'l Acad Sci. (USA) 103:4005. In addition, IgG1 mutants containing L235V, F243L, R292P, Y300L, V305I and P396L mutations which exhibited enhanced binding to Fc$\gamma$RIIIa and concomitantly enhanced ADCC activity in transgenic mice expressing human Fc$\gamma$RIIIa in models of B cell malignancies and breast cancer have been identified. Stavenhagen et al. (2007) Cancer Res. 67:8882; U.S. Pat. No. 8,652,466; Nordstrom et al. (2011) Breast Cancer Res. 13:R123.

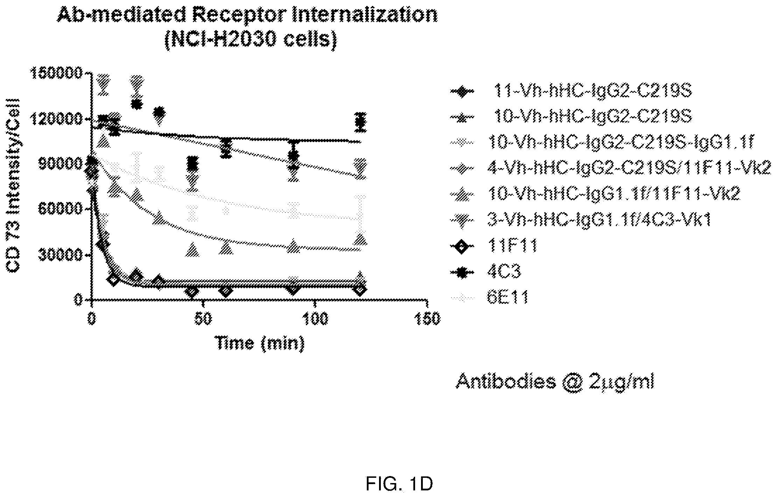

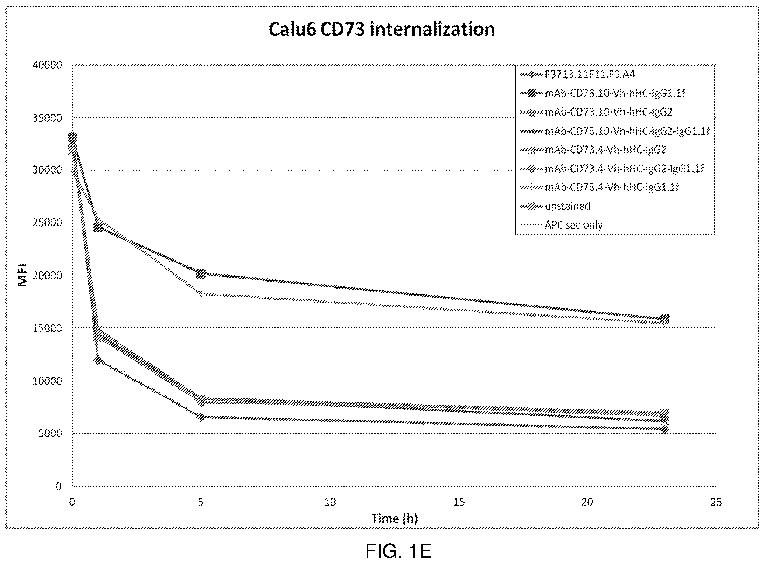

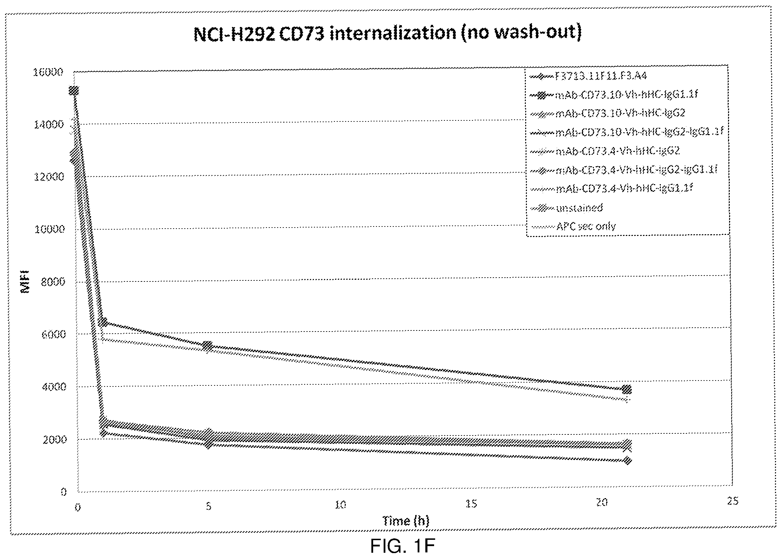

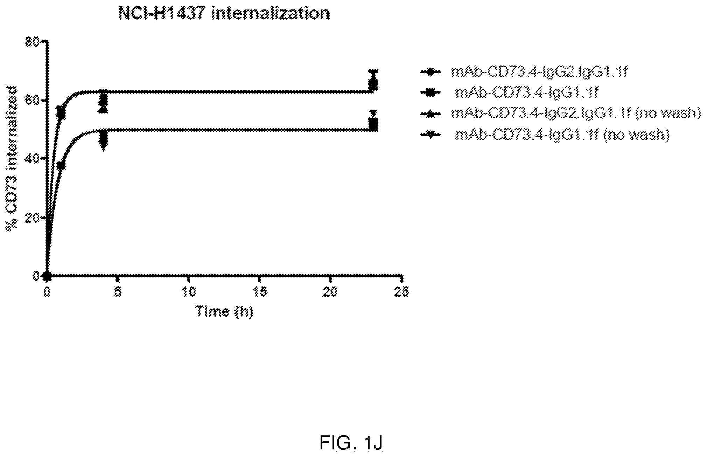

Different IgG isotypes also exhibit differential CDC activity (IgG3>IgG1>>IgG2≈IgG4). Dangl et al. (1988) EMBO J. 7:1989. For uses in which enhanced CDC is desired, it is also possible to introduce mutations that increase binding to C1q. The ability to recruit complement (CDC) may be enhanced by mutations at K326 and/or E333 in an IgG2, such as K326W (which reduces ADCC activity) and E333S, to increase binding to C1q, the first component of the complement cascade. Idusogie et al. (2001) J. Immunol. 166:2571. Introduction of S267E / H268F / S324T (alone or in any combination) into human IgG1 enhances C1q binding. Moore et al. (2010) mAbs 2:181. The Fc region of the IgG1/IgG3 hybrid isotype antibody "113F" of Natsume et al. (2008) Cancer Res. 68:3863 (figure 1 therein) also confers enhanced CDC. *See also* Michaelsen et al. (2009) Scand. J. Immunol. 70:553 and Redpath et al. (1998) Immunology 93:595.

Additional mutations that can increase or decrease effector function are disclosed at Dall'Acqua et al. (2006) J. Immunol. 177:1129. *See also* Carter (2006) Nat. Rev. Immunol. 6:343; Presta (2008) Curr. Op. Immunol. 20:460.

Fc variants that enhance affinity for the inhibitory receptor FcyRIIb may also be used, e.g. to enhance apoptosis-inducing or adjuvant activity. Li & Ravetch (2011) Science 333:1030; Li & Ravetch (2012) Proc. Nat'l Acad. Sci (USA) 109:10966; U.S. Pat. App. Pub. 2014/0010812. Such variants may provide an antibody with immunomodulatory activities related to FcyRIIb+ cells, including for example B cells and monocytes. In one embodiment, the Fc variants provide selectively enhanced affinity to FcyRIIb relative to one or more activating receptors. Modifications for altering binding to FcyRIIb include one or more modifications at a position selected from the group consisting of 234, 235, 236, 237, 239, 266, 267, 268, 325, 326, 327, 328, and 332, according to the EU index. Exemplary substitutions for enhancing FcyRIIb affinity include but are not limited to 234D, 234E, 234F, 234W, 235D, 235F, 235R, 235Y, 236D, 236N, 237D, 237N, 239D, 239E, 266M, 267D, 267E, 268D, 268E, 327D, 327E, 328F, 328W, 328Y, and 332E. Exemplary substitutions include 235Y, 236D, 239D, 266M, 267E, 268D, 268E, 328F, 328W, and 328Y. Other Fc variants for enhancing binding to FcyRIIb include 235Y/267E, 236D/267E, 239D/268D, 239D/267E, 267E/268D, 267E/268E, and 267E/328F. Specifically, the S267E, G236D, S239D, L328F and I332E variants, including the S267E + L328F double variant, of human IgG1 are of particular value in specifically enhancing affinity for the inhibitory FcyRIIb receptor. Chu et al. (2008) Mol. Immunol. 45:3926; U.S. Pat. App. Pub. 2006/024298; WO 2012/087928. Enhanced specificity for Fc$\gamma$RIIb (as distinguished from Fc$\gamma$RIIa$^{R131}$) may be obtained by adding the P238D substitution. Mimoto et al. (2013) Protein. Eng. Des. & Selection 26:589; WO 2012/115241.

*Glycosylation*

Glycosylation of an antibody is modified to increase or decrease effector function. For example, an aglycoslated antibody can be made that lacks all effector function by mutating the conserved asparagine residue at position 297 (e.g. N297A), thus abolishing complement and FcγRI binding. Bolt et al. (1993) Eur. J. Immunol. 23:403. *See also* Tao & Morrison (1989) J. Immunol. 143:2595 (using N297Q in IgG1 to eliminate glycosylation at position 297).

Although aglycosylated antibodies generally lack effector function, mutations can be introduced to restore that function. Aglycosylated antibodies, e.g. those resulting from N297A/C/D/or H mutations or produced in systems *(e.g. E. coli)* that do not glycosylate proteins, can be further mutated to restore FcyR binding, e.g. S298G and/or T299A/G/or H (WO 2009/079242), or E382V and M428I (Jung et al. (2010) Proc. Nat'l Acad. Sci (USA) 107:604).

Additionally, an antibody with enhanced ADCC can be made by altering the glycosylation. For example, removal of fucose from heavy chain Asn297-linked oligosaccharides has been shown to enhance ADCC, based on improved binding to FcγRIIIa. Shields et al. (2002) JBC 277:26733; Niwa et al. (2005) J. Immunol. Methods 306: 151; Cardarelli et al. (2009) Clin. Cancer Res.15:3376 (MDX-1401); Cardarelli et al. (2010) Cancer Immunol. Immunotherap. 59:257 (MDX-1342). Such low fucose antibodies may be produced, *e.g.,* in knockout Chinese hamster ovary (CHO) cells lacking fucosyltransferase *(FUT8)* (Yamane-Ohnuki et al. (2004) Biotechnol. Bioeng. 87:614), or in other cells that generate afucosylated antibodies. *See, e.g.,* Zhang et al. (2011) mAbs 3:289 and Li et al. (2006) Nat. Biotechnol. 24:210 (both describing antibody production in glycoengineered *Pichia pastoris);* Mossner et al. (2010) Blood 115:4393; Shields et al. (2002) J. Biol. Chem. 277:26733; Shinkawa et al. (2003) J. Biol. Chem. 278:3466; EP 1176195B1. ADCC can also be enhanced as described in PCT Publication WO 03/035835, which discloses use of a variant CHO cell line, Lec13, with reduced ability to attach fucose to Asn(297)-linked carbohydrates, also resulting in hypofucosylation of antibodies expressed in that host cell (see also Shields, R.L. et al. (2002) J. Biol. Chem. 277:26733-26740). Alternatively, fucose analogs may be added to culture medium during antibody production to inhibit incorporation of fucose into the carbohydrate on the antibody. WO 2009/135181.

Increasing bisecting GlcNac structures in antibody-linked oligosaccharides also enhances ADCC. PCT Publication WO 99/54342 by Umana et al. describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180).

Additional glycosylation variants have been developed that are devoid of galactose, sialic acid, fucose and xylose residues (so-called GNGN glycoforms), which exhibit enhanced ADCC and ADCP but decreased CDC, as well as others that are devoid of sialic acid, fucose and xylose (so-called G1/G2 glycoforms), which exhibit enhanced ADCC, ADCP and CDC. U.S. Pat. App. Pub. No. 2013/0149300. Antibodies having these glycosylation patterns are optionally produced in genetically modified *N. benthamiana* plants in which the endogenous xylosyl and fucosyl transferase genes have been knocked-out.

Glycoengineering can also be used to modify the anti-inflammatory properties of an IgG construct by changing the $\alpha2,6$ sialyl content of the carbohydrate chains attached at Asn297 of the Fc regions, wherein an increased proportion of $\alpha2,6$ sialylated forms results in enhanced anti-inflammatory effects. *See* Nimmerjahn et al. (2008) Ann. Rev. Immunol. 26:513. Conversely, reduction in the proportion of antibodies having $\alpha2,6$ sialylated carbohydrates may be useful in cases where anti-inflammatory properties are not wanted. Methods of modifying $\alpha2,6$ sialylation content of antibodies, for example by selective purification of $\alpha2,6$ sialylated forms or by enzymatic modification, are provided at U.S. Pat. Appl. Pub. No. 2008/0206246. In other embodiments, the amino acid sequence of the Fc region may be modified to mimic the effect of $\alpha2,6$ sialylation, for example by inclusion of an F241A modification. WO 2013/095966.

Antibodies described herein can contain one or more glycosylation sites in either the light or heavy chain variable region. Such glycosylation sites may result in increased immunogenicity of the antibody or an alteration of the pK of the antibody due to altered antigen binding (Marshall et al (1972) Annu Rev Biochem 41:673-702; Gala and Morrison (2004) J. Immunol 172:5489-94; Wallick et al (1988) J Exp Med 168:1099-109; Spiro (2002) Glycobiology 12:43R-56R; Parekh et al (1985) Nature 316:452-7; Mimura et al. (2000) Mol Immunol 37:697-706). Glycosylation has been known to occur at motifs containing an N-X-S/T sequence.

*Biological half-life*

In certain embodiments, the antibody is modified to increase its biological half-life. Various approaches are possible. For example, this may be done by increasing the binding affinity of the Fc region for FcRn. In one embodiment, the antibody is altered within the CH1 or CL region to contain a salvage receptor binding epitope taken from two loops of a CH2 domain of an Fc region of an IgG, as described in U.S. Patent Nos. 5,869,046 and 6,121,022 by Presta et al. Other exemplary Fc variants that increase binding to FcRn and/or improve pharmacokinetic properties include substitutions at positions 259, 308, and 434, including for example 2591, 308F, 428L, 428M, 434S, 434H, 434F, 434Y, and 434M. Other variants that increase Fc binding to FcRn include: 250E, 250Q, 428L, 428F, 250Q/428L (Hinton et al., 2004, J. Biol. Chem. 279(8): 6213-6216, Hinton et al. 2006 Journal of Immunology 176:346-356), 256A, 272A, 305A, 307A, 31 1A, 312A, 378Q, 380A, 382A, 434A (Shields et al, Journal of Biological Chemistry, 2001, 276(9):6591-6604), 252F, 252Y, 252W, 254T, 256Q, 256E, 256D, 433R, 434F, 434Y, 252Y/254T/256E, 433K/434F/436H (Del' Acqua et al. Journal of Immunology, 2002, 169:5171-5180, Dall'Acqua et al., 2006, Journal of Biological Chemistry 281:23514-23524). *See* U.S. Pat. No. 8,367,805.

Modification of certain conserved residues in IgG Fc (I253/H310/Q311/H433/N434), such as the N434A variant (Yeung et al. (2009) J. Immunol. 182:7663), has been proposed as a way to increase FcRn affinity, thus increasing the half-life of the antibody in circulation. WO 98/023289. The combination Fc variant comprising M428L and N434S has been shown to increase FcRn binding and increase serum half-life up to five-fold. Zalevsky et al. (2010) Nat. Biotechnol. 28:157. The combination Fc variant comprising T307A, E380A and N434A modifications also extends half-life of IgG1 antibodies. Petkova et al. (2006) Int. Immunol. 18:1759. In addition, combination Fc variants comprising M252Y/M428L, M428L/N434H, M428L/N434F, M428L/N434Y, M428L/N434A, M428L/N434M, and M428L/N434S variants have also been shown to extend half-life. WO 2009/086320.

Further, a combination Fc variant comprising M252Y, S254T and T256E , increases half-life-nearly 4-fold. Dall'Acqua et al. (2006) J. Biol. Chem. 281:23514. A related IgG1 modification providing increased FcRn affinity but reduced pH dependence (M252Y / S254T / T256E / H433K / N434F) has been used to create an IgG1 construct ("MST-HN Abdeg") for use as a competitor to prevent binding of *other* antibodies to FcRn, resulting in increased clearance of that other antibody, either endogenous IgG (e.g. in an autoimmune setting) or another exogenous (therapeutic) mAb. Vaccaro et al. (2005) Nat. Biotechnol. 23:1283; WO 2006/130834.

Other modifications for increasing FcRn binding are described in Yeung et al. (2010) J. Immunol. 182:7663-7671; 6,277,375; 6,821,505; WO 97/34631; WO 2002/060919.

In certain embodiments, hybrid IgG isotypes may be used to increase FcRn binding, and potentially increase half-life. For example, an IgG1/IgG3 hybrid variant may be constructed by substituting IgG1 positions in the CH2 and/or CH3 region with the amino acids from IgG3 at positions where the two isotypes differ. Thus a hybrid variant IgG antibody may be constructed that comprises one or more substitutions, e.g., 274Q, 276K, 300F, 339T, 356E, 358M, 384S, 392N, 397M, 422I, 435R, and 436F. In other embodiments described herein, an IgG1/IgG2 hybrid variant may be constructed by substituting IgG2 positions in the CH2 and/or CH3 region with amino acids from IgG1 at positions where the two isotypes differ. Thus a hybrid variant IgG antibody may be constructed that comprises one or more substitutions, e.g., one or more of the following amino acid substitutions: 233E, 234L, 235L, -236G (referring to an insertion of a glycine at position 236), and 327A. *See* U.S. Pat. No. 8,629,113. A hybrid of IgG1/IgG2/IgG4 sequences has been generated that purportedly increases serum half-life and improves expression. U.S. Pat. No. 7,867,491 (sequence number 18 therein).

The serum half-life of the antibodies of the present invention can also be increased by pegylation. An antibody can be pegylated to, for example, increase the biological (e.g., serum) half-life of the antibody. To pegylate an antibody, the antibody, or fragment thereof, typically is reacted with a polyethylene glycol (PEG) reagent, such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies described herein. See for example, EP 0154316 by Nishimura et al. and EP 0401384 by Ishikawa et al.

Alternatively, under some circumstances it may be desirable to decrease the half-life of an antibody of the present invention, rather than increase it. Modifications such as I253A (Hornick et al. (2000) J. Nucl. Med. 41:355) and H435A/R I253A or H310A (Kim et al. (2000) Eur. J. Immunol. 29:2819) in Fc of human IgG1 can decrease FcRn binding, thus decreasing half-life (increasing clearance) for use in situations where rapid clearance is preferred, such a medical imaging. *See also* Kenanova et al. (2005) Cancer Res. 65:622. Other means to enhance clearance include formatting the antigen binding domains of the present invention as antibody fragments lacking the ability to bind FcRn, such as Fab fragments. Such modification can reduce the circulating half-life of an antibody from a couple of weeks to a matter of hours. Selective PEGylation of antibody fragments can then be used to fine-tune (increase) the half-life of the antibody fragments if necessary. Chapman et al. (1999) Nat. Biotechnol. 17:780. Antibody fragments may also be fused to human serum albumin, *e.g.* in a fusion protein construct, to increase half-life. Yeh et al. (1992) Proc. Nat'l Acad. Sci. 89:1904. Alternatively, a bispecific antibody may be constructed with a first antigen binding domain of the present invention and a second antigen binding domain that binds to human serum albumin (HSA). *See* Int'l Pat. Appl. Pub. WO 2009/127691 and patent references cited therein. Alternatively, specialized polypeptide sequences can be added to antibody fragments to increase half-life, e.g. "XTEN" polypeptide sequences. Schellenberger et al. (2009) Nat. Biotechnol. 27:1186; Int'l Pat. Appl. Pub. WO 2010/091122.

*Stability*

A potential protease cleavage site in the hinge of IgG1 constructs can be eliminated by D221G and K222S modifications, increasing the stability of the antibody. WO 2014/043344.

In certain embodiments, the antibodies described herein do not contain asparagine isomerism sites. The deamidation of asparagine may occur on N-G or D-G sequences and may result in the creation of an isoaspartic acid residue that may introduce a kink into the polypeptide chain and may decrease its stability (isoaspartic acid effect).

Each antibody will have a unique isoelectric point (pI), which generally falls in the pH range between 6 and 9.5. The pI for an IgG1 antibody typically falls within the pH range of 7-9.5 and the pI for an IgG4 antibody typically falls within the pH range of 6-8. There is speculation that antibodies with a pI outside the normal range may have some unfolding and instability under in vivo conditions. Thus, it is preferred to have an antibody that contains a pI value that falls in the normal range. This can be achieved either by selecting antibodies with a pI in the normal range or by mutating charged surface residues.

Each antibody will have a characteristic melting temperature, with a higher melting temperature indicating greater overall stability in vivo (Krishnamurthy R and Manning M C (2002) Curr Pharm Biotechnol 3:361-71). Generally, it is preferred that the $T_{M1}$ (the temperature of initial unfolding) be greater than 60° C, preferably greater than 65° C., even more preferably greater than 70° C. The melting point of an antibody can be measured using differential scanning calorimetry (Chen et al (2003) Pharm Res 20:1952-60; Ghirlando et al (1999) Immunol Lett 68:47-52) or circular dichroism (Murray et al. (2002) J. Chromatogr Sci 40:343-9).

In a preferred embodiment, antibodies are selected that do not degrade rapidly. Degradation of an antibody can be measured using capillary electrophoresis (CE) and MALDI-MS (Alexander A J and Hughes D E (1995) Anal Chem 67:3626-32).

When using an IgG4 constant domain, it is usually preferable to include the substitution S228P, which mimics the hinge sequence in IgG1 and thereby stabilizes IgG4 molecules, e.g. reducing Fab-arm exchange between the therapeutic antibody and endogenous IgG4 in the patient being treated. Labrijn et al. (2009) Nat. Biotechnol. 27:767; Reddy et al. (2000) J. Immunol. 164:1925. Similarly, in IgG2 hinge containing antibodies a C219S and/or C220S mutation stabilizes the antibody comprising an IgG2 hinge.

*Aggregation*

In another preferred embodiment, antibodies are selected that have minimal aggregation effects, which can lead to the triggering of an unwanted immune response and/or altered or unfavorable pharmacokinetic properties. Generally, antibodies are acceptable with aggregation of 25% or less, preferably 20% or less, even more preferably 15% or less, even more preferably 10% or less and even more preferably 5% or less. Aggregation can be measured by several techniques, including size-exclusion column (SEC), high performance liquid chromatography (HPLC), and light scattering.

**V. Non-antibody proteins and antibody derivatives**

The invention described herein may also be applied to molecules that are not full length antibodies, provided that they comprise a hinge. For example, IgG fusion proteins with an enhanced biologic activity or lack of effector function may be made. Accordingly, provided herein are fusion proteins comprising an active moiety linked, e.g., covalently linked, to an IgG constant region, e.g., an Fc region, comprising an IgG2 hinge and optionally a CH2 and CH3 domains or portions thereof, or linked to an IgG (e.g., an IgG1) or portion thereof with reduced effector function, e.g., comprising a mutation at P238, e.g., P238K. The Fc may be any Fc of a modified heavy chain constant region described herein, such as the Fc portions of the modified heavy chain constant regions set forth in Tables 5, 6 or in the Sequence Table.

Antibodies described herein may also be used for forming bispecific molecules or molecules for CAR-T therapy. An antibody, or antigen-binding portions thereof, can be derivatized or linked to another functional molecule, *e.g.,* another peptide or protein *(e.g.,* another antibody or ligand for a receptor) to generate a bispecific molecule that binds to at least two different binding sites or target molecules. Antibodies described herein may be derivatized or linked to more than one other functional molecule to generate multi specific molecules that bind to more than two different binding sites and/or target molecules; such multispecific molecules are also intended to be encompassed by the term "bispecific molecule" as used herein. To create a bispecific molecule, an antibody described herein can be functionally linked *(e.g.,* by chemical coupling, genetic fusion, noncovalent association or otherwise) to one or more other binding molecules, such as another antibody, antibody fragment, peptide or binding mimetic, such that a bispecific molecule results.

VI. **Compositions**

Further provided are compositions, e.g., a pharmaceutical compositions, containing one or a combination of antibodies, or antigen-binding portion(s) thereof, described herein, formulated together with a pharmaceutically acceptable carrier. Such compositions may include one or a combination of *(e.g.,* two or more different) antibodies, or immunoconjugates or bispecific molecules described herein. For example, a pharmaceutical composition described herein can comprise a combination of antibodies (or immunoconjugates or bispecifics) that bind to different epitopes on the target antigen or that have complementary activities.

In certain embodiments, a composition comprises an antibody described herein at a concentration of at least 1 mg/ml, 5 mg/ml, 10 mg/ml, 50 mg/ml, 100 mg/ml, 150 mg/ml, 200 mg/ml, 1-300 mg/ml, or 100-300 mg/ml.

Pharmaceutical compositions described herein also can be administered in combination therapy, *i.e.,* combined with other agents. For example, the combination therapy can include an antibody described herein combined with at least one other anti-cancer and/or T-cell stimulating (e.g., activating) agent. Examples of therapeutic agents that can be used in combination therapy are described in greater detail below in the section on uses of the antibodies described herein.

In some embodiments, therapeutic compositions disclosed herein can include other compounds, drugs, and/or agents used for the treatment of cancer. Such compounds, drugs, and/or agents can include, for example, chemotherapy drugs, small molecule drugs or antibodies that stimulate the immune response to a given cancer. In some instances, therapeutic compositions can include, for example, one or more of an anti-CTLA-4 antibody, an anti-PD-1 antibody, an anti-PDL-1 antibody, an anti-OX40 (also known as CD134, TNFRSF4, ACT35 and/or TXGP1L) antibody, or an anti-LAG-3 antibody.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, *i.e.,* antibody, immunoconjugate, or bispecific molecule, may be coated in a material to protect the compound from the action of acids and other natural conditions that may inactivate the compound.

The pharmaceutical compounds described herein may include one or more pharmaceutically acceptable salts. A "pharmaceutically acceptable salt" refers to a salt that retains the desired biological activity of the parent compound and does not impart any undesired toxicological effects (see *e.g.,* Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition described herein also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, *supra,* and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions described herein is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, preferably from about 0.1 per cent to about 70 per cent, most preferably from about 1 per cent to about 30 per cent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms described herein are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Preferred dosage regimens for an antibody described herein include 1 mg/kg body weight or 3 mg/kg body weight via intravenous administration, with the antibody being given using one of the following dosing schedules: (i) every four weeks for six dosages, then every three months; (ii) every three weeks; (iii) 3 mg/kg body weight once followed by 1 mg/kg body weight every three weeks.

In some methods, two or more monoclonal antibodies with different binding specificities are administered simultaneously, in which case the dosage of each antibody administered falls within the ranges indicated. Antibody is usually administered on multiple occasions. Intervals between single dosages can be, for example, weekly, monthly, every three months or yearly. Intervals can also be irregular as indicated by measuring blood levels of antibody to the target antigen in the patient. In some methods, dosage is adjusted to achieve a plasma antibody concentration of about 1-1000 $\mu$g/ml and in some methods about 25-300 $\mu$g/ml.

An antibody can be administered as a sustained release formulation, in which case less frequent administration is required. Dosage and frequency vary depending on the half-life of the antibody in the patient. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions described herein may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions described herein employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of an antibody described herein preferably results in a decrease in severity of disease symptoms, an increase in frequency and duration of disease symptom-free periods, or a prevention of impairment or disability due to the disease affliction. In the context of cancer, a therapeutically effective dose preferably prevents further deterioration of physical symptoms associated with cancer. Symptoms of cancer are well-known in the art and include, for example, unusual mole features, a change in the appearance of a mole, including asymmetry, border, color and/or diameter, a newly pigmented skin area, an abnormal mole, darkened area under nail, breast lumps, nipple changes, breast cysts, breast pain, death, weight loss, weakness, excessive fatigue, difficulty eating, loss of appetite, chronic cough, worsening breathlessness, coughing up blood, blood in the urine, blood in stool, nausea, vomiting, liver metastases, lung metastases, bone metastases, abdominal fullness, bloating, fluid in peritoneal cavity, vaginal bleeding, constipation, abdominal distension, perforation of colon, acute peritonitis (infection, fever, pain), pain, vomiting blood, heavy sweating, fever, high blood pressure, anemia, diarrhea, jaundice, dizziness, chills, muscle spasms, colon metastases, lung metastases, bladder metastases, liver metastases, bone metastases, kidney metastases, and pancreatic metastases, difficulty swallowing, and the like.

A therapeutically effective dose may prevent or delay onset of cancer, such as may be desired when early or preliminary signs of the disease are present. Laboratory tests utilized in the diagnosis of cancer include chemistries, hematology, serology and radiology. Accordingly, any clinical or biochemical assay that monitors any of the foregoing may be used to determine whether a particular treatment is a therapeutically effective dose for treating cancer. One of ordinary skill in the art would be able to determine such amounts based on such factors as the subject's size, the severity of the subject's symptoms, and the particular composition or route of administration selected.

A composition described herein can be administered via one or more routes of administration using one or more of a variety of methods known in the art. As will be appreciated by the skilled artisan, the route and/or mode of administration will vary depending upon the desired results. Preferred routes of administration for antibodies described herein include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, an antibody described herein can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

Therapeutic compositions can be administered with medical devices known in the art. For example, in a preferred embodiment, a therapeutic composition described herein can be administered with a needleless hypodermic injection device, such as the devices disclosed in U.S. Patent Nos. 5,399,163; 5,383,851; 5,312,335; 5,064,413; 4,941,880; 4,790,824; or 4,596,556. Examples of well-known implants and modules for use with antibodies described herein include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. These patents are incorporated herein by reference. Many other such implants, delivery systems, and modules are known to those skilled in the art.

In certain embodiments, the antibodies described herein can be formulated to ensure proper distribution *in vivo*. For example, the blood-brain barrier (BBB) excludes many highly hydrophilic compounds. To ensure that the therapeutic compounds described herein cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery *(see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p120 (Schreier et al. (1994) J. Biol. Chem. 269:9090); see also K. Keinanen; M.L. Laukkanen (1994) FEBS Lett. 346:123; J.J. Killion;

I.J. Fidler (1994) Immunomethods 4:273.

## VII. Uses and Methods

The antibodies, antibody compositions and methods described herein have numerous *in vitro* and *in vivo* utilities involving, for example, the treatment of various disorders, e.g., cancers. For example, antibodies described herein can be administered to cells in culture, in *vitro* or *ex vivo*, or to human subjects, e.g., *in vivo.* Accordingly, provided herein are methods of treatment of a subject comprising administering to the subject an antibody comprising a modified heavy chain constant region, such that treatment occurs. Also provided herein are methods of modifying an immune response in a subject comprising administering to the subject an antibody such that the immune response in the subject is modified. Preferably, the response is enhanced, stimulated or up-regulated. However, in other embodiments, an immune response is inhibited.

Preferred subjects include human patients in whom enhancement of an immune response would be desirable. The methods are particularly suitable for treating human patients having a disorder that can be treated by augmenting an immune response (e.g., the T-cell mediated immune response). In a particular embodiment, the methods are particularly suitable for treatment of cancer *in vivo.* In one embodiment, the subject is a tumor-bearing subject and an immune response against the tumor is stimulated. A tumor may be a solid tumor or a liquid tumor, e.g., a hematological malignancy. In certain embodiments, a tumor is an immunogenic tumor. In certain embodiments, a tumor is non-immunogenic. In certain embodiments, a tumor is PD-L1 positive. In certain embodiments a tumor is PD-L1 negative. A subject may also be a virus-bearing subject and an immune response against the virus is stimulated.

Further provided are methods for inhibiting growth of tumor cells in a subject comprising administering to the subject an antibody described herein such that growth of the tumor is inhibited in the subject. Also provided are methods of treating viral infection in a subject comprising administering to the subject an antibody described herein such that the viral infection is treated in the subject.

Also encompassed herein are methods for depleting Treg cells from the tumor microenvironment of a subject having a tumor, e.g., cancerous tumor, comprising administering to the subject a therapeutically effective amount of an antibody described herein that comprises an Fc that stimulates depletion of $T_{reg}$ cells in the tumor microenvironment. An Fc may, e.g., be an Fc with effector function or enhanced effector function, such as binding or having enhanced binding to one or more activating Fc receptors.

In certain embodiments, an antibody comprising a modified heavy chain constant region binds to a stimulatory molecule and inhibits its activity, i.e., is an antagonist of a stimulatory molecule, or the antibody binds to an inhibitory molecule and stimulates its activity, i.e., is an agonist of an inhibitory molecule. Such antibodies may be used for treating disease in which the immune system or an immune response should be downregulated, e.g., autoimmune diseases or to prevent transplant rejections.

### *Cancer*

Provided herein are methods for treating a subject having cancer, comprising administering to the subject antibody described herein, such that the subject is treated, e.g., such that growth of cancerous tumors is inhibited or reduced and/or that the tumors regress. For example, activation of GITR by anti-GITR antibodies can enhance the immune response to cancerous cells in the patient. The antibody can be used alone to inhibit the growth of cancerous tumors. Alternatively, the antibody can be used in conjunction with another agent, e.g., other immunogenic agents, standard cancer treatments, or other antibodies, as described below.

Cancers whose growth may be inhibited using the antibodies described herein include cancers typically responsive to immunotherapy. Non-limiting examples of cancers for treatment include squamous cell carcinoma, small-cell lung cancer, non-small cell lung cancer, squamous non-small cell lung cancer (NSCLC), non NSCLC, glioma, gastrointestinal cancer, renal cancer (e.g. clear cell carcinoma), ovarian cancer, liver cancer, colorectal cancer, endometrial cancer, kidney cancer (e.g., renal cell carcinoma (RCC)), prostate cancer (e.g. hormone refractory prostate adenocarcinoma), thyroid cancer, neuroblastoma, pancreatic cancer, glioblastoma (glioblastoma multiforme), cervical cancer, stomach cancer, bladder cancer, hepatoma, breast cancer, colon carcinoma, and head and neck cancer (or carcinoma), gastric cancer, germ cell tumor, pediatric sarcoma, sinonasal natural killer, melanoma (e.g., metastatic malignant melanoma, such as cutaneous or intraocular malignant melanoma), bone cancer, skin cancer, uterine cancer, cancer of the anal region, testicular cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the parathyroid gland, cancer of the adrenal gland, sarcoma of soft tissue, cancer of the urethra, cancer of the penis, solid tumors of childhood, cancer of the ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally-induced cancers including those induced by asbestos, virus-related cancers (e.g., human papilloma virus (HPV)-related tumor), and hematologic malignancies derived from either of the two major blood cell lineages, i.e., the myeloid cell line (which produces granulocytes, erythrocytes, thrombocytes, macrophages and mast cells) or lymphoid cell line (which produces B, T, NK and plasma cells), such as all types of luekemias, lymphomas, and myelomas, e.g., acute, chronic, lymphocytic and/or myelogenous leukemias, such as acute leukemia (ALL), acute myelogenous leukemia (AML), chronic lymphocytic leukemia (CLL), and chronic myelogenous leukemia (CML), undifferentiated AML (M0), myeloblastic leukemia (M1), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphomas, such as Hodgkin's lymphoma (HL), non-Hodgkin's lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic (e.g., Ki 1+) large-cell lymphoma, adult T-cell lymphoma/leukemia, mantle cell lymphoma, angio immunoblastic T-cell lymphoma, angiocentric lymphoma, intestinal T-cell lymphoma, primary mediastinal B-cell lymphoma, precursor T-lymphoblastic lymphoma, T-lymphoblastic; and lymphoma/leukaemia (T-Lbly/T-ALL), peripheral T- cell lymphoma, lymphoblastic lymphoma, post-transplantation lymphoproliferative disorder, true histiocytic lymphoma, primary central nervous system lymphoma, primary effusion lymphoma, lymphoblastic lymphoma (LBL), hematopoietic tumors of lymphoid lineage, acute lymphoblastic leukemia, diffuse large B-cell lymphoma, Burkitt's lymphoma, follicular lymphoma, diffuse histiocytic lymphoma (DHL), immunoblastic large cell lymphoma, precursor B-lymphoblastic lymphoma, cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, nonsecretory myeloma, smoldering myeloma (also called indolent myeloma), solitary plasmocytoma, and multiple myelomas, chronic lymphocytic leukemia (CLL), hairy cell lymphoma; hematopoietic tumors of myeloid lineage, tumors of mesenchymal origin, including fibrosarcoma and rhabdomyoscarcoma; seminoma, teratocarcinoma, tumors of the central and peripheral nervous, including astrocytoma, schwannomas; tumors of mesenchymal origin, including fibrosarcoma, rhabdomyoscaroma, and osteosarcoma; and other tumors, including melanoma, xeroderma pigmentosum, keratoacanthoma, seminoma, thyroid follicular cancer and teratocarcinoma, hematopoietic tumors of lymphoid lineage, for example T-cell and B-cell tumors, including but not limited to T-cell disorders such as T-prolymphocytic leukemia (T-PLL), including of the small cell and cerebriform cell type; large granular lymphocyte leukemia (LGL) preferably of the T-cell type; a/d T-NHL hepatosplenic lymphoma; peripheral/post-thymic T cell lymphoma (pleomorphic and immunoblastic subtypes); angiocentric (nasal) T-cell lymphoma; cancer of the head or neck, renal cancer, rectal cancer, cancer of the thyroid gland; acute myeloid lymphoma, as well as any combinations of said cancers. The methods described herein may also be used for treatment of metastatic cancers, refractory cancers (e.g., cancers refractory to previous immunotherapy, e.g., with a blocking CTLA-4 or PD-1 antibody), and recurrent cancers.

## *Combination Therapies*

In addition to the therapies provided above, the antibodies described herein can also be used in combination with another therapy. For example, for cancer treatment, an antibody described herein may be administered to a subject who is also receiving another cancer treatement, such as chemotherapy, radiation, surgery or gene therapy.

Methods of treatment may include coadministration of an antibody described herein (e.g., an antagonist antibody, agonist antibody, and ADC having a modified heavy chain constant region) with another molecule, e.g., antibody (e.g., an antagonist antibody, agonist antibody, and ADC). An antibody described herein that stimulates the immune system may be administered with another molecule that stimulates the immune system, e.g., a molecule that is an agonist of a co-stimulatory molecule or an inhibitor of an inhibitory molecule.

An antibody as described herein alone or with one or more additional immune stimulating antibodies (e.g., CTLA-4 and/or PD-1 and/or PD-L1 and/or LAG-3 blockade) can be combined with standard cancer treatments. For example, an antibody described herein alone or with one or more additional antibodies can be effectively combined with chemotherapeutic regimes. In these instances, it may be possible to reduce the dose of other chemotherapeutic reagent administered with the combination of the instant disclosure (Mokyr et al. (1998) Cancer Research 58: 5301-5304). An example of such a combination is a combination of an antibody described herein, with or without and an additional antibody, further in combination with decarbazine or IL-2 for the treatment of melanoma.

An antibody described herein may be combined with an anti-neoplastic antibody, such as Rituxan® (rituximab), Herceptin® (trastuzumab), Bexxar® (tositumomab), Zevalin® (ibritumomab), Campath® (alemtuzumab), Lymphocide® (eprtuzumab), Avastin® (bevacizumab), and Tarceva® (erlotinib), and the like. Antibodies described herein may also be combined with one or more of the following chemotherapeutic agents: camptothecin (CPT-11), 5-fluorouracil (5-FU), cisplatin, doxorubicin, irinotecan, paclitaxel, gemcitabine, cisplatin, paclitaxel, carboplatin-paclitaxel (Taxol), doxorubicin, 5-fu, or camptothecin + apo21/TRAIL (a 6X combo)); a proteasome inhibitor (e.g., bortezomib or MG132); a Bcl-2 inhibitor (e.g., BH3I-2' (bcl-xl inhibitor), indoleamine dioxygenase-1 (IDO1) inhibitor (e.g., INCB24360), AT-101 (R-(-)-gossypol

EP 4 098 662 A1

derivative), ABT-263 (small molecule), GX-15-070 (obatoclax), or MCL-1 (myeloid leukemia cell differentiation protein-1) antagonists), iAP (inhibitor of apoptosis protein) antagonists (e.g., smac7, smac4, small molecule smac mimetic, synthetic smac peptides (see Fulda et al., Nat Med 2002;8:808-15), ISIS23722 (LY2181308), or AEG-35156 (GEM-640)), HDAC (histone deacetylase) inhibitors, anti-CD20 antibodies (e.g., rituximab), angiogenesis inhibitors (e.g., bevacizumab), antiangiogenic agents targeting VEGF and VEGFR (e.g., Avastin), synthetic triterpenoids (see Hyer et al., Cancer Research 2005;65:4799-808), c-FLIP (cellular FLICE-inhibitory protein) modulators (e.g., natural and synthetic ligands of PPARγ (peroxisome proliferator-activated receptor γ), 5809354 or 5569100), kinase inhibitors (e.g., Sorafenib), Trastuzumab, Cetuximab, Temsirolimus, mTOR inhibitors such as rapamycin and temsirolimus, Bortezomib, JAK2 inhibitors, HSP90 inhibitors, PI3K-AKT inhibitors, Lenalildomide, GSK3β inhibitors, IAP inhibitors and/or genotoxic drugs.

The antibodies and combination antibody therapies described herein can further be used in combination with one or more anti-proliferative cytotoxic agents. Classes of compounds that may be used as anti-proliferative cytotoxic agents include, but are not limited to, the following:

Alkylating agents (including, without limitation, nitrogen mustards, ethylenimine derivatives, alkyl sulfonates, nitrosoureas and triazenes): Uracil mustard, Chlormethine, Cyclophosphamide (CYTOXAN™) fosfamide, Melphalan, Chlorambucil, Pipobroman, Triethylenemelamine, Triethylenethiophosphoramine, Busulfan, Carmustine, Lomustine, Streptozocin, Dacarbazine, and Temozolomide.

Antimetabolites (including, without limitation, folic acid antagonists, pyrimidine analogs, purine analogs and adenosine deaminase inhibitors): Methotrexate, 5-Fluorouracil, Floxuridine, Cytarabine, 6-Mercaptopurine, 6-Thioguanine, Fludarabine phosphate, Pentostatine, and Gemcitabine.

Suitable anti-proliferative agents for combining with antibodies described herein, without limitation, taxanes, paclitaxel (paclitaxel is commercially available as TAXOL™), docetaxel, discodermolide (DDM), dictyostatin (DCT), Peloruside A, epothilones, epothilone A, epothilone B, epothilone C, epothilone D, epothilone E, epothilone F, furanoepothilone D, desoxyepothilone BI, [17]-dehydrodesoxyepothilone B, [18]dehydrodesoxyepothilones B, C12,13-cyclopropyl-epothilone A, C6-C8 bridged epothilone A, trans-9,10-dehydroepothilone D, cis-9,10-dehydroepothilone D, 16-desmethylepothilone B, epothilone B10, discoderomolide, patupilone (EPO-906), KOS-862, KOS-1584, ZK-EPO, ABJ-789, XAA296A (Discodermolide), TZT-1027 (soblidotin), ILX-651 (tasidotin hydrochloride), Halichondrin B, Eribulin mesylate (E-7389), Hemiasterlin (HTI-286), E-7974, Cyrptophycins, LY-355703, Maytansinoid immunoconjugates (DM-1), MKC-1, ABT-751, T1-38067, T-900607, SB-715992 (ispinesib), SB-743921, MK-0731, STA-5312, eleutherobin, 17beta-acetoxy-2 -ethoxy-6-oxo-B-homo-estra-1,3,5(10)-trien-3-ol, cyclostreptin, isolaulimalide, laulimalide, 4-epi-7-dehydroxy-14,16-didemethyl-(+)-discodermolides, and cryptothilone 1, in addition to other microtubuline stabilizing agents known in the art.

Combination treatments can be administered simultaneously or sequentially. In certain examples, combinations are fixed dose combinations.

In cases where it is desirable to render aberrantly proliferative cells quiescent in conjunction with or prior to treatment with the antibodies described herein, hormones and steroids (including synthetic analogs), such as 17a-Ethinylestradiol, Diethylstilbestrol, Testosterone, Prednisone, Fluoxymesterone, Dromostanolone propionate, Testolactone, Megestrolacetate, Methylprednisolone, Methyl-testosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, ZOLADEX™, can also be administered to the patient. When employing the methods or compositions described herein, other agents used in the modulation of tumor growth or metastasis in a clinical setting, such as antimimetics, can also be administered as desired.

Methods for the safe and effective administration of chemotherapeutic agents are known to those skilled in the art. In addition, their administration is described in the standard literature. For example, the administration of many of the chemotherapeutic agents is described in the Physicians' Desk Reference (PDR), e.g., 1996 edition (Medical Economics Company, Montvale, N.J. 07645-1742, USA); the disclosure of which is incorporated herein by reference thereto.

The chemotherapeutic agent(s) and/or radiation therapy can be administered according to therapeutic protocols well known in the art. It will be apparent to those skilled in the art that the administration of the chemotherapeutic agent(s) and/or radiation therapy can be varied depending on the disease being treated and the known effects of the chemotherapeutic agent(s) and/or radiation therapy on that disease. Also, in accordance with the knowledge of the skilled clinician, the therapeutic protocols (e.g., dosage amounts and times of administration) can be varied in view of the observed effects of the administered therapeutic agents on the patient, and in view of the observed responses of the disease to the administered therapeutic agents.

The present disclosure is further illustrated by the following examples, which should not be construed as further limiting. The contents of all figures and all references, Genbank sequences, patents and published patent applications cited throughout this application are expressly incorporated herein by reference. In particular, the disclosures of PCT applications WO 09/045957, WO 09/073533, WO 09/073546, WO 09/054863, PCT/US2013/072918, PCT/US15/61632, and U.S. Patent Publication No. 2011/0150892 are expressly incorporated herein by reference.

44

**EXAMPLES**

*Example 1: Enhanced internalization of anti-CD73 antibodies with an IgG2 hinge relative to the same antibodies with α non-IgG2 hinge*

It had been observed that hybridoma derived anti-CD73 antibody 11F11, which has an IgG2 constant region, is more potent in cellular CD73 inhibition assays than the 11F11 antibody as an IgG1 or IgG1.1 (effectorless IgG1), and more potent than other anti-CD73 antibodies having IgG1 constant regions. Based at least on this observation, it was hypothesized that increased inhibitory activity of anti-CD73 antibodies having IgG2 hinges relative to those having non-IgG2 hinges, such as IgG1 hinges, was due to increased internalization of the antibodies. To test this hypothesis, anti-CD73 antibodies having IgG1 or IgG2 constant regions or portions thereof were tested in internalization assays.

The antibodies that were used are listed in Table 7 which provides the identities of each of the domains of the constant regions (all human) of each antibody, including specific mutations if present.

**Table 7**

| Name of antibody | VH | CH1 | Hinge | CH2 | CH3 | HC SEQ ID NO[1] | LC SEQ ID NO[2] |
|---|---|---|---|---|---|---|---|
| 11F11 | 11F11 | IgG2 | IgG2 | IgG2 | IgG2 | 44 | 72 |
| 4C3 | 4C3 | IgG1 | IgGl | IgG1 | IgG1 | 45 | 73 |
| 6D11 | 6D11 | IgG1 | IgG1 | IgG1 | IgG1 | 46 | 74 |
| CD73.10-IgG2-C219S | CD73.10 | IgG2 | IgG2 (C219S) | IgG2 | IgG2 | 47 | 72 |
| CD73.10-IgG2-C219S-IgG1.1 | CD73.10 | IgG2 | IgG2 (C219S) | IgG1.1 (A330S/P331S) | IgG2 | 48 | 72 |
| CD73.10-IgG1.1 | CD73.10 | IgG1.1 | IgG1.1 (L234A/L235E/G237 A) | IgG1.1 (A330S/P331S) | IgG1.1 | 49 | 72 |
| CD73.4- IgG2-C219S | CD73.10 | IgG2 | IgG2 (C219S) | IgG2 | IgG2 | 50 | 72 |
| CD73.3-IgG1.1 | CD73.3 | IgG1.1 | IgG1.1 (L234A/L235E/G237A) | IgG1.1 (A330S/P331S) | IgG1.1 | 51 | 73 |
| [1] SEQ ID NO of full length heavy chain [2] SEQ ID NO of full length light chain | | | | | | | |

The antibodies were made by expressing the heavy and light chains in HEK293-6E cells, and culture media was harvested 5 days after transfection.

Binding of the constructs to FcγRs was measured. hCD64 and hCD32a-H131 binding data for IgG1.1 and IgG2 molecules were consistent with expected values for the different Fcs. IgG1.1f is the most inert Fc. IgG2 and IgG2-C219S showed typical FcR binding for IgG2. As expected, data for IgG2-C219S-G1.1f suggests significantly weaker binding than wild type IgG1 or IgG2, but increased binding compared to IgG1.1f.

The affinity of the antibodies for human CD73 was measured to determine whether the change of the constant region affects them. The affinities were determined by Surface Plasmon Resonance (SPR) as follows. CD73 binding kinetics and affinity were studied by surface Plasmon resonance (SPR) using a Biacore T100 instrument (GE Healthcare) at 25°C. This experiment tested the binding of the N-terminal domain of hCD73 (consisting of residues 26 - 336 of human CD73; termed N-hCD73) to antibodies that were captured on immobilized protein A surfaces. For these experiments, protein A (Pierce) was immobilized to a density of 3000 - 4000 RU on flow cells 1-4 of a CM5 sensor chip (GE Healthcare) using standard ethyl(dimethylaminopropyl) carbodiimide (EDC) / N-hydroxysuccinimide (NHS) chemistry, with ethanolamine blocking, in a running buffer of 0.01 M HEPES pH 7.4, 0.15 M NaCl, 3 mM EDTA, 0.005% v/v tween 20. Kinetic experiments were performed by first capturing antibodies (5-10ug/ml) on the protein A surfaces using a 30 s contact time at 10ul/min, with binding of 600, 200, 66.7, 22.2, 7.4, and 2.5 nM N-hCD73-his, using a 180 s association time and 360 s dissociation time at a flow rate of 30 ul/min. The running buffer for the kinetic experiments was 10 mM sodium phosphate, 130 mM sodium chloride, 0.05% tween 20, pH 7.1. The surfaces were regenerated after each cycle using two 30 s pulses of 10 mM glycine pH 1.5 at a flow rate of 30 $\mu$l/min. Sensogram data was double-referenced and then fitted to a 1:1 Langmuir model using Biacore T100 evaluation software v2.0.4, to determine the association rate constant (ka), the dissociation rate constant (kd), and the equilibrium dissociation constant (KD).

The results are shown in Table 8. The table compiles data from different experiments. For antibodies for which two sets of numbers are shown, each set corresponds to data obtained in a separate experiment.

**Table 8**

| mAb | Fc | ka (1/Ms) | kd (1/s) | KD (nM) |
|---|---|---|---|---|
| 11F11 | IgG2 | 2.6E+05 | 4.2E-04 | 1.6 |
| | | 2.9 E+05 | 1.6 E-04 | 0.56 |
| 4C3 | IgG1 | 2.2E+04 | 2.4E-03 | 110 |
| | | 2.4 E+04 | 2.2 E-03 | 92 |
| 6E11 | IgG1 | 5.7E+04 | 1.4E-03 | 25 |
| CD73.10 | IgG1.1 | 2.7 E+05 | 1.3 E-03 | 4.7 |
| CD73.10 | IgG2-C219S | 2.2 E+05 | 1.4 E-03 | 6.2 |
| | | 2.2 E+05 | 1.8 E-03 | 8.3 |
| CD73.10 | IgG2-C219S-IgG1.1 | 2.4 E+05 | 1.4 E-03 | 5.7 |
| | | 2.3 E+05 | 1.60E-03 | 6.8 |
| CD73.4 | I IgG2-C219S | 2.9 E+05 | 1.6 E-04 | 0.55 |
| | | 2.8 E+05 | 3.3 E-04 | 1.2 |
| | | 2.9 E+05 | 3.7 E-04 | 1.3 |
| CD73.3 | IgG1.1 | 1.6 E+04 | 3.6 E-03 | 220 |

The results indicate that the presence of the different constant regions in an antibody, e.g., CD73.10, did not change the affinity of the antibody to human CD73.

The internalization of anti-CD73 antibodies was measured in two different assays.

A. High-content internalization assay (2 hour fixed time assay)

The anti-CD73 antibodies used to test anti-CD73 antibody dependent CD73 internalization in Calu6 cells by assessing cellular expression after 2 hours of antibody incubation. Cells (2,000 cells/well) in 20 $\mu$l of complete medium (Gibco RPMI Media 1640 with 10% heat inactivated fetal bovine serum) were plated in 384 BD Falcon plate and grown overnight at 37°C 5% $CO_2$ and 95% humidity. Anti-CD73 antibodies were serially diluted with PBS buffer containing 0.2% BSA, and added 5 $\mu$l/well into the cell plate. The cells were incubated with antibodies for 2 hours at 37°C 5% $CO_2$ and 95% humidity, followed by washing once with PBS buffer. Formaldehyde (final 4% in PBS) was then added into the cell plate at 20ul/well, and the plate was incubated at room temperature for 10 minutes. Afterwards, all liquid was aspirated and cells were washed once with 30ul PBS. Detection antibody (2.5 $\mu$g/well of anti-CD73 Ab CD73.10.IgG2C219S) was added at 15 $\mu$g/well into the fixed cell plate. The cells were incubated at 4°C overnight. On the next day, the plate was washed twice with PBS buffer, followed by adding secondary antibody containing Alexa-488 goat anti human and DAPI, stained for 1 hour at room temperature. After 3 washes in PBS buffer, the plate was imaged on Arrayscan Vti (Cellomics, Pittsburgh, PA). $IC_{50}$ and Ymax were measured. Ymax was determined by comparing to 100nM dose of 11F11 as internal maximum. All calculations were determined as a percentage of internalization compared to this control, which was set to 100%.

The results are provided in Table 9.

**Table 9**

| mAb | Constant region | Epitope bin | EC50 (nM) | Ymax |
|---|---|---|---|---|
| 11F11 | IgG2 | 1 | 0.58 | 98 |
| 4D4 | IgG2 | 1 | 0.38 | 104 |
| 10D2 | IgG1 | 1 | ND | 29 |
| 24H2 | IgG1 | 1 | 8.2 | 51 |
| 7A11 | IgG1 | 1 | 2.59 | 50 |
| CD73.4 | IgG2-C219S-IgG1.1 | 1 | 1.2 | 97 |
| CD73.10 | IgG1.1 | 1 | 6.18 | 64 |
| CD73.10 | IgG2-C219S | 1 | 0.67 | 99 |
| CD73.10 | IgG2-C219S-IgG1.1 | 1 | 0.87 | 99 |
| ND = Not Detected<br>NA = Not Applicable | | | | |

The results show that anti-CD73 antibodies having an IgG2 hinge have a lower EC50 and higher Ymax.

Kinetic internalization studies were performed to assess the rate of internalization. Several cells lines were tested: H2228 cells, HCC15 cells, Calu6 cells, and NCI-H2030. Cells (2,000 cells/well) in 20 $\mu$l of complete medium (Gibco RPMI Media 1640 with 10% heat inactivated fetal bovine serum) were plated in 384 BD Falcon plate and grown overnight at 37°C 5% $CO_2$ and 95% humidity. CD73 antibodies were diluted with PBS buffer containing 0.2% BSA to 10$\mu$g/ml and added 5 $\mu$l/well into the cell plate. The cells were incubated with antibodies for 0-2 hour time course at 37°C, followed by washing once with PBS buffer. The cells were subsequently fixed with formaldehyde (final 4% in PBS) at room temperature for 10 minutes, and then washed once with 30ul PBS. Detection antibody (2.5 $\mu$g/well anti-CD73 Abs CD73.10.IgG2C219S) were diluted with PBS buffer containing 0.2% BSA, and added 15 $\mu$l/well into the fixed cell plate. The plate was incubated at 4°C for overnight. On the next day, after 3 washes in PBS buffer, Secondary antibody Alexa488-goat anti human with DAPI were added. The cells were stained for 60 minutes at room temperature, after 3 washes, images were acquired using Arrayscan Vti (Cellomics, Pittsburgh, PA). The results are provided in Figures 1A - J and Tables 10 and 11. The values in Table 10 derive from the data shown in Figures 1A - J.

**Table 10**

| Cell line | 11F11(IgG2) $T_{1/2}$ (min) | 6E11(IgG1) $T_{1/2}$ (min) | CD73.10.IgG1.1f $T_{1/2}$ (min) |
|---|---|---|---|
| Calu6 | 3.9 | 60.9 | 14.4 |
| HCC44 | 3.3 | 27.9 | 23.5 |
| H2030 | 3.3 | 40.3 | 18.3 |
| H647 | 45.7 | N/A | N/A |
| H2228 | 10.9 | 36.5 | 35.7 |
| HCC15 | 2.2 | 84.4 | 37.9 |
| SKLU1 | 6.8 | 18.0 | 17.2 |
| SKMES1 | 2.2 | 62.8 | 32.3 |
| SW900 | 10.3 | 94.9 | 43.4 |

EP 4 098 662 A1

**Table 11:** $T_{1/2}$ and % internalization of CD73 antibodies in 4 human cell lines

| | H228 cells | | HCC15 cells | | Calu6 cells | | H2030 cells | |
|---|---|---|---|---|---|---|---|---|
| | $T_{1/2}$ min | % internalization | $T_{1/2}$ min | % internalization | $T_{1/2}$ min | % internalization | $T_{1/2}$ min | % internalization |
| CD73.11-IgG2CS | - | - | - | - | 4.1 | 89 | 4.6 | 85 |
| CD73.10-IgG2CS | 9.7 | 93 | 2.6 | 91 | 3.0 | 91 | 3.3 | 85 |
| CD73.10-IgG2CS-IgG1.1f | 9.4 | 92 | 3.0 | 91 | 3.1 | 91 | 4.3 | 87 |
| CD73.4-IgG2CS | 13.8 | 94 | 3.1 | 94 | 6.5 | 88 | 3.7 | 89 |
| CD73.10-IgG1.1f | 35.7 | 33 | 37.9 | 71 | 14.4 | 63 | 18.3 | 67 |
| CD73.3-IgG1.1f | 16.5 | -47 | >240 | 38 | 111.4 | 79 | >120 | 27 |
| 11F11 | 10.9 | 96 | 2.2 | 94 | 3.9 | 87 | 3.3 | 90 |
| 4C3 | 7.6 | -48 | 141.5 | 28 | 0.6 | -6 | >120 | -34 |
| 6E11 | 36.5 | 13 | 84.4 | 64 | 107.4 | 68 | 40.32 | 51 |

The results indicate that 11F11 (an IgG2 antibody) internalized within minutes, reaching a plateau in 30 minutes, whereas 6E11 (an IgG 1 antibody) internalized more slowly, reaching a plateau at about 1hr (Figures 1A-J). Similarly, 11F11 with an IgG1 constant region internalized more slowly than 11F11 with an IgG2 constant region. This trend was observed in several cell lines (Tables 10 and 11 and Figures 1A-J).

B. Internalization measured by flow cytometry

Anti-CD73 antibody mediated internalization of CD73 was also tested by flow cytometry. Indicated cells were incubated with 10μg/mL of the indicated antibody for 30 minutes on ice, washed several times, and transferred to 37 °C for the indicated time. Cells were harvested at the same time after the indicated incubation time. Cells were stained with primary antibody again (same antibody used for initial incubation) followed by anti-human secondary antibody. Cells were then assayed for expression of CD73 by flow cytometry.

The results, which are shown in Figure 1E and Table 11, are consistent with those obtained in the internalization assays described above, and indicate that, all antibodies with IgG2 hinge and CH1 induced rapid and complete internalization. The CD73 levels remained low after 22 hours post wash-out, indicating that internalization is durable.

Similar results shown in Figure 1F and Table 11 were obtained in the NCI-H292 cell line in which the antibody was maintained in culture during the incubation time (no wash-out). Again, these data indicate rapid and significant internalization and maintenance of downregulation of endogenous CD73.

Internalization assays were also conducted with the human SNU-C1 (colon cancer cell line) and NCI-H1437 (non-small cell lung carcinoma cell line) cells. The results, which are shown in Figures 1I and J, also indicate rapid internalization with a maximal level reached within 5 hours and a maximal level of internalization of about 50% for CD73.4.IgG2-C219S-IgG1.1f in SNU-C1 and 60% for NCI-H1437 cells. Figures 1G and H show similar kinetics of internalization of CD73.4.IgG2-C219S-IgG1.1f in Calu6 and NCI-H292 cells. For graphs, which show % of CD73 internalized, this number was obtained as follows:

$$\% \ CD73 \ internalized = 100 - \left( \frac{MFI_{t=x} - MFI_{background}}{MFI_{t=0} - MFI_{background}} \times 100 \right)$$

where for each antibody, $MFI_{t=x}$ is the MFI at a given timepoint and $MFI_{t=0}$ is maximal fluorescence at t=0, and $MFI_{background}$ is the MFI of the secondary Ab only.

**Table 12:** $EC_{50}$ of antibody mediated CD73 internalization in several cell lines (data from Figures 1G-I)

| | Calu6 | | NCI-H292 | | SNU-C1 | | SNU-C1 (no wash) | | NCI-H1437 | | NCI-H1437 (no wash) | |
|---|---|---|---|---|---|---|---|---|---|---|---|---|
| | Ymax (%) | $T_{1/2}$ (hr) | Ymax (%) | $T_{1/2}$ (hr) | Ymax (%) | $T_{1/2}$ (hr) | Ymax (%) | $T_{1/2}$ (hr) | Ymax (%) | $T_{1/2}$ (hr) | Ymax (%) | $T_{1/2}$ (hr) |
| mAb-CD73.4-IgG2-IgG1.1f | 76.8 | 0.5661 | 77.64 | 0.2633 | 48.96 | 0.4954 | 38.39 | 1.025 | 63.12 | 0.3164 | 62.78 | 0.3418 |
| mAb-CD73.4-IgG2 | 75.59 | 0.6003 | 78.42 | 0.2766 | | | | | | | | |
| mAb-CD73.4-IgG1.1f | 44.99 | 1.737 | 51.49 | 0.2087 | 30.58 | 0.9915 | 33.16 | 2.33 | 49.76 | 0.4915 | 49.95 | 0.5384 |

Thus, anti-CD73 antibodies with an IgG2 hinge internalize faster and to a greater extent relative to anti-CD73 antibodies with an IgG1 hinge.

***Example 2: Enhanced agonist activity of GITR antibodies with an IgG2 hinge relative to the same antibodies with an IgG1 hinge***

This Example demonstrates that anti-GITR antibodies comprising an IgG2 hinge have an increased ability to induce IL-2 and IFN-γ secretion from T cells relative to the same antibodies that have an IgG1 hinge.

It had been observed in CHO-OKT3 and 3A9 assays described above that hybridoma derived antibodies, having an IgG2 constant region, are more potent in stimulating cytokine secretion than the same antibodies in which the heavy chain constant region was switched to that of IgG1 or an effectorless IgG1 (IgG1.1). Therefore, the effect of an IgG2 constant region or hinge was further tested on anti-GITR antibodies in these assays.

The heavy chain variable region of an anti-human GITR antibody (SEQ ID NO: 75) was linked to the heavy chain constant regions shown in Table 13. The light chain of the anti-GITR antibodies comprised SEQ ID NO: 77. Table 13 shows the identity of each domain of the constant regions:

**Table 13:** Heavy chain constant regions of antibodies used in this Example

| Name of antibody | CH1 | Hinge | CH2 | CH3 | SEQ ID NO* |
|---|---|---|---|---|---|
| anti-GITR | IgG2 SEQ ID NO:7 | IgG2 SEQ ID NO:8 | IgG2 SEQ ID NO: 9 | IgG2 SEQ ID NO:10 | SEQ ID NO: 52 |
| anti-GITR -IgG2 | IgG2 SEQ ID NO:7 | IgG2 SEQ ID NO:8 | IgG2 SEQ ID NO: 9 | IgG2 SEQ ID NO:10 | SEQ ID NO: 52 |
| anti-GITR -IgG1 | IgG1 SEQ ID NO:2 | IgG1 SEQ ID NO:3 | IgG1 SEQ ID NO: 4 | IgG1 SEQ ID NO:5 | SEQ ID NO: 53 |
| anti-GITR -IgG1.1 | IgG1.1 SEQ ID NO:2 | IgG1.1 (L234A/L235E/G237A) SEQ ID NO:25 | IgG1.1 (A330S/P331S) SEQ ID NO:24 | IgG1.1 SEQ ID NO:5 | SEQ ID NO: 54 |
| anti-GITR -IgG2-IgG1 or anti-GITR.g2.g1 | IgG2 SEQ ID NO:7 | IgG2/IgG1 hybrid SEQ ID NO:22 | IgG1 SEQ ID NO: 4 | IgG1 SEQ ID NO:5 | SEQ ID NO: 55 |
| anti -GITR -IgG2-IgG 1.1 or anti-GITR.g2.g1.1 | IgG2 SEQ ID NO:7 | IgG2 SEQ ID NO:8 | IgG1.1 (A330S/P331S) SEQ ID NO:24 | IgG1 SEQ ID NO:5 | SEQ ID NO: 56 |
| *SEQ ID NO of full-length heavy chain constant region | | | | | |

First, the binding affinities of these GITR antibodies were compared to those of GITR antibodies having an IgG1 hinge. The binding affinities of the anti-GITR antibodies to soluble GITR was determined by Biacore as follows. Anti-GITR antibodies were captured on human kappa coated chips (~5KRUs; Southernbiotech cat#2060-01), and recombinant human GITR (rHGITR/Fc: R&D systems, CAT#689-GR) was flowed across the chip at concentrations of 500 nM, 250 nM, 125 nM, 62 nM, and 31 nM. The capture concentration of the mAb/volume was 2-40 μg/mL (5 μL at 10 μL/min). The antigen association time was 5 minutes at 15 μL/min, the antigen dissociation time was 6 minutes, and regeneration was performed with 50 mM HCl/50 mM NaOH (12 μL each at 100 μL/min).

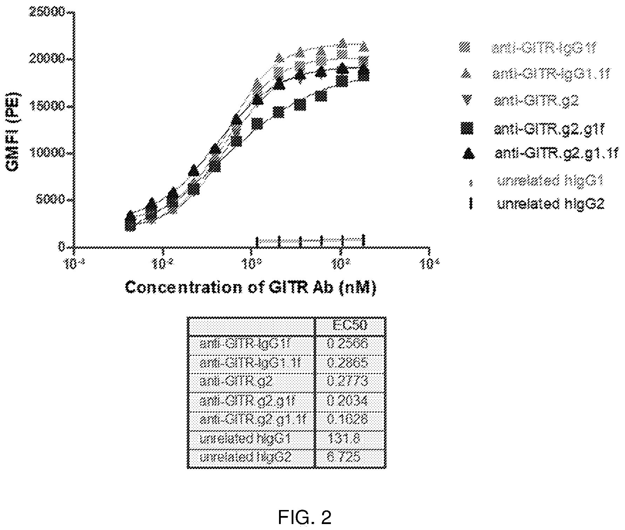

The results, which are shown in Figure 2, indicate that all three GITR antibodies having an IgG2 hinge have similar affinities for activated T cells as GITR antibodies have IgG1 or IgG1.1 constant region.

Next, the ability of GITR antibodies having an IgG1 constant region or IgG2 hinge/IgG1 Fc domain were tested for their ability to induce IL-2 and IFN-γ secretion from human donor T cells stimulated with anti-CD3scFv (OKT3)-expressing CHO cells. The CHO cells expressed low levels of OKT3 to promote suboptimal stimulation to be able to observe agonism by anti-GITR antibodies. CD4+ T cells from a donor were stimulated with OKT3 expressing CHO cells and an anti-GITR antibody, and IL-2 and IFN-γ secretion was measured. The experiments were conducted as follows. For experiments with CD4+ T cells, CD4+T cells were obtained from human PBMCs with RosetteSep Human CD4+ T cell enrichment cocktail (StemCell Technology#15062) according to the manufacturer's protocol. CHO cells expressing anti-CD3scFv (OKT3) (CHO-OKT3) were washed twice with RPMI medium and subjected to irradiation with a dosage of 50K Rad. Cells were harvested and resuspended in culture medium (RPMI-1640 supplemented with 10% Fetal Bovine Serum, 2mM L-glutamine, 55nM β-Mercaptoethanol, 1mM sodium pyruvate, and 100U/mL Penicillin/streptomycin) at $2.5 \times 10^5$/mL. $2.5 \times 10^4$ CHO-OKT3 cells and $1 \times 10^5$ T cells were seeded per well in a 96-well TC grade flat-bottom plate (Costar). Cells were incubated with an 8-point, 4-fold titration of GITR antibody starting at 40 μg/mL. An irrelevant hIgG1 was added at 40 μg/mL as an isotype control. A sample with cells only was included to show baseline activity without any treatment. Supernatant from each sample was harvested at day 2 for IL-2 measurement (only for assays with CD4+ T cells) (BD opt EIA Human IL- 2 ELISA kit; BD Bioscience#555190) and at day 3 for IFN-γ measurement (BD optEIA human IFN-g ELISA Kit; BD Bioscience#555142).

As shown in Figures 3A and B, the antibody with the IgG2 hinge/IgG1 Fc domain (anti-GITR.g2.g1) induced both IL-2 and IFN-γ secretion from T cells to a higher degree than the antibody with the IgG1 constant region (anti-GITR.gl). Similar results were obtained with the effectorless versions of these constant domains (Figure 3C).

To further confirm the increased activation of T cells with the anti-GITR antibodies comprising an IgG2 hinge, IL-2 secretion in a different experimental format was tested. In this experiment, the ability of GITR antibodies to induce IL-2 secretion from 3A9-hGITR cells (mouse T cell hybridoma 3A9 cell line ectopically expressing human GITR) was tested as follows. Mouse T cell hybridoma 3A9 cell line which ectopically expresses human GITR (3A9-hGITR) was cultured on anti-CD3 monoclonal antibody-coated plates in the presence of increasing amounts of the indicated antibodies. $5 \times 10^4$ 3A9-hGITR cells were cultured on plates coated with 1 μg/ml anti-CD3 antibody (Clone 145-2C11; BD Biosciences), and treated with the indicated concentrations of antibodies for 7 hours.

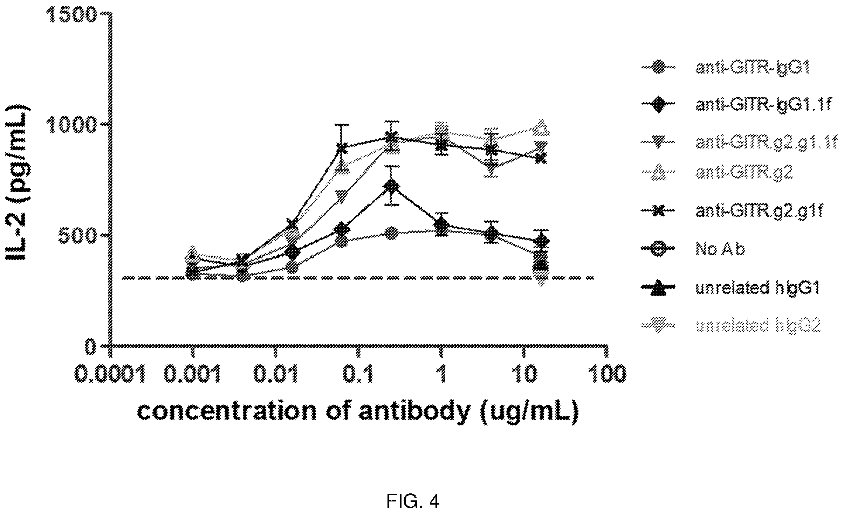

As shown in Figure 4, all antibodies having the IgG2 hinge (anti-GITR.g2, anti-GITR.g2.glf, and anti-GITR.g2.g1.f) induced IL-2 secretion from 3A9-hGITR cells to a higher degree than their IgG1 constant region containing counterparts (anti-GITR.glf and anti-GITR.g1.1f).

These results collectively suggest that anti-GITR antibodies having an IgG2 hinge and g1 or g1.1 constant regions are more potent than the same antibodies having an IgG1 hinge.

### *Example 3: Impact of different hinge/Fc combinations on size of antibody/antigen complexes*

As shown in the above Examples, anti-CD73 antibodies with an IgG2 hinge are better inhibitors of CD73 cellular activity and internalize better than the same antibodies with an IgG1 hinge and anti-GITR antibodies with an IgG2 hinge are more potent agonists than the same antibodies with an IgG1 hinge. Based on this observation, and the fact that an IgG2 hinge is stiffer than an IgG1 hinge, it was hypothesized that larger complexes are formed between an antigen and antibodies having an IgG2 hinge relative to antibodies having an IgG1 hinge. The following experiment was conducted to analyze this hypothesis.

The structure and oligomeric state of CD73/antibody complexes in solution were examined by SEC-MALS and DLS. For these studies, antibodies containing either an IgG1 or IgG2 constant region, were mixed at varying molar ratios with recombinant proteins comprising either the full length extracellular domain of human-CD73 containing a C-terminal polyhistidine tag (amino acid residues 26 - 546 of human-CD73, termed hCD73-his) or a fragment corresponding to the N-terminal domain of human-CD73 (amino acid residues 26 - 336, termed N-hCD73-his).

The oligomeric state of CD73/antibody complexes were examined by size-exclusion chromatography coupled to an in-line multi-angle light scattering detector (SEC-MALS). Isocratic separations were performed on a Shodex PROTEIN KW-803 column connected to an Prominence Shimadzu UFLC in buffer containing 200 mM $K_2HPO_4$, 150 mM NaCl, pH 6.8, containing 0.02% Na azide (0.1 μm filtered) running at 0.5 mL/min. Samples were injected onto the column using a SIL-20AC Prominence Shimadzu autosampler, and data were obtained from three online detectors connected in series: a Prominence SPD-20AD diode array UV/vis spectrophotometer followed by a Wyatt miniDAWN™ TREOS Multi-Angle Light Scattering Detector then a Wyatt Optilab T-rEX Refractive Index Detector. Data were collected and analyzed using Astra (Wyatt) and Labsolutions (Shimadzu) software.

Dynamic light scattering (DLS) studies were performed on a Wyatt DynaPro plate reader in 384 well plates at 25°C. Experimental parameters were 20 acquisitions of 5 s each per measurement, and measurements were recorded in quadruplicate, with the average and standard deviation reported. Intensity autocorrelation functions were fitted using the "Regularization" algorithm in the Dynamics software (Wyatt Technologies).

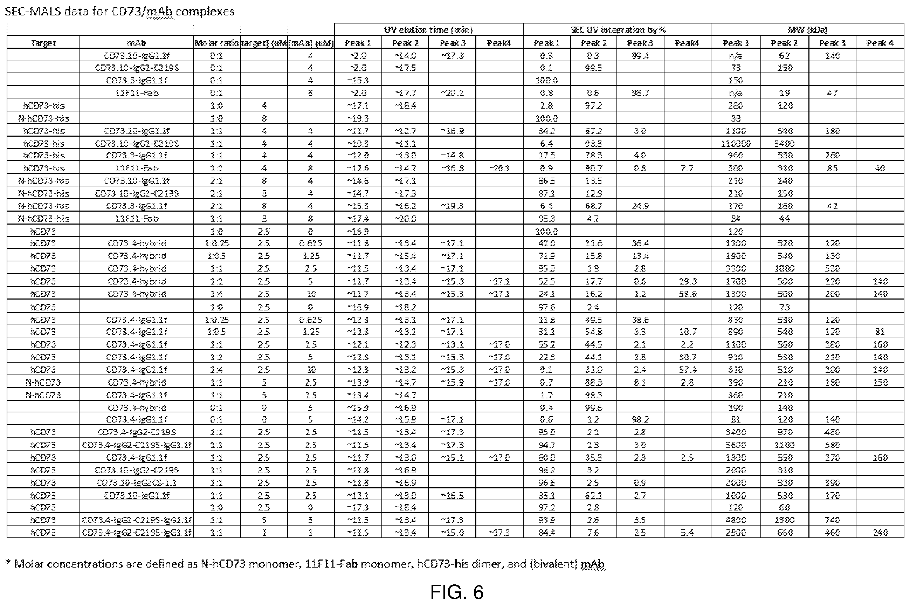

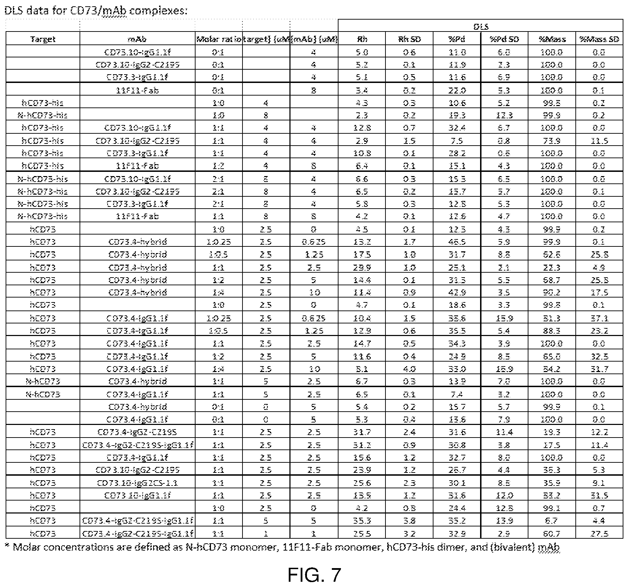

A summary of the SEC-MALS and DLS is provided in Figures 6 and Figure 7. Analysis of the antibodies alone, shows retention times (about 16 - 17 min), masses (140 - 150 kDa), and hydrodynamic radii (5.0 - 5.4 nm) for each antibody that are typical for a monomeric monoclonal antibody. The data for the hCD73-his protein is consistent with the protein adopting the expected dimeric structure in solution; in particular, the mass determined from the SEC-MALS data (120 kDa) is consistent with that expected for a CD73-his dimer (117 kDa) and inconsistent with what would be expected for a hCD73-his monomer (58.5 kDa). The data for N-hCD73 is consistent with the recombinant N-domain protein being monomeric in solution (SEC-MALS measured mass = 38 kDa, compared to expected monomeric mass = 35.0 kDa), which is expected because the region of the full length CD73 extracellular domain that is responsible for dimerization of the protein is contained within the C-terminal domain without contribution of N-domain residues.

Equimolar mixtures of a given antibody with N-hCD73-his were found to elute as a single species in the SEC with shorter retention time than the antibody or N-hCD73-his alone, as well as larger hydrodynamic radii (Rh) by DLS, which is consistent with the formation of complexes. MALS data indicate masses for these complexes of approximately 210 kDa. This is consistent with one N-hCD73-his molecule bound to each of the two Fab domains of a given antibody to form a 1:2 antibody:N-hCD73-his complex.

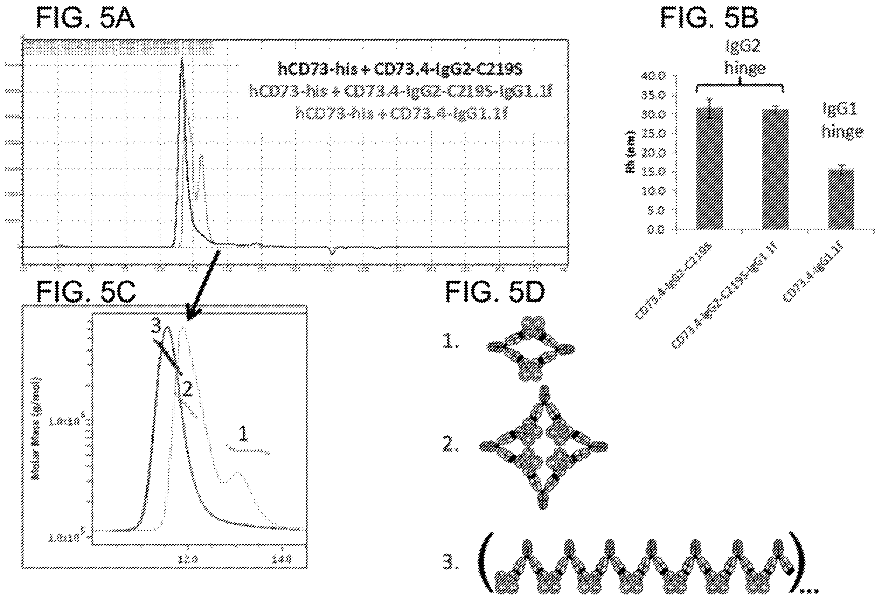

SEC-MALS data for mixtures of anti-CD73 antibodies with hCD73-his dimer shows that the mixture elutes earlier than either the hCD73-his or antibody alone, suggesting that complexes are formed. Comparing the data for mAbs that contain the same variable region but different constant domains, shows that the elution times for the complexes of hCD73-his with mAbs containing a IgG2 constant domains (IgG2-C219S, IgG2-C219S-IgG1.1f) are earlier than those for complexes of hCD73-his with mAbs containing an IgG1.1f constant domain. In addition, the MALS-determined masses for complexes of hCD73-his with mAbs containing an IgG2 constant domain are larger than those for complexes of hCD73-his with mAbs containing an IgG1 constant domain. DLS data further shows that the hydrodynamic radius of complexes of hCD73-his with mAbs containing a IgG2 constant domain are larger than those for complexes of hCD73-his with mAbs containing an IgG1 constant domain. For example, the SEC-MALS and DLS data for CD73.4 with three different constant regions (IgG2-C219S, IgG2-C219S-IgG1.1f, or IgG1.1f) is shown in Figure 5. Here it can be seen that the complex of hCD73-his with CD73.4 containing the IgG2 constant domain have shorter retention times (Figure 5A), larger hydrodynamic radii (Figure 5B) and larger MALS-determined masses (Figure 5C), as compared to the complexes of hCD73-his with CD73.4-IgG1.1f. Based on the MALS masses, a schematic model for the structure and stoichiometry of the complexes between hCD73-his and the antibodies is shown in Figure 5D, where complexes containing CD73.4-IgG1.1f predominantly form smaller 2:2 (peak 1 = ~550 kDa) or 4:4 mAb/CD73 dimer complexes (peak 2 = ~1300 kDa), whereas CD73.4-IgG2-C219S or CD73.4-IgG2-C219S-IgG1.1f form much larger complexes (>3000 kDa) with hCD73-his, for which precise structure and stoichiometry cannot be confidently modeled.

Collectively the SEC-MALS and DLS data demonstrate that larger complexes are formed between hCD73-his and mAbs containing an IgG2 hinge region (IgG2-C219S or IgG2-C219S-IgG1.1f), compared to those containing the IgG1 hinge region (IgG1.1f).

### Example 4: CH1 of IgG2 isotype further improves antibody mediated CD73 internalization

Additional internalization assays were conducted in Calu6 and H292 cells to further discriminate the role of isotype on internalization. The internalization assays were conducted as described in Example 1A and 1B (flow cytometry protocol without the washout step of the antibodies), and the antibodies of varying hybrid isotypes shown in Table 14 were maintained in culture at 10 μg/mL during the incubation time. For the flow cytometry experiments, the method of Example 1B was adapted to high throughput analysis in 96 well plates (as opposed to 48 well plates) and with 50,000 cells per well.

**Table 14:** Constant regions tested with the variable regions of CD73.4:

| Constructs | SEQ ID NO of constant region | Description |
|---|---|---|
| IgG1f | 78 | wild type IgG1f |
| IgG1.1f | 83 | standard inert IgG1.1f |
| IgG2.3 | 79 | IgG2 A-form (C219S) |
| IgG2.5 | 82 | IgG2 B-form (C131S) |
| IgG2.3G1-KH | 81 | CH1, upper hinge and lower hinge/upper CH2 of IgG2.3, all else IgG1f |

(continued)

| Constructs | SEQ ID NO of constant region | Description |
|---|---|---|
| IgG2.5G1-KH | 90 | CH1, upper hinge and lower hinge/upper CH2 of IgG2.5, all else IgG1f |
| IgG2.3G1-AY | 80 | CH1 and upper hinge of IgG2.3, all else IgG1f |
| IgG2.5G1-AY | 89 | CH1 and upper hinge of IgG2.5, all else IgG1f |
| IgG 1-G2.3G1-KH | 93 | CH1 of IgG1, upper hinge and lower hinge/upper CH2 of IgG2.3, all else IgG1f |
| IgG 1-G2.3G1-AY | 92 | CH1 of IgG1, upper hinge of IgG2.3, all else IgG1f |
| IgG2.3G1.1f-KH | 84 | CH1, upper hinge and lower hinge/upper CH2 of IgG2.3, all else IgG1.1f |
| IgG2.5G1.1f-KH | 88 | CH1, upper hinge and lower hinge/upper CH2 of IgG2.5, all else IgG1.1f |
| IgG 1-deltaTHT | 85 | IgG1 with THT sequence removed from hinge |
| IgG2.3-plusTHT | 86 | IgG2.3 with THT sequence (from IgG1) added into hinge |
| IgG2.5-plusTHT | 91 | IgG2.5 with THT sequence (from IgG1) added into hinge |
| IgG2.3-plusGGG | 87 | IgG2.3 with flexible GGG sequence added into hinge |

FcγR binding was shown to be as expected for each construct, i.e., FcγR binding is driven by lower hinge/CH2 region.

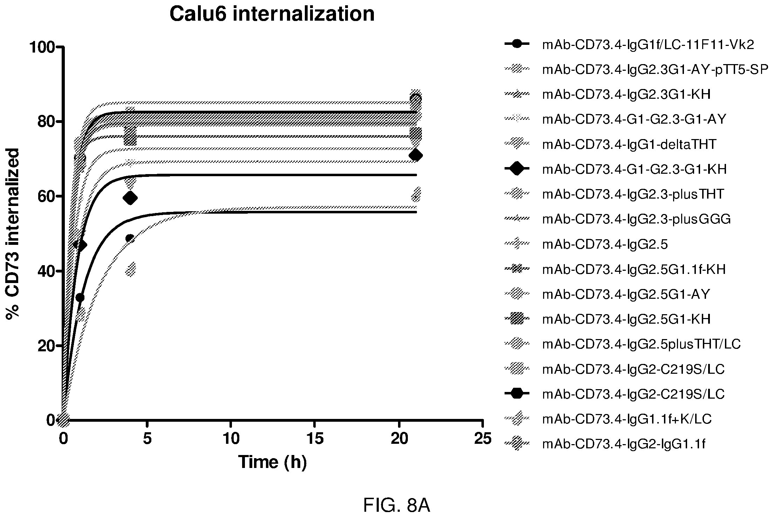

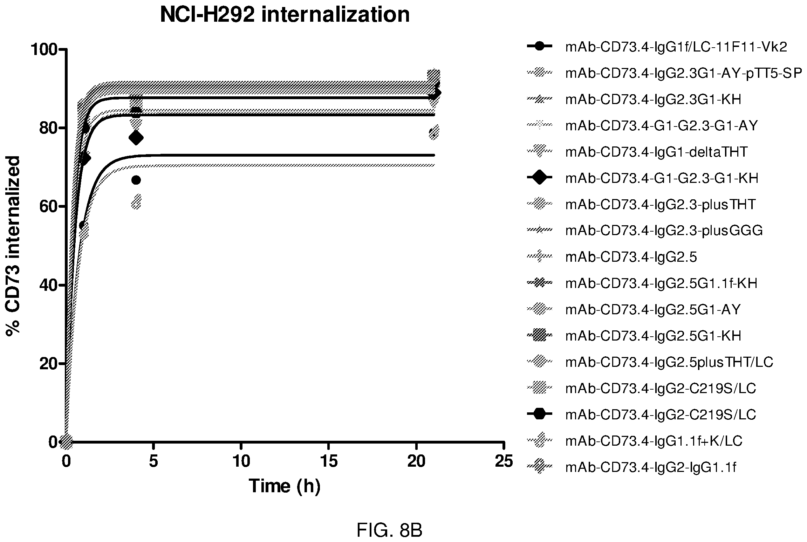

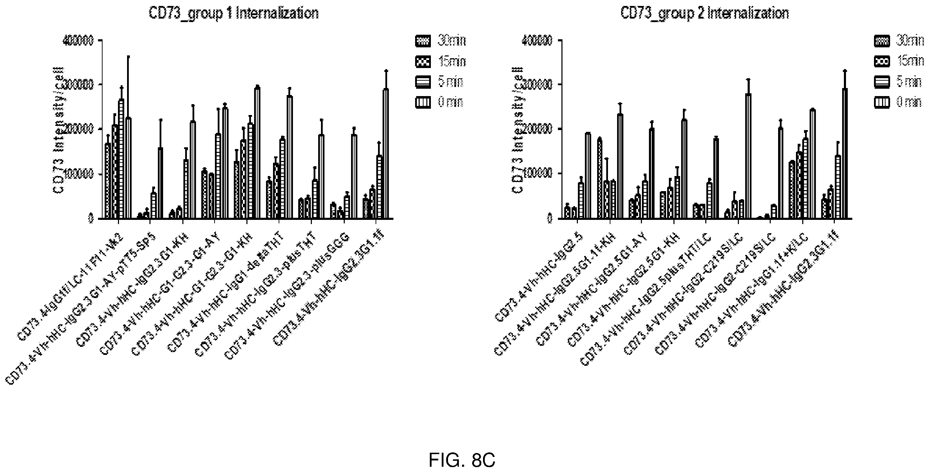

The results are shown in Figures 8A, B and C and in Tables 15 and 16. Data shown in Table 15 were generated using the same protocol described in Example 1B (without washing out the antibodies). Data shown in Table 16 were generated using the same protocol described in Example 1A.

**Table 15:** Ymax and $T_{1/2}$ of antibody mediated CD73 internalization in Calu6 and NCI-292 cells

| | Calu6 | | NCI-H292 | |
|---|---|---|---|---|
| | Ymax (%) | $T_{1/2}$ (hr) | Ymax (%) | $T_{1/2}$ (hr) |
| mAb-CD73.4-IgG1f/LC-11F11-Vk2 | 55.72 | 0.8452 | 73.05 | 0.5014 |
| mAb-CD73.4-IgG2.3G1-AY-pTT5-SP | 85.07 | 0.3326 | 90.25 | 0.272 |
| mAb-CD73.4-IgG2.3G1-KH | 81.62 | 0.3962 | 91.61 | 0.2801 |
| mAb-CD73.4-G1-G2.3-G1-AY | 72.7 | 0.4229 | 84.51 | 0.3083 |
| mAb-CD73.4-IgG1-deltaTHT | 69.27 | 0.5652 | 83.63 | 0.3441 |
| mAb-CD73.4-GI-G2.3-GI-KH | 65.67 | 0.5674 | 83.29 | 0.343 |
| mAb-CD73.4-IgG2.3-plusTHT | 81.19 | 0.3551 | 91.41 | 0.2935 |
| mAb-CD73.4-IgG2.3-plusGGG | 81.72 | 0.3355 | 91.6 | 0.2712 |
| mAb-CD73.4-IgG2.5 | 78.98 | 0.3485 | 89.56 | 0.3057 |
| mAb-CD73.4-IgG2.5G1.1f-KH | 79.63 | 0.3527 | 90.86 | 0.2993 |
| mAb-CD73.4-IgG2.5G1-AY | 81.91 | 0.2901 | 91.3 | 0.2452 |
| mAb-CD73.4-IgG2.5G1-KH | 76 | 0.2837 | 90.75 | 0.256 |
| mAb-CD73.4-IgG2.5plusTHT/LC | 80.15 | 0.2869 | 89.6 | 0.2565 |
| mAb-CD73.4-IgG2-C219S/LC | 82.35 | 0.3725 | 88.91 | 0.2866 |
| mAb-CD73.4-IgG2-C219S/LC | 82.54 | 0.3639 | 87.66 | 0.2845 |

(continued)

|  | Calu6 | | NCI-H292 | |
|---|---|---|---|---|
|  | Ymax (%) | $T_{1/2}$ (hr) | Ymax (%) | $T_{1/2}$ (hr) |
| mAb-CD73.4-IgG1.1f+K/LC | 57.07 | 1.519 | 70.4 | 0.4969 |
| mAb-CD73.4-IgG2CS-IgG1.1f | 80.98 | 0.3508 | 90.35 | 0.2764 |

**Table 16:** Internalization characteristics of CD73.4 with various constant regions in Calu6 cells

| CD73_mAb_Clones | Internalization Max | Speed |
|---|---|---|
| CD73.4-IgG1f/LC-11F11-Vk2 | + | + |
| CD73.4-Vh-hHC-IgG2.3G1-AY-pTT5-SP5 | ++++ | ++++ |
| CD73.4-Vh-hHC-IgG2.3G1-KH | ++++ | +++ |
| CD73.4-Vh-hHC-G1-G2.3-G1-AY | ++ | ++ |
| CD73.4-Vh-hHC-G1-G2.3-G1-KH | ++ | ++ |
| CD73.4-Vh-hHC-IgG1-deltaTHT | ++ | +++ |
| CD73.4-Vh-hHC-IgG2.3-plusTHT | ++++ | ++++ |
| CD73.4-Vh-hHC-IgG2.3-plusGGG | ++++ | ++++ |
| CD73.4-Vh-hHC-IgG2.5 | ++++ | ++++ |
| CD73.4-Vh-hHC-IgG2.5G1.1f-KH | ++ | ++++ |
| CD73.4-Vh-hHC-IgG2.5G1-AY | +++ | ++++ |
| CD73.4-Vh-hHC-IgG2.5G1-KH | +++ | ++++ |
| CD73.4-Vh-hHC-IgG2.5plusTHT/LC | ++++ | ++++ |
| CD73.4-Vh-hHC-IgG2-C219S/LC | ++++ | ++++ |
| CD73.4-Vh-hHC-IgG2-C219S/LC | ++++ | ++++ |
| CD73.4-Vh-hHC-IgG1.1f+K/LC | + | + |
| CD73.4-Vh-hCh-IgG2-C219S-IgG1.1f | ++++ | ++++ |

Figures 8A-C and Tables 15 and 16 indicate that antibodies having a hinge and CH1 domain of the IgG2 isotype are most efficient at driving internalization of CD73, whereas the antibodies that have an IgG1 hinge and CH1 domain correspond to the lower curves in the figure, i.e., lower extent of internalization. In addition, antibodies with only the hinge from IgG2 have an increased internalization compared to a human IgG1 hinge. Thus, antibodies having a hinge and CH1 domain of the IgG2 isotype have superior internalization characteristics relative to the antibodies with an IgG1 isotype.

Thus, anti-CD73 antibody mAb-CD73.4-IgG2CS-IgG1.1f (having an IgG2 hinge with C219S substitution and an IgG2 CH1 domain) induced rapid internalization dependent on cell line tested. The $T_{1/2}$ for internalization ranged from minutes to under an hour. Most cell lines tested had a $T_{1/2}$ under 10 minutes. A nearly complete internalization was induced for some cell lines and most tested had at least a 50% reduction in surface CD73 expression which typically reached maximal levels by 5 hours, much shorter in some cases.

### Example 5: IgG2 CH1 enhances GITR Ab induced IL-2 secretion by CD4+ T cells

This Example shows that a CH1 domain of the IgG2 isotype enhances anti-GITR antibody induced T cell activity, relative to the antibody with a CH1 domain of the IgG 1 isotype.

The same modified heavy chain constant regions that were used in Example 4 were linked to the variable regions of the anti-GITR antibody (of Example 2). Donor CD4+ T cells were incubated with OKT3-scFv expressing CHO cells and the various anti-GITR antibodies, and the level of IL-2 secreted was measured. This was conducted as described in Example 2.

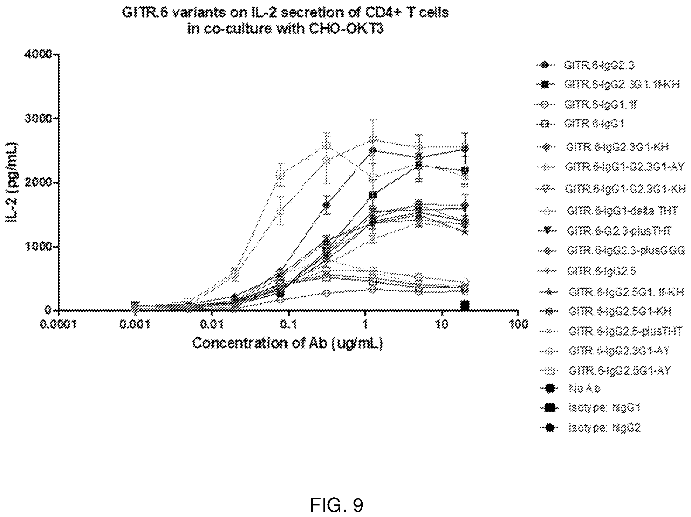

The results, which are shown in Figure 9, indicate that all anti-GITR antibodies having a CH1 domain of the IgG2 isotype, in addition to a hinge of the IgG2 isotype, are more effective at stimulating IL-2 secretion from CD4+ T cells than thos having an IgG1 hinge and CH1.

Thus, this Example shows that the presence of an IgG2 hinge and IgG2 CH1 domain in an agonist anti-GITR antibody further enhances the agonist activity of the antibody relative to the same antibody that does not have a hinge and/or a CH1 domain of the IgG2 isotype. An antibody having both a hinge and a CH1 domain of the IgG2 isotype has a stronger agonist effect relative to an antibody having a hinge, but not CH1, of the IgG2 isotypeAdditionally, an antibody with a CH1 domain from IgG2 has a stronger agonist activity than an antibody with with a CH1 domin from IgG1 isotype. An antibody with a hinge from IgG2 and a CH1 domain from IgG1 has stronger agonist acivity than an antibody with a CH1 and hinge from IgG1 isotype.

***Example 6: Relevance of certain amino acid residues in IgG2 CH1 and hinge in improving antibody mediated CD73 internalization***

Anti-CD73 antibodies (CD73.4) with the heavy chain constant regions shown in Table 17 were prepared and tested as described above in antibody mediated CD73 internalization assays.

**Table 17:** Heavy chain constant regions that were fused to anti-CD73 variable regions

| Description | Constructs | SEO ID NO of constant region |
|---|---|---|
| CH1 domain of IgG2, with all else IgG1. Also, Cys>Ser mutant to reduce potential disulfide heterogeneity; | G2-G1-G1-G1 | 94 |
| | G2.5-G1-G1-G1 | 95 |
| CH1 domain of IgG1 with all else IgG2,3: | G1-G2.3-G2-G2 | 96 |
| Swap CH1 regions in IgG1 with those of IgG2, either separate or together: | G1-KRGEGSSNLP | 97 |
| | G-1-KRGEGS | 98 |
| | G1-SNLF | 99 |
| | IgG1-ITNDRTPR | 100 |
| | G1-SNLFPR | 101 |
| Swap CH1 regions in IgG2 with those of IgG1, either separate or together: | G2-RKEGSGNSFL | 102 |
| | G2-RKEGSG | 103 |
| | G2-NSFL | 104 |
| | IgG2-TIDNTRRP | 105 |
| | G2-NSFLRP | 106 |
| IgG1 with CH2 domain residues of IgG2: | G1-G1-G2-G1-AY | 107 |
| | G1-G1-G2-G1-KH | 108 |
| IgG2 with CH2 domain residues of IgG1: | G2-G2.3-G1-G2-KH | 109 |
| | G2.5-G2.3.G1-G2-KH | 110 |
| | G2-G2.3-G1-G2-AY | 111 |
| | G2.5-G2.3-G1-G2-AY | 112 |

(continued)

| Description | Constructs | SEQ ID NO of constant region |
|---|---|---|
| Swap hinge regions between IgG1 and IgG2: | G1-G2.3-G1-G1-KH | 113 |
| | G2-G1-G2-G2-AY | 114 |
| | G2.5-G1-G2-G2-AY | 115 |
| | G1-G2-G1-G1-AY | 116 |
| | G2-G1-G2-G2-KH | 117 |
| | G2.5-G1-G2-G2-KH | 118 |
| Hinge truncations | IgG1-deltaHinge | 119 |
| | IgG2-deltaHinge | 120 |
| | IgC2,5-deltaHinge | 121 |
| | IgG1.deltaG237 | 122 |
| | IgG2-plusG237 | 123 |
| Other | IgG2.4 | 124 |
| | IgG2.3/4 | 125 |

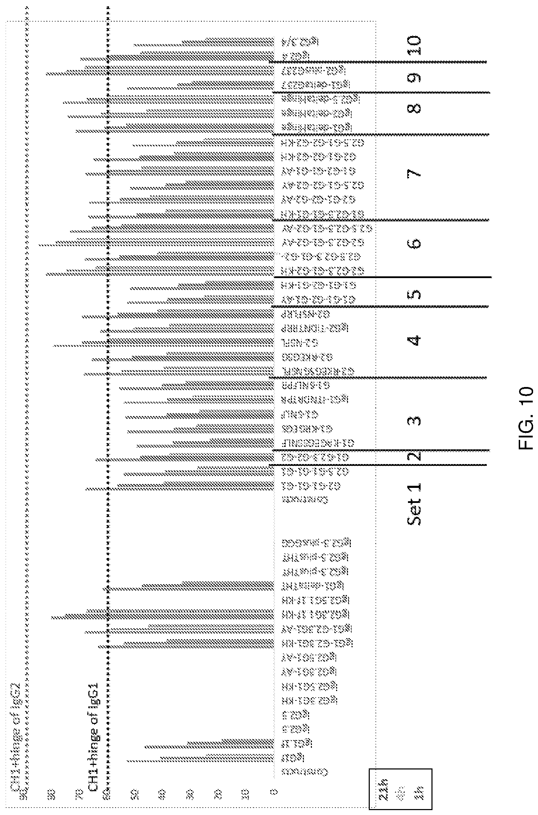

The results, which are shown in Figure 10, provide the following information in the context of CD73 internalization:

• CH2 domain does not appear to have an impact as shown by

   ○ a) very little difference in internalization ability was observed between the antibodies comprising a modified heavy chain constant region with format "AY" (having the IgG2 hinge ERKCCVECPPCPAPPVAG (SEQ ID NO: 8) relative to those with format "KH" (ERKCCVECPPCPAPELLGG (SEQ ID NO: 22) (Set 5, 6 and 7);
   ○ b) CH2 swaps are comparable to wiltype G1 or G2 (Sets 5 and 6); and
   ○ c) residue 237 has no impact on internalization: neither the addition of a "G" residue to an IgG2 hinge nor the deletion of the C terminal "G" in an IgG1 hinge affected internalization (Set 9).
   This suggests that the CH2 domain does not impact internalization (i.e., the CH2 domain can be from IgG1 or IgG2);
• Swapping the CH1 regions indicated in Set 3 (KRGEGSSNLF; KRGEGS; SNLF; ITNDRTPR and SNLFPR) in IgG1 with those of IgG2 provides little benefit, i.e., the internalization remains similar to that of IgG1; see Set 3);
• Swapping the CH1 regions indicated in Set 4 (RKEGSGNSFL; RKEGSG; NSFL; TIDNTRRP and NSFLRP) in IgG2 with those of IgG1 has variable impact: changing NSFL has no impact, whereas the other 2 regions (RKEGSG & RP) are involved (see Set 4). Based on the results of Sets 3 and 4, it appears that there is an interaction between the CH1 region and the hinge, with RKEGSG and RP regions being more important than NSFL region;
• The hinge region impacts internalization, i.e., the hinge of IgG2 provides better internalization relative to the hinge of IgG1 (see Sets 7 and 8). In addition, IgG1 with a deletion (G1-delta-hinge) improves internalization over IgG1. IgG2 with a deletion (G2-delta-hinge) provides a similar level of internalization relative to that of an IgG2 hinge. This suggests that the hinge region impacts internalization, which effect is enhanced by an IgG2 CH1 (G2-G1-G2-G2-AY is comparable to G1-G2-G1-G1-AY);
• IgG2.4 (C220S) has similar or reduced internalization compared to IgG2.3 (C219S). IgG2.3/4 (C219S/C220S) has much reduced internalization compared to IgG2.3 or IgG2.4 alone (see Set 10). This suggests that internalization of an antibody with an IgG2 hinge and C219S is about the same as that of an IgG2 hinge with C220S, both of which are much better than that of an IgG2 hinge with both C219S and C220S;
• IgG2.5 (C131S mutation) has reduced internalization compared to constructs with C131 (see Sets 1,6 and 7).

Thus, these results indicate that the CH1 domain and the hinge are both relevant in the antibody mediated CD73 internalization, and that an antibody having the IgG2 sequences from these domains is internalized with better efficacy relative to an antibody having these regions from IgG1.

### Example 7: Antibodies having an IgG2 hinge and/or CH1 domain form high molecular weight complexes

CD73.4 antibodies having the heavy chain constant regions set forth in Table 14 were also tested for formation of high molecular weight complexes by SEC-MALS and DLS experiments, as described in Example 3.

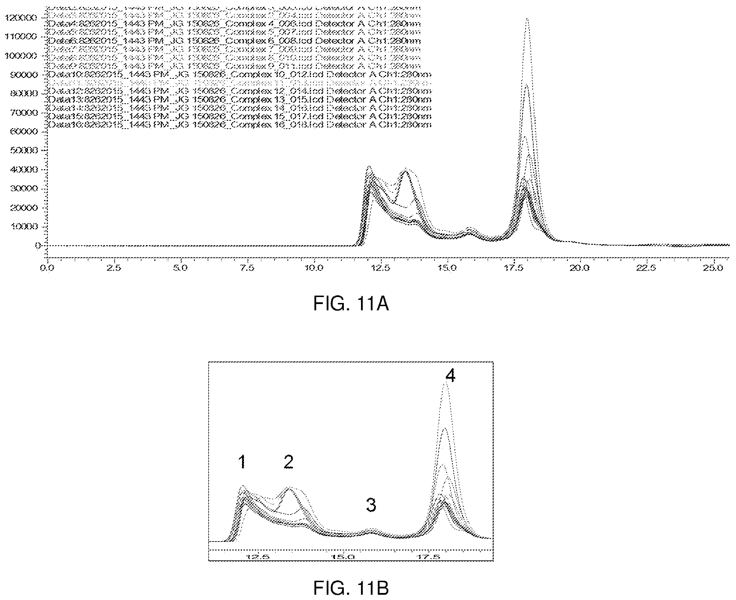

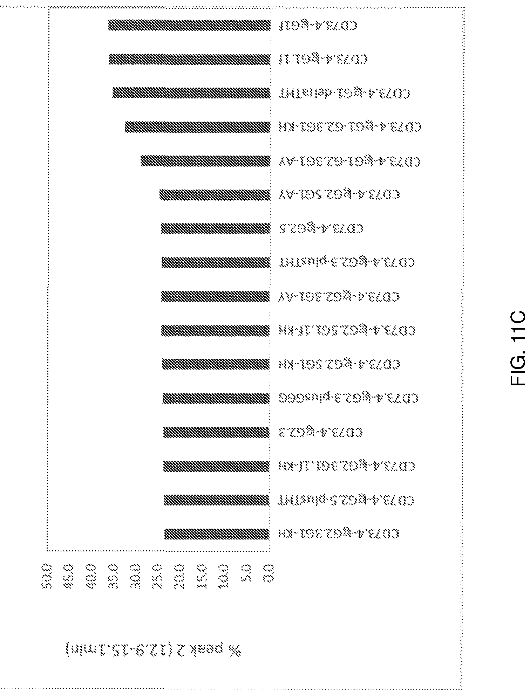

Three out of the 16 antibodies in this study were were previously tested: CD73.4-IgG1.1f, CD73.4-IgG2-C219S (also called CD73.4-IgG2.3), and CD73.4-IgG2-C219S-IgG1.1f (also called CD73.4-IgG2.3G1.1f-KH). SEC-MALS and DLS data of the antibodies alone showed retention times, masses, and hydrodynamic radii for each antibody that are typical for a monomeric monoclonal antibody. Equimolar complexes of each antibody (5.5 uM) with hCD73-his (5.5uM) showed slower retention times for all complexes as compared to antibody or hCD73-his alone indicating the formation of complexes. An overlay of the SEC chromatogram data for each of the 16 complexes is shown in Figure 11A. The chromatogram data can be divided into 4 distinct peaks, which are shown in Figure 11B. Peak 1 contains the largest species, with MALS-determined masses suggesting complexes with mass equivalent of greater than 4:4 hCD73-his:mAb complexes. Peak 2 contains species with MALS-determined masses suggesting complexes of about 2:2 hCD73-his:mAb complexes. Peak 3 is a minor species with low signal and MALS-determined masses suggesting about 1:1 hCD73-his:mAb complexes. Peak 4 corresponds to the elution of the mAbs alone with MALS-determined masses consistent with free antibody. To quantitate the relative amounts of each species, the 4 peaks of each chromatogram were integrated as peak 1 (<12.9 min), peak 2 (12.9 - 15.1 min), peak 3 (15.1 - 16.7 min), peak 4 (16.7 - 19.3 min). The integration also included an additional integrated range called peak 5 (>19.3 min) to account for any low molecular weight species, which were found to be negligible (<3.5% for all complexes). The percentage of each species from this integration is summarized in Table 18. All complexes contained a similar small percentage of peak 3 (about 6-9%), but variable amounts of the other peaks. Most notable is that all complexes between hCD73-his and antibodies containing a CH1 domain from hIgG1 had a significantly greater percentage of smaller complexes (peak 2), whereas those containing CH1 domain from hIgG2 had a greater percentage of larger complexes (peak 1) (Table 18 and Figure 11C). This suggests an important role for not only the hinge region but also the CH1 domain in higher order complex formation.

**Table 18:** Retention times of CD73.4 antibodies with modified heavy chain constant regions

| | UV% | | | | |
|---|---|---|---|---|---|
| | Peak1 | Peak2 | Peak3 | Peak4 | Peak5 |
| Complexes | <12.9 min | 12.9-15.1min | 15.1-16.7min | 16.7-19.3min | >19.3min |
| CD73.4-IgG2.3 + hCD73-his | 37.0 | 23.8 | 7.7 | 28.6 | 2.9 |
| CD73.4-IgG2.3G1.1f-KH + hCD73-his | 36.0 | 23.8 | 7.9 | 29.3 | 3.0 |
| CD73.4-IgG1.1f+ hCD73-his | 28.4 | 36.2 | 7.4 | 25.6 | 2.3 |
| CD73.4-IgG1f + hCD73-his | 26.0 | 36.5 | 7.5 | 27.8 | 2.2 |
| CD73.4-IgG2.3G1-AY + hCD73-his | 30.2 | 24.3 | 8.1 | 34.4 | 3.0 |
| CD73.4-IgG2.3G1-KH + hCD73-his | 34.9 | 23.4 | 7.9 | 30.7 | 3.0 |
| CD734-IgG1-G2.3G1-AY + hCD73-his | 14.6 | 29.2 | 6.4 | 48.3 | 1.6 |
| CD734-IgG1-G2.3G1-KH + hCD73-his | 23.8 | 32.6 | 7.0 | 34.5 | 2.1 |
| CD73.4-IgG1-deltaTHT + hCD73-his | 28.3 | 35.4 | 7.0 | 26.9 | 2.4 |
| CD73.4-IgG2.3-plusTHT + hCD73-his | 30.6 | 24.3 | 8.3 | 33.7 | 3.2 |
| CD73.4-IgG2.3-plusGGG + hCD73-his | 30.0 | 23.9 | 8.2 | 34.9 | 2.9 |
| CD73.4-IgG2.5 + hCD73-his | 31.7 | 24.4 | 8.4 | 32.5 | 3.1 |
| CD73.4-IgG2.5G1.1f-KH + hCD73-his | 30.7 | 24.3 | 8.9 | 32.7 | 3.4 |
| CD73.4-IgG2.5G1-AY + hCD73-his | 26.3 | 24.8 | 8.1 | 38.3 | 2.6 |
| CD73.4-IgG2.5G1-KH + hCD73-his | 21.4 | 24.1 | 7.0 | 45.6 | 1.9 |
| CD73.4-IgG2.5-plusTHT + hCD73-his | 32.6 | 23.5 | 8.3 | 32.6 | 3.0 |

**Example 8: Fc Receptor binding for antibodies with engineered constant domains**

This Example demonstrates that antibodies having modified heavy chain constant regions comprising the CH1 and hinge of IgG2 bind to FcγRs when they contain CH2 and CH3 domains of IgG1.

In addition to antigen binding by the variable domains, antibodies can engage Fc-gamma receptors (FcgRs) through interaction with the constant domains. These interactions mediate effector functions such as antibody-dependent cellular cytotoxicity (ADCC) and antibody-dependent cellular phagocytosis (ADCP). Effector function activity is high for the IgG1 isotype, but very low or absent for IgG2 and IgG4 due to these isotypes having lower affinity for FcgRs. In addition, the effector function of IgG1 can be modified through mutation of amino acid residues within the constant regions to alter FcgR affinity and selectivity.

The binding of antibodies to Fc gamma receptors (FcγRs or FcgRs) was studied using biosensor technologies including Biacore surface plasmon resonance (SPR) and Fortebio Biolayer Interferometry (BLI). SPR studies were performed on a Biacore T100 instrument (GE Healthcare) at 25°C. The Fab fragment from a murine anti-6xHis antibody was immobilized on a CM5 sensor chip using EDC/NHS to a density of -3000 RU. Various his-tagged FcgRs (7 ug/ml) were captured via the C-terminal his-tag using a contact time of 30 s at 10 ul/min, and the binding of 1.0 uM antibody was evaluated in a running buffer of 10 mM NaPO4, 130 mM NaCl, 0.05% p20 (PBS-T) pH 7.1. FcgRs used for these experiments included CD64 (FcgRI), CD32a-H131 (FcgRIIa-H131), CD32a-R131 (FcgRIIa-R131), CD32b (FcgRIIb), CD16a-V158 (FcgRIIIa-V158), CD16b-NA1 (FcgRIIIb-NA1), and CD16B-NA2 (FcgRIIIb-NA2). BLI experiments were performed on a Fortebio Octet RED instrument (Pall, Fortebio) at 25oC in 10 mM NaPO4, 130 mM NaCl, 0.05% p20 (PBS-T) pH 7.1. Antibodies were captured out of undiluted expression supernatants on protein A coated sensors, followed by the binding of 1uM hCD32a-H131, hCD32a-R131, hCD32b, hCD16a-V158, or 0.1 uM hCD64 analytes.

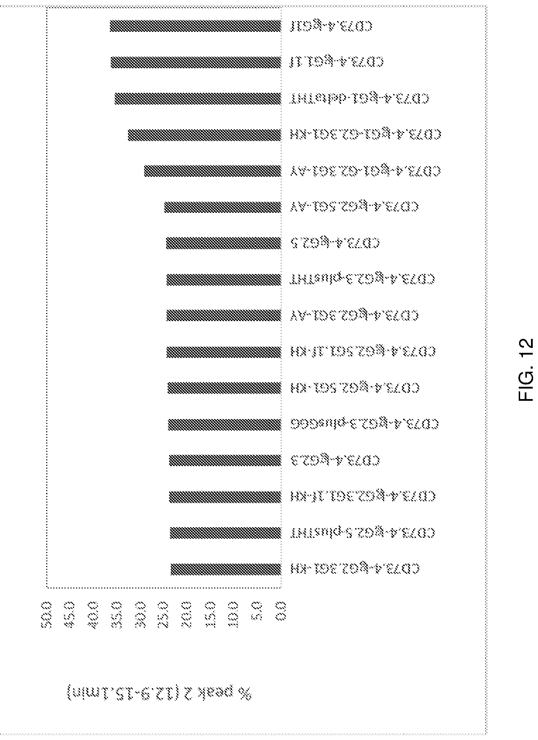

First, antibodies binding to various targets were made that contain modified IgG1 Fc domains including the substitutions S267E (SE) and S267E/L328F (SELF), as well as various combinations of the mutations P238D, P271G, H268D, A330R, G237D, E233D, referred to as V4, V7, V8, V9 and V12. The binding of these antibodies was studied by Biacore SPR with comparison to IgG1f, IgG2.3 (IgG2-C219S) and IgG4.1 (IgG4-S228P) antibodies, as well as an IgG1.1f antibody which has been engineered to reduce binding to all FcgRs. The results, which are shown in Figure 12, demonstrate the expected FcgR binding properties for IgG1f, IgG2.3 and IgG4.1 and the mutated IgG1 antibodies, including increased CD32aH131, CD32a-R131 and CD32b binding for SE and SELF, as well as increased selectivity of the V4, V7, V8, V9 and V12 mutants for CD32b over CD32a-H131 and CD32a-R131, Figure 12.

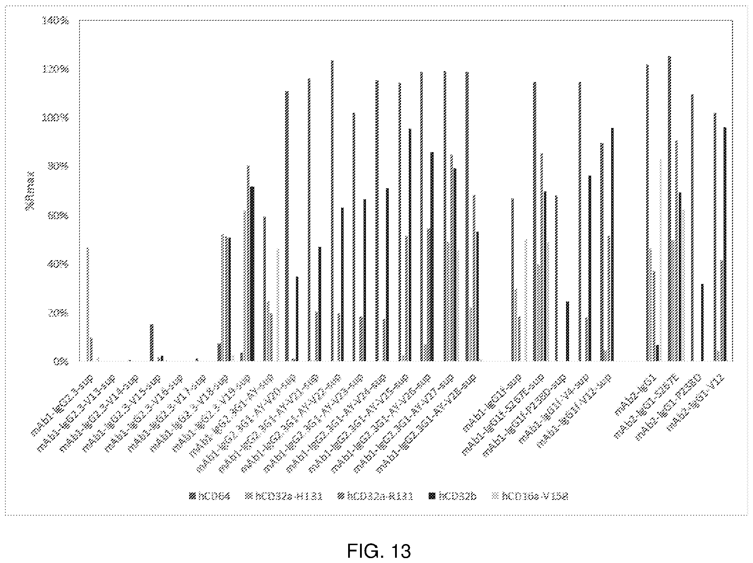

The next set of constructs were used to engineer effector function into the otherwise effector function negative IgG2 isotype. For this study, the mutations described above were introduced in the context of IgG2.3 constant region, or an IgG2.3/IgG1f hybrid termed IgG2.3G1-AY, Table 19. Antibodies were expressed at small scale as supernatants, and tested for binding to FcgRs using Fortebio Octet BioLayer Interferometry biosensor technology. Since the antibodies were present at low concentration in the supernatants, the experiment was performed by capturing antibodies out of the supernatants using protein A coated sensors, followed by binding of FcgR analytes in solution. Purified and supernatant control IgG1f including wild type IgG1, SE, P238D, V4 and V12 antibodies were also included for comparison, and each of these control antibodies demonstrated expected FcgR binding properties, Figure 13. The IgG2.3 antibody also demonstrated the expected binding profile, with appreciable binding to only CD32a-H131. However, all mutations to introduce S267E, L328F, P238D, P271G, H268D, A330R, G237D, or E233D mutations into IgG2.3 failed to recapitulate the FcgR affinity of the corresponding engineered IgG1 mAbs, Figure 13. In contrast, the IgG2.3G1-AY construct was able to fully preserve the FcgR binding properties of wild type IgG1, while retaining the CH1 and hinge regions of IgG2.3. In addition, all IgG2.3G1-AY mutants containing S267E, L328F, P238D, P271G, H268D, A330R, G237D, and E233D demonstrated FcgR binding properties comparable to the IgG1 version mAbs containing the same mutations, Figure 13. This demonstrates the successful engineering of antibodies with CH1 and hinge regions of IgG2 combined with effector function of wild type or mutant IgG1.

Table 19: Engineered IgG2 constructs

| Set | ID | Construct | Seq ID# |
|---|---|---|---|
| 1 | IgG2.3 | hHC-IgG2-C219S | |
| | IgG2.3-V13 | hHC-IgG2-C219S - P238D | |
| | IgG2.3-V14 | hHC-IgG2-C219S - P238D,P271G | |
| | IgG2.3-V15 | hHC-IgG2-C219S - P238D,H268D,P271G | |
| | IgG2.3-V16 | hHC-IgG2-C219S - P238D,P271G,A330R | |
| | IgG2.3-V17 | hHC-IgG2-C219S - P238D,H268D,P271G,A330R | |
| | IgG2.3-V18 | hHC-IgG2-C219S-S267E | |
| | IgG2.3-V19 | hHC-IgG2-C219S -S267E,L328F | |
| 2 | IgG2.3G1 | hHC-IgG2-C219S/hHC-IgG1f | |
| | IgG2.3G1-AY-V20 | hHC-IgG2-C219S/hHC-IgG1f - P238D | |
| | IgG2.3G1-AY-V21 | hHC-IgG2-C219S/hHC-IgG1f - P238D,P271G | |
| | IgG2.3G1-AY-V22 | hHC-IgG2-C219S/hHC-IgG1f-P238D,H268D,P271G | |
| | IgG2.3G1-AY-V23 | hHC-IgG2-C219S/hHC-IgG1f-P238D,P271G,A330R | |
| | IgG2.3G1-AY-V24 | hHC-IgG2-C219S/hHC-IgG1f-P238D,H268D,P271G,A330R | |
| | IgG2.3G1-AY-V25 | hHC-IgG2-C219S/hHC-IgG1f-G237D,P238D,H268D,P271G,A330R | |
| | IgG2.3G1-AY-V26 | hHC-IgG2-C219S/hHC-IgG1f-E233D,G237D,P238D,H268D, P271G, A330R | |
| | IgG2.3G1-AY-V27 | hHC-IgG2-C219S/hHC-IgG1f-S267E | |
| | IgG2.3G1-AY-V28 | hHC-IgG2-C219S/hHC-IgG1f-S267E,L328F | |

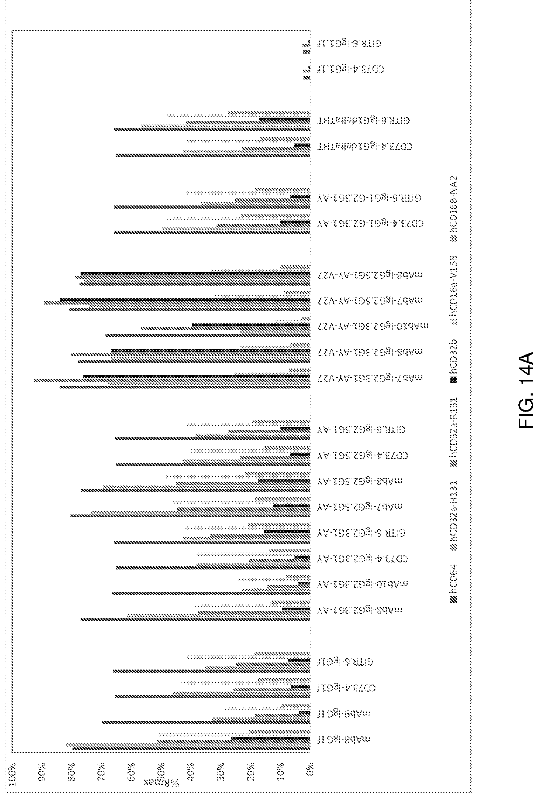

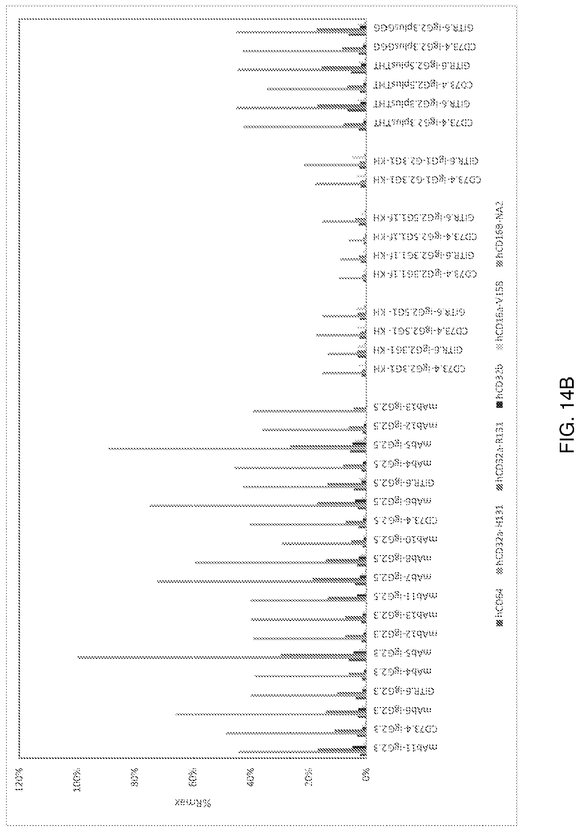

This engineering strategy was further explored by producing other antibodies formatted with IgG2.3G1-AY, IgG2.3G1-AY-S267E (IgG2.3G1-AY-V27), as well as IgG2-B-form variants (IgG2.5G1-AY and IgG2.5G1-AY-V27), and other hybrid antibodies containing different combinations of IgG1 and IgG2 constant domains, and testing the binding of these antibodies to anti-his Fab captured his-tagged FcgRs using Biacore SPR technology. In agreement with the Octet supernatant data, the SPR data showed that the IgG2.3G1-AY and IgG2.3G1-AY-V27 antibodies had comparable FcgR binding properties to IgG1f and IgG1f-S267E respectively, despite containing the CH1 and hinge regions of an A-form IgG2 antibody (IgG2.3) (Figure 14A and B and Table 20). Similar data was also obtained using IgG2.5G1-AY and IgG2.5G1-AY-V27 antibodies, demonstrating the successful engineering of B-form IgG2 antibodies (containing C131S mutation termed IgG2.5) having IgG1f or modified IgG1f like effector functions. Data for several other antibodies with IgG2.3G1-AY, IgG2.3G1-AY-V27, IgG2.5G1-AY, or IgG2.5G1-AY-V27 constant regions but different variable regions shows that this engineering strategy is broadly applicable to other antibodies independent of the variable domains (Figure 14A and B and Table 20). Other constructs that demonstrate IgG1f-like FcgR binding properties are IgG1-G2.3G1-AY, and IgG1deltaTHT, whereas several of the modified constant region constructs were unable to retain IgG1f-like FcgR binding properties, including IgG2.3G1-KH, IgG2.5G1-KH, IgG2.3plusTHT, IgG2.5plusTHT and IgG2.3plusGGG constructs, (Figure 14A and B and Table 20).

**Table 20:** %Rmax values for 1 uM antibodies binding to anti-his Fab captured FcgR-his proteins

| mAb | hCD64 | hCD32aH131 | hCD32a-R131 | hCD32b | hCD16a-V158 | hCD16B-NA2 |
|---|---|---|---|---|---|---|
| mAb8-IgG1f | 80% | 82% | 51% | 27% | 51% | 21% |
| mAb9-IgG1f | 70% | 33% | 19% | 4% | 28% | 10% |
| CD73.4-IgG1f | 65% | 46% | 26% | 6% | 43% | 17% |
| GITR.6-IgG1f | 66% | 35% | 25% | 8% | 41% | 19% |
| | | | | | | |
| CD73.4-IgG1.1f | 2% | 0% | 2% | 1% | 0% | 0% |
| GITR.6-IgG1.1f | 2% | 0% | 3% | 1% | 0% | 0% |
| | | | | | | |
| mAb11-IgG2.3 | 2% | 44% | 17% | 5% | 1% | 0% |
| CD73.4-IgG2.3 | 3% | 48% | 11% | 1% | 1% | 0% |
| mAb6-IgG2.3 | 3% | 66% | 14% | 3% | 1% | 0% |
| GITR.6-IgG2.3 | 4% | 40% | 10% | 1% | 2% | 0% |
| mAb4-IgG2.3 | 1% | 39% | 6% | 1% | 1% | 0% |
| mAb5-IgG2.3 | 6% | 100% | 30% | 4% | 3% | 0% |
| mAb12-IgG2.3 | 2% | 39% | 7% | 1% | 1% | 0% |
| mAb13-IgG2.3 | 2% | 40% | 7% | 1% | 1% | 0% |
| | | | | | | |
| mAb11-IgG2.5 | 0% | 40% | 13% | 3% | 0% | -1% |
| mAb7-IgG2.5 | 4% | 72% | 19% | 2% | 2% | 0% |
| mAb8-IgG2.5 | 3% | 59% | 14% | 3% | 2% | 0% |
| mAb10-IgG2.5 | 1% | 29% | 5% | 1% | 1% | 0% |
| CD73.4-IgG2.5 | 3% | 40% | 7% | 1% | 1% | 0% |
| mAb6-IgG2.5 | 3% | 75% | 17% | 4% | 2% | 0% |
| GITR.6-IgG2.5 | 4% | 43% | 13% | 2% | 2% | 1% |
| mAb4-IgG2.5 | 2% | 46% | 8% | 1% | 1% | 0% |
| mAb5-IgG2.5 | 6% | 89% | 26% | 5% | 4% | 1% |
| mAb12-IgG2.5 | 1% | 36% | 6% | 1% | 1% | 0% |
| mAb13-IgG2.5 | -2% | 39% | 4% | -2% | 0% | -2% |
| | | | | | | |
| mAb8-IgG2.3G1-AY | 77% | 61% | 38% | 10% | 38% | 13% |
| mAb10-IgG2.3G1-AY | 67% | 23% | 14% | 4% | 24% | 8% |
| CD73.4-IgG2.3G1-AY | 65% | 38% | 20% | 5% | 38% | 14% |
| GITR.6-IgG2.3G1-AY | 66% | 43% | 33% | 16% | 42% | 21% |
| | | | | | | |
| mAb7-IgG2.5G1-AY | 80% | 73% | 45% | 12% | 47% | 19% |
| mAb8-IgG2.5G1-AY | 77% | 70% | 45% | 17% | 48% | 22% |
| CD73.4-IgG2.5G1-AY | 65% | 43% | 24% | 7% | 40% | 16% |
| GITR.6-IgG2.5G1-AY | 65% | 38% | 27% | 10% | 41% | 19% |

(continued)

| mAb | hCD64 | hCD32aH131 | hCD32a-R131 | hCD32b | hCD16a-V158 | hCD16B-NA2 |
|---|---|---|---|---|---|---|
| CD73.4-IgG2.3G1-KH | 2% | 15% | 2% | 0% | 2% | 0% |
| GITR.6-IgG2.3G1-KH | 3% | 13% | 3% | 0% | 3% | 1% |
| CD73.4-IgG2.5G1-KH | 2% | 17% | 2% | 0% | 3% | 0% |
| GITR.6-IgG2.5G1-KH | 2% | 15% | 3% | 0% | 3% | 1% |
| CD73.4-IgG2.3G1.1f-KH | 1% | 10% | 1% | 0% | 1% | 0% |
| GITR.6-IgG2.3G1.1f-KH | 2% | 9% | 2% | 0% | 1% | 0% |
| CD73.4-IgG2.5G1.1f-KH | 1% | 6% | 1% | 0% | 1% | 0% |
| GITR.6-IgG2.5G1.1f-KH | 3% | 15% | 4% | 0% | 2% | 0% |
| mAb7-IgG2.3G1-AY-V27 | 84% | 68% | 92% | 76% | 26% | 7% |
| mAb8-IgG2.3G1-AY-V27 | 78% | 67% | 80% | 67% | 24% | 7% |
| mAb10-IgG2.3G1-AY-V27 | 69% | 24% | 57% | 40% | 12% | 3% |
| mAb7-IgG2.5G1-AY-V27 | 81% | 74% | 89% | 84% | 32% | 9% |
| mAb8-IgG2.5G1-AY-V27 | 77% | 76% | 79% | 77% | 33% | 10% |
| CD73.4-IgG1-G2.3G1-AY | 66% | 50% | 31% | 10% | 48% | 23% |
| GITR.6-IgG1-G2.3G1-AY | 66% | 36% | 25% | 7% | 42% | 19% |
| CD73.4-IgG1-G2.3G1-KH | 2% | 18% | 2% | 0% | 4% | 1% |
| GITR.6-IgG1-G2.3G1-KH | 2% | 21% | 2% | 0% | 5% | 1% |
| CD73.4-IgG1deltaTHT | 65% | 43% | 23% | 6% | 42% | 17% |
| GITR.6-IgG1deltaTHT | 66% | 57% | 42% | 17% | 48% | 27% |
| CD73.4-IgG2.3plusTHT | 3% | 42% | 8% | 1% | 1% | 0% |
| GITR.6-IgG2.3plusTHT | 6% | 45% | 17% | 2% | 3% | 1% |
| CD73.4-IgG2.5plusTHT | 2% | 34% | 7% | 1% | 1% | 0% |
| GITR.6-IgG2.5plusTHT | 5% | 44% | 15% | 2% | 3% | 1% |
| CD73.4-IgG2.3plusGGG | 3% | 43% | 8% | 1% | 1% | 0% |
| GITR.6-IgG2.3plusGGG | 6% | 45% | 17% | 2% | 3% | 1% |

Taken together these data show that the sequence immediately C-terminal to the conserved CPPCPAP motif in the hinge region confers FcgR-mediated effector function, whereas the CH1 and upper portions of the hinge of the antibody can be replaced with IgG2 or modified IgG2 sequences, to potentially combine the effector functions of IgG1 and modified IgG1 with the superior internalization or signaling properties of antibodies containing IgG2 CH1 and/or hinge regions.

### Example 9: GITR agonist Ab internalization is enhanced in antibodies having an IgG2 hinge and CH1 domain

To induce GITR expression, cells were incubated for 72h at 37°C with 20ng/ml anti-CD3 + 1000ng/ml CD28. As an alternate method of T-cell activation, large batches of activated CD4+ T-cells were prepared by a three stage culture protocol. Briefly, CD4+ T-cells were stimulated with plate bound CD3 (1.5ug/ml) supplemented with 1ug/ml soluble CD28 for 72h at 37°C, expanded in culture for 14 days in the presence of 20 u/ml IL2 and finally exposed to another round of activation by addition of 10ug/ml PHA, 2u/ml IL2 and 1ug/ml CD28 for 72h at 37°C. Stimulated T cells were seeded into 384 well PDL imaging plates for 2h to adhere the cells, cooled for 15min at 4°C, and then alexa 488 labeled GITR antibodies were added separately for 1h. Plates were finally imaged by HCS and the data were reported as total intensity per cell.

Three different GITR antibodies have been evaluated using the above mentioned T cell activation methods. They are GITR.6 antibody as a G1 isotype and an inert (IgG1.1) isotype unable to bind to Fc receptors, as well as a chimera with the IgG2 hinge in place of the IgG 1 hinge.

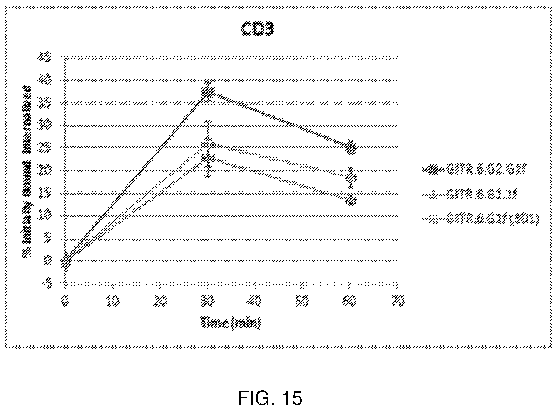

GITR antibody induced internalization was assessed in CD3 stimulated CD4+ T-cells using the alexa quench assay format. Freshly obtained CD4 positive T cells were incubated under as described above to induce GITR expression. After stimulation, cells were resuspended into fresh media and plated for internalization assays as follows. Cells were incubated with antibody as described above, washed with warm media and incubated at 37°C for the indicated times prior to fixation and quenching. Internalized antibody was measured as increased fluorescence above the small unquenchable signal observed at time zero and then normalized against the total fluorescence "unquenched control" initially bound to the cells. As shown in Figure 15, GITR ligation resulted in rapid internalization peaking between 30-60 minutes for each antibody tested while control antibodies were found to maintain localization to the plasma membrane. The results indicate that the IgG2 hinge region enhances GITR ligation induced internalization.

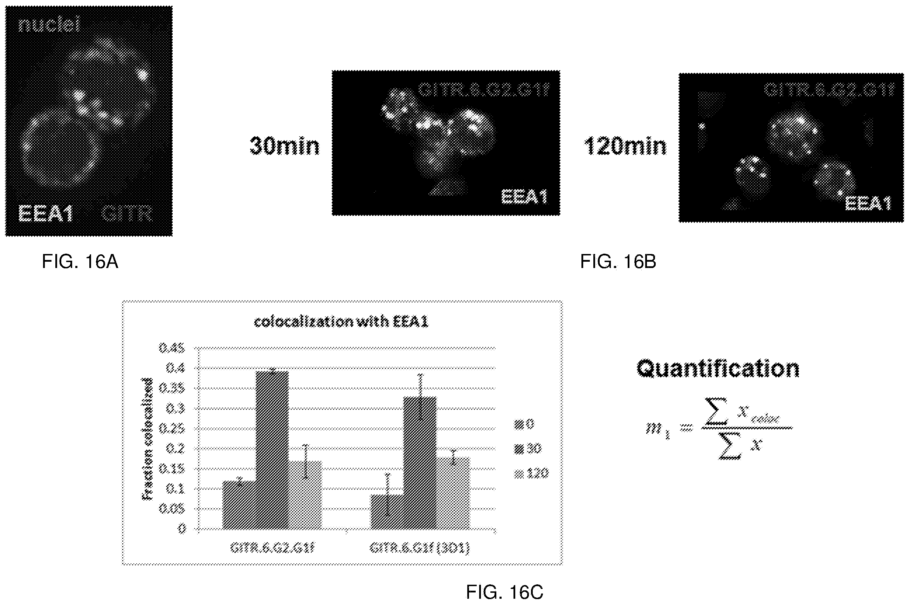

To further dissect the detailed mechanisms of internalization and associated dynamics, antibody endocytosis and delivery into early endosome compartments was analyzed. In this experiment, cells were subjected to pulse chase analysis with unlabeled antibodies. Upon fixation, cells were permeabilized and stained for the early endosome marker EEA1 (cell signaling technology), washed and then detected with alexa fluor - 488 conjugated anti-rabbit secondary antibody (EEA1) and alexa fluor - 647 conjugated anti-human antibody (GITR). Plates were imaged on an Opera confocal system with a 60X water immersion objective. The results indicated clear segregation between the membrane bound anti-GITR antibody staining and intracellular EEA1 signal. Upon warming the cultures, clustering for some antibodies was detected that appears to co-localize with endosomal proteins. Quantification of endosomal co-localization was performed using HCS Studio Software and the results are plotted as the ratio of colocalized pixel intensity relative to total staining (Figure 16). The colocalization of GITR antibody and early endosome is most prominent at 30 minutes. At this tested time point, GITR.6.G2.G1f showed a higher fraction colocalized than the GITR.6.G1f antibody. The colocalization results correlate with the observations made using the alexa quenching method described above and support a model suggesting the G2 hinge has potential advantage over G1 for inducing GITR internalization.

### Example 10: GITR agonist Ab signaling in T cell receptor activated CD4+ and CD8+ T cells is enhanced in antibodies having an IgG2 hinge and CH1 domain

To further investigate the mechanisms for anti-GITR agonist antibodies, several signaling pathways involved in T cell activation, such as NFkB and P38 signaling pathways, were monitored.

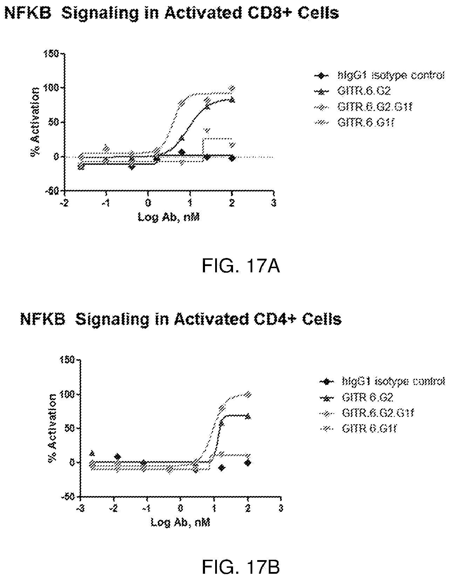

CD4+ and **CD8+** T cells from a healthy donor (M6576) were activated with plate-coated 0.4 μg/ml anti-CD3 and 0.4□g/ml anti-CD28. After 3 days, cells were collected and plated onto 384-well image plates for signaling activation. After cells settled in the plate for 2 hours, they were treated with GITR antibodies for 15 minutes and the signaling events were terminated by adding formaldehyde to a final of 10% into the assays plate. Then the cells were permeabilized and stained with phosphor-p65 NFKB antibody for signaling detection. As shown in Figure 17, GITR.6.G2 and GITR.6.G2.G1f antibodies had higher signaling responses compared to the GITR.6.G1f in both CD4+ and **CD8+** T cells. Although there is no direct evidence of linking internalization and signaling pathway activation, it is intriguing to note that G2 isotype seems to improve both aspects of antibody functional activities compared to the IgG1 for GITR.6.

To quantify the signaling activities for each antibody, both EC50 and Emax for each antibody were calculated, since both parameters are critical to capture the full extent of the signaling event. The response level of GITR.6.G2.G1f is chosen to be the 100% control, and all other antibodies were normalized against it. As shown in Table 21 for both CD4+ and **CD8+** T cell populations activated by anti-CD3 and anti-CD28 antibodies, there were a range of activities for GITR antibodies in terms of both potency (EC50s) and efficacy (Emax%). Although GITR.6.G2, GITR.6.G2.G1f and GITR.6.G1f showed similar potencies (EC50s) around 10nM range, the efficacy (Emax) was quite different for different isotypes, suggesting G1 antibody does not signal as effectively as the G2 or chimeric isotypes.

**Table 21.** Summary of the GITR HuMab NFKB Signaling activities in TCR Activated CD4+ and CD8+ T Cells

|  | *CD4+* T cells | | *CD8+* T cells | |
|---|---|---|---|---|
| Antibody | EC50 (nM) | Emax (%) | EC50 (nM) | Emax (%) |
| GITR.6.G2 | 12.8 | 69 | 9.00 | 85 |
| GITR.6.G2.Gif | 9.00 | 100 | 3.77 | 92 |
| GITR.6.GIf | 7.3 | 10.8 | 20.05 | 27 |
| hIgG1 Isotype Control | Inactive | 4 | Inactive | 6 |

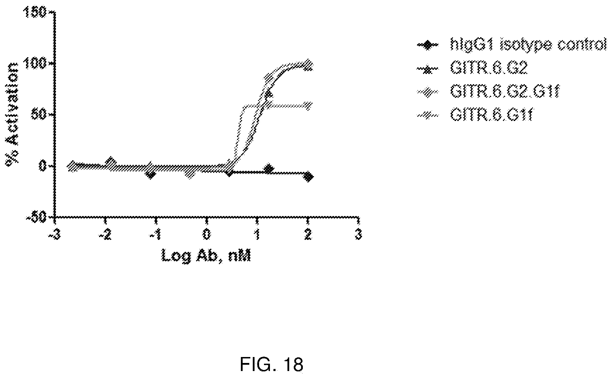

To further confirm if the signaling difference of GITR.6.G2 and GITR.6.G2.G1f compared to GITR.6.G1f is limited to NFkB signaling only or if it holds true for other signaling events as well, a P38MAPK signaling readout was explored. As shown in Figure 18, GITR.6.G2 and GITR.6.G2.G1f antibodies had higher signaling responses compared to the GITR.6.G1f antibody in a CD4+ cell p38 MAPK activation assay. Therefore the better signaling activities for GITR.6 G2 isotype compared with G1 isotype is not only limited to NFkB signaling.

In addition to enhanced agonist activity and internalization, it was also shown that modified heavy chain constant regions can impart enhanced ADCC (to, e.g., an agonist of a stimulatory receptor), as well as provide a new activity to an antibody. For example, it was found that changing the constant heavy chain domain of an antibody that binds to an inhibitory cell surface molecule and prevents the inhibitory activity of the cell surface molecule (an antagonist) to a modified heavy chain constant region described herein, resulted in the antibody losing its ability to be an antagonist, and instead endowed it with agonist activity (of the inhibitory activity).

***Example 11: Confirmation of disulfide bonds of IgG2.3 and IgG2.5 constructs***

The disulfide bond structures in an antibody comprising the constant domain IgG2.3 (A form), IgG2.3G1 (A form) and IgG2.5 (B-form) were confirmed to be correct by comparison of non-reduced to reduced Lys-C digests.

The antibody samples were digested with Lys-C which specifically cleaves peptide bonds on the carboxyl-terminal side of Lysine (K, Lys) residues. Peptides in the digest were separated using a Waters ACQUITY BEH C18 column, 1.7$\mu$m, 2.1×150mm, reverse phase HPLC column and detected with an ultraviolet (UV) detector at 214 nm and Thermo LTQ mass spectrometer.

Lys-C enzymatic digestion and reduction of disulfide bonds: To a vial containing 100$\mu$g of the antibody sample, 120$\mu$L denature buffer was added, resulting in a 3.7M GuHCl, 0.2M Tris pH 7.0 solution. The mixture was incubated at 55°C for 30 minutes. Alkylation of protein was done by adding 1$\mu$l 50mM Iodoacetamide in the above solution, then incubation in the dark at room temperature for 30 minutes. Alkylated sample was diluted with 80$\mu$L dH2O and Waco Lys-C was added at enzyme to substrate ratio as 1:10. The antibodies were digested overnight in the dark at room temperature. After digestion, a 100 $\mu$L aliquot was removed from the Lys-C digested sample and 10$\mu$L of 0.5M DTT was added in. This sample was incubated at room temperature for 1 hour to reduce the disulfide bonds.

The results obtained are as follows:

Disulfide structure of the IgG2.3 and IgG2.3G1 antibodies (A form): Within the Fab region of the heavy chain Cys22 (H) is linked to Cys98 (H) and Cys151 (H) is linked to Cys 207(H). Within the Fc region of the heavy chain Cys265(H) is linked to Cys325 (H) and Cys371 (H) is linked to Cys429 (H). Within the Fab region of the light chain Cys23 (L) is linked to Cys88 (L) and Cys134 (L) is linked to Cys194 (L). The C-terminal of light chain Cys214 (L) is linked to the heavy chain at Cys138 (H). The hinge region of the heavy chain contains three cysteine residues Cys227 (H), Cys230 (H) and Cys233 (H), which provide three inter-chain disulfide bonds. The most likely linkage is Cys227 (H) to Cys227 (H), Cys230 (H) to Cys230 (H) and Cys233 (H) to Cys233 (H) which is the correct theoretical disulfide arrangement of IgG2 A form.

Disulfide structure of the IgG2.5 antibody (B form): Within the Fab region of the heavy chain Cys22 (H) is linked to Cys98 (H) and Cys151 (H) is linked to Cys 207(H). Within the Fc region of the heavy chain Cys264(H) is linked to Cys324 (H) and Cys370 (H) is linked to Cys428 (H). Within the Fab region of the light chain Cys23 (L) is linked to Cys88 (L) and Cys134 (L) is linked to Cys194 (L). The hinge region of the heavy chain contains four cysteine residues Cys226(H), Cys227 (H), Cys230 (H) and Cys233 (H). The C-terminal of light chain Cys214 (L) is linked to a cysteine residue of heavy chain in the hinge region, and rest three cysteine residues provide three inter-chain disulfide bonds. The most likely linkage is Cys214 (L) to Cys226 (H), then Cys227 (H) to Cys227 (H), Cys230 (H) to Cys230 (H) and Cys233 (H) to Cys233 (H), which is the correct theoretical disulfide arrangement of IgG2 B form. Additionally, the disulfide linkages in the hinge region were confirmed using electron transfer dissociation (ETD) triggered tandem mass spectrometry using an ion trap mass spectrometer.

***Example 12: Relevance of certain amino acid residues in IgG2 CH1 and hinge in improving GITR agonism on T cells***

Anti-GITR antibodies (GITR.6) with the heavy chain constant regions shown in Table 17 were prepared and tested in IL-2 production assays as described in Example 2, but in which supernatants were harvested at 40 hours rather than 48 hours.

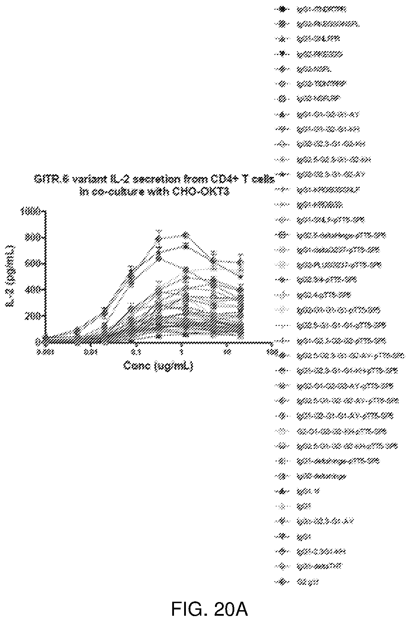

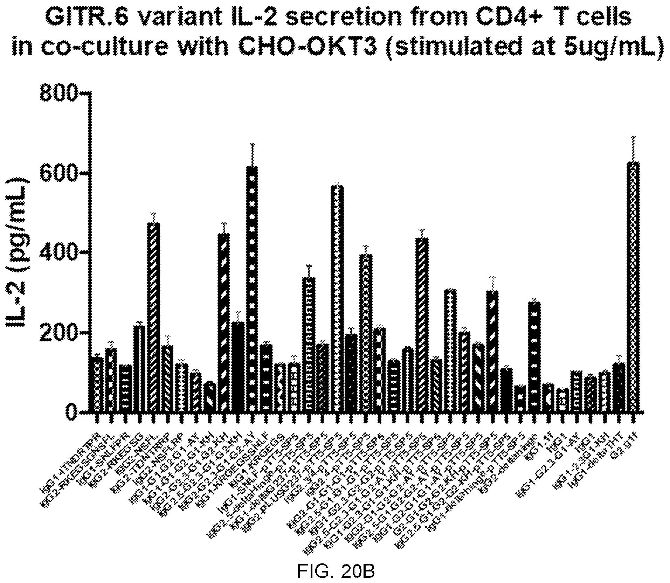

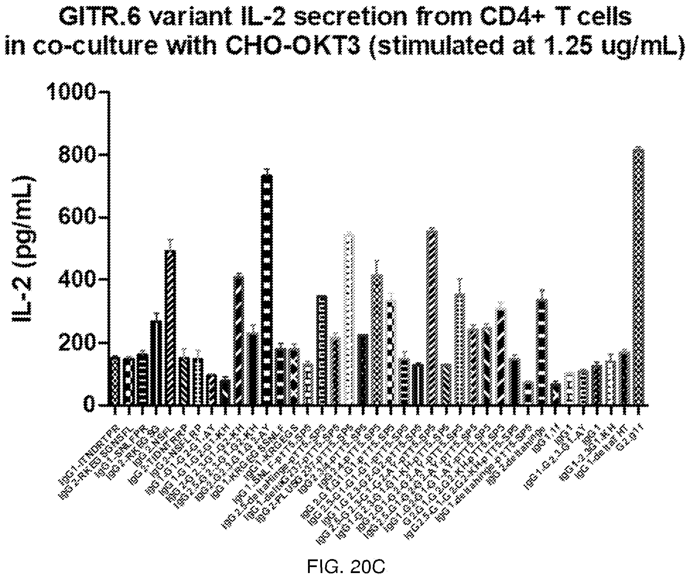

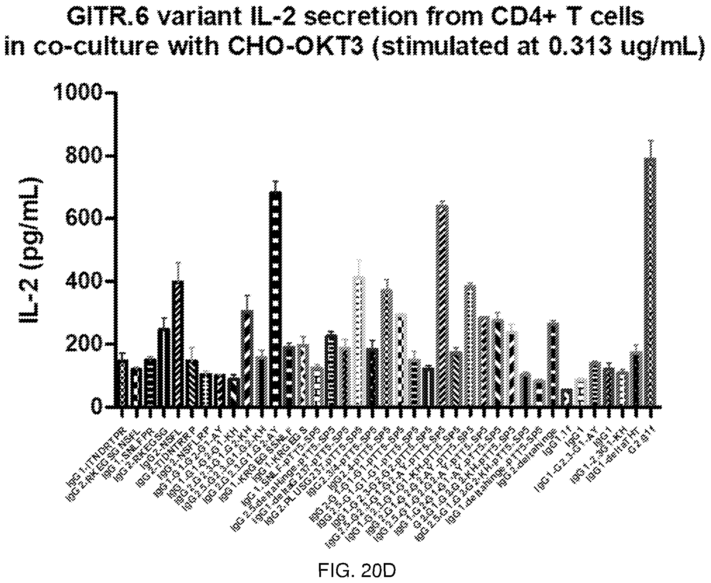

The results, which are shown in Figure 20A-D, were largely in agreement with the CD73 internalization results (see Figure 10) obtained with anti-CD73 antibodies having the same heavy chain constant regions as those used in this Example.

***Example 13: Elimination of effector functions with α P238K mutation***

Variable regions of an antibody were fused to an IgG1 Fc that differs from a wild type IgG1 Fc in a single amino acid residue: P238K (SEQ ID NO: 198). With this single mutation, the antibody demonstrated a lack of effector function, having essentially no detectable binding signal towards the low affinity Fc√Rs hCD32a-H131, hCD32a-R131, hCD32b, hCD16a-V158 or hCD16b-NA2 (see data in Example 14). In addition, the antibody with IgG1 P238K showed significant reduction in binding affinity to the high affinity Fc√R CD64 (see data in Example 14). Binding of the antibody to CD64 demonstrated a faster off-rate (dissociation constant) relative to antibodies with a wild type IgG1 constant domain.

The lack of effector function of an IgG1 Fc having a P238K mutation (SEQ ID NO: 198) was also demonstrated with an antibody variant.

Thus, a human IgG1 Fc with a single mutation (P238K), e.g., wherein the heavy chain constant region comprises the amino acid sequence SEQ ID NO: 198, can be used in any antibodies in which the effector functions are not desirable.

***Example 14: Elimination of effector functions with a P238K and additional mutations***

Additional antibodies were generated with Fcs having mutation(s) to further reduce effector function, preferably both ADCC and CDC. Mutants were generated to further reduce FcR binding as shown in Table 22. In particular, as shown above, P238K eliminates detectable FcR binding except to CD64, so the goal was to combine P238K with additional mutations to reduce CD64 binding. Mutations were tested in the context of IgG1 isotype, IgG2.3 and IgG2.5 isotype and IgG2.3G1 isotype formats. The Fcs used in these antibodies comprise one of the amino acid sequences having SEQ ID NOs: 234-245 and 247-262.

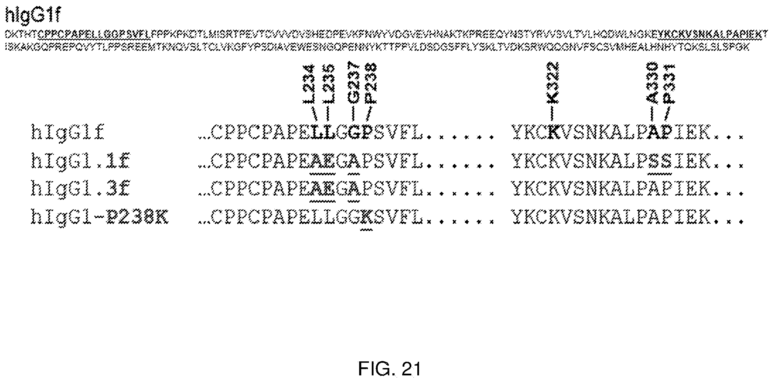

The location of the mutations is shown in FIG. 21.

The binding of human FcyRs to antibodies was studied by surface plasmon resonance using a Biacore 8K system (GE Healthcare). For these studies, protein A was immobilized on flow cells 1-4 of the CM5 sensor chip using standard ethyl (dimethylaminopropyl) carbodiimide (EDC)/N-hydroxysuccinimide (NHS) chemistry, with ethanolamine blocking, in a running buffer of 10 mM HEPES pH 7.4, 150 mM NaCl, 3 mM EDTA, 0.05% surfactant p20, to a density of -3000 RU. Purified antibodies (10 $\mu$g/mL) or expression supernatants (diluted to ~10ug/ml) were captured on the protein A surface to a density of ~1000 - 1200 RU, and the binding of FcyR analytes was tested in running buffer consisting of 10 mM NaPO4, 130 mM NaCl, 0.05% p20, buffer (PBS-T) pH 7.1 at 25°C, using 120 s association time and 120 s dissociation time at a flow rate of 20 $\mu$L/min. The data were analyzed using Biacore 8K evaluation software, by determining the measured binding response as a percentage of theoretical maximum binding response for each antibody (%Rmax), based on the level of captured antibody, assuming 100% fractional activity and only taking into account protein mass without glycosylation, as follows. T compare the FcgR binding of different molecules, the SPR binding data was analyzed by calculating the maximum binding response as a percentage of the theoretical maximum binding response (%Rmax) as generally shown in Eq. 1:

$$\%Rmax \;=\; \frac{(Observed\ Binding\ Response\ Analyte)}{(Theoretical\ Maximum\ Binding\ Response\ Analyte)} \qquad \mathbf{Eq.\ 1}$$

Specifically, the %Rmax was calculated using the equation:

$$\%Rmax = \frac{(Binding\ Response\ Analyte)}{\frac{(Mw\ Analyte)}{(Mw\ Ligand)} \times (Response\ Ligand) \times (analyte{:}ligand\ stoichiometry)} \qquad \textbf{Eq. 2}$$

where "Analyte" is the antibody and "Ligand" is the captured FcgR protein. This analysis does not take into account the mass of glycosylation of antibody or FcgR, and assumes 100% fractional activity for the captured ligand.

The "% Rmax analysis" is particularly useful for evaluating the binding of the "low affinity" FcgRs, e.g., hCD32a-H131, hCD32a-R131, hCD32b, hCD16a-V158, hCD16a-F158, hCD16b-NA1, and hCD16b-NA2, which have relatively fast association and dissociation rates and affinities near or below the analyte concentration tested (1 micromolar ($\mu$M)), so saturation of the surface is generally not achieved under these conditions. In contrast, the "high affinity" FcgR hCD64 binds with higher affinity and slower dissociation kinetics than the other FcgRs, particularly to IgG1 and IgG4, and thus these isotypes do typically saturate the hCD64 surface under micromolar analyte concentrations, and are more difficult to differentiate affinities using %Rmax. For these interactions, differences between antibodies can be easily observed by comparison of the dissociation rates in the sensorgram data.

The results are shown in Table 22 and exemplary sensorgram data are provided in FIG. 21 A-L.

Table 22: Binding of antibodies with wild-type or mutated Fcs to FcγRs shown as percentage of Rmax

| Antibody | Sample | 1uM hCD64 | 10uM hCD32a-H131 | 10uM hCD32a-R131 | 10uM hCD32b | 10uM hCD16a-V158 | 10uM hCD16a-F158 | 0.1uM hCD64 | 1uM hCD32a-H131 | 1uM hCD32a-R131 | 1uM hCD32b | 1uM hCD16a-V158 | 1uM hCD16a-F158 |
|---|---|---|---|---|---|---|---|---|---|---|---|---|---|
| Ab1-hIgG1f | Purified | 126% | 98% | 93% | 61% | 116% | 45% | 126% | 54% | 43% | 13% | 81% | 10% |
| Ab2-hIgG1f | Purified | 123% | 98% | 96% | 73% | 116% | 65% | 124% | 65% | 56% | 20% | 94% | 17% |
| Ab1-NF | Purified | 125% | 97% | 98% | 76% | 124% | 130% | 125% | 55% | 59% | 22% | 123% | 104% |
| Ab33-IgG2.3 | Purified | 16% | 100% | 69% | 29% | 27% | 4% | 2% | 60% | 21% | 7% | 6% | 2% |
| Ab4-hz1-P238K | Purified | 116% | 0% | 1% | 1% | -1% | -1% | 88% | 0% | 0% | 0% | 0% | 0% |
| | | | | | | | | | | | | | |
| Ab2-IgG1.3f-P238K | supernatant | 1% | 1% | 3% | 2% | 0% | 0% | 0% | 1% | 1% | 1% | 0% | 0% |
| Ab2-IgG1f-P238K | supernatant | 109% | -3% | -2% | -2% | -4% | -4% | 89% | 1% | 1% | 1% | 0% | 0% |
| Ab2-IgG1f-L235E-P238K | supernatant | 11% | 1% | 3% | 3% | 0% | -1% | 2% | 0% | 1% | 1% | 0% | 0% |
| Ab2-IgG1f-L235E-P238K-K322A | supernatant | 11% | 2% | 5% | 3% | 0% | 0% | 2% | 1% | 1% | 1% | 1% | 0% |
| Ab2-IgG2.3G1.3f-P238K | supernatant | 2% | 1% | 3% | 1% | 0% | 0% | 0% | 0% | 1% | 1% | 0% | 0% |
| Ab2-IgG2.3G1-L235E-P238K | supernatant | 16% | 1% | 4% | 3% | 1% | 0% | 2% | 1% | 1% | 1% | 1% | 1% |
| Ab2-IgG2.3G1-L235E-P238K-K322A | supernatant | 16% | 2% | 5% | 3% | 0% | 0% | 2% | 1% | 1% | 1% | 1% | 0% |
| Ab2-IgG2.5G1.3f-P238K | supernatant | 2% | 1% | 3% | 2% | 0% | 0% | 0% | 1% | 1% | 1% | 1% | 1% |
| Ab2-IgG2.5G1-L235E-P238K-K322A | supernatant | 15% | 2% | 5% | 3% | 0% | 0% | 2% | 1% | 1% | 1% | 1% | 1% |
| | | | | | | | | | | | | | |
| Ab4-IgG1fa | supernatant | 124% | 99% | 95% | 71% | 116% | 59% | 125% | 63% | 53% | 19% | 91% | 15% |
| Ab4-IgG1.3fa | supernatant | 7% | 2% | 29% | 17% | 4% | 1% | 1% | 0% | 4% | 2% | 1% | 0% |
| Ab4-IgG1fa-P238K | supernatant | 116% | 1% | 1% | 1% | 0% | 0% | 87% | 1% | 1% | 1% | 0% | 0% |
| Ab4-IgG1fa-L235A-P238K | supernatant | 51% | 0% | 1% | 0% | -1% | -1% | 10% | 0% | 0% | 0% | 0% | 0% |
| Ab4-IgG1fa-L235E-P238K | supernatant | 9% | 0% | 3% | 2% | 0% | 0% | 1% | 0% | 1% | 1% | 0% | 0% |
| Ab4-IgG1.3fa-P238K | supernatant | 1% | 0% | 2% | 1% | 0% | -1% | 0% | 0% | 1% | 0% | 0% | 0% |
| Ab4-IgG1fa-L235E-P238K-K322A | supernatant | 11% | 1% | 4% | 1% | -1% | -1% | 2% | 0% | 1% | 0% | 0% | 0% |
| Ab4-IgG2.3 | supernatant | 16% | 98% | 73% | 31% | 34% | 4% | 2% | 66% | 23% | 6% | 7% | 2% |
| Ab4-IgG2.3-P238K | supernatant | 1% | 1% | 2% | 1% | 0% | 0% | 0% | 2% | 2% | 2% | 1% | 1% |
| Ab4-IgG2.3G1 | supernatant | 123% | 95% | 93% | 72% | 113% | 62% | 123% | 62% | 55% | 21% | 90% | 16% |
| Ab4-IgG2.3G1-P238K | supernatant | 116% | 0% | 1% | 1% | -1% | -2% | 91% | 0% | 0% | 0% | 0% | -1% |
| Ab4-IgG2.5G1-P238K | supernatant | 118% | 1% | 2% | 2% | 0% | 0% | 94% | 1% | 1% | 1% | 1% | 1% |
| Ab4-IgG2.3G1-L235E-P238K | supernatant | 13% | 0% | 3% | 3% | 0% | 0% | 2% | 0% | 1% | 1% | 0% | 0% |

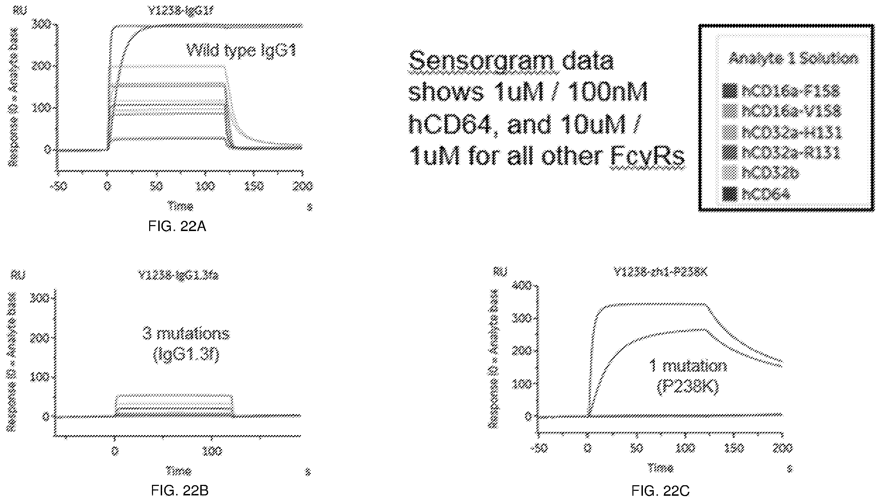

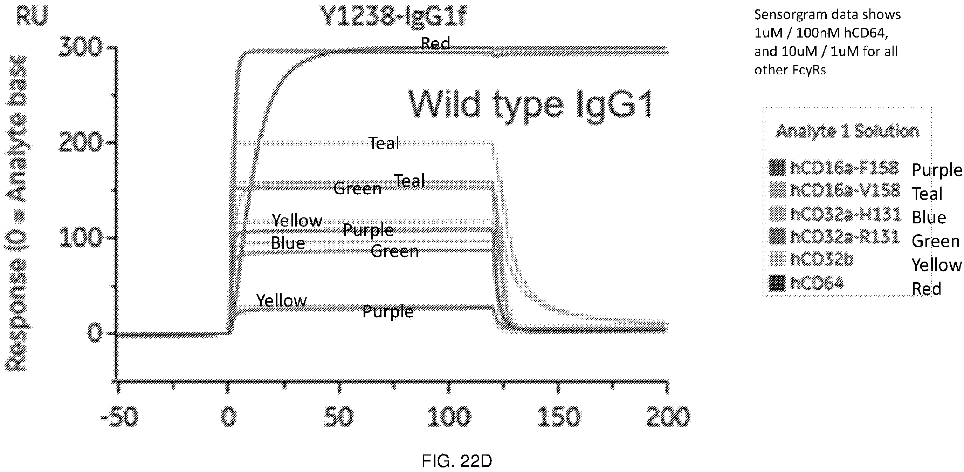

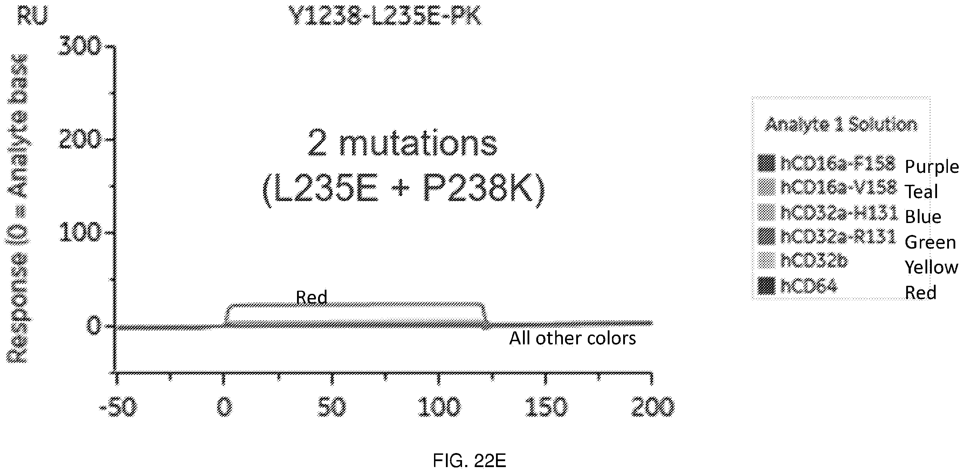

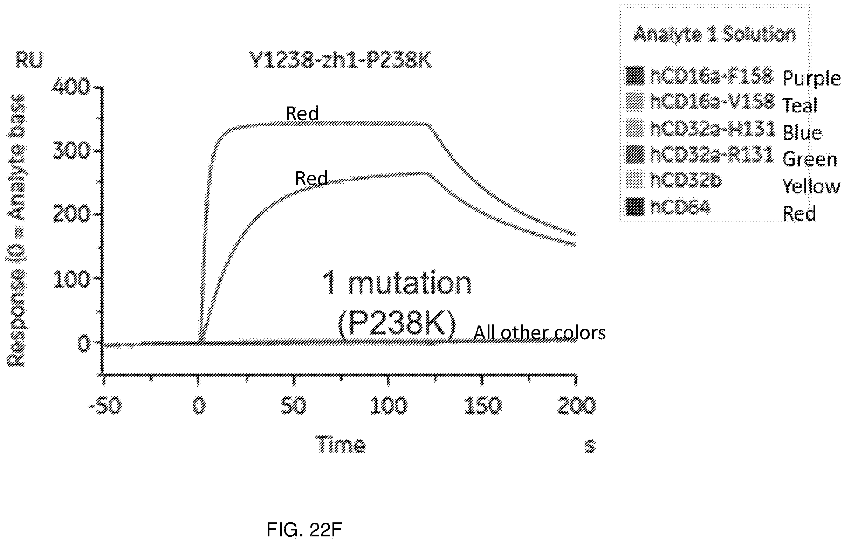

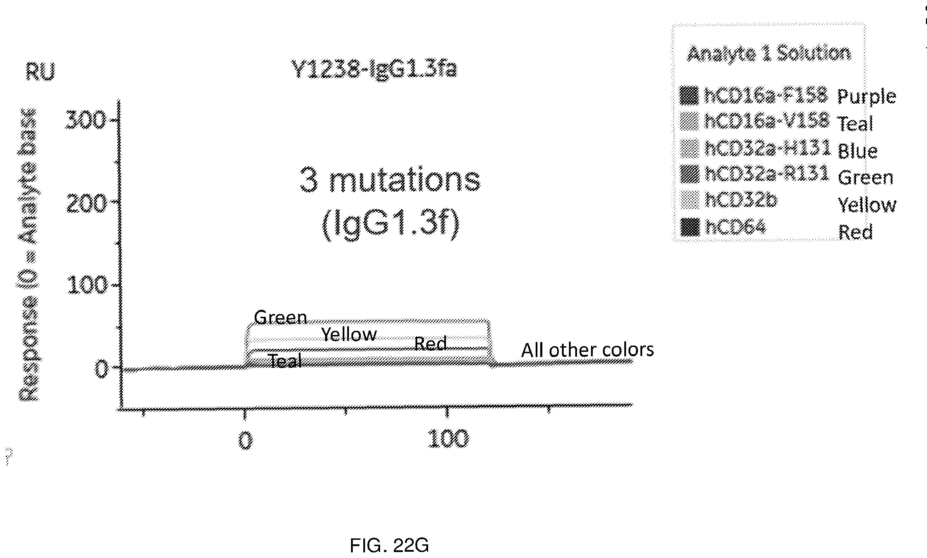

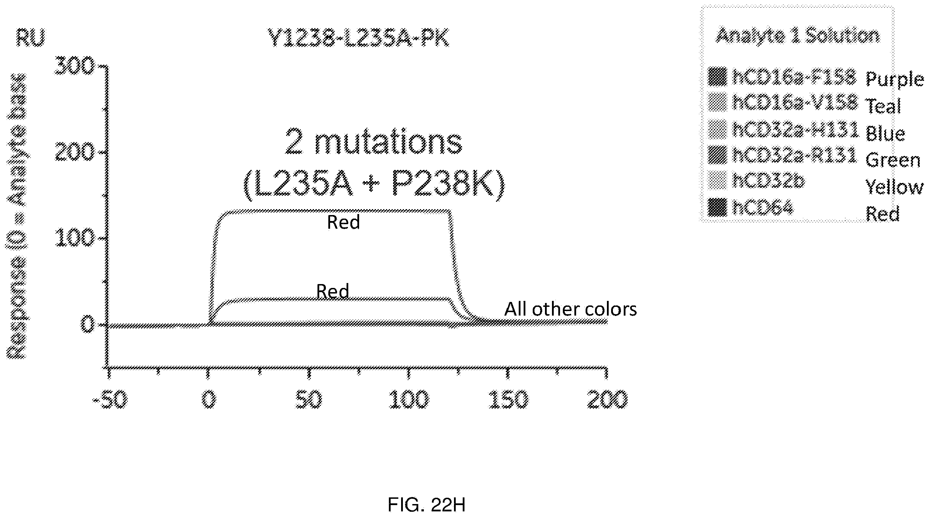

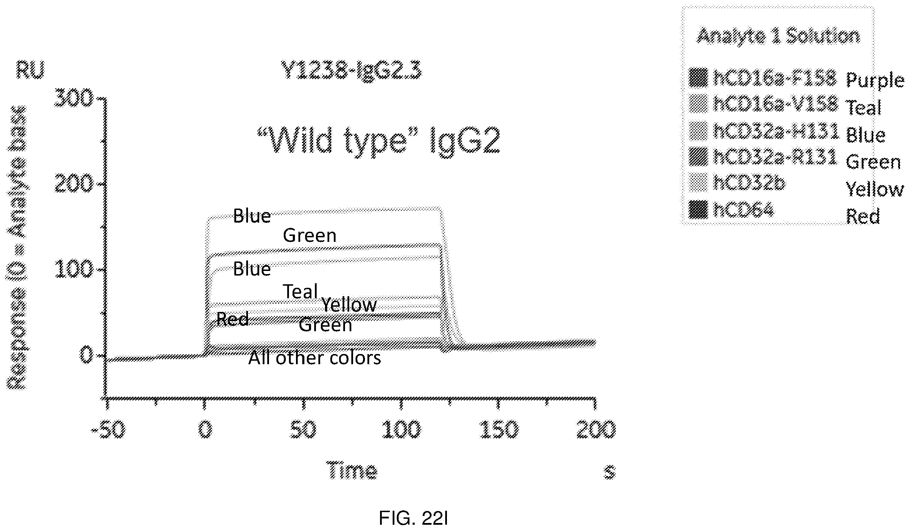

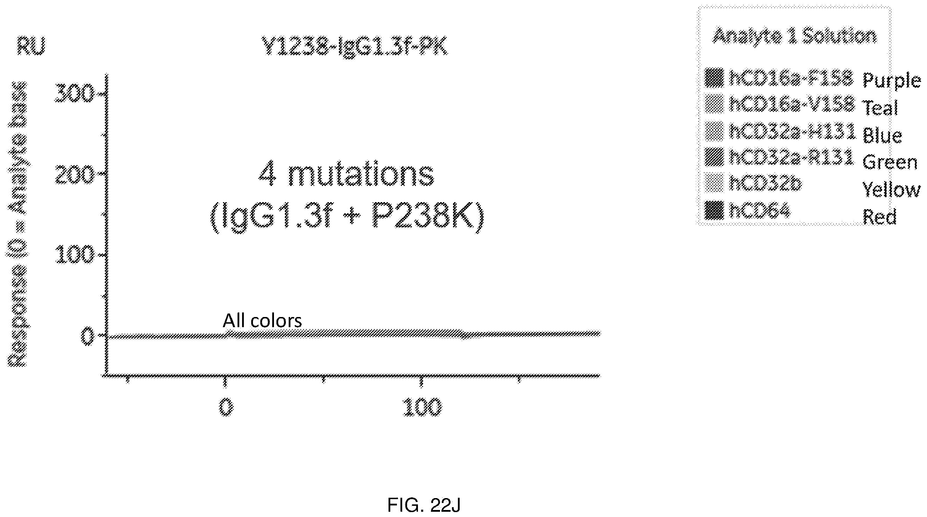

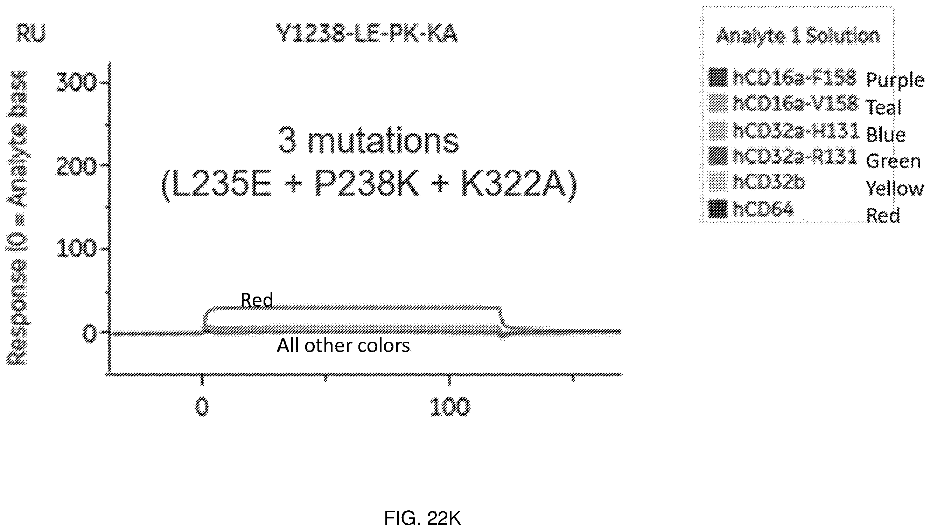

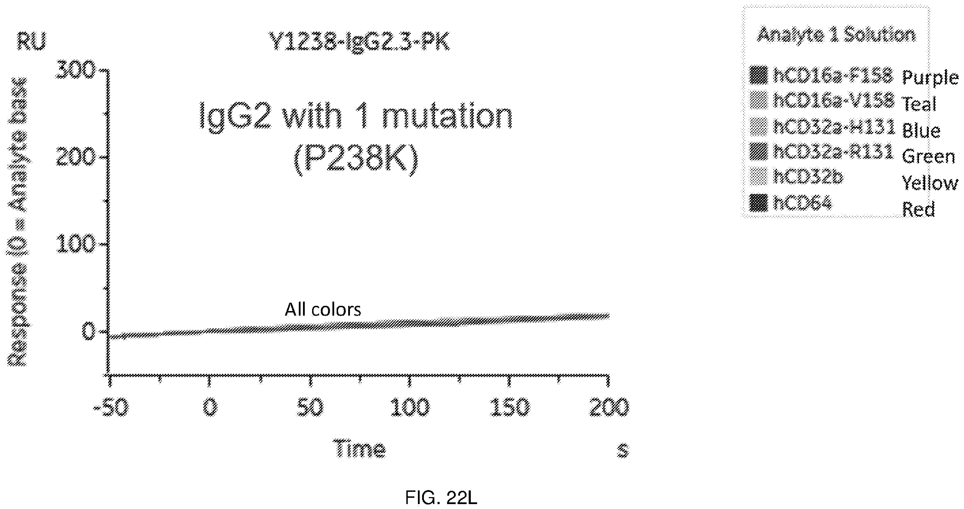

As shown in Table 22 and in FIG. 22, the combination mutants demonstrated very weak FcR binding. Addition of L235 mutations to P238K isotype reduced CD64 binding to similar levels as IgG1.3f. L235E was superior to L235A mutation for reducing CD64 binding. Adding the P238K mutation to IgG2 (IgG2.3-P238K) resulted in a fully inert isotype, demonstrating no detectable binding to any of the FcR proteins. The mutations also showed similar trends in the context of IgG1 and IgG2.xG1 formats. K322A mutation, which reduce clq binding (CDC activity), and was added in some constructs, had minimal impact on FcR binding so not much effect of K322A was observed.

### Example 14: Elimination of effector functions with IgG1.3 Fc

This Example is described in Examples 2 and 3 of co-filed and co-owned PCT application entitled "MODIFIED IgG1 Fc DOMAINS AND ANTI-CD40 DOMAIN ANTIBODY FUSIONS THEREWITH."

This Example shows that an antibody or polypeptide with an IgG1.3 Fc is essentially devoid of binding to CD16, CD32a, CD32b and CD64. This has also been observed when an IgG1.3 Fc was linked to the variable domain of anti-TIM3 antibodies (see WO2018/013818). IgG1.3 was derived from the "IgG1.1" Fc ("IgG1.1" is an IgG1 with L234A, L235E, G237A, A330S and P331S substitutions) by removing A330S and P331S, thereby retaining 3 of the 5 mutations, i.e., L234A, L235E, G237A. It was surprisingly discovered that the absence of A330S and P331S in the IgG1.1 Fc did not significantly affect the inertness of this Fc. Below are exemplary FcγR binding measurements of IgG1.1 and IgG1.3 (and other Fcs for comparative purposes) containing antibodies and fusion proteins, comparing the inertness of IgG1.1 and IgG1.3 in the context of an antibody as well as in the context of a non-antibody protein.

Materials and Methods used in this example include the following.

*FcgR Binding SPR*: FcgR binding can be measured *in vitro* using purified FcγRs using Biacore™ surface plasmon resonance (SPR). Two methods were used herein.

One method tests the binding of purified antibodies or dAb-Fc proteins to His-tagged FcgR proteins (FcgR-His ("FcgR" is used interchangeably with "FcγR") which are captured on the immobilized Fab fragment of an anti-His antibody. These experiments are performed on either a Biacore™ T100 or Biacore™ T200 instrument (GE Healthcare) at 25° C. The Fab fragment from a murine anti-6xHis antibody (generated in house) is immobilized on a CM5 sensor chip using standard ethyl(dimethylaminopropyl) carbodiimide (EDC)/N-hydroxysuccinimide (NHS) chemistry with ethanolamine blocking, to a density of -3000 Resonance Units RU in a running buffer of 10 millimolar (mM) HEPES pH 7.4, 150 mM NaCl, 3 mM EDTA, 0.05% surfactant p20 (HBS-EP+). All remaining studies are performed using a running buffer of 10 mM NaPO$_4$, 130 mM NaCl, 0.05% p20 (PBS-T) at pH 7.1. Various FcgR proteins containing a C-terminal 6x poly-histidine tag (generated in house) were captured on this surface (typically using FcgR-His protein concentration of ~7 μg/ml) using a contact time of 30 seconds (s) at 10 μl/min. Various concentrations of purified antibody or dAb-Fc proteins are tested for binding, for example using an association time of 120 seconds at 30 μl/min, and a dissociation time of 120 seconds at 30 μl/min. FcgR proteins tested in these studies include the "high affinity" FcgR hCD64 (hFcgRI), as well as the "low affinity" FcgRs hCD32a-H131 (FcgRIIa-H131), hCD32a-R131 (FcgRIIa-R131), hCD32b (FcgRIIb), hCD16a-V158 (FcgRIIIa-V158), hCD16a-F158 (FcgRIIIa-F158), hCD16b-NA1 (FcgRIIIb-NA1), and hCD16b-NA2 (FcgRIIIb-NA2).

To quantitatively analyze the binding responses and compare the FcgR binding of different molecules, the SPR binding data can be analyzed by calculating the maximum binding response as a percentage of the theoretical maximum binding response (%Rmax) as generally shown in Eq. 1:

$$\%Rmax \ = \ \frac{(Observed\ Binding\ Response\ Analyte)}{(Theoretical\ Maximum\ Binding\ Response\ Analyte)} \quad \textbf{Eq. 1}$$

Specifically, the %Rmax is calculated using the equation:

$$\%Rmax \ = \ \frac{(Binding\ Response\ Analyte)}{\frac{(Mw\ Analyte)}{(Mw\ Ligand)} \times (Response\ Ligand) \times (analyte{:}ligand\ stoichiometry)} \quad \textbf{Eq. 2}$$

where "Analyte" is the antibody or dAb-Fc and "Ligand" is the captured FcgR protein. This analysis does not take into account the mass of glycosylation of antibody, dAb-Fc or FcgR, and assumes 100% fractional activity for the captured ligand.

The "%Rmax analysis" is particularly useful for evaluating the binding of the "low affinity" FcgRs, e.g., hCD32a-H131, hCD32a-R131, hCD32b, hCD16a-V158, hCD16a-F158, hCD16b-NA1, and hCD16b-NA2, which have relatively fast association and dissociation rates and affinities near or below the analyte concentration tested (1 micromolar (μM)), so saturation of the surface is generally not achieved under these conditions. In contrast, the "high affinity" FcgR hCD64 binds with higher affinity and slower dissociation kinetics than the other FcgRs, particularly to IgG1 and IgG4, and thus these isotypes do typically saturate the hCD64 surface under micromolar analyte concentrations, and are more difficult to differentiate affinities using %Rmax. For these interactions, differences between antibodies can be easily observed by comparison of the dissociation rates in the sensorgram data.

A second SPR assay for testing the interaction between antibodies or dAb-Fc proteins with FcgR proteins is a protein A capture method. These experiments are also performed on either a Biacore™ T100 or Biacore™ T200 instrument (GE Healthcare) at 25° C. For these studies, protein A is immobilized on flow cells 1-4 of a CM5 sensor chip using standard ethyl (dimethylaminopropyl) carbodiimide (EDC)/N-hydroxysuccinimide (NHS) chemistry, with ethanolamine blocking, in a running buffer of 10 mM HEPES pH 7.4, 150 mM NaCl, 3 mM EDTA, 0.05% surfactant p20, to a density of ~3000 RU. Antibody or dAb-Fc proteins (typically -3-10 μg/ml) are captured on the protein A surface, and the binding of FcgR analytes are tested in running buffer consisting of 10 mM NaPO$_4$, 130 mM NaCl, 0.05% p20, buffer (PBS-T) at pH 7.1 and at 25° C, using for example, 120 sec association time and 180 sec dissociation time at a flow rate of 30 μL/min.

The protein A capture assay can also be used to analyze unpurified supernatants containing antibody or dAb-Fc molecules. For this analysis, the antibody or dAb-Fc proteins can be captured from either undiluted supernatants or supernatants diluted with running buffer. To quantitatively analyze the binding responses and compare the FcgR binding of different molecules, the SPR binding data can be analyzed by calculating the %Rmax using Eq. 1 above, wherein Analyte is the purified FcgR protein, and Ligand is the captured antibody or dAb-Fc protein.

In addition to %Rmax analysis, quantitative analysis of the kinetics and affinity of binding can be performed by testing a titration of FcgR analyte for binding to protein A captured antibodies or dAb-Fc proteins. For example, FcgR in a 3:1 serial dilution can be titrated from 10 μM down to either 0.15 nM (hCD64) or 1.5 nM (all other FcgRs). These kinetic data can be fit to either a 1:1 Langmuir model or to a steady-state binding model using Biacore™ T200 evaluation software to obtain kinetic and affinity values.

*dAb-Fcs*: The dAb-Fcs studied in this example are shown in Table 23. In these sequences, the single variable domain 3h56-269 residues are amino acids 1- 118 (underlined). The linker AST is double-underlined.

**Table 23**

| Seq # | Sample ID | Sequence |
|---|---|---|
| 263 | 3h56-269-IgG4.1 OR BMS-986090 | <u>EVQLLESGGGLVQPGGSLRLSCAASGFTFRDYEMWWVRQAPGKGLERVSA INPQGTRTYYADSVKGRFTISRDNSKNTLYLQMNSLRAEDTAVYYCAKLP FRFSDRGQGTLVTVSS<u>AST</u></u>ESKYGPPCPPCPAPEFLGGPSVFLFPPKPKD TLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLD SDGSFFLYSRLTVDKSRWQEGNVFSCSVMHEALHNHYTQKSLSLSLGK |
| 264 | 3h56-269-CT | <u>EVQLLESGGGLVQPGGSLRLSCAASGFTFRDYEMWWVRQAPGKGLERVSA INPQGTRTYYADSVKGRFTISRDNSKNTLYLQMNSLRAEDTAVYYCAKLP FRFSDRGQGTLVTVSS<u>AST</u></u>EPKSSDKTHTSPPSPAPELLGGSSVFLFPPK PKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 265 | 3h56-269-IgG1.1f | <u>EVQLLESGGGLVQPGGSLRLSCAASGFTFRDYEMWWVRQAPGKGLERVSA INPQGTRTYYADSVKGRFTISRDNSKNTLYLQMNSLRAEDTAVYYCAKLPFRF SDRGQGTLVTVSS<u>AST</u></u>EPKSCDKTHTCPPCPAPEAEGAPSVFLFPPKPKDTL MISRTPEVTCVVVDVSHEDPEVKFNWYVDGVEVHNAKTKPREEQYNSTYRV VSVLTVLHQDWLNGKEYKCKVSNKALPSSIEKTISKAKGQPREPQVYTLPPSR EEMTKNQVSLTCLVKGFYPSDIAVEWESNGQPENNYKTTPPVLDSDGSFFLY SKLTVDKSRWQQGNVFSCSVMHEALHNHYTQKSLSLSPGK |
| 266 | 3h56-269-IgG1.3f | <u>EVQLLESGGGLVQPGGSLRLSCAASGFTFRDYEMWWVRQAPGKGLERVSA INPQGTRTYYADSVKGRFTISRDNSKNTLYLQMNSLRAEDTAVYYCAKLPFRF SDRGQGTLVTVSS<u>AST</u></u>EPKSCDKTHTCPPCPAPEAEGAPSVFLFPPKPKDTL MISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSR EEMTKNQVSLTCLVKGFYPSDIAVEWESNGQPENNYKTTPPVLDSDGSFFLY SKLTVDKSRWQQGNVFSCSVMHEALHNHYTQKSLSLSPGK |
| 267 | 3h56-269-IgG1-D265A | <u>EVQLLESGGGLVQPGGSLRLSCAASGFTFRDYEMWWVRQAPGKGLERVSA INPQGTRTYYADSVKGRFTISRDNSKNTLYLQMNSLRAEDTAVYYCAKLPFRF SDRGQGTLVTVSS<u>AST</u></u>EPKSCDKTHTCPPCPAPELLGGPSVFLFPPKPKDTLM ISRTPEVTCVVVAVSHEDPEVKFNWYVDGVEVHNAKTKPREEQYNSTYRVV SVLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYS KLTVDKSRWQQGNVFSCSVMHEALHNHYTQKSLSLSPGK |

The sequences of IgG1.1f and IgG1.3f, each starting with "EPK" (i.e., the sequences in SEQ ID NOs: 77 and 78) that are shown in Table 23 are identical to the sequences starting at EPK in SEQ ID NOs 83 and 248, respectively.

*Control mAb*: A control monoclonal antibody (1F4) was also formatted with similar Fc domain mutations. The individual chain sequences are shown in Table 24, including the sequence (SEQ ID NO: 268) of the portion of the 1F4 heavy chain including the variable region and CH1 region. This sequence is underlined in the heavy chain sequences (SEQ ID NOs: 269-275). The pair of heavy chain and light chain sequences for each 1F4 mAb variant is shown in Table 25.

**Table 24**

| Seq ID No. | Sequence identity | Sequence |
|---|---|---|
| 268 | 1F4 Heavy chain variable region and CH1 | EVQLLESGGGLVQPGGSLRLSCAASGFTFSIYAMSWVRQAPG KGLEWVSAISDSGGRTYFADSVRGRFTISRDNSKNTLSLQMNS LRAEDTAVYYCAKVDYSNYLFFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKRV |
| 269 | 1F4 Light chain variable region and CL | EIVLTQSPGTLSLSPGERATLSCRASQSISSSYLAWYQQKPGQA PRLLIYGASSRATGIPDRFSGSGSGTDFTLTISRLEPEDFAVYYC QQYGSSPYTFGQGTKLEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC |
| 270 | 1F4-IgG1f heavy chain | EVQLLESGGGLVQPGGSLRLSCAASGFTFSIYAMSWVRQAPG KGLEWVSAISDSGGRTYFADSVRGRFTISRDNSKNTLSLQMNS LRAEDTAVYYCAKVDYSNYLFFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKRVEPKSCDKTHTCPPCPAPELLGGPSVFLFPPKPKDTLM ISRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPGK |
| 271 | 1F4-IgG4.1 heavy chain | EVQLLESGGGLVQPGGSLRLSCAASGFTFSIYAMSWVRQAPG KGLEWVSAISDSGGRTYFADSVRGRFTISRDNSKNTLSLQMNS LRAEDTAVYYCAKVDYSNYLFFDYWGQGTLVTVSSASTKGP SVFPLAPCSRSTSESTAALGCLVKDYFPEPVTVSWNSGALTSG VHTFPAVLQSSGLYSLSSVVTVPSSSLGTKTYTCNVDHKPSNT KVDKRVESKYGPPCPPCPAPEFLGGPSVFLFPPKPKDTLMISRT PEVTCVVVDVSQEDPEVQFNWYVDGVEVHNAKTKPREEQFN STYRVVSVLTVLHQDWLNGKEYKCKVSNKGLPSSIEKTISKA KGQPREPQVYTLPPSQEEMTKNQVSLTCLVKGFYPSDIAVEW ESNGQPENNYKTTPPVLDSDGSFFLYSRLTVDKSRWQEGNVF SCSVMHEALHNHYTQKSLSLSLG |

(continued)

| Seq ID No. | Sequence identity | Sequence |
|---|---|---|
| 272 | 1F4-IgG1.1f heavy chain | EVQLLESGGGLVQPGGSLRLSCAASGFTFSIYAMSWVRQAPGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAEGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPSSIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 273 | 1F4-IgG1.3f heavy chain | EVQLLESGGGLVQPGGSLRLSCAASGFTFSIYAMSWVRQAPGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAEGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 274 | 1F4-D265A heavy chain | EVQLLESGGGLVQPGGSLRLSCAASGFTFSIYAMSWVRQAGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIVEWESNGQPENNYKTTPPVLDSDGSFFLYSKLTVDKSRWQQGNVFSCSVMHEALHNHYTQKSLSLSPGK |
| 275 | 1F4-CT heavy chain | EVQLLESGGGLVQPGGSLRLSCAASGFTFSIYAMSWVRQAPGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTSPPSPAPELLGGSSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

**Table 25**

| mAb name | HC | LC |
|---|---|---|
| 1F4-TgG1f | SEQ #: 270 | SEQ #: 269 |
| 1F4-IgG4.1 | SEQ #: 271 | SEQ #: 269 |
| 1F4-IgG1.1f | SEQ #: 272 | SEQ #: 269 |
| 1F4-IgG1.3f | SEQ #: 273 | SEQ #: 269 |
| 1F4-D265A | SEQ #: 274 | SEQ #: 269 |
| 1F4-CT | SEQ #: 275 | SEQ #: 269 |

**Results:** dAb-Fc molecules were produced with mutations in the Fc domain to reduce FcgR binding. Specifically, the anti-CD40 domain antibody 3h56-269 was formatted with the following Fc domain variants: IgG1.1f, IgG1.3f, and IgC1-D265A. In each of 3h-56-269-IgG1.1f (SEQ ID NO: 77), 3h-56-269-IgG1.3f (SEQ ID NO: 78), and 3h-56-269-IgG1-D265A (SEQ ID NO: 79), amino acids 1-116 are 3h-56-269 dAb, amino acids 117-119 are a linker, and amino acids 120-351 are the Fc domain.

Each these dAb-Fc fusion proteins, as well as each of 3h56-269-IgG4.1 and 3h56-269-CT, was confirmed to bind with high affinity to purified human-CD40 monomer (hCD40monomer, generated in house) as measured by Biacore™ SPR. As shown in Table 26, the KD values range between 7.3 nM and 11.5 nM for the different Fc variants. Each of the dAb-Fc molecules also bound human CD40 with high avidity, as measured by SPR using hCD40-Fc on the surface of a sensor chip and the dAb-Fc molecules as soluble analytes in solution, where data for 250 nM and 25 nM dAb-Fc analyte injections were fit to a 1:1 Langmuir model to estimate avidity-influenced apparent KD values ($KD_{apparent}$) for all dAb-Fcs as <1 nM. See Table 26.

**Table 26:** SPR data for binding of dAb-Fc molecules to human CD40.

| Ligand | hCD40monomer binding to dAb-Fc molecules captured on immobilized protein A surface (Affinity) | | | dAb-Fc binding to immobilized hCD40-Fc surface (Avidity) |
|---|---|---|---|---|
| | ka (1/Ms) | kd (1/s) | KD (nM) | $KD_{apparent}$ (nM) |
| 3h56-269-IgG4.1 | 8.5E+03 | 9.7E-05 | 11.5 | <1 |
| 3h56-269-CT | 1.6E+04 | 1.3E-04 | 8.0 | <1 |
| | 1.6E+04 | 1.2E-04 | 7.3 | <1 |
| 3h56-269-CT (UCOE-CHO)* | 1.6E+04 | 1.3E-04 | 7.7 | <1 |
| | 1.9E+04 | 1.4E-04 | 7.0 | <1 |
| 3h56-269-IgG1.1f | 9.6E+03 | 1.0E-04 | 10.8 | <1 |
| 3h56-269-IgG1.3f | 9.9E+03 | 9.1E-05 | 9.2 | <1 |
| | 1.1E+04 | 1.1E-04 | 9.8 | <1 |
| 3h56-269-IgG1-D265A | 1.1E+04 | 9.9E-05 | 9.0 | <1 |
| *3h-56-269-CT expressed and purified from UCOE-CHO cells. | | | | |

The FcgR binding properties of the dAb-Fc molecules and the various control monoclonal 1F4 antibodies were characterized by SPR. The first assay involved binding of 1 μM or 10 μM dAb-Fcs or a human-IgGlf antibody control (1F4-IgG1f) to anti-His Fab captured FcgR-His surfaces. These data are shown in Table 27.

**Table** 27: %Rmax data for 1µM or 10µM dAb-Fcs or 1F4-IgG1f antibody control binding to anti-His Fab captured hFcgR-His proteins.

| Sample | Conc (µM) | Anti-His Fab captured FcgR | | | | | |
| --- | --- | --- | --- | --- | --- | --- | --- |
| | | High affinity FcgR | Low Affinity FcgR | | | | |
| | | hCD64 | hCD32aH131 | hCD32a-R131 | hCD32b | hCD16a-V158 | hCD16b-NA2 |
| 1F4-IgG1f | 1 | 65% | 31% | 19% | 5% | 31% | 13% |
| 3h56-269-IgG4.1 | 1 | 68% | 27% | 30% | 20% | 6% | 1% |
| 3h56-269-IgG1-D265A | 1 | 68% | 7% | 2% | 0% | 1% | 0% |
| 3h56-269-IgGl.lf | 1 | 11% | 1% | 10% | 3% | 0% | 0% |
| 3h56-269-IgG1.3f | 1 | 12% | 1% | 8% | 3% | 1% | 0% |
| 3h56-269-CT | 1 | 72% | 0% | 1% | 0% | 1% | 0% |
| 1F4-IgG1f | 10 | 65% | 62% | 51% | 24% | 52% | 36% |
| 3h56-269-IgG4.1 | 10 | 69% | 65% | 66% | 57% | 27% | 9% |
| 3h56-269-IgG1-D265A | 10 | 69% | 33% | 17% | 2% | 2% | -1% |
| 3h56-269-IgGl.lf | 10 | 39% | 6% | 43% | 21% | 3% | 2% |
| 3h56-269-IgG1.3f | 10 | 39% | 6% | 37% | 19% | 5% | 4% |
| 3h56-269-CT | 10 | 70% | 2% | 10% | 3% | 6% | -1% |

In another assay, FcgR analytes (at 1 µM or 10 µM) were tested for binding to protein A-captured dAb-Fc surfaces (data shown in Table 28) and for binding to antibody surfaces (data shown in Table 29).

**Table 28:** %RMax data for 1 µM or 10 µM FcgRs binding to protein A-captured dAb-Fc proteins.

| Sample | Cone (µM) | Protein A-captured dAb-Fc protein | | | | |
| --- | --- | --- | --- | --- | --- | --- |
| | | 3h56-269-IgG4.1 | 3h56-269-IgG1-D265A | 3h56-269-IgG1.1f | 3h56-269-IgG1.3f | 3h56-269-CT |
| hCD64 | 1 | 99% | 41% | 1 % | 2% | 80% |
| hCD32a-H131 | 1 | 29% | 3% | 0% | 1% | 1% |
| hCD32a-R131 | 1 | 31% | 1% | 4% | 5% | 1% |
| hCD32b | 1 | 19% | 0% | 1% | 2% | 1% |
| hCD16a-V158 | 1 | 12% | 0% | 0% | 1% | 1% |

(continued)

| Sample | Cone (μM) | Protein A-captured dAb-Fc protein | | | | |
|---|---|---|---|---|---|---|
| | | 3h56-269-IgG4.1 | 3h56-269-IgG1-D265A | 3h56-269-IgG1.1f | 3h56-269-IgG1.3f | 3h56-269-CT |
| hCD16B-NA2 | 1 | 2% | 0% | 0% | 0% | 0% |
| hCD64 | 10 | 119% | 85% | 3% | 7% | 114% |
| hCD32a-H131 | 10 | 70% | 18% | 4% | 6% | 7% |
| hCD32a-R131 | 10 | 71% | 4% | 18% | 26% | 7% |
| hCD32b | 10 | 59% | 1% | 9% | 12% | 4% |
| hCD16a-V158 | 10 | 47% | 2% | 2% | 6% | 7% |
| hCD16B-NA2 | 10 | 13% | 0% | 1% | 3% | 1% |

**Table 29:** %Rmax data for 1 μM or 10 μM FcgRs binding to protein A captured antibodies.

| Sample | Conc (μM) | Protein A-captured antibodies | | | | | |
|---|---|---|---|---|---|---|---|
| | | 1F4-IgG1f | 1F4-IgG4.1 | 1F4-D265A | 1F4-IgG1.1f | 1F4-IgG1.3f | 1F4-CT |
| hCD64 | 1 | 138% | 126% | 96% | 8% | 5% | 120% |
| hCD32a-H131 | 1 | 62% | 29% | 7% | 2% | 1% | 2% |
| hCD32a-R131 | 1 | 48% | 33% | 3% | 5% | 3% | 2% |
| hCD32b | 1 | 11% | 17% | 1% | 1% | 1% | 1% |
| hCD16a-V158 | 1 | 97% | 15% | 1% | 3% | 2% | 2% |
| hCD16B-NA2 | 1 | 33% | 4% | 1% | 4% | 3% | 0% |
| hCD64 | 10 | 155% | 139% | 131% | 17% | 14% | 131% |
| hCD32a-H131 | 10 | 99% | 79% | 38% | 7% | 6% | 11% |
| hCD32a-R131 | 10 | 101% | 87% | 17% | 28% | 24% | 13% |
| hCD32b | 10 | 55% | 68% | 4% | 11% | 12% | 8% |
| hCD16a-V158 | 10 | 125% | 59% | 2% | 5% | 7% | 11% |
| hCD16B-NA2 | 10 | 81% | 16% | -2% | 4% | 6% | 1% |

Based on the binding responses or lack thereof in these experiments, a subset of the higher affinity dAb-Fc/FcgR or Ab/FcgR interactions with strongest binding responses were selected for kinetic/affinity characterization using analyte titrations (FcgR analytes binding to protein A captured antibodies or dAb-Fcs). These data are presented in Table 30.

**Table 30:** KD values (in nM) for purified FcgR analytes binding to protein A captured antibodies or dAb-Fcs.

| Sample | hCD64 | hCD32aH131 | hCD32a-R131 | hCD32b | hCD16a-V158 | hCD16B-NA2 |
|---|---|---|---|---|---|---|
| 1F4-IgG1f | 0.2 | 920 | 1400 | >5000 | 430 | 4800 |
| 1F4-IgG4.1 | 0.58 | 3700 | 2400 | 3100 | >5000 | >5000 |
| 3h56-269-IgG4.1 | 2.8 | >5000 | 2200 | >5000 | >5000 | >5000 |

(continued)

| Sample | hCD64 | hCD32aH131 | hCD32a-R131 | hCD32b | hCD16a-V158 | hCD16B-NA2 |
|---|---|---|---|---|---|---|
| 3h56-269-IgG1-D265A | 62 | | | | | |
| 3h56-269-IgGl.lf | >5000 | | | | | |
| 3h56-269-IgG1.3f | >5000 | >5000 | >5000 | >5000 | >5000 | >5000 |
| 3h56-269-CT | 4.6 | >5000 | >5000 | >5000 | >5000 | >5000 |

Collectively, these FcgR binding SPR data show that the IgG1f and IgG4.1 isotype molecules have significantly higher FcgR affinity across all FcgRs as compared to the modified Fc variant IgG1-D265A, IgG1.1f, IgG1.3f, or CT molecules. Of the modified Fc variants, the hCD64 binding affinity was the strongest for 3h56-269-CT (KD = 4.6 nM), weaker for 3h56-269-IgG1-D265A (KD = 62 nM), and the weakest for 3h56-269-IgG1.1f and 3h56-269-IgG1.3f, for which affinity was too weak to quantitate under the conditions tested ($K_D > 5 \mu M$, which is half of the highest analyte concentration tested). All of the other FcgR interactions (hCD32aH131, hCD32a-R131, hCD32b, hCD16a-V158, hCD16b-NA2) for the IgG1-D265A, IgG1.1f, **IgG1.3f** and CT variants were also too weak to obtain reliable KD values ($K_D > 5 \mu M$). However, differences in the relative binding responses can be observed in the %Rmax data. For example, the IgG1-D265A variant has stronger binding response for hCD32a-H131 as compared to the IgG1.1f, **IgG1.3f** or CT variants (Table 28). In contrast, the IgG1.1f and IgG1.3f variants have stronger binding responses for hCD32a-R131 as compared to the IgG1-D265A and CT variants (Table 28).

IgG1.3 containing fusion protein or antibody were assessed by DSC, icIEF and mass spectrometry. Materials and methods are described below.

*Differential Scanning Calorimetry*: DSC experiments were performed on a MicroCal VP-Capillary DSC instrument (Malvern Instruments, Malvern, UK) in 10 mM NaPO$_4$, 130 mM NaCl pH 7.1. Samples of 1 mg/ml dAb-Fc or antibody were tested using a scan range of 10-110° C and a scan rate of 90° C/hr. Data were analyzed using MicroCal-Origin 7.0 software.

*Imaged Capillary Isoelectric Focusing*: icIEF experiments were performed on a ProteinSimple iCE3™ System (ProteinSimple, San Jose, CA). For these studies the dAb-Fc or antibody samples, typically at 2 mg/ml concentration, were mixed with a carrier ampholyte mixture consisting of 2 M urea, 0.35% methylcellulose, 1% Pharmalyte 5-8, 3% Pharmalyte 8-10.5, and pI markers 5.85 and 10.10, to a final protein concentration of 0.20 mg/mL, and analyzed using a pre-focusing time of 1 min at 1.5 kV and a focusing time of 10 min at 3 kV.

*Mass Spectrometry*: For mass spectrometry (mass spec) analysis, samples were reduced using 100 mM DTT, and N-deglycosylation was performed with peptide:N-Glycosidase (FPNGaseF). Liquid chromatography-mass spectrometry (LC/MS) instrumentation used was a Waters Synapt® G2 (Waters Corporation, Milford, MA) with a Waters Acquity® UPLC (ultra-performance liquid chromatography). The UPLC column was a Waters Acquity® BEH (ethylene bridged hybrid particle) C4 (2.1 x 150 mm, 300 Å, 1.7 um particle). The gradient was 10% to 38% (Mobile phase B) in 10 min at 200 μL/min flow rate. Mobile phase A was 0.1% formic acid in water. Mobile phase B was 0.1% formic acid in acetonitrile. Column temperature was 60° C. Data analysis was performed manually with the aid of Waters MassLynx™ software; spectral deconvolution was performed with the MaxEntl algorithm.

*Accelerated Stability Studies*: Accelerated stability studies were conducted by first extensively dialyzing dAb-Fc molecules in target formulation buffers at 4° C. Samples were recovered and concentrated using Amicon® Ultra Centrifugal Filter Units (Merck KgaA, Germany) and prepared at different target concentrations in dialysis buffer. These samples were incubated at various temperatures, typically 4° C, 25° C, 32° C, and/or 40° C for several weeks, with aliquots removed and analyzed by analytical size exclusion chromatography. Analytical size exclusion chromatography was conducted on an Agilent 1260 HPLC, using a Shodex™ K403-4F column (Showa Denko America, Inc., New York, NY) in a mobile phase of 100 mM Sodium Phosphate, 150 mM Sodium Chloride, pH 7.3, flow rate of 0.3 ml/min.

*Results- Differential scanning calorimetry:* DSC can be used to measure the thermal stability of a protein. The best fit Tm values are summarized in Table 31.

**Table 31:** Thermal melting temperature (Tm) values for dAb-Fc molecules as determined by DSC.

| Sample | Tm dAb and CH2 domains | | Tm CH3 domain (°C) |
|---|---|---|---|
| | Tm1 (°C) | Tm2 (°C) | |
| 3h56-269-IgG4.1 | 62.8 | | 69.6 |

(continued)

| Sample | Tm dAb and CH2 domains | | Tm CH3 domain (°C) |
| --- | --- | --- | --- |
| | Tm1 (°C) | Tm2 (°C) | |
| 3h56-269-CT | 55.4 | 60.4 | 83.2 |
| 3h56-269-IgG1.1f | 59.0 | 61.6 | 82.3 |
| 3h56-269-IgG1.3f | 57.0 | 62.8 | 81.9 |
| 3h56-269-IgG1-D265A | 56.4 | 61.4 | 82.4 |

Based on the characteristic thermal denaturation profiles for IgG Fc domains, the Fc CH3 domain transition for 3h56-269-IgG4.1 was assigned as the transition with midpoint (Tm) value of 69.6°C; and the Fc CH3 domain of the various IgG1 molecules was assigned as the transition with Tm near ~82 - 83°C. The denaturation of the dAb domain and CH2 domain for the dAb-Fcs were assigned to the transition(s) below 65°C, which differ between the different constructs, both in the onset of thermal denaturation ($T_{onset}$), the shape of the unfolding transition, and the best fit Tm values. For example, the thermal transition for the dAb and CH2 domains of 3h56-269-IgG4.1 appears as a single overlapping or cooperative transition, with Tm value of 62.8° C. The denaturation profile for the dAb and CH2 domains of 3h56-269-IgG1-D265A, 3h56-269-IgG1.1f and 3h56-269-IgG1.3f are all consistent with a more asymmetrical transition, which was best described by two transitions having Tm values between ~56 - 63° C. 3h56-269-CT had the lowest $T_{onset}$, beginning to unfold near 40° C, with a broad thermal transition and the lowest fitted Tm values of Tm1 = 55.4 °C and Tm2 = 60.4 °C.

*Results - Imaged capillary isoelectric focusing (icIEF):* Imaged capillary isoelectric focusing (icIEF) can be used to characterize sample homogeneity or heterogeneity. The ability to generate a homogeneous product is another important developability criterion. Consequently, during the discovery and optimization of a novel protein therapeutic, various analytical methods are utilized to characterize and quantitate sample heterogeneities, and to select for the most homogeneous molecules.

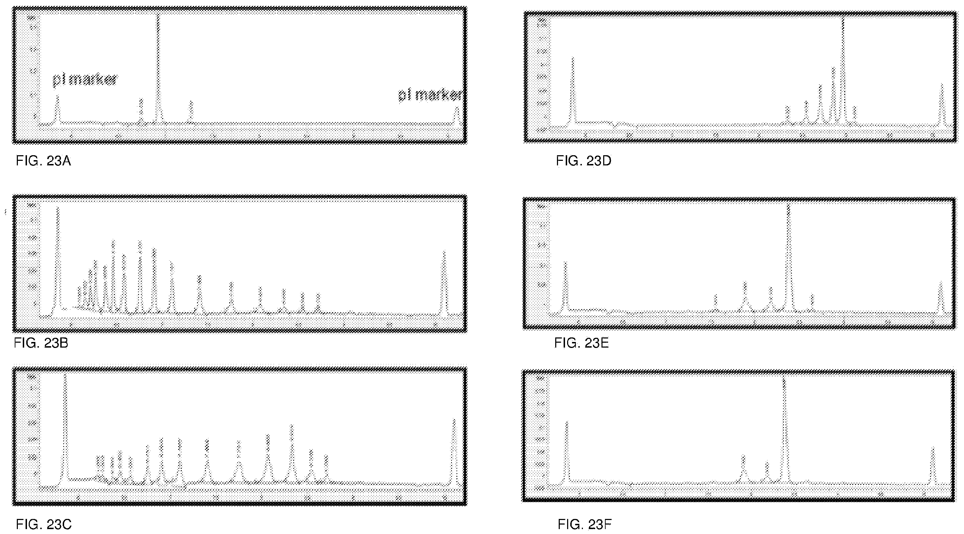

The charge profiles for dAb-Fc molecules were characterized by icIEF. The data are shown in FIG. 23. The icIEF profiles for 3h56-269-IgG4.1 (FIG. 23A), 3h56-269-IgG1.1f (FIG. 23E) and 3h56-269-IgG1.3f (FIG. 23F) are all relatively simple, each consisting of a distinct main peak with area of 69-86%, and between two and four charge variants in lower abundance. This icIEF profile is similar to the typical profile obtained for an antibody. The main peak for 3h56-269-IgG1-D265A (FIG. 23D) is somewhat lower abundance (49%) with a corresponding higher level of acidic variants with at least six detectable species. In contrast, the profile for 3h56-269-CT (FIG. 23B) is highly heterogeneous, consisting of at least 16 different species and no clear main peak. The icIEF profile for 3h56-269-CT expressed in a different cell line (UCOE-CHO) was equally heterogeneous (FIG. 23C), although the distribution of the charge variants was considerably different from the HEK293-expressed material.

*Results- Mass spectrometry:* Typical glycosylation on the Fc domain of IgG or Fc-containing proteins is a mixture of GOF, G1F and some G2F species. Other glycoforms, such as sialylated or non-fucosylated forms, are generally found in much lower abundance or at undetectable levels.

To characterize the glycosylation profiles of the dAb-Fc proteins, and to compare the dAb-Fc proteins to control antibodies with similar Fc mutations, mass spectrometry experiments were conducted. The data are shown in Table 32.

Table 32: Detectable glycoforms in dAb-Fc and antibody molecules as determined by mass spectrometry.

| Sample | G0F | G1F | G2F | G2FS1 | G2FS2 |
| --- | --- | --- | --- | --- | --- |
| 3h56-269-IgG4.1 | 67% | 29% | 4% | | |
| 3h56-269-IgG1.1f | 32% | 58% | 9% | | |
| 3h56-269-IgG1.3f | 42% | 55% | 3% | | |
| 3h56-269-IgG1-D265A | 4% | 37% | 43% | 13% | 2% |
| 1F4-IgG1f | 68% | 32% | | | |
| 1F4-IgG1.3f | 26% | 64% | 10% | | |
| 1F4-D265A | 27% | 40% | 27% | 4% | 2% |

The mass spectrometry data for the control antibodies 1F4-IgG1f and 1F4-IgG1.3f, as well as for dAb-Fc antibodies 3h56-269-IgG4.1, 3h56-269-IgG1.1f, 3h56-269-IgG1.3f, showed that these proteins consist of a typical mixture of GOF, G1F glycoforms, with a lower abundance of G2F species.

Thus, IgGl.3 (heavy chain constant region and Fc) is essentially devoid of binding to CD16, CD32a, CD32b and CD64 and has good biophysical properties. This has also been observed when an IgG1.3 Fc was linked to the variable domain of an anti-TIM3 antibodies (see WO2018/013818). An anti-TIM3 antibody comprising IgG1.3 was shown to have good thermal stability (Tml = 68.1°C, Tm2 = 80.3°C, Tm3 = 82.6°C) and thermal reversibility (95.6 % at 74°C, 25.5 % at 80°C), which suggests that the molecule retains its structural integrity under thermal stress and has robust refolding properties when stress is released.

EP 4 098 662 A1

**SEQUENCE TABLE 33**

| SEQ ID NO | Description | Sequence |
|---|---|---|
| 1 | Full-length IgG1 wild-type | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 2 | CH1 IgG1 wild-type | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKV |
| 3 | Hinge IgG1 wild-type | EPKSCDKTHTCPPCPAPELLGG |
| 4 | CH2 IgG1 wild-type | PSVFLFPPKPKDTLMISRTPEVTCVVVDVSHEDPEVKFNWYVDG VEVHNAKTKPREEQYNSTYRVVSVLTVLHQDWLNGKEYKCKV SNKALPAPIEKTISKAK |
| 5 | CH3 IgG1 wild-type | GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 6 | Full-length IgG2 wild-type | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 7 | CH1 IgG2 wild-type | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTV |
| 8 | Hinge IgG2 wild-type | ERKCCVECPPCPAPPVAG |

(continued)

| SEQ ID NO | Description | Sequence |
|---|---|---|
| 9 | CH2 IgG2 wild-type | PSVFLFPPKPKDTLMISRTPEVTCVVVDVSHEDPEVQFNWYVDG VEVHNAKTKPREEQFNSTFRVVSVLTVVHQDWLNGKEYKCKVS NKGLPAPIEKTISKTK |
| 10 | CH3 IgG2 wild-type | GQPREPQVYTLPPSREEMTKNQVSLTCLVKGFYPSDIAVEWESN GQPENNYKTTPPMLDSDGSFFLYSKLTVDKSRWQQGNVFSCSV MHEALHNHYTQKSLSLSPGK |
| 11 | Full-length IgG3 wild-type | ASTKGPSVFPLAPCSRSTSGGTAALGCLVKDYFPEPVTVSWNSG ALTSGVHTFPAVLQSSGLYSLSSVVTVPSSSLGTQTYTCNVNHKP SNTKVDKRVELKTPLGDTTHTCPRCPEPKSCDTPPPCPRCPEPKS CDTPPPCPRCPEPKSCDTPPPCPRCPAPELLGGPSVFLFPPKPKDT LMISRTPEVTCVVVDVSHEDPEVQFKWYVDGVEVHNAKTKPRE EQYNSTFRVVSVLTVLHQDWLNGKEYKCKVSNKALPAPIEKTIS KTKGQPREPQVYTLPPSREEMTKNQVSLTCLVKGFYPSDIAVEW ESSGQPENNYNTTPPMLDSDGSFFLYSKLTVDKSRWQQGNIFSC SVMHEALHNRFTQKSLSLSPGK |
| 12 | CH1 IgG3 wild-type | ASTKGPSVFPLAPCSRSTSGGTAALGCLVKDYFPEPVTVSWNSG ALTSGVHTFPAVLQSSGLYSLSSVVTVPSSSLGTQTYTCNVNHKP SNTKVDKRV |
| 13 | Hinge IgG3 wild-type | ELKTPLGDTTHTCPRCPE |
| 14 | CH2 IgG3 wild-type | PKSCDTPPPCPRCPEPKSCDTPPPCPRCPEPKSCDTPPPCPRCPAPE LLGGPSVFLFPPKPKDTLMISRTPEVTCVVVDVSHEDPEVQFKW YVDGVEVHNAKTKPREEQYNSTFRVVSVLTVLHQDWLNGKEY KCKVSNKALPAPIEKTISKTK |
| 15 | CH3 IgG3 wild-type | GQPREPQVYTLPPSREEMTKNQVSLTCLVKGFYPSDIAVEWESS GQPENNYNTTPPMLDSDGSFFLYSKLTVDKSRWQQGNIFSCSVM HEALHNRFTQKSLSLSPGK |

(continued)

| SEQ ID NO | Description | Sequence |
|---|---|---|
| 16 | Full-length IgG4 wild-type | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKYGPPCPSCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |
| 17 | CH1 IgG4 wild-type | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRV |
| 18 | Hinge IgG4 wild-type | ESKYGPPCPSCPAPEFLGG |
| 19 | CH2 IgG4 wild-type | PSVFLFPPKPKDTLMISRTPEVTCVVVDVSQEDPEVQFNWYVDG VEVHNAKTKPREEQFNSTYRVVSVLTVLHQDWLNGKEYKCKV SNKGLPSSIEKTISKAK |
| 20 | CH3 IgG4 wild-type | GQPREPQVYTLPPSQEEMTKNQVSLTCLVKGFYPSDIAVEWESN GQPENNYKTTPPVLDSDGSFFLYSRLTVDKSRWQEGNVFSCSVM HEALHNHYTQKSLSLSLGK |
| 21 | Modified IgG2 Hinge (C219S) | ERKSCVECPPCPAPPVAG |
| 22 | IgG2/IgG1 hybrid hinge | ERKCCVECPPCPAPELLGG |
| 23 | IgG2 C219S/IgG1 hybrid hinge | ERKSCVECPPCPAPELLGG |
| 24 | Modified CH2 IgG1 (A330S/P331S) | PSVFLFPPKPKDTLMISRTPEVTCVVVDVSHEDPEVKFNWYVDG VEVHNAKTKPREEQYNSTYRVVSVLTVLHQDWLNGKEYKCKV SNKALPSSIEKTISKAK |
| 25 | IgG1.1 Hinge (L234A/L235E/G237A) | EPKSCDKTHTCPPCPAPEAEGA |

EP 4 098 662 A1

(continued)

| SEQ ID NO | Description | Sequence |
|---|---|---|
| 26 | IgG1-IgG2-IgG1 (IgG1-IgG2/IgG1/IgG1(SEQ#22)-IgG1-IgG1) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVERKCCVECPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 27 | IgG1-IgG2-IgG12 (IgG1-IgG2(SEQ#8)-IgG1-IgG1) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVERKCCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 28 | IgG2-IgG1 (IgG2-IgG2/IgG1(SEQ#22)-IgG1-IgG1) | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |

| SEQ ID NO | Description | Sequence |
|---|---|---|
| 29 | IgG2-IgG 12 (IgG2-IgG2(SEQ#8)-IgG1-IgG1) | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPG |
| 30 | IgG1-IgG2-IgG1.1 (IgG1-IgG2(SEQ#8)-IgG1(A330S/P331S)-IgG1) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVERKCCVECPPCPAPPVAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPSSIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPG |
| 31 | IgG2-IgG1.1 (IgG2-IgG2(SEQ#8)-IgG1(A330S/P331S)-IgG1) | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPSSIEKTISKAKGQ PREPQVYTLPPSREEMTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 32 | IgG1-IgG2CS-IgG1 (IgG1-IgG2(C219S)-IgG1-IgG1) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVERKSCVECPPCPAPPVAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPG |

EP 4 098 662 A1

(continued)

| SEQ ID NO | Description | Sequence |
|---|---|---|
| 33 | IgG1-IgG2CS-IgG12 (IgG1-IgG2(C219S)-IgG1-IgG1) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVERKSCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 34 | IgG2CS-IgG1 (IgG2-IgG2(C219S)-IgG1-IgG1) | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |
| 35 | IgG2CS-IgG12 (IgG2-IgG2(C219S)-IgG1-IgG1) | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG |

| SEQ ID NO | Description | Sequence |
|---|---|---|
| 36 | IgG1-IgG2CS-TgG1.1 (IgG1-IgG2(C219S)-IgG1(A330S/P331S)-IgG1) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVERKSCVECPPCPAPPVAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPSSIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPG |
| 37 | IgG2CS-IgG1.1 (IgG2-IgG2(C219S)-IgG1(A330S/P331S)-IgG1) | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPSSIEKTISKAKGQ PREPQVYTLPPSREEMTKNQVSLTCLVKGFYPSDIAVEWESNGQ PENNYKTTPPVLDSDGSFFLYSKLTVDKSRWQQGNVFSCSVMH EALHNHYTQKSLSLSPG |
| 38 | Ab 11F11 VH | QVQLVESGGGVVQPGRSLRLSCATSGFTFSNYGMHWVRQAPGK GLEWVAVILYDGSNKYYPDSVKGRFTISRDNSKNTLYLQMNSL RAEDTAVYYCARGGSSWYPDSFDIWGQGTMVTVSS |
| 39 | Ab 4C3 VH | EVQLVESGGGLVQPGRSLRLSCAASGFTFDDYAMHWVRQAPGK GLEWVSGISWKSGSIGYADSVKGRFTISRDNAKNSLYLQMNSLR AEDTALYYCVKGYYVILTGLDYWGQGTLVTVSS |
| 40 | Ab CD73.10 VH | QVQLVESGGGVVQPGRSLRLSCAASGFTFSNYGMHWVRQAPG KGLEWVAVIWYDESNKYYPDSVKGRFTISRDNSKNTLYLQMNS LRAEDTAVYYCARGGSSWYPDSFDIWGQGTMVTVSS |
| 41 | Ab CD73.3 VH (4C3 / V94A) | EVQLVESGGGLVQPGRSLRLSCAASGFTFDDYAMHWVRQAPGK GLEWVSGISWKSGSIGYADSVKGRFTISRDNAKNSLYLQMNSLR AEDTVLYYCVKGYYVILTGLDYWGQGTLVTVSS |

| SEQ ID NO | Description | Sequence |
|---|---|---|
| 42 | Ab 6E11 VH | EVQLVESGGALVQPGRSLRLSCAASGFTFDDYAMHWVRQAPGKGLEWVSGITWNSGGIGYADSVKGRFTISRDNAKNSLYLQMNSLRAEDTALYYCAKDRYYSSWLLFDNWGQGILVTVSS |
| 43 | Ab CD73.4 VH | QVQLVESGGGVVQPGRSLRLSCAASGFTFSNYGMHWVRQAPGKGLEWVAVILYDGSNKYYPDSVKGRFTISRDNSKNTLYLQMNSLRAEDTAVYYCARGGSSWYPDSFDIWGQGTMVTVSS |
| 44 | Ab 11F11 full-length HC | QVQLVESGGGVVQPGRSLRLSCATSGFTFSNYGMHWVRQAPGKGLEWVAVILYDGSNKYYPDSVKGRFTISRDNSKNTLYLQMNSLRAEDTAVYYCARGGSSWYPDSFDIWGQGTMVTVSSASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 45 | Ab 4C3 full-length HC | EVQLVESGGGLVQPGRSLRLSCAASGFTFDDYAMHWVRQAPGKGLEWVSGISWKSGSIGYADSVKGRFTISRDNAKNSLYLQMNSLRAEDTALYYCVKGYYVILTGLDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

EP 4 098 662 A1

84

| SEQ ID NO | Description | Sequence |
|---|---|---|
| 46 | Ab 6E11 full-length HC | EVQLVESGGALVQPGRSLRLSCAASGFTFDDYAMHWVRQAPGK GLEWVSGITWNSGGIGYADSVKGRFTISRDNAKNSLYLQMNSLR AEDTALYYCAKDRYYSSWLLFDNWGQGILVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPREP QVYTLPPSRDELTKNQVSLTCLVKGFYPSDIAVEWESNGQPENN YKTTPPVLDSDGSFFLYSKLTVDKSRWQQGNVFSCSVMHEALH NHYTQKSLSLSPGK |
| 47 | Ab CD73.10-IgG2-C219S full-length HC | QVQLVESGGGVVQPGRSLRLSCAASGFTFSNYGMHWVRQAPG KGLEWVAVIWYDESNKYYPDSVKGRFTISRDNSKNTLYLQMNS LRAEDTAVYYCARGGSSWYPDSFDIWGQGTMVTVSSASTKGPS VFPLAPCSRSTSESTAALGCLVKDYFPEPVTVSWNSGALTSGVH TFPAVLQSSGLYSLSSVVTVPSSNFGTQTYTCNVDHKPSNTKVD KTVERKSCVECPPCPAPPVAGPSVFLFPPKPKDTLMISRTPEVTC VVVDVSHEDPEVQFNWYVDGVEVHNAKTKPREEQFNSTFRVVS VLTVVHQDWLNGKEYKCKVSNKGLPAPIEKTISKTKGQPREPQV YTLPPSREEMTKNQVSLTCLVKGFYPSDIAVEWESNGQPENNYK TTPPMLDSDGSFFLYSKLTVDKSRWQQGNVFSCSVMHEALHNH YTQKSLSLSPG |
| 48 | Ab CD73.10-IgG2-C219S-IgG1.1 full-length HC | QVQLVESGGGVVQPGRSLRLSCAASGFTFSNYGMHWVRQAPG KGLEWVAVIWYDESNKYYPDSVKGRFTISRDNSKNTLYLQMNS LRAEDTAVYYCARGGSSWYPDSFDIWGQGTMVTVSSASTKGPS VFPLAPCSRSTSESTAALGCLVKDYFPEPVTVSWNSGALTSGVH TFPAVLQSSGLYSLSSVVTVPSSNFGTQTYTCNVDHKPSNTKVD KTVERKSCVECPPCPAPPVAGPSVFLFPPKPKDTLMISRTPEVTC VVVDVSHEDPEVKFNWYVDGVEVHNAKTKPREEQYNSTYRVV SVLTVLHQDWLNGKEYKCKVSNKALPSSIEKTISKAKGQPREPQ VYTLPPSREEMTKNQVSLTCLVKGFYPSDIAVEWESNGQPENNY KTTPPMLDSDGSFFLYSKLTVDKSRWQQGNVFSCSVMHEALHN HYTQKSLSLSPG |

EP 4 098 662 A1

| SEQ ID NO | Description | Sequence |
|---|---|---|
| 49 | Ab CD73.10-IgG1.1 full-length HC (IgG1.1 - IgG1.1 (L234A/L235E/G237A)-IgG1.1(A330S/P331S)-IgG1.1) | QVQLVESGGGVVQPGRSLRLSCAASGFTFSNYGMHWVRQAPG KGLEWVAVIWYDESNKYYPDSVKGRFTISRDNSKNTLYLQMNS LRAEDTAVYYCARGGSSWYPDSFDIWGQGTMVTVSSASTKGPS VFPLAPSSKSTSGGTAALGCLVKDYFPEPVTVSWNSGALTSGVH TFPAVLQSSGLYSLSSVVTVPSSSLGTQTYICNVNHKPSNTKVDK KVEPKSCDKTHTCPPCPAPEAEGAPSVFLFPPKPKDTLMISRTPE VTCVVVDVSHEDPEVKFNWYVDGVEVHNAKTKPREEQYNSTY RVVSVLTVLHQDWLNGKEYKCKVSNKALPSSIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPG |
| 50 | Ab CD73.4-IgG2-C219S full-length HC | QVQLVESGGGVVQPGRSLRLSCAASGFTFSNYGMHWVRQAPG KGLEWVAVILYDGSNKYYPDSVKGRFTISRDNSKNTLYLQMNS LRAEDTAVYYCARGGSSWYPDSFDIWGQGTMVTVSSASTKGPS VFPLAPCSRSTSESTAALGCLVKDYFPEPVTVSWNSGALTSGVH TFPAVLQSSGLYSLSSVVTVPSSNFGTQTYTCNVDHKPSNTKVD KTVERKSCVECPPCPAPPVAGPSVFLFPPKPKDTLMISRTPEVTC VVVDVSHEDPEVQFNWYVDGVEVHNAKTKPREEQFNSTFRVVS VLTVVHQDWLNGKEYKCKVSNKGLPAPIEKTISKTKGQPREPQV YTLPPSREEMTKNQVSLTCLVKGFYPSDIAVEWESNGQPENNYK TTPPMLDSDGSFFLYSKLTVDKSRWQQGNVFSCSVMHEALHNH YTQKSLSLSPG |

EP 4 098 662 A1

| SEQ ID NO | Description | Sequence |
|---|---|---|
| 51 | Ab CD73.3-IgG1.1 full-length HC (IgG1.1 - IgG1.1(L234A/L235E/G237A)-IgG1.1 (A330S/P331S)-IgG1.1) | EVQLVESGGGLVQPGRSLRLSCAASGFTFDDYAMHWVRQAPGK GLEWVSGISWKSGSIGYADSVKGRFTISRDNAKNSLYLQMNSLR AEDTVLYYCVKGYYVILTGLDYWGQGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPEAEGAPSVFLFPPKPKDTLMISRTPEVTC VVVDVSHEDPEVKFNWYVDGVEVHNAKTKPREEQYNSTYRVV SVLTVLHQDWLNGKEYKCKVSNKALPSSIEKTISKAKGQPREPQ VYTLPPSREEMTKNQVSLTCLVKGFYPSDIAVEWESNGQPENNY KTTPPVLDSDGSFFLYSKLTVDKSRWQQGNVFSCSVMHEALHN HYTQKSLSLSPG |
| 52 | Full-length heavy chain constant region IgG2-IgG2-IgG2-IgG2 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 53 | Full-length heavy chain constant region IgG1-IgG1-IgG1-IgG1 | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |

EP 4 098 662 A1

| SEQ ID NO | Description | Sequence |
|---|---|---|
| 54 | Full-length heavy chain constant region IgG1-IgG1.1 (L234A/L235E/G237A)-IgG1.1 (A330S/P331S)-IgG1 | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPE_AEGA_PSVFLFPPKPKDTL MISRTPEVTCVVVDVSHEDPEVKFNWYVDGVEVHNAKTKPREE QYNSTYRVVSVLTVLHQDWLNGKEYKCKVSNKALP_SS_IEKTISK AKGQPREPQVYTLPPSR_EEM_TKNQVSLTCLVKGFYPSDIAVEWE SNGQPENNYKTTPPVLDSDGSFFLYSKLTVDKSRWQQGNVFSCS VMHEALHNHYTQKSLSLSPG |
| 55 | Full-length heavy chain constant region IgG2-IgG2/IgG1 hybrid-IgG1-IgG1 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPE_LLGG_PSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPG |
| 56 | Full-length heavy chain constant region IgG2-IgG2-IgG1.1(A330/P331S)-IgG1 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALP_SS_IEKTISKAKGQ PREPQVYTLPPSR_EEM_TKNQVSLTCLVKGFYPSDIAVEWESNGQ PENNYKTTPPVLDSDGSFFLYSKLTVDKSRWQQGNVFSCSVMH EALHNHYTQKSLSLSPG |
| 57 | Table 2 - hinge domain | VDKRV |
| 58 | Table 2 - hinge domain | VDKTV |
| 59 | Table 2 - hinge domain | EPKSCDKTHT |
| 60 | Table 2 - hinge domain | ERK |
| 61 | Table 2 - hinge domain | ELKTPLGDTTHT |
| 62 | Table 2 - hinge domain | EPKS |

EP 4 098 662 A1

| SEQ ID NO | Description | Sequence |
|---|---|---|
| 63 | Table 2 - hinge domain | ESKYGPP |
| 64 | Table 2 - hinge domain | CPPCP |
| 65 | Table 2 - hinge domain | CCVECPPCP |
| 66 | Table 2 - hinge domain | CPRCP |
| 67 | Table 2 - hinge domain | EPKSCDTPPCPRCP |
| 68 | Table 2 - hinge domain | CDTPPCPRCP |
| 69 | Table 2 - hinge domain | CPSCP |
| 70 | Table 2 - hinge domain | APELLGG |
| 71 | Table 2 - hinge domain | APPVAG |
| 72 | Light chain 11F11 | DIQMTQSPSSLSASVGDRVTITCRASQGISSWLAWYQQKPEKAP KSLIYAASSLQSGVPSRFSGSGSGTDFTLTISSLQPEDFATYYCQQ YNSYPLTFGGGTKVEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |
| 73 | Light chain 4C3 | EIVLTQSPGTLSLSPGERATLSCRASQSVSSYLAWYQQKPGQAPR LLIYGASSRATGIPDRFSGSGSGTDFTLTISRLEPEDFAVYYCQQY GSSPLTFGGGTKVEIKRTVAAPSVFIFPPSDEQLKSGTASVVCLLN NFYPREAKVQWKVDNALQSGNSQESVTEQDSKDSTYSLSSTLTL SKADYEKHKVYACEVTHQGLSSPVTKSFNRGEC |
| 74 | Light chain 6D11 | EIVLTQSPGTLSLSPGERATLSCRASQSVSSSYLAWYQQKPGQAP RLLIYGASSRATGIPDRFSGSGSGTDFTLTISRLEPEDFAVYYCQH YGSSFTFGPGTKVDIKRTVAAPSVFIFPPSDEQLKSGTASVVCLLN NFYPREAKVQWKVDNALQSGNSQESVTEQDSKDSTYSLSSTLTL SKADYEKHKVYACEVTHQGLSSPVTKSFNRGEC |
| 75 | Anti-GITR AbVH | QVQLVESGGGVVQPGRSLRLSCAASGFTFSSYGMHWVRQAPGK GLEWVAVIWYEGSNKYYADSVKGRFTISRDNSKNTLYLQMNSL RAEDTAVYYCARGGSMVRGDYYYGMDVWGQGTTVTVSS |

| SEQ ID NO | Description | Sequence |
|---|---|---|
| 76 | Anti-GITR Ab VL | AIQLTQSPSSLSASVGDRVTITCRASQGISSALAWYQQKPGKAPK LLIYDASSLESGVPSRFSGSGSGTDFTLTISSLQPEDFATYYCQQF NSYPYTFGQGTKLEIK |
| 77 | Anti-GITR Ab LC | AIQLTQSPSSLSASVGDRVTITCRASQGISSALAWYQQKPGKAPK LLIYDASSLESGVPSRFSGSGSGTDFTLTISSLQPEDFATYYCQQF NSYPYTFGQGTKLEIKRTVAAPSVFIFPPSDEQLKSGTASVVCLL NNFYPREAKVQWKVDNALQSGNSQESVTEQDSKDSTYSLSSTL TLSKADYEKHKVYACEVTHQGLSSPVTKSFNRGEC |
| 78 | IgG1f | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 79 | IgG2.3 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

EP 4 098 662 A1

| SEQ ID NO | Description | Sequence |
|---|---|---|
| 80 | IgG2.3G1-AY | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 81 | IgG2.3G1-KH | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 82 | IgG2.5 | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 83 | IgG1.1f | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPEAEGAPSVFLFPPKPKDTL MISRTPEVTCVVVDVSHEDPEVKFNWYVDGVEVHNAKTKPREE QYNSTYRVVSVLTVLHQDWLNGKEYKCKVSNKALPSSIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |

(continued)

| SEQ ID NO | Description | Sequence |
|---|---|---|
| 84 | IgG2.3G1.1f-KH | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPSSIEKTISKAKGQ PREPQVYTLPPSREEMTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 85 | IgG1-deltaTHT | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 86 | IgG2.3-plusTHT | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVETHTCPPCPAPPVAGPSVFLFPPKPKDTLMI SRTPEVTCVVVDVSHEDPEVQFNWYVDGVEVHNAKTKPREEQF NSTFRVVSVLTVVHQDWLNGKEYKCKVSNKGLPAPIEKTISKTK GQPREPQVYTLPPSREEMTKNQVSLTCLVKGFYPSDIAVEWESN GQPENNYKTTPPMLDSDGSFFLYSKLTVDKSRWQQGNVFSCSV MHEALHNHYTQKSLSLSPGK |

(continued)

| SEQ ID NO | Description | Sequence |
|---|---|---|
| 87 | IgG2.3-plusGGG | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVEGGGCPPCPAPPVAGPSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 88 | IgG2.5G1.1f-KH | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPSSIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 89 | IgG2.5G1-AY | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 90 | IgG2.5G1-KH | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO | Description | Sequence |
|---|---|---|
| 91 | IgG2.5-plusTHT | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVETHTCPPCPAPPVAGPSVFLFPPKPKDTLMI SRTPEVTCVVVDVSHEDPEVQFNWYVDGVEVHNAKTKPREEQF NSTFRVVSVLTVVHQDWLNGKEYKCKVSNKGLPAPIEKTISKTK GQPREPQVYTLPPSREEMTKNQVSLTCLVKGFYPSDIAVEWESN GQPENNYKTTPPMLDSDGSFFLYSKLTVDKSRWQQGNVFSCSV MHEALHNHYTQKSLSLSPGK |
| 92 | IgGl-G2.3Gl-AY | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVERKSCVECPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 93 | IgGl-G2.3Gl-KH | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVERKSCVECPPCPAPPVAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

EP 4 098 662 A1

(continued)

| SEQ ID NO | Description | Sequence |
|---|---|---|
| 94 | G2-G1-G1-G1 | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |
| 95 | G2.5-G1-G1-G1 | ASTKGPSVFPLAPSSRSTSESTAALGCLVKDYFPEPVTVSWNSGALTSGV HTFPAVLQSSGLYSLSSVVTVPSSNFGTQTYTCNVDHKPSNTKVDKTVER KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |

| SEQ ID NO | Description | Sequence |
|---|---|---|
| 96 | G1-G2.3-G2-G2 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEP KSCVECPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 97 | G1-KRGEGSSNLF | ASTKGPSVFPLAPSSRSTSESTAALGCLVKDYFPEPVTVSWNSGALTSGV HTFPAVLQSSGLYSLSSVVTVPSSNFGTQTYICNVNHKPSNTKVDKRVEP KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |

EP 4 098 662 A1

| SEQ ID NO | Description | Sequence |
|---|---|---|
| 98 | G1-KRGEGS | ASTKGPSVFPLAPSSRSTSESTAALGCLVKDYFPEPVTVSWNSGA LTSGV HTFPAVLQSSGLYSLSSVVTVPSSSLGTQTYICNVNHKPSNTKVD KRVEP KSCDKTHTCPPCPAPELLGGPSVFLFPPKPKDTLMISRTPEVTCV VVDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPGK |
| 99 | GI-SNLF | ASTKGPSVFPLAPSSKSTSGGTAALGCLVKDYFPEPVTVSWNSG ALTSGV HTFPAVLQSSGLYSLSSVVTVPSSNFGTQTYICNVNHKPSNTKVD KRVEP KSCDKTHTCPPCPAPELLGGPSVFLFPPKPKDTLMISRTPEVTCV VVDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPGK |

EP 4 098 662 A1

| SEQ ID NO | Description | Sequence |
|---|---|---|
| 100 | IgG1-ITNDRTPR | ASTKGPSVFPLAPSSKSTSGGTAALGCLVKDYFPEPVTVSWNSGALTSGV HTFPAVLQSSGLYSLSSVVTVPSSSLGTQTYTCNVDHKPSNTKVDKTVER KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |
| 101 | G1-SNLFPR | ASTKGPSVFPLAPSSKSTSGGTAALGCLVKDYFPEPVTVSWNSGALTSGV HTFPAVLQSSGLYSLSSVVTVPSSNFGTQTYICNVNHKPSNTKVDKRVER KSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPG |

EP 4 098 662 A1

| SEQ ID NO | Description | Sequence |
|---|---|---|
| 102 | G2-RKEGSGNSFL | ASTKGPSVFPLAPCSKSTSGGTAALGCLVKDYFPEPVTVSWNSGALTSGV HTFPAVLQSSGLYSLSSVVTVPSSSLGTQTYTCNVDHKPSNTKVDKTVER KSCVECPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 103 | G2-RKEGSG | ASTKGPSVFPLAPCSKSTSGGTAALGCLVKDYFPEPVTVSWNSGALTSGV HTFPAVLQSSGLYSLSSVVTVPSSNFGTQTYTCNVDHKPSNTKVDKTVER KSCVECPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |

EP 4 098 662 A1

| SEQ ID NO | Description | Sequence |
|---|---|---|
| 104 | G2-NSFL | ASTKGPSVFPLAPCSRSTSESTAALGCLVKDYFPEPVTVSWNSGA LTSGV HTFPAVLQSSGLYSLSSVVTVPSSSLGTQTYTCNVDHKPSNTKV DKTVER KSCVECPPCPAPPVAGPSVFLFPPKPKDTLMISRTPEVTCVVVDV S HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPMLDSDGSFFLYSKLT VDKSRW QQGNVFSCSVMHEALHNHYTQKSLSLSPGK |
| 105 | IgG2-TIDNTRRP | ASTKGPSVFPLAPCSRSTSESTAALGCLVKDYFPEPVTVSWNSGA LTSGV HTFPAVLQSSGLYSLSSVVTVPSSNFGTQTYICNVNHKPSNTKVD KRVEP KSCVECPPCPAPPVAGPSVFLFPPKPKDTLMISRTPEVTCVVVDV S HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPMLDSDGSFFLYSKLT VDKSRW QQGNVFSCSVMHEALHNHYTQKSLSLSPGK |

EP 4 098 662 A1

| SEQ ID NO | Description | Sequence |
|---|---|---|
| 106 | G2-NSFLRP | ASTKGPSVFPLAPCSRSTSESTAALGCLVKDYFPEPVTVSWNSGALTSGV HTFPAVLQSSGLYSLSSVVTVPSSSLGTQTYTCNVDHKPSNTKVDKTVEP KSCVECPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 107 | G1-G1-G2-G1-AY | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEP KSCDKTHTCPPCPAPELLGGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |

EP 4 098 662 A1

| SEQ ID NO | Description | Sequence |
|---|---|---|
| 108 | G1-G1-G2-G1-KH | ASTKGPSVFPLAPSSKSTSGGTAALGCLVKDYFPEPVTVSWNSG ALTSGV HTFPAVLQSSGLYSLSSVVTVPSSSLGTQTYICNVNHKPSNTKVD KRVEP KSCDKTHTCPPCPAPPVAGPSVFLFPPKPKDTLMISRTPEVTCVV VDVS HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPGK |
| 109 | G2-G2.3-G1-G2-KH | ASTKGPSVFPLAPCSRSTSESTAALGCLVKDYFPEPVTVSWNSGA LTSGV HTFPAVLQSSGLYSLSSVVTVPSSNFGTQTYTCNVDHKPSNTKV DKTVER KSCVECPPCPAPPVAGPSVFLFPPKPKDTLMISRTPEVTCVVVDV S HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPMLDSDGSFFLYSKLT VDKSRW QQGNVFSCSVMHEALHNHYTQKSLSLSPGK |
| 110 | G2.5-G2.3-G1-G2-KH | ASTKGPSVFPLAPSSRSTSESTAALGCLVKDYFPEPVTVSWNSGA LTSGV HTFPAVLQSSGLYSLSSVVTVPSSNFGTQTYTCNVDHKPSNTKV DKTVER |

EP 4 098 662 A1

102

| SEQ ID NO | Description | Sequence |
|---|---|---|
|  |  | KSCVECPPCPAPPVAGPSVFLFPPKPKDTLMISRTPEVTCVVVDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 111 | G2-G2.3-G1-G2-AY | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KSCVECPPCPAPELLGGPSVFLFPPKPKDTLMISRTPEVTCVVVDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPG |

EP 4 098 662 A1

(continued)

| SEQ ID NO | Description | Sequence |
|---|---|---|
| 112 | G2.5-G2.3-G1-G2-AY | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 113 | G1-G2.3-G1-G1-KH | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCVECPPCPAPPVAGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

| SEQ ID NO | Description | Sequence |
|---|---|---|
| 114 | G2-G1-G2-G2-AY | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KSCDKTHTCPPCPAPELLGGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 115 | G2.5-G1-G2-G2-AY | ASTKGPSVFPLAPSSRSTSESTAALGCLVKDYFPEPVTVSWNSGALTSGV HTFPAVLQSSGLYSLSSVVTVPSSNFGTQTYTCNVDHKPSNTKVDKTVER KSCDKTHTCPPCPAPELLGGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |

(continued)

| SEQ ID NO | Description | Sequence |
|---|---|---|
| 116 | G1-G2-G1-G1-AY | ASTKGPSVFPLAPSSKSTSGGTAALGCLVKDYFPEPVTVSWNSG ALTSGV HTFPAVLQSSGLYSLSSVVTVPSSSLGTQTYICNVNHKPSNTKVD KRVEP KSCVECPPCPAPELLGGPSVFLFPPKPKDTLMISRTPEVTCVVVD VS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPGK |
| 117 | G2-G1-G2-G2-KH | ASTKGPSVFPLAPCSRSTSESTAALGCLVKDYFPEPVTVSWNSGA LTSGV HTFPAVLQSSGLYSLSSVVTVPSSNFGTQTYTCNVDHKPSNTKV DKTVER KSCDKTHTCPPCPAPPVAGPSVFLFPPKPKDTLMISRTPEVTCVV VDVS HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPMLDSDGSFFLYSKLT VDKSRW QQGNVFSCSVMHEALHNHYTQKSLSLSPG |

| SEQ ID NO | Description | Sequence |
|---|---|---|
| 118 | G2.5-G1-G2-G2-KH | ASTKGPSVFPLAPSSRSTSESTAALGCLVKDYFPEPVTVSWNSGA LTSGV HTFPAVLQSSGLYSLSSVVTVPSSNFGTQTYTCNVDHKPSNTKV DKTVER KSCDKTHTCPPCPAPPVAGPSVFLFPPKPKDTLMISRTPEVTCVV VDVS HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPMLDSDGSFFLYSKLT VDKSRW<br><br>QQGNVFSCSVMHEALHNHYTQKSLSLSPGK |
| 119 | IgG1-deltaHinge | ASTKGPSVFPLAPSSKSTSGGTAALGCLVKDYFPEPVTVSWNSG ALTSGV HTFPAVLQSSGLYSLSSVVTVPSSSLGTQTYICNVNHKPSNTKVD KRVEP KCPPCPAPELLGGPSVFLFPPKPKDTLMISRTPEVTCVVVDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPGK |

107

EP 4 098 662 A1

| SEQ ID NO | Description | Sequence |
|---|---|---|
| 120 | IgG2-deltaHinge | ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |
| 121 | IgG2.5-deltaHinge | ASTKGPSVFPLAPSSRSTSESTAALGCLVKDYFPEPVTVSWNSGALTSGV HTFPAVLQSSGLYSLSSVVTVPSSNFGTQTYTCNVDHKPSNTKVDKTVER KCPPCPAPPVAGPSVFLFPPKPKDTLMISRTPEVTCVVVDVS HEDPEVQFNWYVDGVEVHNAKTKPREEQFNSTFRVVSVLTVVHQDWLNGK EYKCKVSNKGLPAPIEKTISKTKGQPREPQVYTLPPSREEMTKNQVSLTC LVKGFYPSDIAVEWESNGQPENNYKTTPPMLDSDGSFFLYSKLTVDKSRW QQGNVFSCSVMHEALHNHYTQKSLSLSPGK |

EP 4 098 662 A1

| SEQ ID NO | Description | Sequence |
|---|---|---|
| 122 | IgG1-deltaG237 | ASTKGPSVFPLAPSSKSTSGGTAALGCLVKDYFPEPVTVSWNSG ALTSGV HTFPAVLQSSGLYSLSSVVTVPSSSLGTQTYICNVNHKPSNTKVD KRVEP KSCDKTHTCPPCPAPELLGPSVFLFPPKPKDTLMISRTPEVTCVV VDVS HEDPEVKFNWYVDGVEVHNAKTKPREEQYNSTYRVVSVLTVL HQDWLNGK EYKCKVSNKALPAPIEKTISKAKGQPREPQVYTLPPSREEMTKNQ VSLTC LVKGFYPSDIAVEWESNGQPENNYKTTPPVLDSDGSFFLYSKLT VDKSRW QQGNVFSCSVMHEALHNHYTQKSLSLSPG |
| 123 | IgG2-plusG237 | ASTKGPSVFPLAPCSRSTSESTAALGCLVKDYFPEPVTVSWNSGA LTSGV HTFPAVLQSSGLYSLSSVVTVPSSNFGTQTYTCNVDHKPSNTKV DKTVER KSCVECPPCPAPPVAGGPSVFLFPPKPKDTLMISRTPEVTCVVVD VS HEDPEVQFNWYVDGVEVHNAKTKPREEQFNSTFRVVSVLTVVH QDWLNGK EYKCKVSNKGLPAPIEKTISKTKGQPREPQVYTLPPSREEMTKNQ VSLTC LVKGFYPSDIAVEWESNGQPENNYKTTPPMLDSDGSFFLYSKLT VDKSRW QQGNVFSCSVMHEALHNHYTQKSLSLSPGK |

EP 4 098 662 A1

(continued)

| SEQ ID NO | Description | Sequence |
|---|---|---|
| 124 | IgG2.4 | ASTKGP SVFPLAPCSR STSESTAALG CLVKDYFPEP VTVSWNSGAL TSGVHTFPAV LQSSGLYSLS SVVTVPSSNF GTQTYTCNVD HKPSNTKVDK TVERKCSVEC PPCPAPPVAG PSVFLFPPKP KDTLMISRTP EVTCVVVDVS HEDPEVQFNW YVDGVEVHNA KTKPREEQFN STFRVVSVLT VVHQDWLNGK EYKCKVSNKG LPAPIEKTIS KTKGQPREPQ VYTLPPSREE MTKNQVSLTC LVKGFYPSDI AVEWESNGQP ENNYKTTPPM LDSDGSFFLY SKLTVDKSRW QQGNVFSCSV MHEALHNHYT QKSLSLSPGK |
| 125 | IgG2.3/4 | ASTKGP SVFPLAPCSR STSESTAALG CLVKDYFPEP VTVSWNSGAL TSGVHTFPAV LQSSGLYSLS SVVTVPSSNF GTQTYTCNVD HKPSNTKVDK TVERKSSVEC PPCPAPPVAG PSVFLFPPKP KDTLMISRTP EVTCVVVDVS HEDPEVQFNW YVDGVEVHNA KTKPREEQFN STFRVVSVLT VVHQDWLNGK EYKCKVSNKG LPAPIEKTIS KTKGQPREPQ VYTLPPSREE MTKNQVSLTC LVKGFYPSDI AVEWESNGQP ENNYKTTPPM LDSDGSFFLY SKLTVDKSRW QQGNVFSCSV MHEALHNHYT QKSLSLSPGK |
| 126 | Hinge IgG2 C220S | ERKCSVECPPCPAPPVAG |
| 127 | IgG2/IgG1 hybrid hinge C220S | ERKCSVECPPCPAPELLGG |
| 128 | Wildtype IgG2 hinge portion | ERKCCVECPPCPAP |
| 129 | IgG2 hinge portion C219S | ERKSCVECPPCPAP |
| 130 | IgG2 hinge portion C220S | ERKCSVECPPCPAP |

| SEQ ID NO | Description | Sequence |
|---|---|---|
| 131 | IgG2 hinge portion C219X | ERKXCVECPPCPAP |
| 132 | IgG2 hinge portion C220X | ERKCXVECPPCPAP |
| 133 | IgG2 CH1 +IgG2 hinge (wildtype) | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAG |
| 134 | IgG2 with C219X | ERKXCVECPPCPAPPVAG |
| 135 | IgG2 with C220X | ERKCXVECPPCPAPPVAG |
| 136 | IgG2/IgG1 hybrid with C219X | ERKXCVECPPCPAPELLGG |
| 137 | IgG2/IgG1 hybrid with C220X | ERKCVECPPCPAPELLGG |
| 138 | IgG2/IgG1 hybrid deltaG | ERKCCVECPPCPAPELLG |
| 139 | IgG2/IgG1 hybrid with C219S deltaG | ERKSCVECPPCPAPELLG |
| 140 | IgG2/IgG1 hybrid with C220S deltaG | ERKCSVECPPCPAPELLG |
| 141 | IgG2/IgG1 hybrid with C219X deltaG | ERKXCVECPPCPAPELLG |
| 142 | IgG2/IgG1 hybrid with C220X deltaG | ERKCXVECPPCPAPELLG |
| 143 | Wildtype IgG2 with C-terminal X | ERKCCVECPPCPAPPVAGX |
| 144 | IgG2 with C219S with C-terminal X | ERKSCVECPPCPAPPVAGX |
| 145 | IgG2 with C220S with C-terminal X | ERKCSVECPPCPAPPVAGX |
| 146 | IgG2 with C219X with C-terminal X | ERKXCVECPPCPAPPVAGX |
| 147 | IgG2 with C220X with C-terminal X | ERKCXVECPPCPAPPVAGX |
| 148 | IgG2 hinge portion | PVAG |
| 149 | IgG1 hinge portion | SCDKTHT |
| 150 | IgG1 hinge portion 1 | ELLG |
| 151 | IgG1 hinge portion 2 | ELLGG |

EP 4 098 662 A1

| SEQ ID NO | Description | Sequence |
|---|---|---|
| 152 | IgG2.3-V13 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGDSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 153 | IgG2.3-V14 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGDSVFLFPPKPKDTLMISRTPEVTCVVVDVSHEDGEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 154 | IgG2.3-V15 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGDSVFLFPPKPKDTLMISRTPEVTCVVVDVSDEDGEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 155 | IgG2.3-V16 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGDSVFLFPPKPKDTLMISRTPEVTCVVVDVSHEDGEVQFNWYVDGVEVHNAKTKPREEQFNSTFRVVSVLTVVHQDWLNGKEYKCKVSNKGLPRPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |

(continued)

| SEQ ID NO | Description | Sequence |
|---|---|---|
| 156 | IgG2.3-V17 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGDSVFLFPPKPKDTLMISRTPEVTCVVVDVSDEDGEVQFNWYVDGVEVHNAKTKPREEQFNSTFRVVSVLTVVHQDWLNGKEYKCKVSNKGLPRPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 157 | IgG2.3-V18 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGPSVFLFPPKPKDTLMISRTPEVTCVVVDVEHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 158 | IgG2.3-V19 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPPVAGPSVFLFPPKPKDTLMISRTPEVTCVVVDVEHEDPEVQFNWYVDGVEVHNAKTKPREEQFNSTFRVVSVLTVVHQDWLNGKEYKCKVSNKGFPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 159 | IgG2.3G1 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO | Description | Sequence |
|---|---|---|
| 160 | IgG2.3G1-V20 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGDSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 161 | IgG2.3G1-V21 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGDSVFLFPPKPKDTLMISRTPEVTCVVVDVSHEDGEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSKLTVDKSRWQQGNVFSCSVMHEALHNHYTQKSLSLSPGK |
| 162 | IgG2.3G1-V22 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGDSVFLFPPKPKDTLMISRTPEVTCVVVDVSDEDGEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 163 | IgG2.3G1-V23 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGDSVFLFPPKPKDTLMISRTPEVTCVVVDVSHEDGEVKFNWYVDGVEVHNAKTKPREEQYNSTYRVVSVLTVLHQDWLNGKEYKCKVSNKALPRPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

114

(continued)

| SEQ ID NO | Description | Sequence |
|---|---|---|
| 164 | IgG2.3G1-V24 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGDSVFLFPPKPKDTLMISR TPEVTCVVVDVSDEDGEVKFNWYVDGVEVHNAKTKPREEQYN STYRVVSVLTVLHQDWLNGKEYKCKVSNKALPRPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 165 | IgG2.3G1-V25 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGDDSVFLFPPKPKDTLMISR TPEVTCVVVDVSDEDGEVKFNWYVDGVEVHNAKTKPREEQYN STYRVVSVLTVLHQDWLNGKEYKCKVSNKALPRPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 166 | IgG2.3G1-V26 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPDLLGDDSVFLFPPKPKDTLMISR TPEVTCVVVDVSDEDGEVKFNWYVDGVEVHNAKTKPREEQYN STYRVVSVLTVLHQDWLNGKEYKCKVSNKALPRPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 167 | IgG2.3G1-V27 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGPSVFLFPPKPKDTLMISR TPEVTCVVVDVEHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

(continued)

| SEQ ID NO | Description | Sequence |
|---|---|---|
| 168 | IgG2.3G1-V28 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGGPSVFLFPPKPKDTLMISRTPEVTCVVVDVEHEDPEVKFNWYVDGVEVHNAKTKPREEQYNSTYRVVSVLTVLHQDWLNGKEYKCKVSNKAFPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 169 | IgG2.3G1-AY-V9-D270E | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGDDSVFLFPPKPKDTLMISRTPEVTCVVVDVSHEEGEVKFNWYVDGVEVHNAKTKPREEQYNSTYRVVSVLTVLHQDWLNGKEYKCKVSNKALPRPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 170 | IgG2.3G1-AY-V11 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELLGDDSVFLFPPKPKDTLMISRTPEVTCVVVDVSDEDGEVKFNWYVDGVEVHNAKTKPREEQYNSTYRVVSVLTVLHQDWLNGKEYKCKVSNKALPRPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 171 | IgG2.5G1-AY-V9-D270E | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPELLGDDSVFLFPPKPKDTLMISRTPEVTCVVVDVSHEEGEVKFNWYVDGVEVHNAKTKPREEQYNSTYRVVSVLTVLHQDWLNGKEYKCKVSNKALPRPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO | Description | Sequence |
|---|---|---|
| 172 | IgG2.5G1-AY-V11 | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGDDSVFLFPPKPKDTLMISR TPEVTCVVVDVSDEDGEVKFNWYVDGVEVHNAKTKPREEQYN STYRVVSVLTVLHQDWLNGKEYKCKVSNKALPRPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 173 | IgG1f-GASDALIE | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLAGPDVFLFPPKPKDTL MISRTPEVTCVVVDVSHEDPEVKFNWYVDGVEVHNAKTKPREE QYNSTYRVVSVLTVLHQDWLNGKEYKCKVSNKALPLPEEKTIS KAKGQPREPQVYTLPPSREEMTKNQVSLTCLVKGFYPSDIAVEW ESNGQPENNYKTTPPVLDSDGSFFLYSKLTVDKSRWQQGNVFSC SVMHEALHNHYTQKSLSLSPGK |
| 174 | IgGlf-G236A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLAGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 175 | IgG2.3G1-AY-G236A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

| SEQ ID NO | Description | Sequence |
|---|---|---|
| 176 | IgG2.3G 1 -AY-GASDALIE | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLAGPDVFLFPPKPKDTLMISR |
| | | TPEVTCVVVDVSHEDPEVKFNWYVDGVEVHNAKTKPREEQYN STYRVVSVLTVLHQDWLNGKEYKCKVSNKALPLPEEKTISKAK GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 177 | IgG2.5G1-AY-G236A | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLAGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 178 | IgG2.5G 1 -AY-GASDALIE | STKGPSVFPLAPSSRSTSESTAALGCLVKDYFPEPVTVSWNSGAL TSGVHTFPAVLQSSGLYSLSSVVTVPSSNFGTQTYTCNVDHKPSN TKVDKTVERKCCVECPPCPAPELLAGPDVFLFPPKPKDTLMISRT PEVTCVVVDVSHEDPEVKFNWYVDGVEVHNAKTKPREEQYNS TYRVVSVLTVLHQDWLNGKEYKCKVSNKALPLPEEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

| SEQ ID NO | Description | Sequence |
|---|---|---|
| 179 | IgG2.3G1.1f-AY | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPSSIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 180 | IgG2.3G1.3f-AY | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 181 | IgG2.3G1-AY-D265A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGPSVFLFPPKPKDTLMISR TPEVTCVVVAVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 182 | IgG2.3G1-AY-N297A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGPSVFLFPPKPKDTLMISR TPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

| SEQ ID NO | Description | Sequence |
|---|---|---|
| 183 | IgG2.5G1.1f-AY | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPSSIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 184 | IgG2.5G1.3f-AY | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 185 | IgG2.5G1-AY-D265A | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGGPSVFLFPPKPKDTLMISR TPEVTCVVVAVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

EP 4 098 662 A1

| SEQ ID NO | Description | Sequence |
|---|---|---|
| 186 | IgG2.5G1-AY-N297A | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGGPSVFLFPPKPKDTLMISR TPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 187 | CT | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTSPPSPAPELLGGSSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 188 | CTf | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTSPPSPAPELLGGSSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 189 | IgG2.3-CT | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVESPPSPAPELLGGSSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |

(continued)

| SEQ ID NO | Description | Sequence |
|---|---|---|
| 190 | IgG2.5-CT | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVESPPSPAPELLGGSSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 191 | IgG1fa-C226S | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTSPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 192 | IgG1fa-C229S | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPSPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 193 | IgG1fa-C226S ,C229S | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTSPPSPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

| SEQ ID NO | Description | Sequence |
|---|---|---|
| 194 | IgG1fa-P238S | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLGGSSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 195 | IgG1fa-C226A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTAPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 196 | IgG1fa-C229A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPAPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 197 | IgG1fa-C226A,C229A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTAPPAPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |

EP 4 098 662 A1

| SEQ ID NO | Description | Sequence |
|---|---|---|
| 198 | IgG1 fa-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLGGKSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 199 | IgG2.3-R133K | ASTKGPSVFPLAPCSKSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 200 | IgG2.3-E137G | ASTKGPSVFPLAPCSRSTSGSTAALGCLVKDYFPEPVTVSWNSG ALTSGVHTFPAVLQSSGLYSLSSVVTVPSSNFGTQTYTCNVDHK PSNTKVDKTVERKSCVECPPCPAPPVAGPSVFLFPPKPKDTLMIS RTPEVTCVVVDVSHEDPEVQFNWYVDGVEVHNAKTKPREEQFN STFRVVSVLTVVHQDWLNGKEYKCKVSNKGLPAPIEKTISKTKG QPREPQVYTLPPSREEMTKNQVSLTCLVKGFYPSDIAVEWESNG QPENNYKTTPPMLDSDGSFFLYSKLTVDKSRWQQGNVFSCSVM HEALHNHYTQKSLSLSPGK |
| 201 | IgG2.3-S138G | ASTKGPSVFPLAPCSRSTSEGTAALGCLVKDYFPEPVTVSWNSG ALTSGVHTFPAVLQSSGLYSLSSVVTVPSSNFGTQTYTCNVDHK PSNTKVDKTVERKSCVECPPCPAPPVAGPSVFLFPPKPKDTLMIS RTPEVTCVVVDVSHEDPEVQFNWYVDGVEVHNAKTKPREEQFN STFRVVSVLTVVHQDWLNGKEYKCKVSNKGLPAPIEKTISKTKG QPREPQVYTLPPSREEMTKNQVSLTCLVKGFYPSDIAVEWESNG QPENNYKTTPPMLDSDGSFFLYSKLTVDKSRWQQGNVFSCSVM HEALHNHYTQKSLSLSPGK |

| SEQ ID NO | Description | Sequence |
|---|---|---|
| 202 | IgG2.3-E137G-S138G | ASTKGPSVFPLAPCSRSTSGGTAALGCLVKDYFPEPVTVSWNSG ALTSGVHTFPAVLQSSGLYSLSSVVTVPSSNFGTQTYTCNVDHK PSNTKVDKTVERKSCVECPPCPAPPVAGPSVFLFPPKPKDTLMIS RTPEVTCVVVDVSHEDPEVQFNWYVDGVEVHNAKTKPREEQFN STFRVVSVLTVVHQDWLNGKEYKCKVSNKGLPAPIEKTISKTKG QPREPQVYTLPPSREEMTKNQVSLTCLVKGFYPSDIAVEWESNG QPENNYKTTPPMLDSDGSFFLYSKLTVDKSRWQQGNVFSCSVM HEALHNHYTQKSLSLSPGK |
| 203 | IgG2.3-T214R | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKRVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 204 | IgG2.3-R217P | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEPKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 205 | IgG2.3-R217S | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVESKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

| SEQ ID NO | Description | Sequence |
|---|---|---|
| 206 | IgG2.3-V224A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCAECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 207 | IgG2.3-E225A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVACPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 208 | IgG2.3-R133A | ASTKGPSVFPLAPCSASTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 209 | IgG2.3-E137D | ASTKGPSVFPLAPCSRSTSDSTAALGCLVKDYFPEPVTVSWNSG ALTSGVHTFPAVLQSSGLYSLSSVVTVPSSNFGTQTYTCNVDHK PSNTKVDKTVERKSCVECPPCPAPPVAGPSVFLFPPKPKDTLMIS RTPEVTCVVVDVSHEDPEVQFNWYVDGVEVHNAKTKPREEQFN STFRVVSVLTVVHQDWLNGKEYKCKVSNKGLPAPIEKTISKTKG QPREPQVYTLPPSREEMTKNQVSLTCLVKGFYPSDIAVEWESNG QPENNYKTTPPMLDSDGSFFLYSKLTVDKSRWQQGNVFSCSVM HEALHNHYTQKSLSLSPGK |

| SEQ ID NO | Description | Sequence |
|---|---|---|
| 210 | IgG2.3-E137Q | ASTKGPSVFPLAPCSRSTSQSTAALGCLVKDYFPEPVTVSWNSG ALTSGVHTFPAVLQSSGLYSLSSVVTVPSSNFGTQTYTCNVDHK PSNTKVDKTVERKSCVECPPCPAPPVAGPSVFLFPPKPKDTLMIS RTPEVTCVVVDVSHEDPEVQFNWYVDGVEVHNAKTKPREEQFN STFRVVSVLTVVHQDWLNGKEYKCKVSNKGLPAPIEKTISKTKG QPREPQVYTLPPSREEMTKNQVSLTCLVKGFYPSDIAVEWESNG QPENNYKTTPPMLDSDGSFFLYSKLTVDKSRWQQGNVFSCSVM HEALHNHYTQKSLSLSPGK |
| 211 | IgG2.3-S138T | ASTKGPSVFPLAPCSRSTSETTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 212 | IgG2.3-S138E | ASTKGPSVFPLAPCSRSTSEETAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 213 | IgG2.3-E137A-S1381 | ASTKGPSVFPLAPCSRSTSAITAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

| SEQ ID NO | Description | Sequence |
|---|---|---|
| 214 | IgG2.3-E137I-S 138A | ASTKGPSVFPLAPCSRSTSIATAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 215 | IgG2.3-R217G | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEGKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 216 | IgG2.3-R217A | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEAKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 217 | IgG2.3-R217I | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEIKSCVECPPCPAPPVAGPSVFLFPPKPKDTLMISRTP EVTCVVVDVSHEDPEVQFNWYVDGVEVHNAKTKPREEQFNSTF RVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

EP 4 098 662 A1

(continued)

| SEQ ID NO | Description | Sequence |
|---|---|---|
| 218 | IgG2.3-R217E | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEEKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 219 | IgG2.3-R217K | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVEKKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 220 | IgG2.3-V224I | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCIECPPCPAPPVAGPSVFLFPPKPKDTLMISRTP EVTCVVVDVSHEDPEVQFNWYVDGVEVHNAKTKPREEQFNSTF RVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 221 | IgG2.3-E225D | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVDCPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

EP 4 098 662 A1

129

| SEQ ID NO | Description | Sequence |
|---|---|---|
| 222 | IgG2-G4.1-G4-G4 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKYGPPCPPCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |
| 223 | IgG4-G2.3-G2-G2 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 224 | IgG2-G4.1-G2-G2 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKYGPPCPPCPAPEFLGGPSVFLFPPKPKDTLMISR TPEVTCVVVDVSHEDPEVQFNWYVDGVEVHNAKTKPREEQFNS TFRVVSVLTVVHQDWLNGKEYKCKVSNKGLPAPIEKTISKTKGQ PREPQVYTLPPSREEMTKNQVSLTCLVKGFYPSDIAVEWESNGQ PENNYKTTPPMLDSDGSFFLYSKLTVDKSRWQQGNVFSCSVMH EALHNHYTQKSLSLSPGK |
| 225 | IgG4-G2.3-G4-G4 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKSCVECPPCPAPPVAGPSVFLFPPKPKDTLMISRT PEVTCVVVDVSQEDPEVQFNWYVDGVEVHNAKTKPREEQFNST YRVVSVLTVLHQDWLNGKEYKCKVSNKGLPSSIEKTISKAKGQP REPQVYTLPPSQEEMTKNQVSLTCLVKGFYPSDIAVEWESNGQP ENNYKTTPPVLDSDGSFFLYSRLTVDKSRWQEGNVFSCSVMHE ALHNHYTQKSLSLSLGK |

| SEQ ID NO | Description | Sequence |
|---|---|---|
| 226 | IgG2-G2.3-G4-G4 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSQEDPEVQFNWYVDGVEVHNAKTKPREEQFNST YRVVSVLTVLHQDWLNGKEYKCKVSNKGLPSSIEKTISKAKGQP REPQVYTLPPSQEEMTKNQVSLTCLVKGFYPSDIAVEWESNGQP ENNYKTTPPVLDSDGSFFLYSRLTVDKSRWQEGNVFSCSVMHE ALHNHYTQKSLSLSLGK |
| 227 | IgG4-G4.1-G2-G2 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKYGPPCPPCPAPEFLGGPSVFLFPPKPKDTLMISR TPEVTCVVVDVSHEDPEVQFNWYVDGVEVHNAKTKPREEQFNS TFRVVSVLTVVHQDWLNGKEYKCKVSNKGLPAPIEKTISKTKGQ PREPQVYTLPPSREEMTKNQVSLTCLVKGFYPSDIAVEWESNGQ PENNYKTTPPMLDSDGSFFLYSKLTVDKSRWQQGNVFSCSVMH EALHNHYTQKSLSLSPGK |
| 228 | IgG4-G4.1-G1-G1 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKYGPPCPPCPAPEFLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 229 | IgG4.1 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKYGPPCPPCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |

EP 4 098 662 A1

131

| SEQ ID NO | Description | Sequence |
|---|---|---|
| 230 | IgG4.1-R214T | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKTVESKYGPPCPPCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |
| 231 | IgG4.1-S217R | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVERKYGPPCPPCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |
| 232 | IgG4.1-S217P | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVEPKYGPPCPPCPAPEFLGGPSVFLFPPKPKDTLMISR TPEVTCVVVDVSQEDPEVQFNWYVDGVEVHNAKTKPREEQFNS TYRVVSVLTVLHQDWLNGKEYKCKVSNKGLPSSIEKTISKAKGQ PREPQVYTLPPSQEEMTKNQVSLTCLVKGFYPSDIAVEWESNGQ PENNYKTTPPVLDSDGSFFLYSRLTVDKSRWQEGNVFSCSVMHE ALHNHYTQKSLSLSLGK |
| 233 | IgG1fa | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPG |

EP 4 098 662 A1

| SEQ ID NO | Description | Sequence |
|---|---|---|
| 234 | IgG1.3fa | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPEAEGAPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 235 | IgG1 fa-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELLGGKSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 236 | IgG 1.3fa-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPEAEGAKSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTIS KAKGQPREPQVYTLPPSRDELTKNQVSLTCLVKGFYPSDIAVEW ESNGQPENNYKTTPPVLDSDGSFFLYSKLTVDKSRWQQGNVFSC SVMHEALHNHYTQKSLSLSPGK |
| 237 | IgG1fa-L235E-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELEGGKSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |

| SEQ ID NO | Description | Sequence |
|---|---|---|
| 238 | IgG1fa-L235A-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELAGGKSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTIS KAKGQPREPQVYTLPPSRDELTKNQVSLTCLVKGFYPSDIAVEW ESNGQPENNYKTTPPVLDSDGSFFLYSKLTVDKSRWQQGNVFSC SVMHEALHNHYTQKSLSLSPGK |
| 239 | IgG1fa-L235E-P238K-K322A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELEGGKSVFLFPPKPKDTL MISRTPEVTCVVVDVSHEDPEVKFNWYVDGVEVHNAKTKPREE QYNSTYRVVSVLTVLHQDWLNGKEYKCAVSNKALPAPIEKTISK AKGQPREPQVYTLPPSRDELTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 240 | IgG2.3 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGPSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |
| 241 | IgG2.3-P238K | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPPVAGKSVFLFPPKPKDTLMISRT PEVTCVVVDVSHEDPEVQFNWYVDGVEVHNAKTKPREEQFNST FRVVSVLTVVHQDWLNGKEYKCKVSNKGLPAPIEKTISKTKGQP REPQVYTLPPSREEMTKNQVSLTCLVKGFYPSDIAVEWESNGQP ENNYKTTPPMLDSDGSFFLYSKLTVDKSRWQQGNVFSCSVMHE ALHNHYTQKSLSLSPGK |

| SEQ ID NO | Description | Sequence |
|---|---|---|
| 242 | IgG2.3G1 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 243 | IgG2.3G1-P238K | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELLGGKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 244 | IgG2.3G1-L235E-P238K | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPELEGGKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 245 | IgG2.5G1-P238K | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGGKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

EP 4 098 662 A1

(continued)

| SEQ ID NO | Description | Sequence |
|---|---|---|
| 246 | hIgG1f | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 247 | hIgG1f-P238K | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGKSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 248 | hIgG1.3f | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAEGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 249 | hIgG1.3f-P238K | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAEGAKSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

EP 4 098 662 A1

| SEQ ID NO | Description | Sequence |
|---|---|---|
| 250 | hIgG1f-L235E-P238K | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELEGGKSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 251 | hIgG1f-L235E-P23SK-K322A | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKRVEPKSCDKTHTCPPCPAPELEGGKSVFLFPPKPKDTL MISRTPEVTCVVVDVSHEDPEVKFNWYVDGVEVHNAKTKPREE QYNSTYRVVSVLTVLHQDWLNGKEYKCAVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK |
| 252 | IgG2.3G1.3f | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 253 | IgG2.3G1.3f-P238K | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKSCVECPPCPAPEAEGAKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |

(continued)

| SEQ ID NO | Description | Sequence |
|---|---|---|
| 244 | IgG2.3G1-L235E-P238K | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELEGGKSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 254 | IgG2.3G1-L235E-P238K-K322A | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKSCVECPPCPAPELEGGKSVFLFPPKPKDTLMISRTPEVTCVVVDVSHEDPEVKFNWYVDGVEVHNAKTKPREEQYNSTYRVVSVLTVLHQDWLNGKEYKCAVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 255 | IgG2.5 | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| 256 | IgG2.5-P238K | ASTKGPSVFPLAPSSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGKSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |

| SEQ ID NO | Description | Sequence |
|---|---|---|
| 257 | IgG2.5G1 | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 258 | IgG2.5G1-P238K | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELLGGKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 259 | IgG2.5G1.3f | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPEAEGAPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 260 | IgG2.5G1.3f-P238K | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPEAEGAKSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |

EP 4 098 662 A1

| SEQ ID NO | Description | Sequence |
|---|---|---|
| 261 | IgG2.5G1-L235E-P238K | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELEGGKSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 262 | IgG2.5G 1-L235E-P238K-K322A | ASTKGPSVFPLAPSSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPELEGGKSVFLFPPKPKDTLMISR TPEVTCVVVDVSHEDPEVKFNWYVDGVEVHNAKTKPREEQYN STYRVVSVLTVLHQDWLNGKEYKCAVSNKALPAPIEKTISKAKG QPREPQVYTLPPSREEMTKNQVSLTCLVKGFYPSDIAVEWESNG QPENNYKTTPPVLDSDGSFFLYSKLTVDKSRWQQGNVFSCSVM HEALHNHYTQKSLSLSPGK |
| 263 | 3h56-269-IgG4.1 OR BMS-986090 | EVQLLESGGGLVQPGGSLRLSCAASGFTFRDYEMWWVRQAPGKGLER VSA INPQGTRTYYADSVKGRFTISRDNSKNTLYLQMNSLRAEDTAVYYCAKLP FRFSDRGQGTLVTVSSASTESKYGPPCPPCPAPEFLGGPSVFLFPPKPKD TLMISRTPEVTCVVVDVSQEDPEVQFNWYVDGVEVHNAKTKPREEQF NST YRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVL D SDGSFFLYSRLTVDKSRWQEGNVFSCSVMHEALHNHYTQKSLSLSLGK |

| SEQ ID NO | Description | Sequence |
|---|---|---|
| 264 | 3h56-269-CT | EVQLLESGGGLVQPGGSLRLSCAASGFTFRDYEMWWVRQAPGKGLER<br>VSA<br>INPQGTRTYYADSVKGRFTISRDNSKNTLYLQMNSLRAEDTAVYYCAKLP<br>FRFSDRGQGTLVTVSSASTEPKSSDKTHTSPPSPAPELLGGSSVFLFPPK<br>PKDTLMISRTPEVTCVVVDVSHEDPEVKFNWYVDGVEVHNAKTKPREE<br>QY<br>NSTYRVVSVLTVLHQDWLNGKEYKCKVSNKALPAPIEKTISKAKGQPREP<br>QVYTLPPSRDELTKNQVSLTCLVKGFYPSDIAVEWESNGQPENNYKTTP<br>P<br>VLDSDGSFFLYSKLTVDKSRWQQGNVFSCSVMHEALHNHYTQKSLSLSP<br>GK |
| 265 | 3h56-269-IgG1.1f | EVQLLESGGGLVQPGGSLRLSCAASGFTFRDYEMWWVRQAPGKGLER<br>VSAINPQGTRTYYADSVKGRFTISRDNSKNTLYLQMNSLRAEDTAVYYC<br>AKLPFRFSDRGQGTLVTVSSASTEPKSCDKTHTCPPCPAPEAEGAPSVFL<br>FPPKPKDTLMISRTPEVTCVVVDVSHEDPEVKFNWYVDGVEVHNAKTK<br>PREEQYNSTYRVVSVLTVLHQDWLNGKEYKCKVSNKALPSSIEKTISKAK<br>GQPREPQVYTLPPSREEMTKNQVSLTCLVKGFYPSDIAVEWESNGQPE<br>NNYKTTPPVLDSDGSFFLYSKLTVDKSRWQQGNVFSCSVMHEALHNHY<br>TQKSLSLSPGK |
| 266 | 3h56-269-IgG1.3f | EVQLLESGGGLVQPGGSLRLSCAASGFTFRDYEMWWVRQAPGKGLER<br>VSAINPQGTRTYYADSVKGRFTISRDNSKNTLYLQMNSLRAEDTAVYYC<br>AKLPFRFSDRGQGTLVTVSSASTEPKSCDKTHTCPPCPAPEAEGAPSVFL<br>FPPKPKDTLMISRTPEVTCVVVDVSHEDPEVKFNWYVDGVEVHNAKTK<br>PREEQYNSTYRVVSVLTVLHQDWLNGKEYKCKVSNKALPAPIEKTISKAK<br>GQPREPQVYTLPPSREEMTKNQVSLTCLVKGFYPSDIAVEWESNGQPE<br>NNYKTTPPVLDSDGSFFLYSKLTVDKSRWQQGNVFSCSVMHEALHNHY<br>TQKSLSLSPGK |

| SEQ ID NO | Description | Sequence |
|---|---|---|
| 267 | 3h56-269-IgG1-D265A | EVQLLESGGGLVQPGGSLRLSCAASGFTFRDYEMWWVRQAPGKGLER VSAINPQGTRTYYADSVKGRFTISRDNSKNTLYLQMNSLRAEDTAVYYC AKLPFRFSDRGQGTLVTVSSASTEPKSCDKTHTCPPCPAPELLGGPSVFLF PPKPKDTLMISRTPEVTCVVVAVSHEDPEVKFNWYVDGVEVHNAKTKP REEQYNSTYRVVSVLTVLHQDWLNGKEYKCKVSNKALPAPIEKTISKAKG QPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 268 | 1F4 Heavy chain variable region and CH1 | EVQLLESGGGLVQPGGSLRLSCAASGFTFSIYAMSWVRQA PGKGLEWVSAISDSGGRTYFADSVRGRFTISRDNSKNTLSL QMNSLRAEDTAVYYCAKVDYSNYLFFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRV |
| 269 | 1F4 Light chain variable region and CL | EIVLTQSPGTLSLSPGERATLSCRASQSISSSYLAWYQQKPG QAPRLLIYGASSRATGIPDRFSGSGSGTDFTLTISRLEPEDF AVYYCQQYGSSPYTFGQGTKLEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

EP 4 098 662 A1

142

EP 4 098 662 A1

| SEQ ID NO | Description | Sequence |
|---|---|---|
| 270 | 1F4-IgG1f heavy chain | EVQLLESGGGLVQPGGSLRLSCAASGFTFSIYAMSWVRQA PGKGLEWVSAISDSGGRTYFADSVRGRFTISRDNSKNTLSL QMNSLRAEDTAVYYCAKVDYSNYLFFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPGK |
| 271 | 1F4-IgG4.1 heavy chain | EVQLLESGGGLVQPGGSLRLSCAASGFTFSIYAMSWVRQA PGKGLEWVSAISDSGGRTYFADSVRGRFTISRDNSKNTLSL QMNSLRAEDTAVYYCAKVDYSNYLFFDYWGQGTLVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEY KCKVSNKGLPSSIEKTISKAKGQPREPQVYTLPPSQEEMTK NQVSLTCLVKGFYPSDIAVEWESNGQPENNYKTTPPVLDS DGSFFLYSRLTVDKSRWQEGNVFSCSVMHEALHNHYTQK SLSLSLG |

143

| SEQ ID NO | Description | Sequence |
|---|---|---|
| 272 | 1F4-IgG1.1f heavy chain | EVQLLESGGGLVQPGGSLRLSCAASGFTFSIYAMSWVRQA PGKGLEWVSAISDSGGRTYFADSVRGRFTISRDNSKNTLSL QMNSLRAEDTAVYYCAKVDYSNYLFFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRVEPKSCDKTHTCPPCPAPEAEGA PSVFLFPPKPKDTLMISRTPEVTCVVVDVSHEDPEVKFNW YVDGVEVHNAKTKPREEQYNSTYRVVSVLTVLHQDWLN GKEYKCKVSNKALPSSIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPG |
| 273 | 1F4-IgG1.3f heavy chain | EVQLLESGGGLVQPGGSLRLSCAASGFTFSIYAMSWVRQA PGKGLEWVSAISDSGGRTYFADSVRGRFTISRDNSKNTLSL QMNSLRAEDTAVYYCAKVDYSNYLFFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRVEPKSCDKTHTCPPCPAPEAEGA PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPG |

EP 4 098 662 A1

(continued)

| SEQ ID NO | Description | Sequence |
|---|---|---|
| 274 | 1F4-D265A heavy chain | EVQLLESGGGGLVQPGGSLRLSCAASGFTFSIYAMSWVRQAGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIVEWESNGQPENNYKTTPPVLDSDGSFFLYSKLTVDKSRWQQGNVFSCSVMHEALHNHYTQKSLSLSPGK |
| 275 | 1F4-CT heavy chain | EVQLLESGGGGLVQPGGSLRLSCAASGFTFSIYAMSWVRQAPGKGLEWVSAISDSGGRTYFADSVRGRFTISRDNSKNTLSLQMNSLRAEDTAVYYCAKVDYSNYLFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTSPPSPAPELLGGSSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

Those skilled in the art will recognize or be able to ascertain, using no more than routine experimentation, many equivalents of the specific embodiments described herein described herein. Such equivalents are intended to be encompassed by the following clauses

The following numbered clauses are part of the description:

1. An antibody comprising a modified heavy chain constant region, wherein the modified heavy chain constant region comprises a CH1 domain, a hinge, a CH2 domain, and a CH3 domain in order from N- to C- terminus, wherein the hinge is of an IgG2 isotype and at least one of the CH1, CH2, or CH3 domains is not of an IgG2 isotype.

2. The antibody of clause 1, wherein the hinge is a wildtype human IgG2 hinge, or comprises an amino acid sequence that is at least 95% identical to the amino acid sequence of a wildtype human IgG2 hinge.

3. The antibody of clause 1 or 2, wherein the hinge contains one or more modifications that reduces disulfide bond formation.

4. The antibody of any one of the preceding clauses , wherein the hinge comprises the amino acid substitution C219S.

5. The antibody of any one of the preceding clauses , wherein the hinge comprises the amino acid sequence of any one of SEQ ID NO: 8, 21-23, 126-132 or 134-147 or a sequence that comprises 1-3 amino acids inserted between CVE and CPP.

6. The antibody of any one of the preceding clauses , wherein the CH1 domain is an IgG2 CH1 domain.

7. The antibody of any one of the preceding clauses , wherein the CH1 domain is a wildtype human IgG2 CH1 domain, or comprises an amino acid sequence that is at least 95% identical to the amino acid sequence of a wildtype human IgG2 CH1 domain.

8. The antibody of any one of the preceding clauses , wherein the IgG2 CH1 domain comprises the amino acid sequence

ASTKGPSVFPLAPCSRSTSESTAALGCLVKDYFPEPVTVSWNSGALTSGVHTFPAVLQSS

GLYSLSSVVTVPSSNFGTQTYTCNVDHKPSNTKVDKTV (SEQ ID NO: 7).

9. The antibody of any one of the preceding clauses , wherein the CH2 domain is an IgG1 CH2 domain.

10. The antibody of any one of the preceding clauses wherein the CH2 domain is a wildtype human IgG1 CH2 domain, or comprises an amino acid sequence that is at least 95% identical to the amino acid sequence of a wildtype human IgG1 CH2 domain.

11. The antibody of any one of the preceding clauses , wherein the CH2 domain comprises one or more modifications which reduces or eliminates effector functions.

12. The antibody of any one of the preceding clauses , wherein the CH2 domain comprises amino acid substitutions A330S and P331S.

13. The antibody of any one of the preceding clauses , wherein the CH2 domain comprises the amino acid sequence

PSVFLFPPKPKDTLMISRTPEVTCVVVDVSHEDPEVKFNWYVDGVEVHNAKTKPREEQY

NSTYRVVSVLTVLHQDWLNGKEYKCKVSNKALPAPIEKTISKAK (SEQ ID NO: 4).

14. The antibody of any one of the preceding clauses , wherein the CH3 domain is an IgG1 CH3 domain.

15. The antibody of any one of the preceding clauses , wherein the CH3 domain is a wildtype human IgG1 CH3 domain, or comprises an amino acid sequence that is at least 95% identical to the amino acid sequence of a wildtype human IgG1 CH3 domain.

16. The antibody of any one of the preceding clauses , wherein the CH3 domain comprises the amino acid sequence

GQPREPQVYTLPPSREEMTKNQVSLTCLVKGFYPSDIAVEWESNGQPENNYKTTPPVLD SDGSFFLYSKLTVDKSRWQQGNVFSCSVMHEALHNHYTQKSLSLSPGK (SEQ ID NO: 5).

17. The antibody of any one of the preceding clauses , wherein the CH3 domain comprises amino acid substitutions E356D and M358L.

18. The antibody of any one of clauses 1-17, which has at least one enhanced property or a new introduced property relative to the same antibody that comprises an IgG1 hinge and CH1 domain.

19. The antibody of clause 18, wherein the antibody has at least one enhanced property selected from agonist activity, antibody mediated receptor internalization, ADCC, receptor mediated signaling, antagonist activity, immuno-modulating activity and anti-tumor activity; or a newly introduced property, which is agonist activity.

20. An antibody comprising a modified heavy chain constant region comprising a CH1 domain, a hinge, a CH2 domain, and a CH3 domain in order from N- to C- terminus, and wherein

(a) the CH1 domain comprises the amino acid sequence of SEQ ID NO: 7 or an amino acid sequence that differs therefrom in at most 5 amino acids or which is at least 95% identical to SEQ ID NO: 7, and wherein at least one of C131, R133, E137, S138 or R217 are not substituted or deleted;
(b) a hinge comprising any one of SEQ ID NO: 8, 21-23, 126-132 or 134-147 or a sequence that comprises 1-3 amino acids inserted between CVE and CPP, or which differs therefrom in at most 5 amino acids, wherein the hinge does not comprise a substitution or deletion at both C219 and C220;
(c) the antibody has at least one enhanced property or a new introduced property relative to the same antibody that comprises an IgG1 hinge and CH1 domain; and
(d) the modified heavy chain constant region is not a wildtype IgG2 constant region or an IgG2 constant region comprising C219S and/or C220S.

21. The antibody of clause 20, wherein the hinge comprises the amino acid sequence ERKXCVECPPCPAP (SEQ ID NO: 129) or ERKCXVECPPCPAP (SEQ ID NO: 130), wherein X is any amino acid except cysteine.

22. The antibody of clause 21, wherein the hinge comprises the amino acid sequence ERKSCVECPPCPAP (SEQ ID NO: 131) or ERKCSVECPPCPAP (SEQ ID NO: 132).

23. The antibody of any one of clauses 20-22, wherein at least one of P233, V234, A235 or G237 is deleted or substituted with another amino acid residue.

24. The antibody of clause 23, wherein P233, V234, A235 and G237 are deleted or substituted with another amino acid residue.

25. The antibody of any one of clauses 20-24, wherein none of amino acid residues R133, E137, S138 and R217 are substituted or deleted.

26. The antibody of clause 24, wherein none of amino acid residues C131, R133, E137, S138 and R217 are substituted or deleted.

27. The antibody of any one of clauses 20-26, wherein N192 is substituted with another amino acid.

28. The antibody of any one of clauses 20-27, wherein F193 is substituted with another amino acid

29. The antibody of any one of clauses 20-28, wherein the antibody comprises a CH2 domain that is at least 95% identical to that of wildtype IgG1.

30. The antibody of any one of clauses 20-29, wherein the antibody comprises a CH3 domain that is at least 95% identical to that of wildtype IgG1.

31. The antibody of any one of clauses 28-30, wherein the CH2 and/or CH3 domain is not a wildtype IgG1 CH2 and/or CH3 domain, and wherein the antibody has an effector function that is more potent than that of wildtype IgG1.

32. The antibody of any one of clauses 28-30, wherein the CH2 and/or CH3 domain is not a wildtype IgG1 CH2 and/or CH3 domain, and wherein the antibody has an effector function that less potent than that of wildtype IgG1.

33. The antibody of any one of clauses 20-32, wherein the antibody comprises a CH2 domain that is at least 95% identical to that of wildtype IgG4.

34. The antibody of any one of clauses 20-33, wherein the antibody comprises a CH3 domain that is at least 95% identical to that of wildtype IgG4.

35. The antibody of any one of clauses 20-34, wherein the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

36. The antibody of clause 35, wherein

(a) the CH1 domain is a wildtype human IgG2 CH1 domain;
(b) the hinge comprises SEQ ID NO: any one of SEQ ID NO: 8, 21-23, 126-132 or 134-147 or a sequence that comprises 1-3 amino acids inserted between CVE and CPP;
(c) the CH2 domain is a wildtype human IgG1 CH2 domain or a modified CH2 domain conferring enhanced or reduced effector function to the antibody; and
(d) the CH3 domain is a wildtype human IgG1 CH3 domain or a modified CH3 domain conferring enhanced or reduced effector function to the antibody.

37. The antibody of any one of the preceding clauses, comprising the amino acid sequence set forth in any one of SEQ ID NOs: 26-37, 54-56, 78-125, 152-232, 234-245 and 247-262 or an amino acid sequence that is at least 95% identical to SEQ ID NOs: 26-37, 54-56, 78-125, 152-232, 234-245 and 247-262.

38. An antibody comprising a modified heavy chain constant region, wherein the heavy chain constant region comprises a CH1 domain and a hinge comprising the sequence ASTKGPSVFPLAP**CSR**STS**ES**TAALGCLVKDY-FPEPVTVSWNSGALTSGVHTFPAVLQSS GLYSLSSVVTVPSS*NF*GTQTYTCNVDHKPSNTKVDKTVE**RK**C-*C*VECPPCPAP*PVAG* (SEQ ID NO: 133), or an amino acid sequence that differs from SEQ ID NO: 133 in at most 10 amino acids or is at least 90% identical to SEQ ID NO: 133, wherein

at least one of C131, R133, E137, S138 and R217 is not substituted with another amino acid or deleted;
C219 and C220 may be substituted with another amino acid or deleted, but C219 and C220 may not both be substituted or deleted;
1-3 amino acids may be inserted between CVE and CPP in the hinge;
the hinge optionally comprises an additional amino acid at the C-terminus, e.g., G;
one or more of amino acids P233, V234, A235 and G237 may be substituted with another amino acid (e.g., the corresponding amino acid from IgG1) or deleted;
the CH2 and CH3 domains may be wildtype or modified IgG1, IgG2, IgG3 or IgG4 CH2 and CH3 domains;
the modified heavy chain constant region is not a wildtype IgG2 heavy chain constant region or a wildtype IgG2 heavy constant domain with C219S or C220S; and the antibody has at least one enhanced property or a new introduced property relative to the same antibody that comprises an IgG1 hinge and CH1 domain.

39. The antibody of clause 38, wherein the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

40. The antibody of clause 38 or 39, wherein none of amino acids C131; R133; E137; S138; R217 are substituted with another amino acid or deleted.

41. The antibody of any one of clauses 38-40, wherein N192 and/or F193 are not substituted or are N192S and/or F193L, respectively.

42. The antibody of any one of clauses 38-41, wherein C219 is C219S.

43. The antibody of any one of clauses 38-41, wherein C220 is C220S.

44. The antibody of any one of clauses 38-43, wherein P233-G237 are substituted or deleted.

45. The antibody of any one of clauses 38-43, wherein V234-G237 are substituted or deleted.

46. The antibody of any one of clauses 38-43, wherein A235-G237 are substituted or deleted.

47. The antibody of any one of clauses 38-43, wherein G237 is substituted or deleted.

48. The antibody of any one of clauses 38-43, wherein P233 is substituted or deleted.

49. The antibody of any one of clauses 38-43, wherein P233-V234 are substituted or deleted.

50. The antibody of any one of clauses 38-43, wherein P233-A235 are substituted or deleted.

51. The antibody of any of clauses 38-50, wherein the antibody has effector function.

52. The antibody of any of clauses 38-50, wherein the antibody does not have effector function.

53. The antibody of any one of clauses 38-52, wherein the antibody comprises a wildtype or modified IgG1 CH2 domain.

54. The antibody of any one of clauses 38-52, wherein the antibody comprises a wildtype or modified IgG1 CH3 domain.

55. An antibody comprising a modified heavy chain constant region, wherein the heavy chain constant region comprises a CH1 domain comprising the sequence ASTKGPSVFPLAP**CSR**STS**ES**TAALGCLVKDYF-PEPVTVSWNSGALTSGVHTFPAVLQSS GLYSLSSVVTVPSS*NF*GTQTYTCNVDHKPSNTKVDKTV<u>E</u> (SEQ ID NO: 7), or an amino acid sequence that differs from SEQ ID NO: 7 in at most 10 amino acids or is at least 90% identical to SEQ ID NO: 7, wherein

at least one of C131, R133, E137, S138 and R217 is not substituted or deleted;
the modified heavy chain constant region is not a wildtype IgG2 heavy chain constant region or a wildtype IgG2 heavy constant domain with C219S or C220S; and
the antibody has at least one enhanced property or a new introduced property relative to the same antibody that comprises an IgG1 hinge and CH1 domain.

56. The antibody of clause 55, wherein the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

57. The antibody of clause 55 or 56, wherein none of amino acids C131; R133; E137 and S138 are substituted with another amino acid or deleted.

58. The antibody of any one of clauses 55-57, wherein N192 and/or F193 are not substituted or are N192S and/or F193L, respectively.

59. The antibody of any one of clauses 55-58, wherein the antibody has effector function.

60. The antibody of any one of clauses 55-58, wherein the antibody does not have effector function.

61. The antibody of any one of clauses 55-60, wherein the antibody comprises a wildtype or modified IgG1 CH2 domain.

62. The antibody of any one of clauses 55-61, wherein the antibody comprises a wildtype or modified IgG1 CH3 domain.

63. An antibody comprising a modified heavy chain constant region, wherein the heavy chain constant region comprises a hinge comprising the sequence E**RK***CC*VECPPCPAP*PVAG* (SEQ ID NO: 8), or an amino acid sequence that differs from SEQ ID NO: 8 in at most 5 amino acids, wherein

C219 and C220 may be substituted with another amino acid or deleted, but C219 and C220 may not both be substituted or deleted;
one or more of amino acids P233, V234, A235 and G237 may be substituted or deleted;
1-3 amino acids may be inserted between CVE and CPP in the hinge;
the hinge optionally comprises an additional amino acid at the C-terminus, e.g., G;
the CH2 and CH3 domains may be wildtype or modified IgG1, IgG2, IgG3 or IgG4 CH2 and CH3 domains;
the modified heavy chain constant region is not a wildtype IgG2 heavy chain constant region or a wildtype IgG2 heavy constant domain with C219S or C220S; and
the antibody has at least one enhanced property or a new introduced property relative to the same antibody that comprises an IgG1 hinge and CH1 domain.

64. The antibody of clause 63, wherein the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

65. The antibody of any one of clauses 63-64, wherein C219 is C219S.

66. The antibody of any one of clauses 63-64, wherein C220 is C220S.

67. The antibody of any one of clauses 63-66, wherein P233-G237 are mutated or deleted.

68. The antibody of any one of clauses 63-66, wherein V234-G237 mutated or are deleted.

69. The antibody of any one of clauses 63-66, wherein A235-G237 mutated or are deleted.

70. The antibody of any one of clauses 63-66, wherein G237 mutated or is deleted.

71. The antibody of any one of clauses 63-66, wherein P233 mutated or is deleted.

72. The antibody of any one of clauses 63-66, wherein P233-V234 mutated or are deleted.

73. The antibody of any one of clauses 63-66, wherein P233-A235 mutated or are deleted.

74. The antibody of any one of clauses 63-73, wherein the antibody has effector function.

75. The antibody of any one of clauses 63-73, wherein the antibody does not have effector function.

76. The antibody of any one of clauses 63-75, wherein the antibody comprises a wildtype or modified IgG1 CH2 domain.

77. The antibody of any one of clauses 63-76, wherein the antibody comprises a wildtype or modified IgG1 CH3 domain.

78. An antibody comprising a modified heavy chain constant region, wherein the heavy chain constant region comprises an IgG1 or IgG2 hinge, and wherein the hinge is lacking 1-7 amino acids, and wherein the antibody has at least one enhanced property or a new introduced property relative to the same antibody that comprises an IgG1 hinge and CH1 domain.

79. The antibody of clause 78, wherein the antibody has at least one enhanced property selected from agonist activity, antibody mediated receptor internalization, ADCC, receptor mediated signaling, antagonist activity, immuno-modulating activity or anti-tumor activity; or a newly introduced property, which is agonist activity.

80. The antibody of clause 78 or 79, wherein the hinge is an IgG2 hinge that is lacking 1-4 amino acids.

81. The antibody of clause 80, wherein the IgG2 hinge is lacking amino acids C219, C220, V222 and E224.

82. The antibody of clause 78 or 79, wherein the hinge is an IgG1 hinge that is lacking amino acids S219, C220, D221, K222, T223, H224 and T225.

83. The antibody of anyone of clauses 78-82, wherein the antibody comprises an IgG2 CH1 domain that is wildtype or modified.

84. The antibody of anyone of clauses 78-82, wherein the antibody comprises an IgG1 CH1 domain that is wildtype or modified.

85. The antibody of anyone of clauses 78-84, wherein the antibody comprises an IgG2 CH2 domain.

86. The antibody of anyone of clauses 78-84, wherein the antibody comprises an IgG1 CH2 domain.

87. The antibody of anyone of clauses 78-86, wherein the antibody comprises an IgG2 CH3 domain.

88. The antibody of anyone of clauses 78-86, wherein the antibody comprises an IgG1 CH3 domain.

89. The antibody of any one of the preceding clauses , which is a human or humanized antibody, or antigen binding portion thereof.

90. The antibody of any one of of the preceding clauses, wherein the antibody binds specifically to an antigen that is involved in immune regulation.

91. The antibody of clause 90, wherein the antibody is an agonist of a costimulatory receptor or an antagonist of an inhibitory receptor.

92. The antibody of clause 91, wherein the costimulatory receptor is selected from the group of B7-1, B7-2, CD28, 4-1BB, GITR, OX40, ICOS, CD70, CD27, CD40, DR3 or CD28H.

93. The antibody of clause 91, wherein the inhibitory receptor is selected from the group of CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1 and TIM-4.

94. The antibody of clause 90, wherein the antigen is CD73 or CD39.

95. An antibody that binds specifically to a costimulatory receptor and comprises a modified heavy chain constant region selected from the group of SEQ ID NOs: 26-37, 54-56, 78-125, 152-232, 234-245 and 247-262.

96. The antibody of clause 95, wherein the costimulatory receptor is GITR, OX40, 4-1BB, CD28, ICOS, CD40L, CD27 or any other TNFR superfamily member.

97. The antibody of clause 95 or 96, wherein the antibody exhibits enhanced or altered agonist activity relative to an antibody having the same variable regions and light chain, but comprising an IgG1 heavy chain constant region.

98. An antibody that binds specifically to a cell surface molecule and triggers antibody mediated internalization of the cell surface molecule, and comprises a modified heavy chain constant region selected from the group of SEQ ID NOs: 26-37, 54-56, 78-125, 152-232, 234-245 and 247-262.

99. The antibody of clause 98, wherein the cell surface molecule is CD73.

100. The antibody of clause 98 or 99, wherein the antibody possesses enhanced or altered internalization properties relative to an antibody having the same variable regions and light chain, but comprising an IgG1 heavy chain constant region.

101. An antibody that binds specifically to an inhibitory receptor and comprises a modified heavy chain constant region selected from the group of SEQ ID NOs: 26-37, 54-56, 78-125, 152-232, 234-245 and 247-262.

102. The antibody of clause 101, wherein the inhibitory receptor is CTLA-4, PD-1, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1 and TIM-4.

103. The antibody of clause 101 or 102, wherein the antibody exhibits more potent or altered antagonist activity or introduces a new activity relative to the same antibody having an IgG1 heavy chain constant region.

104. An antibody that binds specifically to a cell surface molecule and triggers intracellular signaling, wherein the antibody comprises a modified heavy chain constant region selected from the group of SEQ ID NOs 26-37, 54-56, 78-125, 152-232, 234-245 and 247-262.

105. The antibody of clause 104, wherein the intracellular signaling mediates agonist activity, antagonist activity, internalization of the cell surface molecule, or ADCC.

106. The antibody of clause 104 or 105, wherein the antibody triggers more potent intracellular signaling relative to to an antibody having the same variable regions and light chain, but comprising an IgG1 heavy chain constant region.

107. An antibody that binds specifically to a cell surface molecule and triggers formation of high molecular weight antibody-cell surface molecule complexes, wherein the antibody comprises a modified heavy chain constant region selected from the group of SEQ ID NOs: 26-37, 54-56, 78-125, 152-232, 234-245 and 247-262.

108. The antibody of clause 107, wherein the antibody triggers formation of higher molecular weight complexes relative to an antibody having the same variable regions and light chain, but comprising an IgG1 heavy chain constant region.

109. An antibody that binds specifically to a cell surface molecule and triggers clustering or oligomerization of the cell surface molecule, wherein the antibody comprises a modified heavy chain constant region selected from the group of SEQ ID NOs: 26-37, 54-56, 78-125, 152-232, 234-245 and 247-262.

110. The antibody of clause 109, wherein the antibody triggers more clustering or oligomerization of the cell surface molecule relative to an antibody having the same variable regions and light chain, but comprising an IgG1 heavy chain constant region.

111. A bispecific molecule comprising the antibody of any one of the preceding clauses, linked to a molecule having a second binding specificity.

112. An immunoconjugate comprising the antibody of any one of the preceding clauses, linked to a second agent.

113. A composition comprising the antibody, bispecific or immunoconjugate, of any one of clauses 1-112, and a carrier.

114. The composition of clause 44, further comprising one or more additional therapeutic agents.

115. The composition of clause 45, wherein the additional therapeutic agent stimulates the immune system.

116. The composition of clause 46, wherein the therapeutic agent is an antagonist of a checkpoint inhibitor or a co-stimulatory receptor.

117. A method of preparing an antibody comprising a modified heavy chain constant region, wherein the antibody comprises a CH1 domain, a hinge, a CH2 domain, and a CH3 domain in order from N- to C- terminus, comprising the steps of:

(a) providing an antibody comprising a hinge and/or a CH1 domain that is not an IgG2 hinge and/or IgG2 CH1 domain;

(b) replacing the hinge and/or the CH1 domain with an IgG2 hinge and/or IgG2 CH1 domain, respectively.

118. A method of increasing internalization of an antibody by a cell, comprising:

(a) providing an antibody comprising a hinge and/or a CH1 domain that is not an IgG2 hinge and/or IgG2 CH1 domain;

(b) replacing the hinge and/or the CH1 domain with an IgG2 hinge and/or IgG2 CH1 domain, respectively.

119. The method of clause 118, wherein internalization of the antibody is increased compared to internalization of the same antibody comprising a hinge of a non-IgG2 isotype, e.g., an antibody comprising an IgG1 constant region.

120. A method of increasing the agonist activity of an antibody, comprising:

(a) providing an antibody comprising a hinge and/or a CH1 domain that is not an IgG2 hinge and/or IgG2 CH1 domain;

(b) replacing the hinge and/or the CH1 domain with an IgG2 hinge and/or IgG2 CH1 domain, respectively.

121. The method of clause 120, wherein the agonist activity is increased compared to agonist activity of the same antibody comprising a hinge of a non-IgG2 isotype, e.g., an antibody comprising an IgG1 constant region.

122. The method of any one of clauses 117-121, wherein the IgG2 hinge is a wildtype human IgG2 hinge, or comprises an amino acid sequence that is at least 95% identical to the amino acid sequence of a wildtype human IgG2 hinge.

123. The method of any one of clauses 117-122, comprising the step of replacing at least one of the CH1, CH2, or CH3 domains with a CH1, CH2, or CH3 domain of a different isotype, respectively.

124. The method of any one of clauses 117-123, comprising the steps of

(a) replacing the CH1 domain with an IgG2 CH1 domain;
(b) replacing the CH2 domain with an IgG1 CH2 domain; and/or
(c) replacing the CH3 domain with an IgG1 CH3 domain.

125. The method of any one of clauses 117-124, comprising the steps of

(a) replacing the CH1 domain with a wildtype human IgG2 CH1 domain, or a domain at least 95% identical thereto;
(b) replacing the CH2 domain with a wildtype human IgG1 CH2 domain, or a domain at least 95% identical thereto; and/or
(c) replacing the CH3 domain with a wildtype human IgG1 CH3 domain, or a domain at least 95% identical thereto.

126. The method of any one of clauses 117-125, comprising the step of
replacing the heavy chain constant region with a heavy chain constant region comprising any one of SEQ ID NOs: 26-37, 54-56, 78-125, 152-232, 234-245 and 247-262 or a region at least 95% identical to SEQ ID NOs: 26-37, 54-56, 78-125, 152-232, 234-245 and 247-262.

127. The method of any one of clauses 117-126, wherein the hinge is modified to reduce disulfide bond formation.

128. The method of any one of clauses 117-127, wherein the hinge comprises amino acid substitution C219S.

129. The method of claim any one of clauses 117-128, wherein the hinge comprises an amino acid sequence set forth in any one of SEQ ID NO: 8, 21-23, 126-132 or 134-147 or a sequence that comprises 1-3 amino acids inserted between CVE and CPP.

130. The method of any one of clauses 117-129, wherein the CH1 domain comprises the amino acid sequence

ASTKGPSVFPLAPCSRSTSESTAALGCLVKDYFPEPVTVSWNSGALTSGVHTFPAVLQSS
GLYSLSSVVTVPSSNFGTQTYTCNVDHKPSNTKVDKTV (SEQ ID NO: 7).

131. The method of any one of clauses 117-130, wherein the CH2 domain is modified to reduce or eliminate effector functions.

132. The method of any one of clauses 117-131, wherein the CH2 domain comprises amino acid substitutions A330S and P331S.

133. The method of any one of clauses 117-132, wherein the CH2 domain comprises the amino acid sequence

PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAK (SEQ ID NO: 4).

134. The method of any one of clauses 117-133, wherein the CH2 domain comprises amino acid substitutions A330S and P331S.

135. The method of any one of clauses 117-134, wherein the CH3 domain comprises the amino acid sequence

GQPREPQVYTLPPSR<u>EE</u><u>M</u>TKNQVSLTCLVKGFYPSDIAVEWESNGQPENNYKTTPPVLD
SDGSFFLYSKLTVDKSRWQQGNVFSCSVMHEALHNHYTQKSLSLSPGK (SEQ ID NO: 5).

136. An antibody, or antigen binding portion thereof, produced by the method of any one of clauses 117-135.

137. The antibody, or antigen binding portion thereof, of clause 136, which is a human or humanized antibody.

138. A method of treating a subject, comprising administering the antibody, or antigen binding portion thereof, of any one of the precedint clauses .

139. The method of clause 138, further comprising the step of administering one or more additional therapeutic agents.

140. The method of clause 139, wherein the therapeutic agent stimulates the immune system.

141. The method of clause 140, wherein the therapeutic agent is a checkpoint inhibitor or a co-stimulatory molecule.

142. A method of treating a subject, comprising administering the composition, bispecific molecule, or immuncon-jugate of any one of the preceding clauses.

143. An antibody (or antigen binding fragment thereof) comprising a modified heavy chain constant domain comprising a human IgG heavy chain constant domain, wherein amino acid at position 238 is not P, and the modified heavy chain constant domain has reduced effector function relative to the same IgG heavy chain constant domain, wherein amino acid at position 238 is proline.

144. The antibody of clause 143, wherein the IgG heavy chain constant domain is a human IgG 1 heavy chain constant domain.

145. The antibody of clause 143 or 144, wherein the amino acid at position 238 is K.

146. The antibody of any one of clauses 143-145, wherein the modified heavy chain constant region comprises an amino acid sequence that is at least 90% identical to SEQ ID NO: 198, wherein P238 is not P (or not P nor S), and is, e.g., P238K.

147. The antibody of clause 146, wherein the modified heavy chain constant region comprises an amino acid sequence that is at least 95% identical to SEQ ID NO: 198, wherein P238 is not P (or not P nor S), and is, e.g., P238K.

148. The antibody of clause 147, wherein the modified heavy chain constant region comprises an amino acid sequence that is at least 99% identical to SEQ ID NO: 198, wherein P238 is not P (or not P nor S), and is, e.g., P238K.

149. The antibody of any one of clauses 143-148, wherein the modified heavy chain constant region does not comprise one or more of the non P238 amino acid modifications disclosed in U.S. Patent No. 5,637,481 (the contents of which are specifically incorporated by reference herein), e.g., C220S, C226S and C229S.

150. The antibody of any one of clauses 143-149, wherein the modified heavy chain constant region comprises an amino acid sequence consisting of SEQ ID NO: 198.

151. The antibody of any one of clauses 143-150, wherein the antibody is an antigen binding fragment of an antibody (e.g., a Dab or scFv) that is linked to the modified heavy chain constant region.

152. The antibody of any one of clauses 143-150, wherein the antibody comprises a heavy chain variable domain linked to the modified heavy chain constant domain and a light chain variable domain linked to a light chain constant domain.

153. The antibody of any one of clauses 143-152, wherein the antibody is a full length antibody (heavy and light chain are full length heavy and light chain, respectively).

154. The antibody of any one of clauses 143-153, wherein the modified heavy chain constant domain does not comprise one or more other mutations that reduce effector function.

155. The antibody of clause 154, wherein the modified heavy chain constant domain does not comprise one or more other mutations disclosed herein that reduce effector function.

156. The antibody of any one of clauses 143-153, wherein the modified heavy chain constant domain comprises 1-3 other mutations that reduce effector function.

157. The antibody of clause 156, wherein the modified heavy chain constant domain comprises 1-3 other mutations disclosed herein that reduce effector function.

158. The antibody of any one of clauses 143-157, wherein the effector function of the antibody is about the same as that of an IgG2 antibody.

159. The antibody of any one of clauses 143-158, wherein the antibody has lower binding affinity to the low affinity Fc√Rs relative to the antibody with a wildtype IgG1.

160. The antibody of clause 159, wherein the antibody has no detectable binding to the low affinity Fc√Rs.

161. The antibody of clause 159 or 160, wherein the low affinity Fc√Rs are hCD32a-H131, hCD32a-R131, hCD32b, hCD16a-V158 and hCD16b-NA2.

162. The antibody of clause 161, wherein the antibody has no detectable binding (e.g., with 10 $\mu$M antibody concentration) towards the flow affinity Fc√Rs hCD32a-H131, hCD32a-R131, hCD32b, hCD16a-V158 or hCD16b-NA2.

163. The antibody of any one of clauses 143-162, wherein the antibody binds to the high affinity FcgR CD64 (hFcgRI) with a faster off-rate (dissociation rate) relative to the antibody with a wild-type IgG1 constant region.

164. The antibody of any one of clauses 143-163, wherein the antibody has no detectable binding (e.g., with 10 $\mu$M antibody concentration) towards the low affinity Fc√Rs hCD32aH131, hCD32a-R131, hCD32b, hCD16a-V158 or hCD16b-NA2 and has a faster off-rate relative to the antibody with a wild-tpe constant region.

165. The antibody of any one of clauses 159-164, wherein binding affinity and off-rate to Fc√Rs is determined by Biacore.

166. The antibody of any one of clauses 143-165, wherein the antibody has superior thermal stability relative to the antibody with a wildtype IgG1 constant region.

167. The antibody of any one of clauses 143-166, wherein the antibody has reduced heterogeneity relative to the antibody with a wildtype IgG1 constant region.

168. An antibody comprising a heavy chain constant region comprising the mutations L234A, L235E and G337A, but does not contain a mutation at A330 and/or P331 that reduces effector function (e.g., does not comprise A330S and/or P331S), wherein the antibody has reduced effector function (reduced ADCC and optionally reduced CDC) relative to the same antibody without these mutations.

169. The antibody of clause 168, which binds to an inhibitory receptor on an immune cell, e.g., a T cell.

170. The antibody of clauses 168 or 169, which binds with reduced affinity to the FcγRs hCD32a-H131, hCD32a-R131, hCD32b, hCD16a-V158, hCD16b-NA2 and hCD64, relative to the same antibody without these mutations.

171. The antibody of any of clauses 168-170, wherein the heavy chain constant region comprises an amino acid sequence that is at least 95% identical to SEQ ID NO: 234 or 248.

172. The antibody of any of clauses 168-171, wherein the heavy chain constant region comprises the amino acid sequence SEQ ID NO: 234 or 248, including 1-5, 1-4, 1-3, 1-2 or 1 amino acid substitution (e.g., a conservative amino acid substitution), wherein the amino acid substitutions do not significantly increase binding to one or more FcγRs.

173. The antibody of any of clauses 168-172, wherein the heavy chain constant region comprises the amino acid sequence SEQ ID NO: 234 or 248, including or excluding the C-terminal lysine.

174. The antibody of any of clauses 168-172, wherein the heavy chain constant region comprises the amino acid sequence SEQ ID NO: 236, 249, 252, 253, 259 or 260, including or excluding the C-terminal lysine.

175. The antibody of any of clauses 168-174, wherein the antibody is a full length antibody (or comprises a full length heavy chain and a full length light chain), with or without C-terminal lysine.

176. The antibody of any of clauses 168-175, wherein the antibody is an antagonist of a checkpoint inhibitor or an agonist of a checkpoint stimulator.

177. The antibody of any of clauses 168-176, wherein the antibody is not an antibody disclosed in WO 2018/013818.

178. The antibody of any of clauses 168-177, wherein the antibody does not bind to human TIM3.

179. A fusion protein comprising a heavy chain constant region comprising the mutations L234A, L235E and G337A, but does not contain a mutation at A330 and/or P331 that reduces effector function (e.g., does not comprise A330S and/or P331S), wherein the antibody has reduced effector function (reduced ADCC and optionally reduced CDC) relative to the same antibody without these mutations.

180. The fusion protein of clause 179, which binds with reduced affinity to the FcγRs hCD32aH131, hCD32a-R131, hCD32b, hCD16a-V158, hCD16b-NA2 and hCD64, relative to the same antibody without these mutations.

181. The fusion protein of clause 179 or 180, wherein the heavy chain constant region comprises an amino acid sequence that is at least 95% identical to SEQ ID NO: 234 or 248.

182. The fusion protein of any of clauses 179-181, wherein the heavy chain constant region comprises the amino acid sequence SEQ ID NO: 234 or 248, including 1-5, 1-4, 1-3, 1-2 or 1 amino acid substitution (e.g., a conservative amino acid substitution), wherein the amino acid substitutions do not significantly increase binding to one or more FcγRs.

183. The fusion protein of any of clauses 179-182, wherein the heavy chain constant region comprises the amino acid sequence SEQ ID NO: 234 or 248, including or excluding the C-terminal lysine.

184. The antibody of any of clauses 179-183, wherein the heavy chain constant region comprises the amino acid sequence SEQ ID NO: 236, 249, 252, 253, 259 or 260, including or excluding the C-terminal lysine.

185. An antibody comprising a heavy chain constant region comprising an amino acid sequence set forth in the Sequence Table or a sequence that is at least 95% identical thereto.

**Claims**

1. An antibody or a fusion protein comprising a heavy chain constant region comprising the mutations L234A, L235E and G237A, wherein the antibody or fusion protein does not contain a mutation at A330 and P331 that reduces effector function, wherein the antibody or fusion protein has reduced effector function relative to the same antibody or fusion protein without these mutations, and wherein the antibody does not bind to TIM3.

2. The antibody or fusion protein of claim 1, wherein the heavy chain constant region does not comprise A330S and P331S.

3. The antibody or fusion protein of claim 1 or 2, wherein the effector function is antibody-dependent cell-mediated cytotoxicity (ADCC).

4. The antibody or fusion protein of any of claims 1-3, wherein the antibody or fusion protein has additionally reduced complement mediated cytotoxicity (CDC).

5. The antibody of any one of claims 1-4, which binds to an inhibitory receptor on an immune cell, wherein the immune cell is optionally a T cell.

6. The antibody or fusion protein of any of claims 1-5, wherein the heavy chain constant region comprises an amino acid sequence that is at least 95% identical to SEQ ID NO: 234 or 248.

7. The antibody or fusion protein of any of claims 1-6, wherein the heavy chain constant region comprises the amino acid sequence SEQ ID NO: 234 or 248, including 1-5, 1-4, 1-3, 1-2 or 1 amino acid substitution, wherein the amino acid substitutions do not significantly increase binding to one or more FcyRs.

8. The antibody or fusion protein of claim 7, wherein the amino acid substitution is a conservative amino acid substitution.

9. The antibody or fusion protein of any of claims 1-8, wherein the heavy chain constant region comprises the amino acid sequence SEQ ID NO: 234 or 248, including or excluding the C-terminal lysine.

10. The antibody or fusion protein of any of claims 1-8, wherein the heavy chain constant region comprises the amino acid sequence SEQ ID NO: 236, 249, 252, 253, 259 or 260, including or excluding the C-terminal lysine.

11. The antibody of any of claims 1-10, wherein the antibody is a full-length antibody, with or without C-terminal lysine.

12. The antibody of any of claims 1-11, wherein the antibody is an antagonist of a checkpoint inhibitor or an agonist of a checkpoint stimulator.

FIG. 1A

**Ab-mediated Receptor Internalization (HCC15 cells)**

FIG. 1B

FIG. 1C

Ab-mediated Receptor Internalization
(NCI-H2030 cells)

Legend:
- 11-Vh-hHC-IgG2-C219S
- 10-Vh-hHC-IgG2-C219S
- 10-Vh-hHC-IgG2-C219S-IgG1.1f
- 4-Vh-hHC-IgG2-C219S/11F11-Vk2
- 10-Vh-hHC-IgG1.1f/11F11-Vk2
- 3-Vh-hHC-IgG1.1f/4C3-Vk1
- 11F11
- 4C3
- 6E11

Antibodies @ 2µg/ml

FIG. 1D

EP 4 098 662 A1

FIG. 1E

FIG. 1F

EP 4 098 662 A1

Calu6 CD73 internalization

FIG. 1G

FIG. 1H

SNU-C1 internalization

Legend:
- mAb-CD73.4-IgG2.IgG1.1f
- mAb-CD73.4-IgG1.1f
- mAb-CD73.4-IgG2.IgG1.1(no wash)
- mAb-CD73.4-IgG1.1f (no wash)

FIG. 1I

FIG. 1J

FIG. 2

**GITR Abs on IFN-g secretion by CD4 T cells stimulated with OKT3-expressing CHO cells - Donor#2**

anti-GITR.IgG1
anti-GITR.g2.g1f
No Ab
unrelated hIgG1

FIG. 3A

EP 4 098 662 A1

**GITR Abs on IL-2 secretion by CD4 T cells stimulated with OKT3 expressing CHO cells - Donor #3**

FIG. 3B

GITR Abs on IL-2 secretion by CD4 T cells stimulated
with OKT3 expressing CHO cells - Donor #3

anti-GITR-IgG1.1f
anti-GITR.g2.g1.1f
No Ab
unrelated hIgG1

FIG. 3C

EP 4 098 662 A1

anti-GITR-IgG1
anti-GITR-IgG1.1f
anti-GITR.g2.g1.1f
anti-GITR.g2
anti-GITR.g2.g1f
No Ab
unrelated hgG1
unrelated hgG2

IL-2 (pg/mL)

1500
1000
500
0

0.0001 0.001 0.01 0.1 1 10 100

concentration of antibody (ug/mL)

FIG. 4

FIG. 5A

FIG. 5B

FIG. 5C

FIG. 5D

SEC-MALS data for CD73/mAb complexes

| Target | mAb | Molar ratio | target] (uM) | mAb] (uM) | UV elution time (min) | | | | SEC UV integration by % | | | | MW (kDa) | | | |
|---|---|---|---|---|---|---|---|---|---|---|---|---|---|---|---|---|
| | | | | | Peak 1 | Peak 2 | Peak 3 | Peak4 | Peak 1 | Peak 2 | Peak 3 | Peak4 | Peak 1 | Peak 2 | Peak 3 | Peak 4 |
| | CD73.10-IgG1.1f | 0:1 | | 4 | ~2.0 | ~14.0 | ~17.3 | | 0.3 | 0.3 | 99.4 | | n/a | 62 | 140 | |
| | CD73.10-IgG2-C219S | 0:1 | | 4 | ~2.0 | ~17.5 | | | 0.1 | 99.5 | | | 73 | 150 | | |
| | CD73.3-IgG1.1f | 0:1 | | 4 | ~16.3 | | | | 100.0 | | | | 150 | | | |
| | 11F11-Fab | 0:1 | | 8 | ~2.0 | ~17.7 | ~20.2 | | 0.8 | 0.6 | 98.7 | | n/a | 19 | 47 | |
| hCD73-his | | 1:0 | 4 | | ~17.1 | ~18.4 | | | 2.8 | 97.2 | | | 280 | 120 | | |
| N-hCD73-his | | 1:0 | 8 | | ~19.3 | | | | 100.0 | | | | 38 | | | |
| hCD73-his | CD73.10-IgG1.1f | 1:1 | 4 | 4 | ~11.7 | ~12.7 | ~16.9 | | 34.2 | 67.2 | 3.0 | | 1100 | 540 | 180 | |
| hCD73-his | CD73.10-IgG2-C219S | 1:1 | 4 | 4 | ~10.3 | ~11.1 | | | 6.4 | 93.3 | | | 11000 | 3400 | | |
| hCD73-his | CD73.3-IgG1.1f | 1:1 | 4 | 4 | ~12.0 | ~13.0 | ~14.8 | | 17.5 | 78.3 | 4.0 | | 960 | 530 | 260 | |
| hCD73-his | 11F11-Fab | 1:2 | 4 | 8 | ~12.6 | ~14.7 | ~16.8 | ~20.1 | 2.9 | 88.7 | 0.8 | 7.7 | 300 | 310 | 85 | 40 |
| N-hCD73-his | CD73.10-IgG1.1f | 2:1 | 8 | 4 | ~14.6 | ~17.1 | | | 86.5 | 13.5 | | | 210 | 140 | | |
| N-hCD73-his | CD73.10-IgG2-C219S | 2:1 | 8 | 4 | ~14.7 | ~17.3 | | | 87.1 | 12.9 | | | 210 | 350 | | |
| N-hCD73-his | CD73.3-IgG1.1f | 2:1 | 8 | 4 | ~15.3 | ~16.2 | ~19.3 | | 6.4 | 68.7 | 24.9 | | 170 | 180 | 42 | |
| N-hCD73-his | 11F11-Fab | 1:1 | 8 | 8 | ~17.4 | ~20.0 | | | 95.3 | 4.7 | | | 84 | 44 | | |
| hCD73 | | 1:0 | 2.5 | 0 | ~16.9 | | | | 100.0 | | | | 120 | | | |
| hCD73 | CD73.4-hybrid | 1:0.25 | 2.5 | 0.625 | ~11.8 | ~13.4 | ~17.1 | | 42.0 | 21.6 | 36.4 | | 1200 | 520 | 120 | |
| hCD73 | CD73.4-hybrid | 1:0.5 | 2.5 | 1.25 | ~11.7 | ~13.4 | ~17.1 | | 71.9 | 15.8 | 13.4 | | 1900 | 540 | 130 | |
| hCD73 | CD73.4-hybrid | 1:1 | 2.5 | 2.5 | ~11.5 | ~13.4 | ~17.1 | | 95.3 | 1.9 | 2.8 | | 3300 | 1000 | 530 | |
| hCD73 | CD73.4-hybrid | 1:2 | 2.5 | 5 | ~11.7 | ~13.4 | ~15.3 | ~17.1 | 52.5 | 17.7 | 0.6 | 29.3 | 1700 | 500 | 220 | 140 |
| hCD73 | CD73.4-hybrid | 1:4 | 2.5 | 10 | ~11.7 | ~13.4 | ~15.3 | ~17.1 | 24.1 | 16.2 | 1.2 | 58.6 | 1300 | 500 | 200 | 140 |
| hCD73 | | 1:0 | 2.5 | 0 | ~16.9 | ~18.2 | | | 97.6 | 2.4 | | | 120 | 73 | | |
| hCD73 | CD73.4-IgG1.1f | 1:0.25 | 2.5 | 0.625 | ~12.3 | ~13.1 | ~17.1 | | 11.8 | 49.5 | 38.6 | | 830 | 530 | 120 | |
| hCD73 | CD73.4-IgG1.1f | 1:0.5 | 2.5 | 1.25 | ~12.3 | ~13.1 | ~17.1 | | 31.1 | 54.8 | 3.3 | 10.7 | 890 | 540 | 120 | 81 |
| hCD73 | CD73.4-IgG1.1f | 1:1 | 2.5 | 2.5 | ~12.1 | ~12.3 | ~13.1 | ~17.0 | 55.2 | 44.5 | 2.1 | 2.2 | 1100 | 560 | 280 | 160 |
| hCD73 | CD73.4-IgG1.1f | 1:2 | 2.5 | 5 | ~12.3 | ~13.1 | ~15.3 | ~17.0 | 22.3 | 44.1 | 2.8 | 30.7 | 910 | 530 | 210 | 140 |
| hCD73 | CD73.4-IgG1.1f | 1:4 | 2.5 | 10 | ~12.3 | ~13.2 | ~15.3 | ~17.0 | 9.1 | 31.0 | 2.4 | 57.4 | 810 | 510 | 200 | 140 |
| N-hCD73 | CD73.4-hybrid | 1:1 | 5 | 2.5 | ~13.9 | ~14.7 | ~15.9 | ~17.0 | 0.7 | 88.3 | 8.1 | 2.8 | 390 | 210 | 180 | 150 |
| N-hCD73 | CD73.4-IgG1.1f | 1:1 | 5 | 2.5 | ~13.4 | ~14.7 | | | 1.7 | 98.3 | | | 360 | 210 | | |
| | CD73.4-hybrid | 0:1 | 0 | 5 | ~15.9 | ~16.9 | | | 0.4 | 99.6 | | | 290 | 140 | | |
| | CD73.4-IgG1.1f | 0:1 | 0 | 5 | ~14.2 | ~15.9 | ~17.1 | | 0.6 | 1.2 | 98.2 | | 81 | 120 | 140 | |
| hCD73 | CD73.4-IgG2-C219S | 1:1 | 2.5 | 2.5 | ~11.5 | ~13.4 | ~17.3 | | 95.0 | 2.1 | 2.8 | | 3400 | 970 | 480 | |
| hCD73 | CD73.4-IgG2-C219S-IgG1.1f | 1:1 | 2.5 | 2.5 | ~11.5 | ~13.4 | ~17.3 | | 94.7 | 2.3 | 3.0 | | 3600 | 1100 | 580 | |
| hCD73 | CD73.4-IgG1.1f | 1:1 | 2.5 | 2.5 | ~11.7 | ~13.0 | ~15.1 | ~17.0 | 60.0 | 35.3 | 2.3 | 2.5 | 1300 | 550 | 270 | 160 |
| hCD73 | CD73.10-IgG2-C219S | 1:1 | 2.5 | 2.5 | ~11.8 | ~16.9 | | | 96.2 | 3.2 | | | 2000 | 310 | | |
| hCD73 | CD73.10-IgG2CS-1.1 | 1:1 | 2.5 | 2.5 | ~11.8 | ~16.9 | | | 96.6 | 2.5 | 0.9 | | 2000 | 320 | 390 | |
| hCD73 | CD73.10-IgG1.1f | 1:1 | 2.5 | 2.5 | ~12.1 | ~13.0 | ~16.5 | | 35.1 | 62.1 | 2.7 | | 1000 | 530 | 170 | |
| hCD73 | | 1:0 | 2.5 | 0 | ~17.3 | ~18.4 | | | 97.2 | 2.8 | | | 120 | 60 | | |
| hCD73 | CD73.4-IgG2-C219S-IgG1.1f | 1:1 | 5 | 5 | ~11.5 | ~13.4 | ~17.3 | | 93.9 | 2.6 | 3.5 | | 2800 | 1300 | 740 | |
| hCD73 | CD73.4-IgG2-C219S-IgG1.1f | 1:1 | 1 | 1 | ~11.5 | ~13.4 | ~15.8 | ~17.3 | 84.4 | 7.6 | 2.5 | 5.4 | 2800 | 660 | 460 | 240 |

\* Molar concentrations are defined as N-hCD73 monomer, 11F11-Fab monomer, hCD73-his dimer, and (bivalent) mAb

FIG. 6

DLS data for CD73/mAb complexes:

| Target | mAb | Molar ratio | target} (uM) | (mAb) (uM) | DLS Rh | Rh SD | %Pd | %Pd SD | %Mass | %Mass SD |
|---|---|---|---|---|---|---|---|---|---|---|
| | CD73.10-IgG1.1f | 0:1 | | 4 | 5.0 | 0.6 | 11.0 | 6.0 | 100.0 | 0.0 |
| | CD73.10-IgG2-C219S | 0:1 | | 4 | 5.2 | 0.1 | 11.9 | 3.3 | 100.0 | 0.0 |
| | CD73.3-IgG1.1f | 0:1 | | 4 | 5.1 | 0.5 | 11.6 | 6.9 | 100.0 | 0.0 |
| | 11F11-Fab | 0:1 | | 8 | 3.4 | 0.2 | 22.0 | 5.3 | 100.0 | 0.1 |
| hCD73-his | | 1:0 | 4 | | 4.3 | 0.3 | 10.6 | 5.2 | 99.8 | 0.2 |
| N-hCD73-his | | 1:0 | 8 | | 2.3 | 0.2 | 19.3 | 12.3 | 99.9 | 0.2 |
| hCD73-his | CD73.10-IgG1.1f | 1:1 | 4 | 4 | 12.8 | 0.7 | 32.4 | 6.7 | 100.0 | 0.0 |
| hCD73-his | CD73.10-IgG2-C219S | 1:1 | 4 | 4 | 2.9 | 1.5 | 7.5 | 0.8 | 73.9 | 11.5 |
| hCD73-his | CD73.3-IgG1.1f | 1:1 | 4 | 4 | 10.8 | 0.1 | 28.2 | 0.6 | 100.0 | 0.0 |
| hCD73-his | 11F11-Fab | 1:2 | 4 | 8 | 6.4 | 0.1 | 15.1 | 4.3 | 100.0 | 0.0 |
| N-hCD73-his | CD73.10-IgG1.1f | 2:1 | 8 | 4 | 6.6 | 0.3 | 15.3 | 6.5 | 100.0 | 0.0 |
| N-hCD73-his | CD73.10-IgG2-C219S | 2:1 | 8 | 4 | 6.5 | 0.2 | 15.7 | 5.7 | 100.0 | 0.1 |
| N-hCD73-his | CD73.3-IgG1.1f | 2:1 | 8 | 4 | 5.8 | 0.3 | 12.8 | 5.3 | 100.0 | 0.0 |
| N-hCD73-his | 11F11-Fab | 1:1 | 8 | 8 | 4.2 | 0.1 | 17.6 | 4.7 | 100.0 | 0.0 |
| hCD73 | | 1:0 | 2.5 | 0 | 4.5 | 0.1 | 12.3 | 4.3 | 99.9 | 0.2 |
| hCD73 | CD73.4-hybrid | 1:0.25 | 2.5 | 0.625 | 13.2 | 1.7 | 46.5 | 5.9 | 99.9 | 0.1 |
| hCD73 | CD73.4-hybrid | 1:0.5 | 2.5 | 1.25 | 17.5 | 1.0 | 31.7 | 8.8 | 62.6 | 25.8 |
| hCD73 | CD73.4-hybrid | 1:1 | 2.5 | 2.5 | 29.9 | 1.0 | 25.1 | 2.1 | 22.3 | 4.9 |
| hCD73 | CD73.4-hybrid | 1:2 | 2.5 | 5 | 14.4 | 0.1 | 31.3 | 5.5 | 68.7 | 25.8 |
| hCD73 | CD73.4-hybrid | 1:4 | 2.5 | 10 | 11.4 | 0.9 | 42.9 | 3.5 | 90.2 | 17.5 |
| hCD73 | | 1:0 | 2.5 | 0 | 4.7 | 0.1 | 18.6 | 3.3 | 99.8 | 0.1 |
| hCD73 | CD73.4-IgG1.1f | 1:0.25 | 2.5 | 0.625 | 10.4 | 1.5 | 38.6 | 15.9 | 51.3 | 37.1 |
| hCD73 | CD73.4-IgG1.1f | 1:0.5 | 2.5 | 1.25 | 12.9 | 0.6 | 39.5 | 5.4 | 88.3 | 23.2 |
| hCD73 | CD73.4-IgG1.1f | 1:1 | 2.5 | 2.5 | 14.7 | 0.5 | 34.3 | 3.9 | 100.0 | 0.0 |
| hCD73 | CD73.4-IgG1.1f | 1:2 | 2.5 | 5 | 11.6 | 0.4 | 24.9 | 8.5 | 65.0 | 32.5 |
| hCD73 | CD73.4-IgG1.1f | 1:4 | 2.5 | 10 | 5.1 | 4.0 | 33.0 | 15.9 | 54.2 | 31.7 |
| N-hCD73 | CD73.4-hybrid | 1:1 | 5 | 2.5 | 6.7 | 0.3 | 13.9 | 7.0 | 100.0 | 0.0 |
| N-hCD73 | CD73.4-IgG1.1f | 1:1 | 5 | 2.5 | 6.5 | 0.1 | 7.4 | 3.2 | 100.0 | 0.0 |
| | CD73.4-hybrid | 0:1 | 0 | 5 | 5.4 | 0.2 | 15.7 | 5.7 | 99.9 | 0.1 |
| | CD73.4-IgG1.1f | 0:1 | 0 | 5 | 5.3 | 0.4 | 13.6 | 7.9 | 100.0 | 0.0 |
| hCD73 | CD73.4-IgG2-C219S | 1:1 | 2.5 | 2.5 | 31.7 | 2.4 | 31.6 | 11.4 | 19.3 | 12.2 |
| hCD73 | CD73.4-IgG2-C219S-IgG1.1f | 1:1 | 2.5 | 2.5 | 31.2 | 0.9 | 30.8 | 3.8 | 17.5 | 11.4 |
| hCD73 | CD73.4-IgG1.1f | 1:1 | 2.5 | 2.5 | 15.6 | 1.2 | 32.7 | 8.0 | 100.0 | 0.0 |
| hCD73 | CD73.10-IgG2-C219S | 1:1 | 2.5 | 2.5 | 23.9 | 1.2 | 26.7 | 4.4 | 36.3 | 5.3 |
| hCD73 | CD73.10-IgG2CS-1.1 | 1:1 | 2.5 | 2.5 | 25.6 | 2.3 | 30.1 | 8.8 | 35.9 | 9.1 |
| hCD73 | CD73.10-IgG1.1f | 1:1 | 2.5 | 2.5 | 13.9 | 1.2 | 31.6 | 12.0 | 83.2 | 31.5 |
| hCD73 | | 1:0 | 2.5 | 0 | 4.2 | 0.8 | 24.4 | 12.8 | 99.1 | 0.7 |
| hCD73 | CD73.4-IgG2-C219S-IgG1.1f | 1:1 | 5 | 5 | 35.3 | 3.8 | 35.2 | 13.9 | 6.7 | 4.4 |
| hCD73 | CD73.4-IgG2-C219S-IgG1.1f | 1:1 | 1 | 1 | 25.5 | 3.2 | 32.9 | 2.9 | 60.7 | 27.5 |

* Molar concentrations are defined as N-hCD73 monomer, 11F11-Fab monomer, hCD73-his dimer, and (bivalent) mAb

FIG. 7

**Calu6 internalization**

Legend:
- mAb-CD73.4-IgG1f/LC-11F11-Vk2
- mAb-CD73.4-IgG2.3G1-AY-pTT5-SP
- mAb-CD73.4-IgG2.3G1-KH
- mAb-CD73.4-G1-G2.3-G1-AY
- mAb-CD73.4-IgG1-deltaTHT
- mAb-CD73.4-G1-G2.3-G1-KH
- mAb-CD73.4-IgG2.3-plusTHT
- mAb-CD73.4-IgG2.3-plusGGG
- mAb-CD73.4-IgG2.5
- mAb-CD73.4-IgG2.5G1.1f-KH
- mAb-CD73.4-IgG2.5G1-AY
- mAb-CD73.4-IgG2.5G1-KH
- mAb-CD73.4-IgG2.5plusTHT/LC
- mAb-CD73.4-IgG2-C219S/LC
- mAb-CD73.4-IgG2-C219S/LC
- mAb-CD73.4-IgG1.1f+K/LC
- mAb-CD73.4-IgG2-IgG1.1f

FIG. 8A

EP 4 098 662 A1

**NCI-H292 internalization**

Legend:
- mAb-CD73.4-IgG1f/LC-11F11-Vk2
- mAb-CD73.4-IgG2.3G1-AY-pTT5-SP
- mAb-CD73.4-IgG2.3G1-KH
- mAb-CD73.4-G1-G2.3-G1-AY
- mAb-CD73.4-IgG1-deltaTHT
- mAb-CD73.4-G1-G2.3-G1-KH
- mAb-CD73.4-IgG2.3-plusTHT
- mAb-CD73.4-IgG2.3-plusGGG
- mAb-CD73.4-IgG2.5
- mAb-CD73.4-IgG2.5G1.1f-KH
- mAb-CD73.4-IgG2.5G1-AY
- mAb-CD73.4-IgG2.5G1-KH
- mAb-CD73.4-IgG2.5plusTHT/LC
- mAb-CD73.4-IgG2-C219S/LC
- mAb-CD73.4-IgG2-C219S/LC
- mAb-CD73.4-IgG1.1f+K/LC
- mAb-CD73.4-IgG2-IgG1.1f

FIG. 8B

FIG. 8C

FIG. 9

FIG. 10

EP 4 098 662 A1

FIG. 11A

FIG. 11B

181

FIG. 12

FIG. 13

FIG. 14A

FIG. 14B

FIG. 15

FIG. 16A

30min

FIG. 16B

120min

FIG. 16C

**Quantification**

$$m_1 = \frac{\sum x_{colux}}{\sum x}$$

EP 4 098 662 A1

## NFKB Signaling in Activated CD8+ Cells

**FIG. 17A**

## NFKB Signaling in Activated CD4+ Cells

**FIG. 17B**

FIG. 18

FIG. 19

FIG. 20A

GITR.6 variant IL-2 secretion from CD4+ T cells in co-culture with CHO-OKT3 (stimulated at 5ug/mL)

FIG. 20B

FIG. 20C

GITR.6 variant IL-2 secretion from CD4+ T cells in co-culture with CHO-OKT3 (stimulated at 1.25 ug/mL)

GITR.6 variant IL-2 secretion from CD4+ T cells
in co-culture with CHO-OKT3 (stimulated at 0.313 ug/mL)

FIG. 20D

EP 4 098 662 A1

**hIgG1f**

OKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKT
ISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

|  | L234 L235 G237 P238 | | K322 | A330 P331 |
|---|---|---|---|---|
| hIgG1f | ...CPPCPAPE**LL**G**GP**SVFL...... | YKC**K**VSNKAL**P**A**P**IEK... |
| hIgG1.1f | ...CPPCPAPE**AE**G**A**PSVFL...... | YKCKVSNKALP**SS**IEK... |
| hIgG1.3f | ...CPPCPAPE**AE**G**A**PSVFL...... | YKCKVSNKALP**A**PIEK... |
| hIgG1-P238K | ...CPPCPAPELLGG**K**SVFL...... | YKCKVSNKALPAPIEK... |

FIG. 21

FIG. 22A

FIG. 22B

FIG. 22C

FIG. 22D

FIG. 22E

EP 4 098 662 A1

FIG. 22F

FIG. 22G

FIG. 22H

EP 4 098 662 A1

FIG. 22I

EP 4 098 662 A1

FIG. 22J

FIG. 22K

EP 4 098 662 A1

FIG. 22L

EP 4 098 662 A1

FIG. 23A

FIG. 23B

FIG. 23C

FIG. 23D

FIG. 23E

FIG. 23F

Europäisches
Patentamt

European
Patent Office

Office européen
des brevets

# EUROPEAN SEARCH REPORT

**Application Number**

EP 22 16 7890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAZQUEZ-LOMBARDI RODRIGO ET AL: "Potent antitumour activity of interleukin-2-Fc fusion proteins requires Fc-mediated depletion of regulatory T-cells", NATURE COMMUNICATIONS, NATURE PUBLISHING GROUP, GB, vol. 8, 12 May 2017 (2017-05-12), XP002773463, ISSN: 2041-1723, DOI: 10.1038/NCOMMS15373 * page 4, left-hand column, paragraph 1; figure 3a * & Vazquez-Lombardi Rodrigo: "Potent antitumour activity of interleukin-2-Fc fusion proteins requires Fc-mediated depletion of regulatory T-cells Supplementary Information", , 12 May 2017 (2017-05-12), XP055966485, Retrieved from the Internet: URL:https://www.nature.com/articles/ncomms 15373 [retrieved on 2022-09-29] * figure 7 * ----- -/-- | 1-12 | INV. C07K16/28 |
| | | | TECHNICAL FIELDS SEARCHED (IPC) |
| | | | C07K |

The present search report has been drawn up for all claims

| Place of search | Date of completion of the search | Examiner |
|---|---|---|
| Munich | 30 September 2022 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 16 7890

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LATOUR S ET AL: "Bidirectional negative regulation of human T and dendritic cells by CD47 and its cognate receptor signal-regulator protein-alpha: down-regulation of IL-12 responsiveness and inhibition of dendritic cell activation", THE JOURNAL OF IMMUNOLOGY, WILLIAMS & WILKINS CO, US, vol. 167, no. 5, 1 September 2001 (2001-09-01), pages 2547-2554, XP002457665, ISSN: 0022-1767 * page 2548, right-hand column, paragraph 3 * | 1-12 | |
| X | WO 2012/168199 A1 (NOVO NORDISK AS [DK]; ZAHN STEFAN [DK] ET AL.) 13 December 2012 (2012-12-13) * page 3, line 29 - page 4, line 4 * | 1-12 | |
| Y | US 2013/209445 A1 (LAZAR GREGORY A [US] ET AL) 15 August 2013 (2013-08-15) * paragraphs [0018], [0030], [0249], [0250]; figure 41; table 10 * | 10 | TECHNICAL FIELDS SEARCHED (IPC) |
| X | WO 2016/106302 A1 (SQUIBB BRISTOL MYERS CO [US]) 30 June 2016 (2016-06-30) | 1-9,11, 12 | |
| Y | * paragraph [00374]; sequence 47 * | 10 | |
| X,D,P | WO 2018/013818 A2 (BRISTOL MYERS SQUIBB CO [US]) 18 January 2018 (2018-01-18) * paragraph [0057]; sequence 92 * | 1-12 | |
| X,P | WO 2017/152085 A1 (BRISTOL MYERS SQUIBB CO [US]) 8 September 2017 (2017-09-08) * page 2, paragraph 2; sequence 402 * | 1-12 | |

The present search report has been drawn up for all claims

| Place of search | Date of completion of the search | Examiner |
|---|---|---|
| Munich | 30 September 2022 | Cilensek, Zoran |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 16 7890

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2022

| Patent document cited in search report | Publication date | Patent family member(s) | | Publication date |
|---|---|---|---|---|
| WO 2012168199 A1 | 13-12-2012 | AU | 2012266487 A1 | 21-11-2013 |
| | | BR | 112013031198 A2 | 29-11-2016 |
| | | CA | 2838497 A1 | 13-12-2012 |
| | | CN | 103596982 A | 19-02-2014 |
| | | DK | 2718322 T3 | 03-12-2018 |
| | | DK | 3424953 T3 | 02-11-2020 |
| | | EP | 2718322 A1 | 16-04-2014 |
| | | EP | 3424953 A1 | 09-01-2019 |
| | | EP | 3798230 A1 | 31-03-2021 |
| | | ES | 2693647 T3 | 13-12-2018 |
| | | IL | 229254 A | 28-09-2017 |
| | | JP | 6141834 B2 | 07-06-2017 |
| | | JP | 6416177 B2 | 31-10-2018 |
| | | JP | 2014523408 A | 11-09-2014 |
| | | JP | 2017081941 A | 18-05-2017 |
| | | KR | 20140036261 A | 25-03-2014 |
| | | PL | 2718322 T3 | 31-07-2019 |
| | | PL | 3424953 T3 | 25-01-2021 |
| | | PT | 3424953 T | 03-11-2020 |
| | | RU | 2013155029 A | 20-07-2015 |
| | | TW | 201311724 A | 16-03-2013 |
| | | US | 2013004514 A1 | 03-01-2013 |
| | | US | 2013295116 A1 | 07-11-2013 |
| | | US | 2015044231 A1 | 12-02-2015 |
| | | US | 2017073421 A1 | 16-03-2017 |
| | | US | 2020017599 A1 | 16-01-2020 |
| | | US | 2021238300 A1 | 05-08-2021 |
| | | WO | 2012168199 A1 | 13-12-2012 |
| US 2013209445 A1 | 15-08-2013 | US | 2013209445 A1 | 15-08-2013 |
| | | US | 2013209457 A1 | 15-08-2013 |
| WO 2016106302 A1 | 30-06-2016 | AR | 103268 A1 | 26-04-2017 |
| | | AU | 2015369683 A1 | 10-08-2017 |
| | | AU | 2021201231 A1 | 11-03-2021 |
| | | BR | 112017013385 A2 | 06-02-2018 |
| | | CA | 2971732 A1 | 30-06-2016 |
| | | CL | 2017001660 A1 | 23-03-2018 |
| | | CN | 107207594 A | 26-09-2017 |
| | | CN | 110256558 A | 20-09-2019 |
| | | CO | 2017006989 A2 | 05-01-2018 |
| | | EA | 201791171 A1 | 30-11-2017 |
| | | EP | 3237448 A1 | 01-11-2017 |
| | | IL | 285287 A | 30-09-2021 |
| | | JP | 6180663 B2 | 16-08-2017 |
| | | JP | 6633032 B2 | 22-01-2020 |

EPO FORM P0459

For more details about this annex : see Official Journal of the European Patent Office, No. 12/82

page 1 of 3

## ANNEX TO THE EUROPEAN SEARCH REPORT
### ON EUROPEAN PATENT APPLICATION NO.

**EP 22 16 7890**

This annex lists the patent family members relating to the patent documents cited in the above-mentioned European search report.
The members are as contained in the European Patent Office EDP file on
The European Patent Office is in no way liable for these particulars which are merely given for the purpose of information.

**30-09-2022**

| Patent document cited in search report | Publication date | Patent family member(s) | Publication date |
|---|---|---|---|
| | | JP 2017520512 A | 27-07-2017 |
| | | JP 2018035138 A | 08-03-2018 |
| | | JP 2020062026 A | 23-04-2020 |
| | | JP 2021177759 A | 18-11-2021 |
| | | KR 20170099966 A | 01-09-2017 |
| | | MA 40662 A1 | 30-06-2020 |
| | | MY 187045 A | 27-08-2021 |
| | | PE 20171244 A1 | 24-08-2017 |
| | | PH 12017501166 A1 | 11-12-2017 |
| | | SG 10202006538T A | 28-08-2020 |
| | | SG 11201705063V A | 28-07-2017 |
| | | TN 2017000267 A1 | 19-10-2018 |
| | | TW 201629102 A | 16-08-2016 |
| | | US 2016176963 A1 | 23-06-2016 |
| | | US 2019112375 A1 | 18-04-2019 |
| | | US 2021324072 A1 | 21-10-2021 |
| | | UY 36471 A | 30-06-2016 |
| | | WO 2016106302 A1 | 30-06-2016 |
| | | ZA 201704981 B | 28-08-2019 |
| | | ZA 201806307 B | 30-03-2022 |
| WO 2018013818 A2 | 18-01-2018 | AU 2017297506 A1 | 21-02-2019 |
| | | BR 112019000431 A2 | 09-07-2019 |
| | | CA 3030765 A1 | 18-01-2018 |
| | | CL 2019000100 A1 | 10-05-2019 |
| | | CL 2021000698 A1 | 13-08-2021 |
| | | CN 109757103 A | 14-05-2019 |
| | | CO 2019000372 A2 | 18-01-2019 |
| | | EP 3484920 A2 | 22-05-2019 |
| | | IL 264011 A | 31-01-2019 |
| | | JP 7027401 B2 | 01-03-2022 |
| | | JP 2019533981 A | 28-11-2019 |
| | | JP 2022078102 A | 24-05-2022 |
| | | KR 20190027384 A | 14-03-2019 |
| | | PE 20190418 A1 | 19-03-2019 |
| | | SG 102019119720 A | 27-02-2020 |
| | | SG 11201900026T A | 30-01-2019 |
| | | TW 201811829 A | 01-04-2018 |
| | | US 2018016336 A1 | 18-01-2018 |
| | | US 2018298097 A1 | 18-10-2018 |
| | | US 2019248893 A1 | 15-08-2019 |
| | | US 2020190186 A1 | 18-06-2020 |
| | | UY 37325 A | 31-01-2018 |
| | | WO 2018013818 A2 | 18-01-2018 |
| WO 2017152085 A1 | 08-09-2017 | AU 2017228470 A1 | 30-08-2018 |

For more details about this annex : see Official Journal of the European Patent Office, No. 12/82

EPO FORM P0459

**page 2 of 3**

## ANNEX TO THE EUROPEAN SEARCH REPORT
## ON EUROPEAN PATENT APPLICATION NO.

EP 22 16 7890

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2022

| Patent document cited in search report | Publication date | Patent family member(s) | Publication date |
|---|---|---|---|
| | | BR 112018067368 A2 | 15-01-2019 |
| | | CA 3016187 A1 | 08-09-2017 |
| | | CN 109476740 A | 15-03-2019 |
| | | EA 201891983 A1 | 28-02-2019 |
| | | EP 3423494 A1 | 09-01-2019 |
| | | IL 261395 A | 31-10-2018 |
| | | JP 2019514844 A | 06-06-2019 |
| | | JP 2022104961 A | 12-07-2022 |
| | | KR 20180118725 A | 31-10-2018 |
| | | KR 20220033522 A | 16-03-2022 |
| | | SG 10201913033U A | 30-03-2020 |
| | | SG 11201806861S A | 27-09-2018 |
| | | US 2019284293 A1 | 19-09-2019 |
| | | WO 2017152085 A1 | 08-09-2017 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62511178
- US 62599221
- US 20080248028
- WO 2007038868 A2, Davies
- US 8709431 B2
- US 7778814 B2, Domling
- US 8394922 B2, Cheng
- US 20140227295 A1, Cong
- US 65458530 B1, Boger
- US 8461117 B2, Sufi
- US 20120301490 A1, Zhang
- US 20070275904 A1, Vite
- US 6844869 B2, Senter
- US 7498298 B2, Doronina
- US 20130059800 A1, Howard
- US 20130028919 A1
- WO 2013041606 A1
- US 5208020 A, Chari
- US 7374762 B2, Amphlett
- US 7087600 B
- US 6989452 B
- US 7129261 B
- WO 02096910 A
- WO 07038658 A
- WO 07051081 A
- WO 07059404 A
- WO 08083312 A
- WO 081103693 A
- US 20060024317 A
- US 20060004081 A
- US 20060247295 A
- US 20090074660 A1, Korman
- US 8097703 B2, Rao-Naik
- US 20100143368 A1, King
- US 7387776 B2, Keler
- US 8124738 B2, Terrett
- US 6984720 B1, Korman
- US 8008449 B2, Korman
- US 20090297438 A1, Huang
- US 7875278 B2, Cardarelli
- US 20100034826 A1, Terrett
- US 20100209432 A1, Terrett
- US 7335748 B2, Harkins
- US 8268970 B2
- US 20100092484 A1, Xu
- WO 2006121168 A
- WO 2012145493 A
- WO 2009014708 A
- WO 03099196 A
- WO 2009114335 A
- WO 2011066389 A
- WO 2011161699 A
- WO 2013173223 A
- US 7635757 B
- US 8217149 B
- US 20090317368 A
- WO 2007005874 A
- US 7943743 B
- WO 07005874 A
- US 2009145493 A
- WO 01114424 A
- WO 9842752 A
- WO 0037504 A
- US 6207156 B
- US 2011007023 A
- WO 2006105021 A
- WO 2011028683 A
- JP 2008278814 B
- US 5624821 A
- US 6277375 B
- US 6737056 B
- US 6194551 B
- US 7317091 B
- US 8101720 B
- WO 0042072 A
- WO 0158957 A
- WO 04016750 A
- WO 04029207 A
- WO 04035752 A
- WO 04074455 A
- WO 04099249 A
- WO 04063351 A
- WO 05070963 A
- WO 05040217 A
- WO 05092925 A
- WO 06020114 A
- WO 9951642 A
- WO 9429351 A
- US 5648260 A
- WO 88007089 A
- WO 9958572 A
- US 5834597 A
- US 20070148167
- WO 2012142515 A
- WO 2006020114 A
- WO 2004074455 A
- US 8652466 B
- US 20140010812

- US 2006024298
- WO 2012087928 A
- WO 2012115241 A
- WO 2009079242 A
- EP 1176195 B1
- WO 03035835 A
- WO 2009135181 A
- WO 9954342 A, Umana
- US 20130149300
- US 20080206246
- WO 2013095966 A
- US 5869046 A
- US 6121022 A, Presta
- US 8367805 B
- WO 98023289 A
- WO 2009086320 A
- WO 2006130834 A
- WO 6277375 A
- WO 6821505 A
- WO 9734631 A
- WO 2002060919 A
- US 8629113 B
- US 7867491 B
- EP 0154316 A, Nishimura
- EP 0401384 A, Ishikawa
- WO 2009127691 A
- WO 2010091122 A

- WO 2014043344 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 4522811 A
- US 5374548 A
- US 5399331 A
- US 5416016 A, Low
- WO 09045957 A
- WO 09073533 A
- WO 09073546 A
- WO 09054863 A
- US 2013072918 W
- US 1561632 W
- US 20110150892 A
- WO 2018013818 A

**Non-patent literature cited in the description**

- **KABAT et al.** Sequences of Proteins of Immunological Interest. National Institutes of Health, 1991
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **CHAN ; CARTER.** *Nat. Rev. Immunol.,* 2010, vol. 10, 301
- **KABAT et al.** Sequences of Proteins of Immunological Interest. Public Health Service, NIH, 1992
- **CHOTHIA et al.** *Nature,* 1989, vol. 342, 877-883
- **MACCALLUM et al.** *J. Mol. Biol.,* vol. 262, 732-745
- **MAKABE et al.** *Journal of Biological Chemistry,* 2008, vol. 283, 1156-1166
- **JEFFERIES et al.** *mAbs,* 2009, vol. 1, 1
- **JEFFRIES et al.** *mAbs,* 2009, vol. 1, 1
- **ROUX et al.** *J. Immunol.,* 1998, vol. 161, 4083
- **ROUX et al.** *J Immunol,* 1998, vol. 161, 4083
- **ALLEN et al.** *Biochemistry,* 2009, vol. 48, 3755
- **LONBERG.** *Nature Biotech,* 2005, vol. 23 (9), 1117-1125
- **SONGSIVILAI ; LACHMANN.** *Clin. Exp. Immunol.,* 1990, vol. 79, 315-321

- **KOSTELNY et al.** *J. Immunol.,* 1992, vol. 148, 1547-1553
- Epitope Mapping Protocols in Methods in Molecular Biology. 1996, vol. 66
- *J. Mol. Biol.,* 1970, (48), 444-453
- **ALTSCHUL et al.** *J. Mol. Biol.,* 1990, vol. 215, 403-10
- **ALTSCHUL.** *Nucleic Acids Res,* 1997, vol. 25 (17), 3389-3402
- **SCHRAMA et al.** *Nature Rev. Drug Disc.,* 2006, vol. 5, 147
- **LEE et al.** *J. Am. Chem. Soc.,* 1987, vol. 109, 3464-3466
- **ROSENBLATT et al.** *J. Immunotherapy,* 2011, vol. 34, 409
- **HURWITZ et al.** *Proc. Natl. Acad. Sci. USA,* 1998, vol. 95 (17), 10067-10071
- **CAMACHO et al.** *J. Clin. Oncology,* 2004, vol. 22 (145
- **MOKYR et al.** *Cancer Res.,* 1998, vol. 58, 5301-5304
- **SARMAY et al.** *Molec. Immunol.,* 1992, vol. 29 (5), 633-9
- **HORTON et al.** *J. Immunol.,* 2011, vol. 186, 4223
- **CHU et al.** *Mol. Immunol.,* 2008, vol. 45, 3926

- **GROSS et al.** *Immunity,* 2001, vol. 15, 289
- **IDUSOGIE et al.** *J. Immunol.,* 2000, vol. 164, 4178
- **TAO et al.** *J. Exp. Med.,* 1993, vol. 178, 661
- **CANFIELD ; MORRISON.** *J. Exp. Med.,* 1991, vol. 173, 1483
- **ROTHER et al.** *Nat. Biotechnol.,* 2007, vol. 25, 1256
- **MUELLER et al.** *Mol. Immunol.,* 1997, vol. 34, 441
- **LABRIJN et al.** *Curr. Op. Immunol.,* 2008, vol. 20, 479
- **DUNCAN ; WINTER.** *Nature,* 1988, vol. 332, 563
- **CHAPPEL et al.** *Proc. Nat'l Acad. Sci. (USA),* 1991, vol. 88, 9036
- **SONDERMANN et al.** *Nature,* 2000, vol. 406, 267
- **STROHL.** *Current Opinion in Biotechnology,* 2009, vol. 20, 685-691
- **ARMOUR et al.** *Eur. J. Immunol.,* 1999, vol. 29, 2613
- **ALEGRE et al.** *Transplantation,* 1994, vol. 57, 1537
- **COLE et al.** *J. Immunol.,* 1997, vol. 159, 3613
- **REDDY et al.** *J. Immunol.,* 2000, vol. 164, 1925
- **OGANESYAN et al.** *Acta Crystallogr. D. Biol. Crystallogr.,* 2008, vol. 64, 700
- **LABRIJN.** *Curr. Op. Immunol.,* 2008, vol. 20, 479
- **DAVIS et al.** *J. Immunol.,* 2007, vol. 34, 2204
- **GLAESNER et al.** *Diabetes Metab. Res. Rev.,* 2010, vol. 26, 287
- **HUTCHINS et al.** *Proc. Nat'l Acad. Sci. (USA),* 1995, vol. 92, 11980
- **AN et al.** *MAbs,* 2009, vol. 1, 572
- **MCEARCHERN et al.** *Blood,* 2007, vol. 109, 1185
- **VAFA et al.** *Methods,* 2014, vol. 65, 114
- **RICHARDS et al.** *Mol. Cancer Therap.,* 2008, vol. 7, 2517
- **MOORE et al.** *mAbs,* 2010, vol. 2, 181
- **SHIELDS et al.** *J. Biol. Chem.,* 2001, vol. 276, 6591
- **IDUSOGIE et al.** *J. Immunol.,* 2001, vol. 166, 2571
- **SHIELDS et al.** *J. Biol. Chem.,* 2001, vol. 276, 6591-6604
- **LAZAR et al.** *Proc. Nat'l Acad Sci. (USA),* 2006, vol. 103, 4005
- **AWAN et al.** *Blood,* 2010, vol. 115, 1204
- **DESJARLAIS ; LAZAR.** *Exp. Cell Res.,* 2011, vol. 317, 1278
- **LAZAR et al.** *Proc. Nat'l Acad Sci.,* vol. 103, 4005
- **STAVENHAGEN et al.** *Cancer Res.,* 2007, vol. 67, 8882
- **NORDSTROM et al.** *Breast Cancer Res,* 2011, vol. 13, R123
- **DANGL et al.** *EMBO J.,* 1988, vol. 7, 1989
- **NATSUME et al.** *Cancer Res.,* 2008, vol. 68, 3863
- **MICHAELSEN et al.** *Scand. J. Immunol.,* 2009, vol. 70, 553
- **REDPATH et al.** *Immunology,* 1998, vol. 93, 595
- **DALL'ACQUA et al.** *J. Immunol.,* 2006, vol. 177, 1129
- **CARTER.** *Nat. Rev. Immunol.,* 2006, vol. 6, 343
- **PRESTA.** *Curr. Op. Immunol.,* 2008, vol. 20, 460
- **LI ; RAVETCH.** *Science,* 2011, vol. 333, 1030
- **LI ; RAVETCH.** *Proc. Nat'l Acad. Sci (USA),* 2012, vol. 109, 10966
- **MIMOTO et al.** *Protein. Eng. Des. & Selection,* 2013, vol. 26, 589
- **BOLT et al.** *Eur. J. Immunol.,* 1993, vol. 23, 403
- **TAO ; MORRISON.** *J. Immunol.,* 1989, vol. 143, 2595
- **JUNG et al.** *Proc. Nat'l Acad. Sci (USA),* 2010, vol. 107, 604
- **SHIELDS et al.** *JBC,* 2002, vol. 277, 26733
- **NIWA et al.** *J. Immunol. Methods,* 2005, vol. 306, 151
- **CARDARELLI et al.** *Clin. Cancer Res.,* 2009, vol. 15, 3376
- **CARDARELLI et al.** *Cancer Immunol. Immunotherap.,* 2010, vol. 59, 257
- **YAMANE-OHNUKI et al.** *Biotechnol. Bioeng.,* 2004, vol. 87, 614
- **ZHANG et al.** *mAbs,* 2011, vol. 3, 289
- **LI et al.** *Nat. Biotechnol.,* 2006, vol. 24, 210
- **MOSSNER et al.** *Blood,* 2010, vol. 115, 4393
- **SHIELDS et al.** *J. Biol. Chem.,* 2002, vol. 277, 26733
- **SHINKAWA et al.** *J. Biol. Chem.,* 2003, vol. 278, 3466
- **SHIELDS, R.L. et al.** *J. Biol. Chem.,* 2002, vol. 277, 26733-26740
- **UMANA et al.** *Nat. Biotech.,* 1999, vol. 17, 176-180
- **NIMMERJAHN et al.** *Ann. Rev. Immunol.,* 2008, vol. 26, 513
- **MARSHALL et al.** *Annu Rev Biochem,* 1972, vol. 41, 673-702
- **GALA ; MORRISON.** *J. Immunol,* 2004, vol. 172, 5489-94
- **WALLICK et al.** *J Exp Med,* 1988, vol. 168, 1099-109
- **SPIRO.** *Glycobiology,* 2002, vol. 12, 43R-56R
- **PAREKH et al.** *Nature,* 1985, vol. 316, 452-7

- **MIMURA et al.** *Mol Immunol,* 2000, vol. 37, 697-706
- **HINTON et al.** *J. Biol. Chem.,* 2004, vol. 279 (8), 6213-6216
- **HINTON et al.** *Journal of Immunology,* 2006, vol. 176, 346-356
- **SHIELDS et al.** *Journal of Biological Chemistry,* 2001, vol. 276 (9), 6591-6604
- **DEL' ACQUA et al.** *Journal of Immunology,* 2002, vol. 169, 5171-5180
- **DALL'ACQUA et al.** *Journal of Biological Chemistry,* 2006, vol. 281, 23514-23524
- **YEUNG et al.** *J. Immunol.,* 2009, vol. 182, 7663
- **ZALEVSKY et al.** *Nat. Biotechnol.,* 2010, vol. 28, 157
- **PETKOVA et al.** *Int. Immunol.,* 2006, vol. 18, 1759
- **DALL'ACQUA et al.** *J. Biol. Chem.,* 2006, vol. 281, 23514
- **VACCARO et al.** *Nat. Biotechnol.,* 2005, vol. 23, 1283
- **YEUNG et al.** *J. Immunol.,* 2010, vol. 182, 7663-7671
- **HORNICK et al.** *J. Nucl. Med.,* 2000, vol. 41, 355
- **KIM et al.** *Eur. J. Immunol.,* 2000, vol. 29, 2819
- **KENANOVA et al.** *Cancer Res.,* 2005, vol. 65, 622
- **CHAPMAN et al.** *Nat. Biotechnol.,* 1999, vol. 17, 780
- **YEH et al.** *Proc. Nat'l Acad. Sci.,* 1992, vol. 89, 1904
- **SCHELLENBERGER et al.** *Nat. Biotechnol.,* 2009, vol. 27, 1186
- **KRISHNAMURTHY R ; MANNING M C.** *Curr Pharm Biotechnol,* 2002, vol. 3, 361-71
- **CHEN et al.** *Pharm Res,* 2003, vol. 20, 1952-60
- **GHIRLANDO et al.** *Immunol Lett,* 1999, vol. 68, 47-52
- **MURRAY et al.** *J. Chromatogr Sci,* 2002, vol. 40, 343-9
- **ALEXANDER A J ; HUGHES D E.** *Anal Chem,* 1995, vol. 67, 3626-32
- **LABRIJN et al.** *Nat. Biotechnol.,* 2009, vol. 27, 767
- **BERGE, S.M. et al.** *J. Pharm. Sci.,* 1977, vol. 66, 1-19
- Sustained and Controlled Release Drug Delivery Systems. Marcel Dekker, Inc, 1978
- **V.V. RANADE.** *J. Clin. Pharmacol.,* 1989, vol. 29, 685
- **UMEZAWA et al.** *Biochem. Biophys. Res. Commun.,* 1988, vol. 153, 1038
- **P.G. BLOEMAN et al.** *FEBS Lett,* 1995, vol. 357, 140
- **M. OWAIS et al.** *Antimicrob. Agents Chemother.,* 1995, vol. 39, 180
- **BRISCOE et al.** *Am. J. Physiol.,* 1995, vol. 1233, 134
- **SCHREIER et al.** *J. Biol. Chem.,* vol. 269, 9090
- **K. KEINANEN ; M.L. LAUKKANEN.** *FEBS Lett,* 1994, vol. 346, 123
- **J.J. KILLION ; I.J. FIDLER.** *Immunomethods,* 1994, vol. 4, 273
- **MOKYR et al.** *Cancer Research,* 1998, vol. 58, 5301-5304
- **FULDA et al.** *Nat Med,* 2002, vol. 8, 808-15
- **HYER et al.** *Cancer Research,* 2005, vol. 65, 4799-808